(19) Europäisches Patentamt
European Patent Office
Office européen des brevets

(11) EP 4 527 416 A1

(12) **EUROPEAN PATENT APPLICATION**
published in accordance with Art. 153(4) EPC

(43) Date of publication:
**26.03.2025 Bulletin 2025/13**

(21) Application number: **23806970.2**

(22) Date of filing: **17.05.2023**

(51) International Patent Classification (IPC):
***A61K 47/68** (2017.01)* ***A61K 39/00** (2006.01)*
***C07D 401/00** (2006.01)* ***A61P 35/00** (2006.01)*

(52) Cooperative Patent Classification (CPC):
**A61K 31/454; A61K 31/5025; A61K 31/513; A61K 31/5377; A61K 31/551; A61K 39/00; A61K 47/64; A61K 47/65; A61K 47/68; A61P 35/00; C07D 401/00**

(86) International application number:
**PCT/CN2023/094714**

(87) International publication number:
**WO 2023/222019 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.05.2022 CN 202210540244**

(71) Applicant: **SystImmune, Inc.**
**Redmond WA 98052 (US)**

(72) Inventors:
- **ZHU, Yi**
  **Redmond, WA 98052 (US)**
- **WAN, Weili**
  **Chengdu, Sichuan 611130 (CN)**
- **ZHUO, Shi**
  **Chengdu, Sichuan 6111300 (CN)**
- **ZHU, Guili**
  **Chengdu, Sichuan 611130 (CN)**
- **YU, Tianzi**
  **Chengdu, Sichuan 611130 (CN)**

(74) Representative: **Tirotta, Ilaria et al**
**Marchi & Partners S.r.l.**
**Via Vittor Pisani, 13**
**20124 Milano (IT)**

Remarks:
The complete document including Reference Table(s) and the Sequence Listing(s) can be downloaded from the EPO website

(54) **LIGAND-DRUG CONJUGATE AND USE THEREOF**

(57) Provided are a ligand-drug conjugate and use thereof. Specifically, provided are a ligand-drug conjugate represented by formula I or a stereoisomer thereof, a prodrug thereof, a pharmaceutically acceptable salt thereof, or a pharmaceutically acceptable solvate thereof.

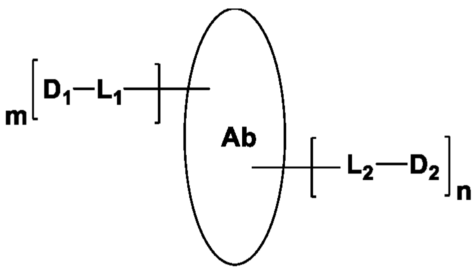

I

EP 4 527 416 A1

## Description

Technical field

The present invention relates to the technical field of medicine, in particular to ligand-drug conjugate and use thereof.

Background art

Antibody-drug conjugate (ADC) is a macromolecule of a novel therapy, which has both strong lethality of micromolecule drug and high specific targeting property of antibody, and can accurately put the drug to tumor parts or/and tumor cells by means of the specificity of the antibody to avoid killing normal cells *in vivo,* thereby reducing adverse reactions in the treatment process. ADC consists of three parts, namely an antibody, a drug and a linker. When the antibody part of the ADC specifically binds to a specific antigen on the surface of tumor cells, the cell membrane of the tumor cells will sink inwards to form endosome to engulf the ADC, which is also called internalization. The antibody part or linker of the ADC will be degraded by some enzyme in the cell, thereby releasing the drug, and the released drug will start to kill tumor cells. In the development process of ADC drug, linker + drug (payload) is an important intermediate material and consists of a linker and a cytotoxic molecule (drug). At the present stage, the cytotoxic molecule in the payload mainly comprises tubulin inhibitors and topoisomerase inhibitors.

Contents of the invention

To improve the therapeutic efficacy of ligand-drug conjugate (e.g., antibody-drug conjugate ADC) and increase the therapeutic window, the present invention provides a ligand-drug conjugate represented by formula I, or a stereoisomer thereof, a prodrug thereof, a pharmaceutically acceptable salt thereof or a pharmaceutically acceptable solvate thereof. The ligand-drug conjugate is coupled with two different drugs, namely a repair inhibitor with low toxicity and low killing activity and a damage agent with high killing activity; the ligand (e.g., antibody) is coupled with the low-toxicity repair inhibitor and the high-toxicity damage agent at the same time, and the obtained dual-drug ADC drug has higher killing activity than the ADC of ligand (e.g., antibody) coupled with a single drug. The ligand-drug conjugate (e.g., the dual-drug ADC) has remarkably stronger tumor inhibition effect at a cellular level.

To this end, in the first aspect, the present invention provides a ligand-drug conjugate represented by formula I, or a stereoisomer thereof, a prodrug thereof, a pharmaceutically acceptable salt thereof or a pharmaceutically acceptable solvate thereof,

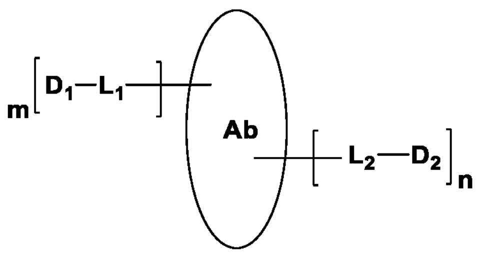

formula I

wherein:

- Ab is a targeting moiety selected from the group consisting of an antibody, an antibody fragment, a targeting protein and an Fc-fusion protein;
- $D_1$ is a first drug linked to $L_1$;
- $D_2$ is a second drug selected from DNA damage repair inhibitors, and is linked to $L_2$;
- $L_1$ is a first linking unit linking the first drug to the targeting moiety;
- $L_2$ is a second linking unit linking the second drug to the targeting moiety;
- m is an integer selected from 0 to 20 (e.g., 0, 1, 2, 3, 4, 5, 6, 7, 8, 9, 10, 11, 12, 13, 14, 15, 16, 17, 18, 19 or 20);
- n is an integer selected from 1 to 20 (e.g., 1, 2, 3, 4, 5, 6, 7, 8, 9, 10, 11, 12, 13, 14, 15, 16, 17, 18, 19 or 20).

In certain embodiments, the second drug is selected from the group consisting of an ATR inhibitor, an ATM inhibitor, a PARP inhibitor, a WEE1 inhibitor, a CHK1 inhibitor, and a DNA-PK inhibitor.

In certain embodiments, the ATR inhibitor is selected from the group consisting of

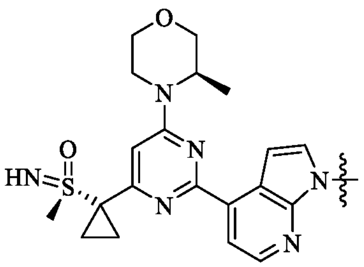,
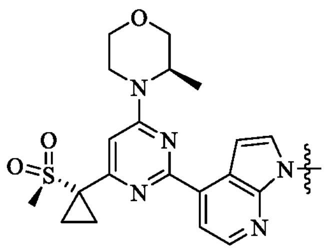,
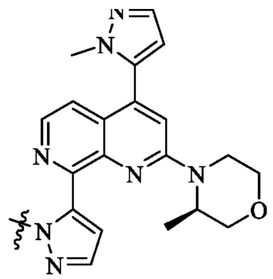,
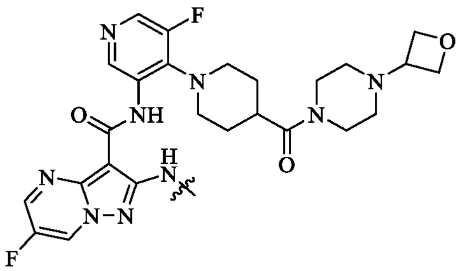

and a compound represented by formula A or a deuteride thereof:

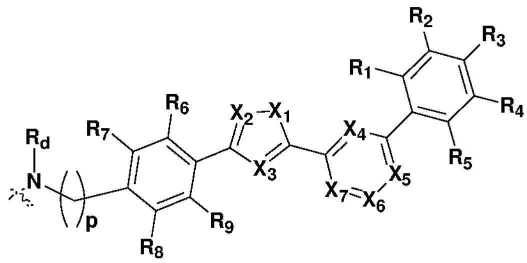
formula A

wherein,

$X_1$ is selected from the group consisting of O, S, NH, and $CH_2$;
$X_2$, $X_3$ each are independently selected from the group consisting of N, and CH;
$X_4$, $X_5$, $X_6$ each are independently selected from the group consisting of N, and CH;
$X_7$ is selected from the group consisting of N, and $C(R_0)$;
$R_0$ is selected from the group consisting of H, C1-C6 alkyl, and $R_bR_aN$-;
$R_a$, $R_b$ each are independently selected from the group consisting of H, and C1-C6 alkyl;
$R_1$, $R_2$, $R_3$, $R_4$, $R_5$ each are independently selected from the group consisting of H, C1-C6 alkyl, and

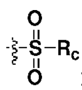 ;

$R_c$ is selected from the group consisting of H, and C1-C6 alkyl;
$R_6$, $R_7$, $R_8$, $R_9$ each are independently selected from the group consisting of H, and C1-C6 alkyl;
$R_d$ is selected from the group consisting of H, and C1-C6 alkyl;
p is selected from the group consisting of 1, 2, 3, 4, 5, and 6.

In certain embodiments, $X_1$ is selected from the group consisting of O, and S.
In certain embodiments, $X_1$ is O.
In certain embodiments, $X_2$ is N, $X_3$ is CH.
In certain embodiments, $X_4$, and $X_6$ are N, $X_5$ is CH.
In certain embodiments, $X_7$ is $C(R_0)$.
In certain embodiments, $R_0$ is $R_bR_aN$-.

In certain embodiments, $R_a$, $R_b$ are each H.

In certain embodiments, $R_1$, $R_2$, $R_3$, $R_4$, $R_5$ each are independently selected from the group consisting of H, and

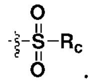

.

In certain embodiments, any one of $R_1$, $R_2$, $R_3$, $R_4$, $R_5$ is

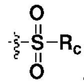

,

with the rest being H.

In certain embodiments, $R_3$ is

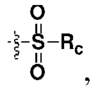

,

$R_1$, $R_2$, $R_4$, and $R_5$ are H.

In certain embodiments, $R_c$ is selected from C1-C6 alkyl.

In certain embodiments, $R_c$ is isopropyl.

In certain embodiments, $R_6$, $R_7$, $R_8$, $R_9$ are H.

In certain embodiments, $R_d$ is selected from C1-C6 alkyl.

In certain embodiments, $R_d$ is methyl.

In certain embodiments, p is selected from the group consisting of 1, 2, and 3.

In certain embodiments, p is 1.

In certain embodiments, the compound represented by formula A is

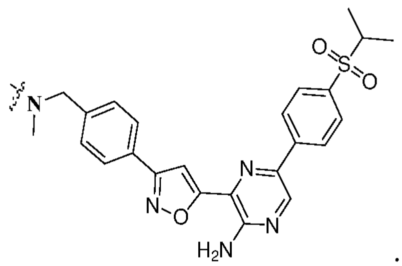

.

In certain embodiments, the PARP inhibitor is selected from the group consisting of

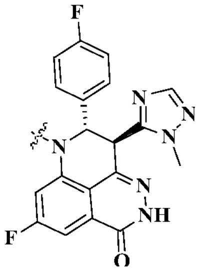 , 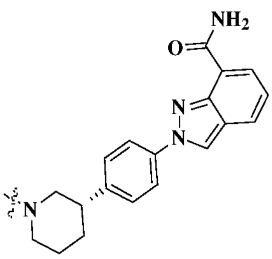 , 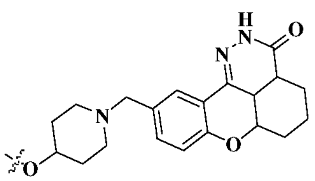 , 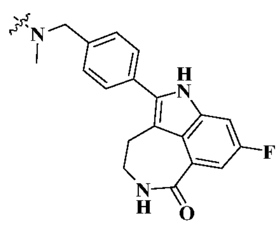 ,

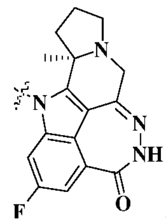

, and

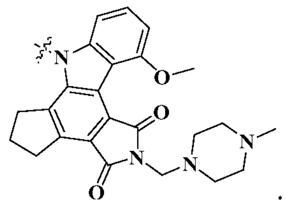

.

In certain embodiments, the ATM inhibitor is selected from the group consisting of

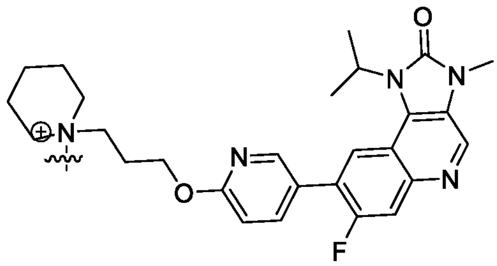

,

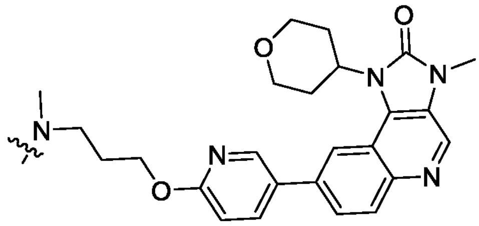

,

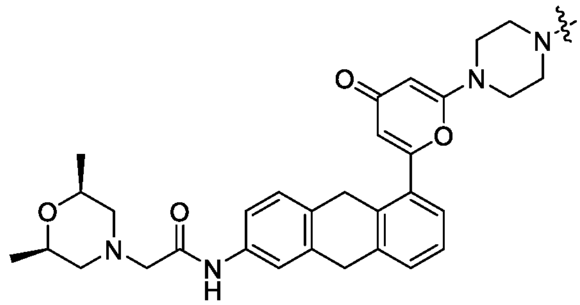

,

and

In certain embodiments, the WEE1 inhibitor is

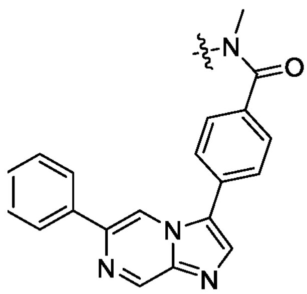

.

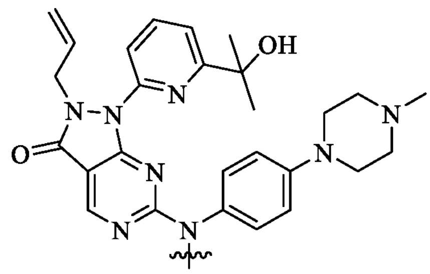

.

In certain embodiments, the CHK1 inhibitor is selected from the group consisting of

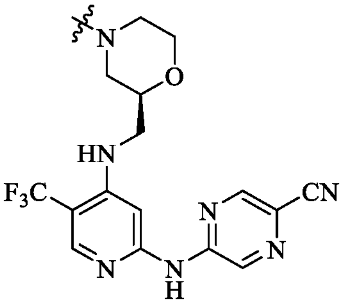
,
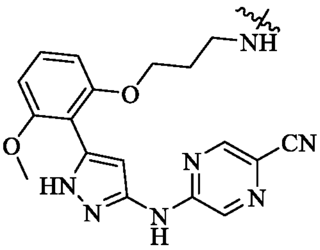
,
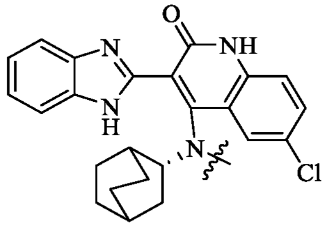

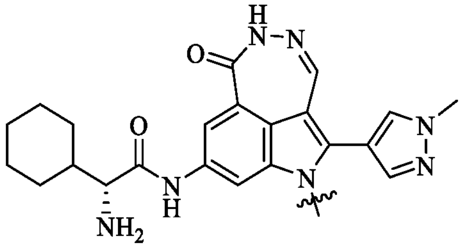
,
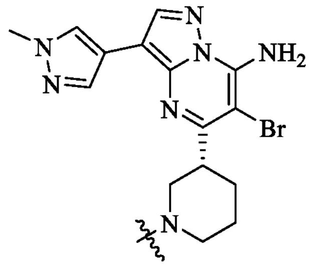
, and
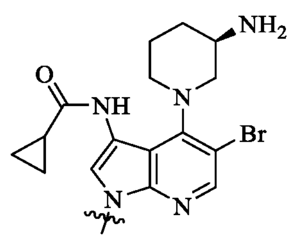
.

In certain embodiments, the DNA-PK inhibitor is selected from the group consisting of

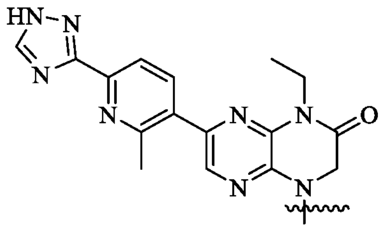
,
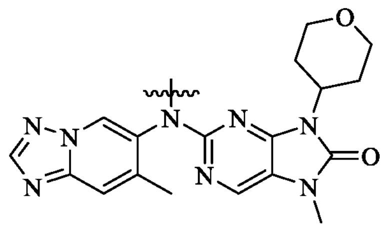
,
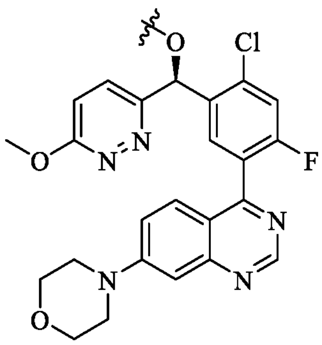
,

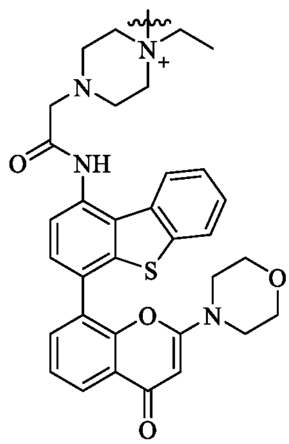
, and
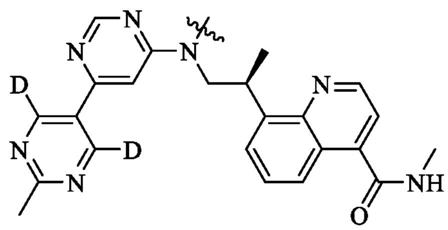
.

In certain embodiments, the second linking unit is a breakable or non-breakable linking unit.

In certain embodiments, $L_2$ is $L_{21}$-$L_{22}$-$L_{23}$-$L_{24}$, and $L_{24}$ is linked to $D_2$, $L_{21}$ is linked to Ab (preferably, $L_{21}$ is coupled to Ab via thiol group),

$L_{21}$ is selected from the group consisting of

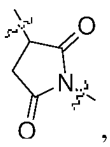
,

and

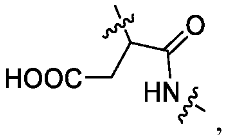
,

with its N terminal linked to $L_{22}$,
$L_{22}$ is selected from the group consisting of:

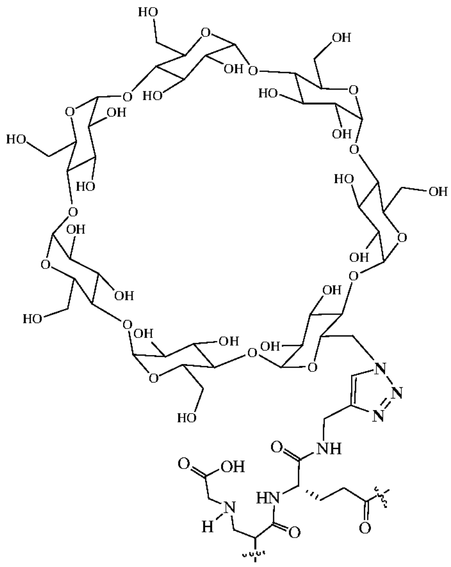
,
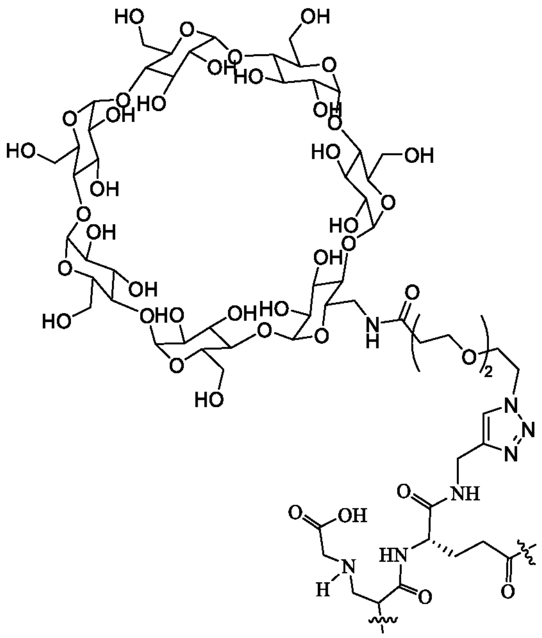
,

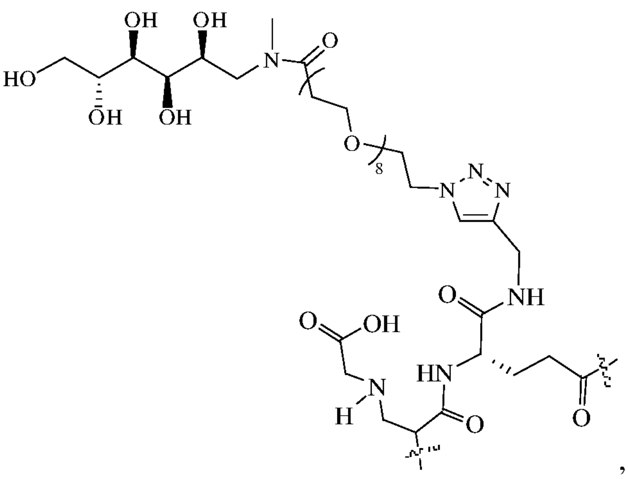
, and
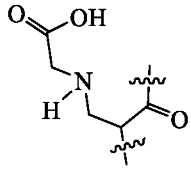
,

with its carbonyl terminal linked to $L_{23}$;
$L_{23}$ is selected from an amino acid residue or a peptide residue consisting of 2-10 amino acid residues, with its carbonyl terminal linked to $L_{24}$;
$L_{24}$ is

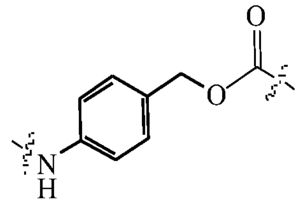

or a direct bond, and when $L_{24}$ is

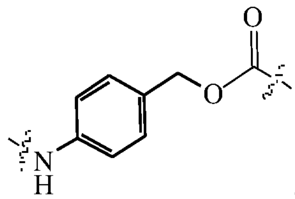

,

its carbonyl terminal is linked to $D_2$.

In certain embodiments, $L_{23}$ is a peptide residue consisting of 2-4 (preferably 2) amino acid residues, with its carbonyl terminal linked to $L_{24}$, preferably, the amino acid is selected from the group consisting of valine, alanine, phenylalanine, glycine, lysine, citrulline, serine, glutamic acid, and aspartic acid, more preferably, the amino acid is selected from the group consisting of valine, alanine, and citrulline.

In certain embodiments, $L_{23}$ is selected from the group consisting of valine-alanine (Val-Ala), and valine-citrulline (Val-Cit), with its carbonyl terminal linked to $L_{24}$.

In certain embodiments, $L_{23}$ is selected from the group consisting of

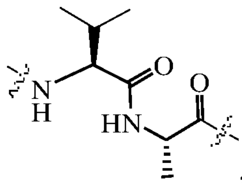

,

and

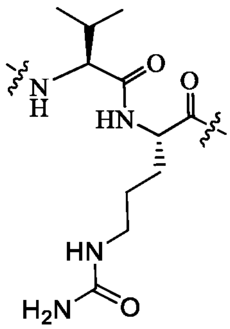

,

with its carbonyl terminal linked to $L_{24}$.

In certain embodiments, $L_2$ is selected from the group consisting of:

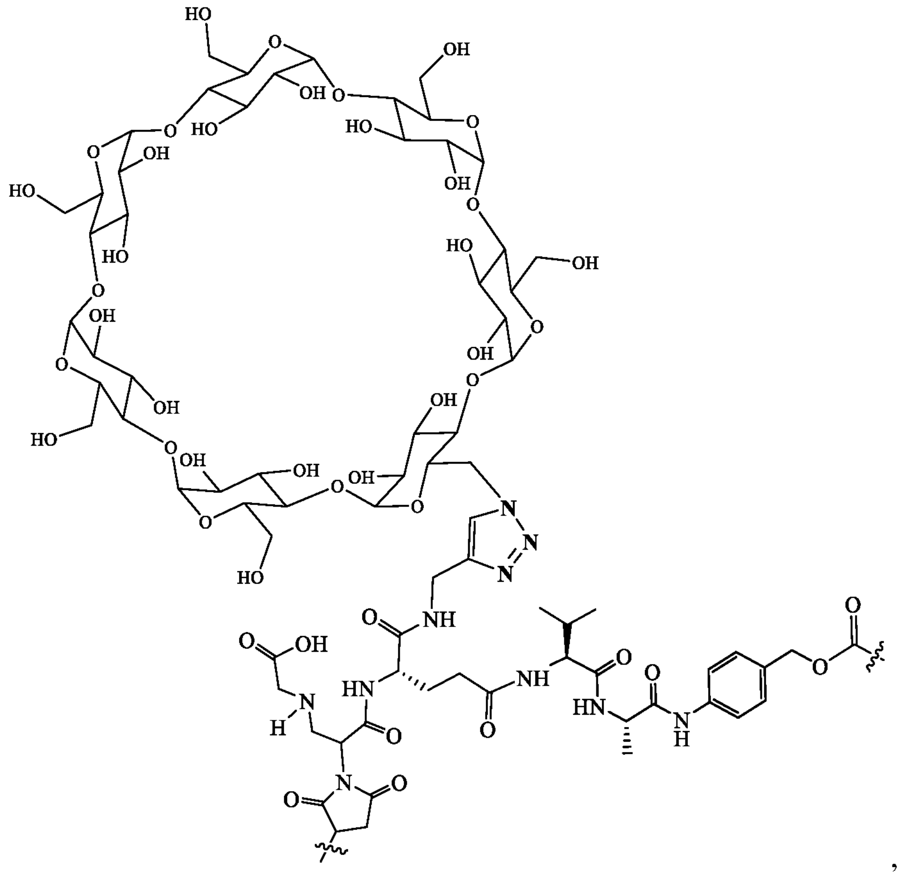

,

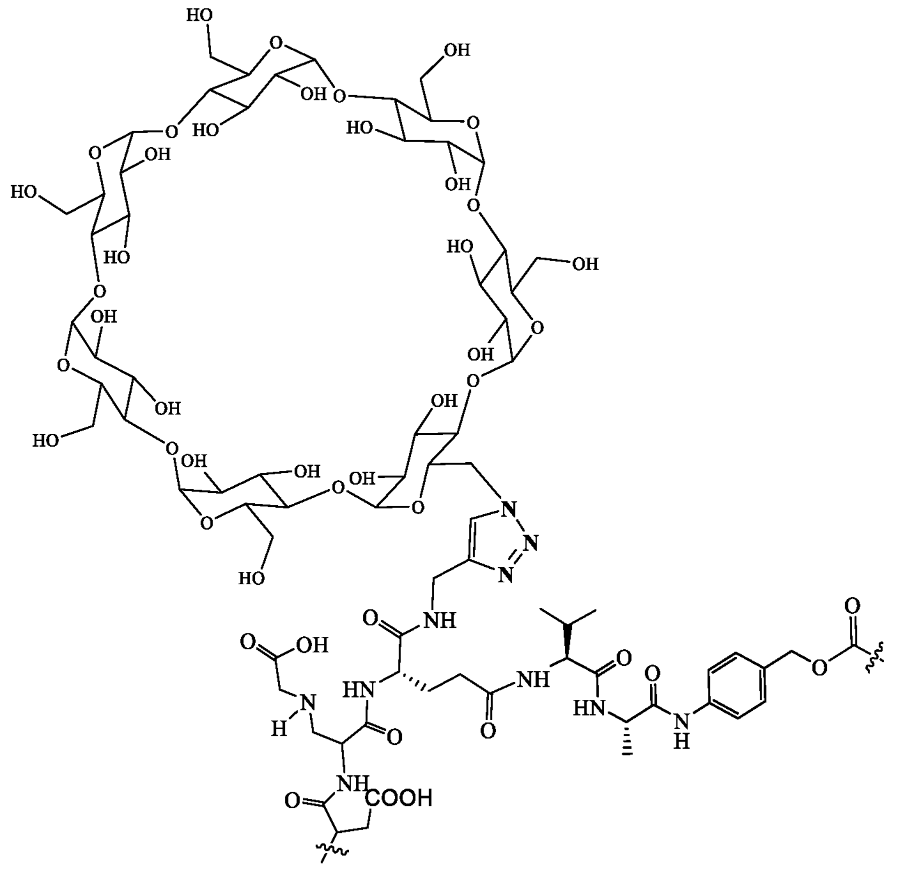

,

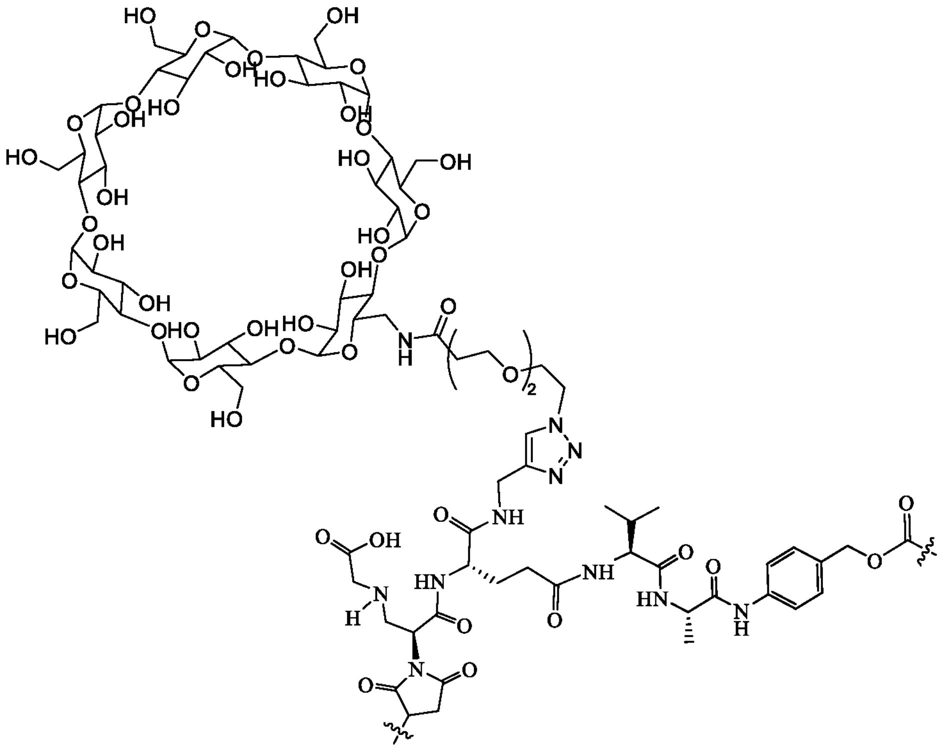

,

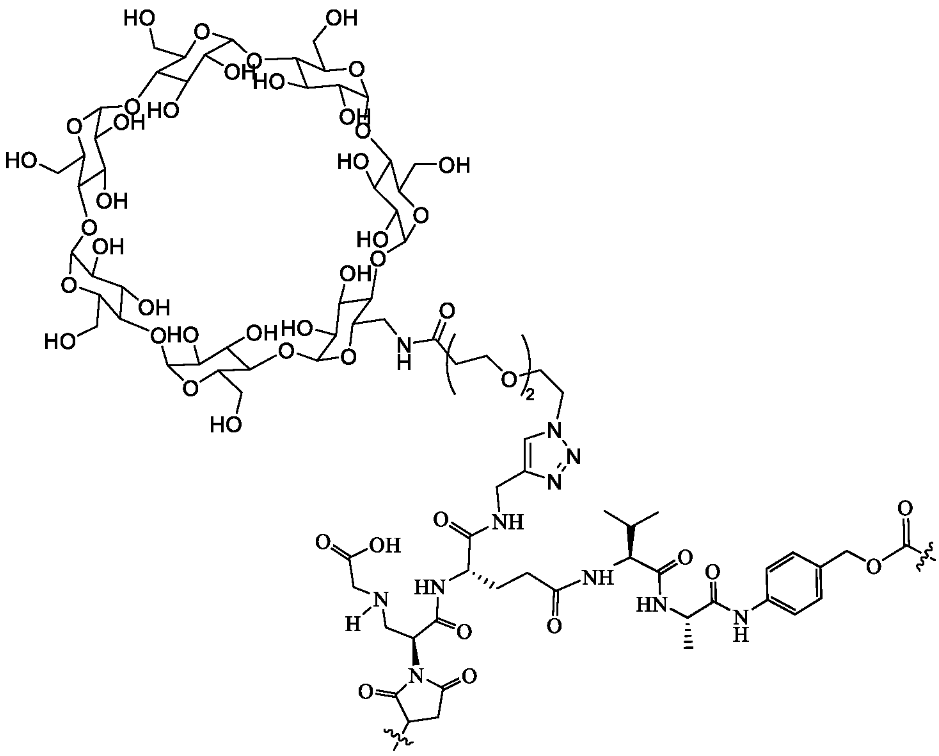

,

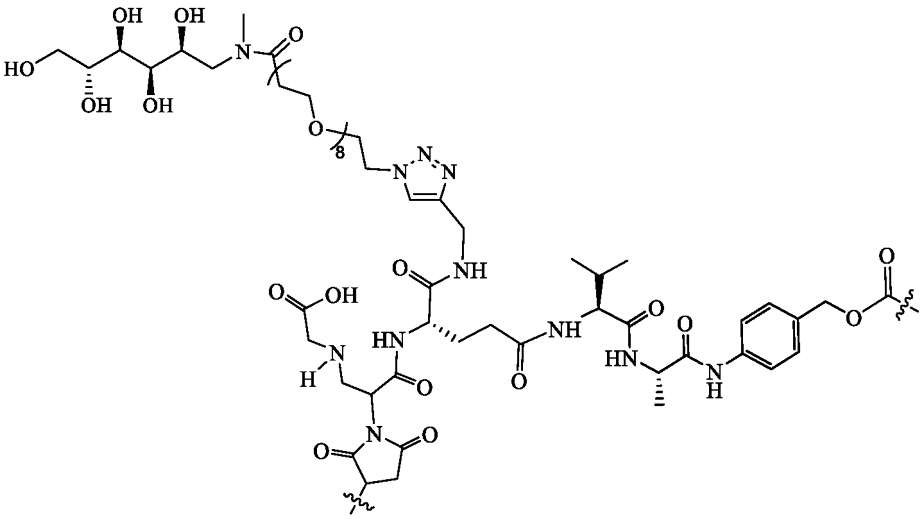

,

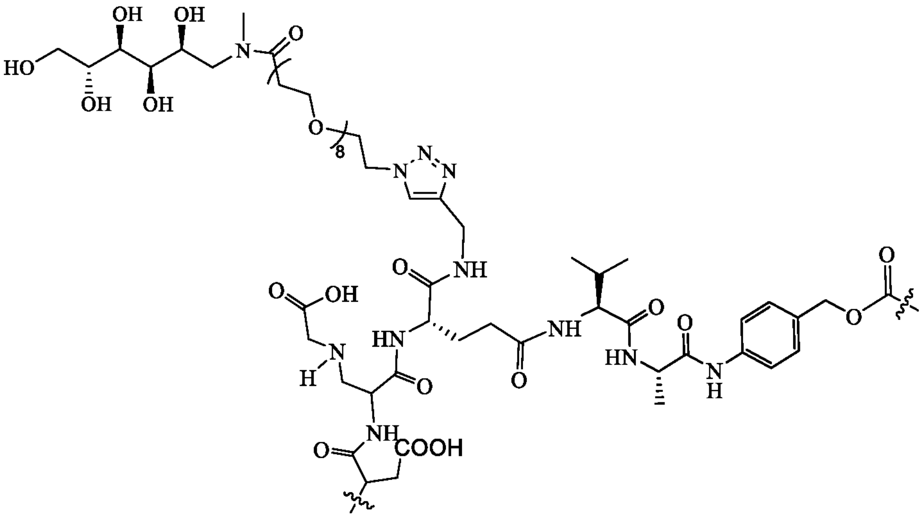

,

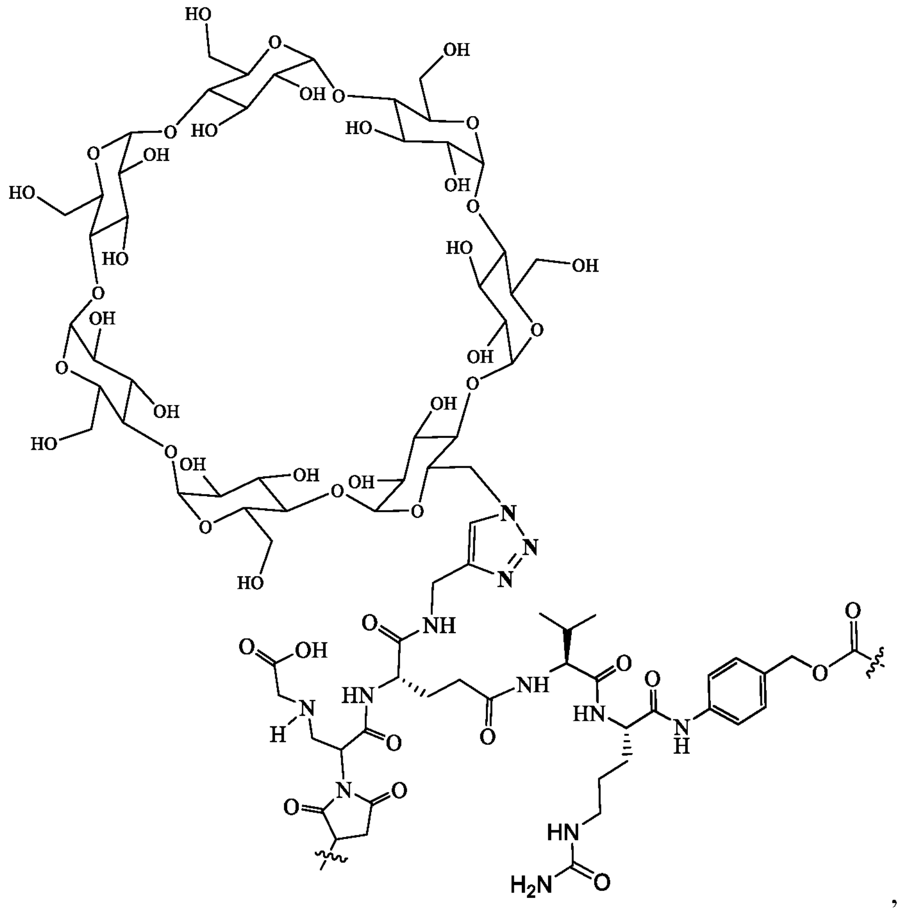

,

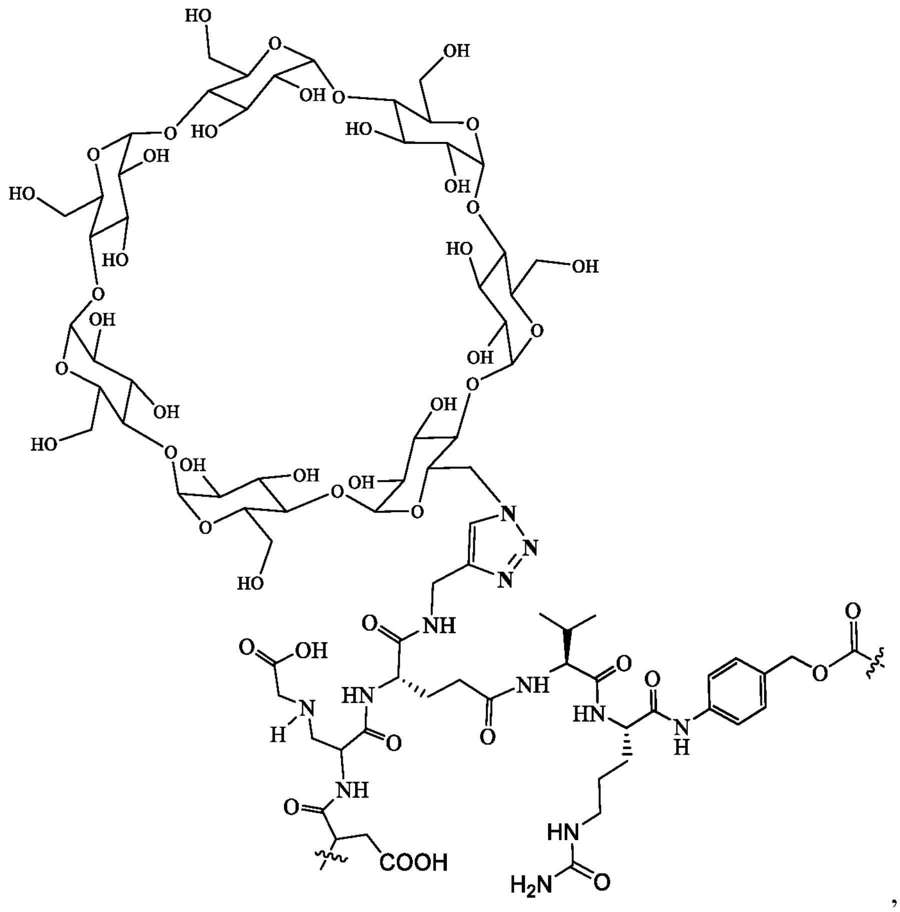

,

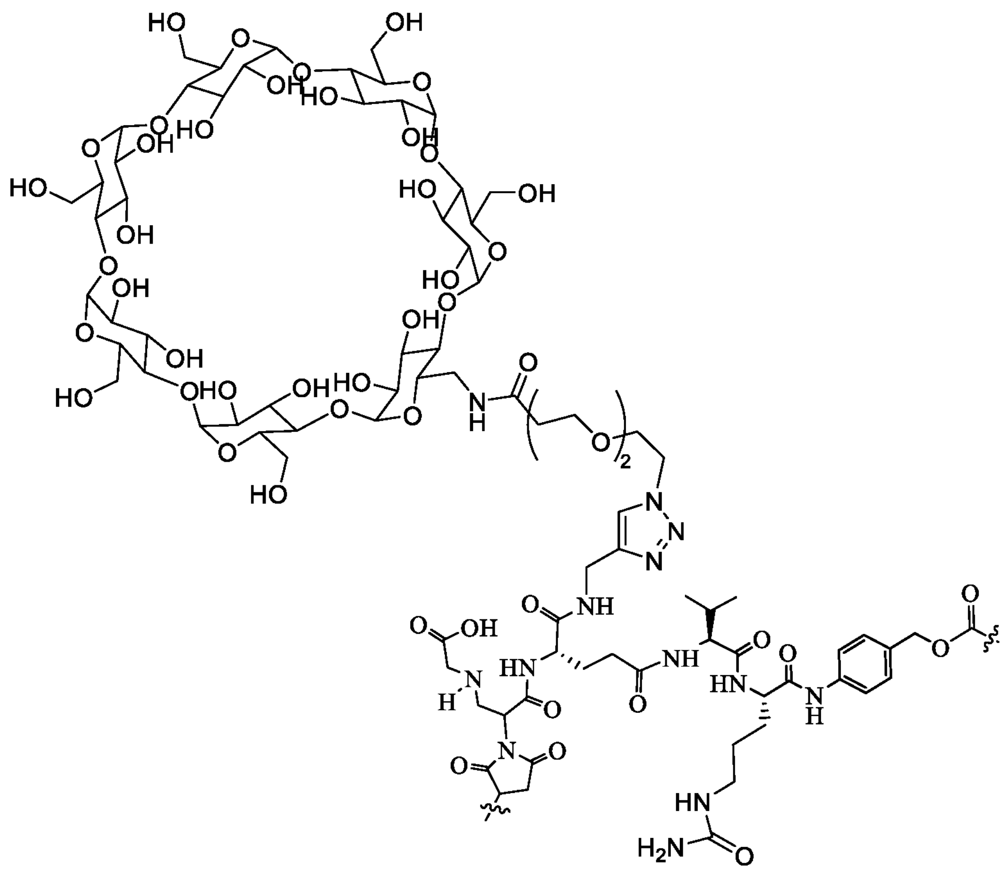

,

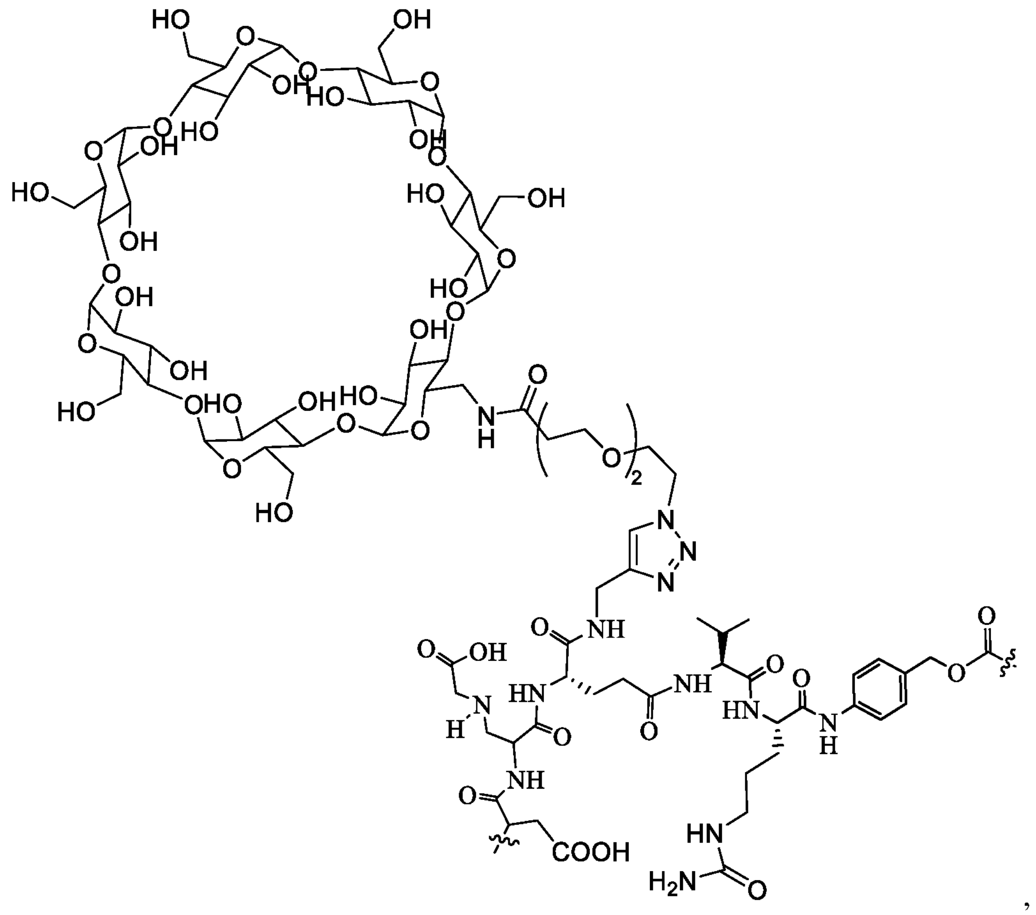

,

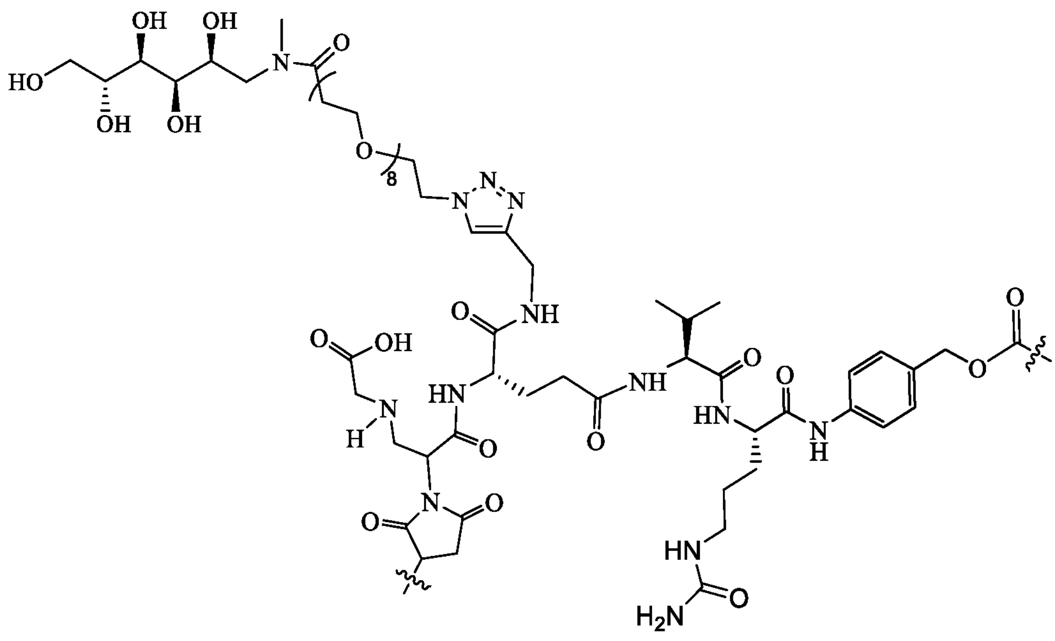

,

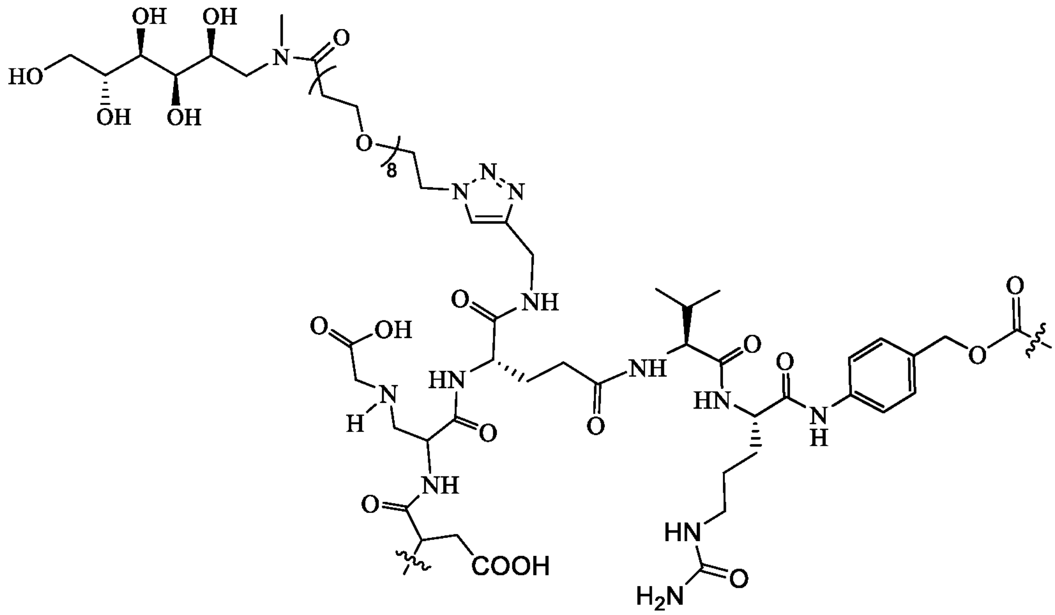

,

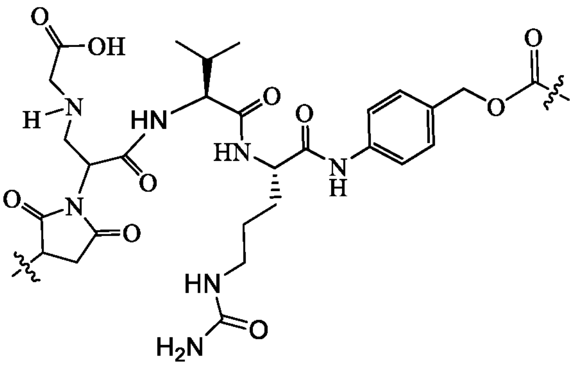

,

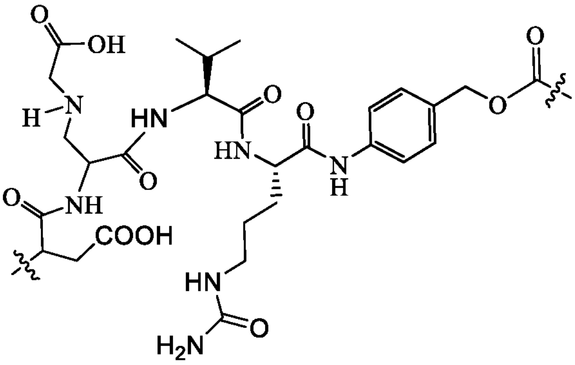

,

and

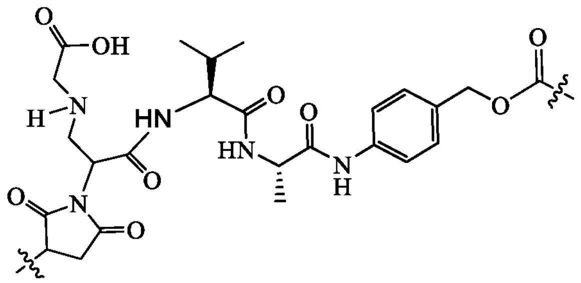

,

with its carbonyl terminal linked to $D_2$.

In certain embodiments, $D_2$-$L_2$- is selected from the group consisting of:

| | |
|---|---|
| R01 | 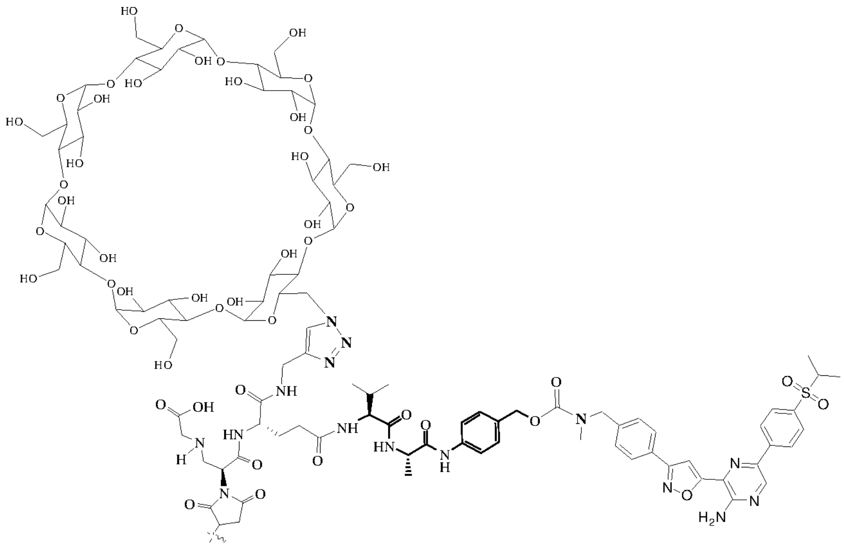 |
| R01-1 | 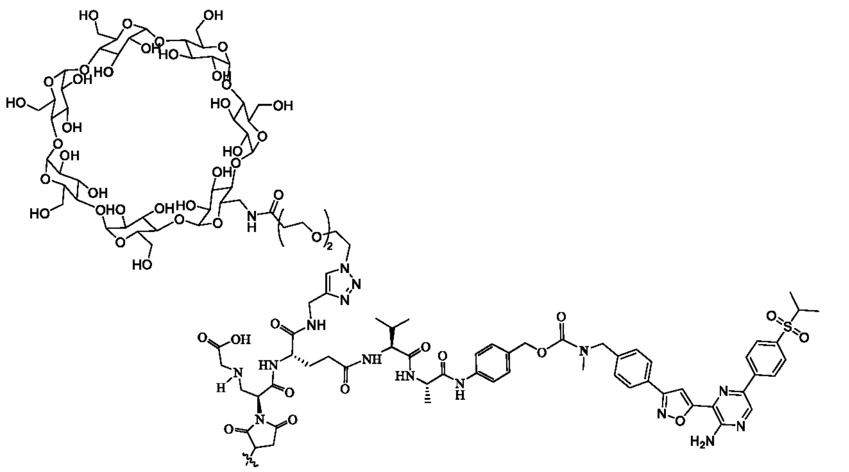 |

(continued)

| | |
|---|---|
| R01-2 | 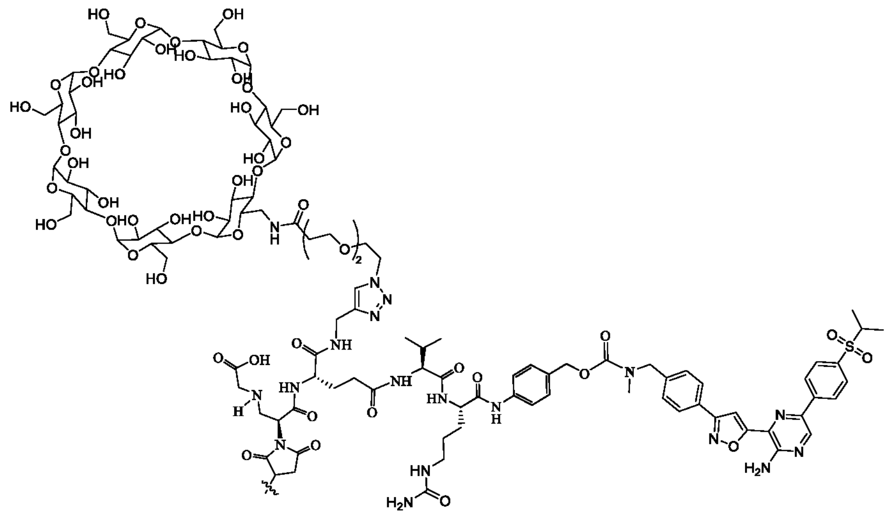 |
| R01-3 | 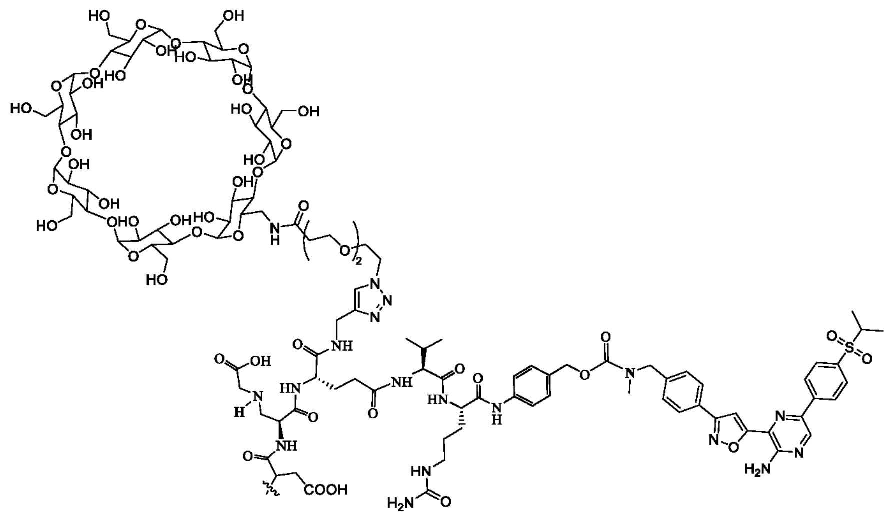 |
| R01-4 | 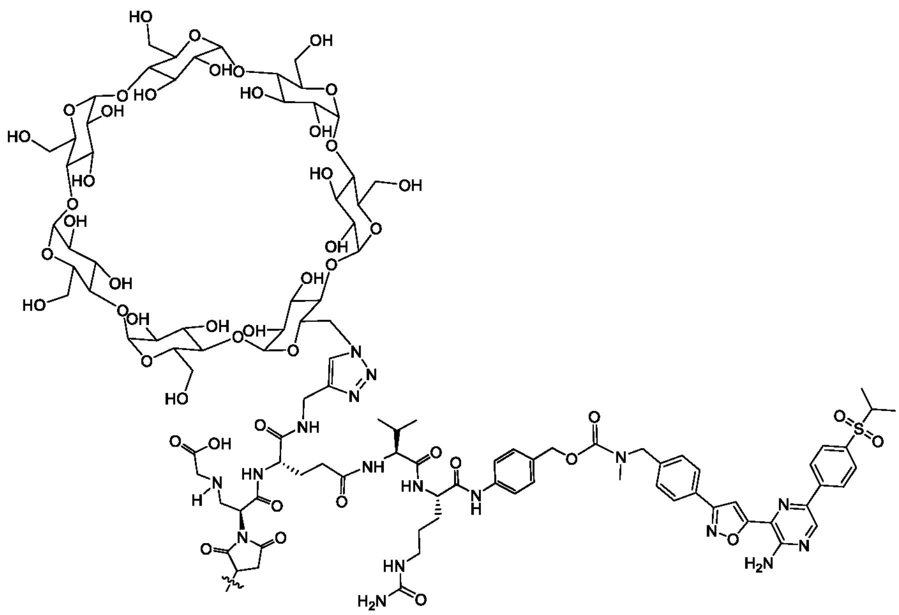 |

(continued)

| | |
|---|---|
| R02 | 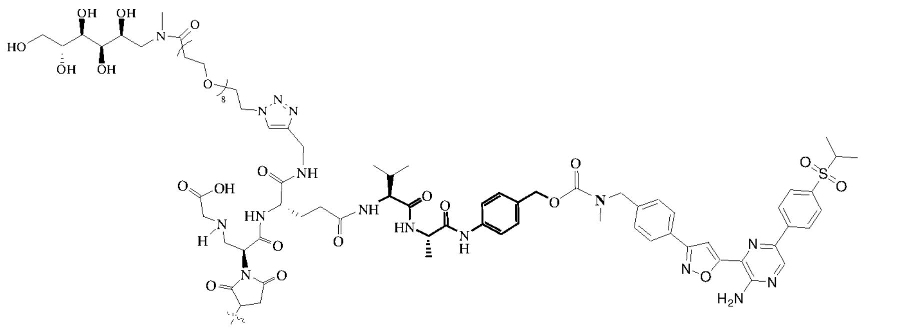 |
| R02-1 | 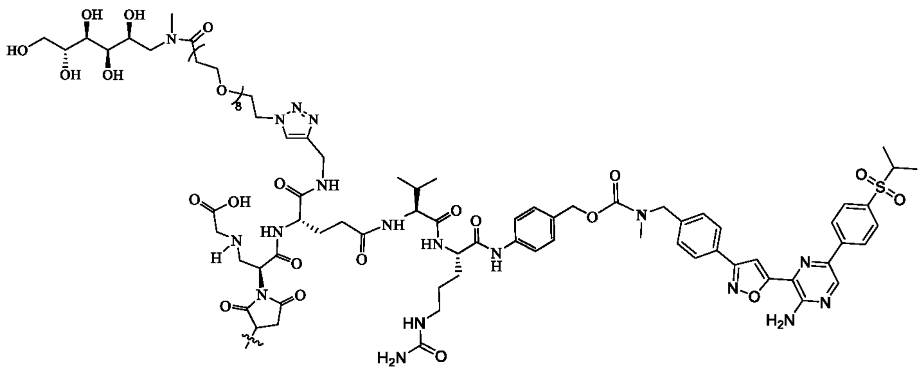 |
| R03 | 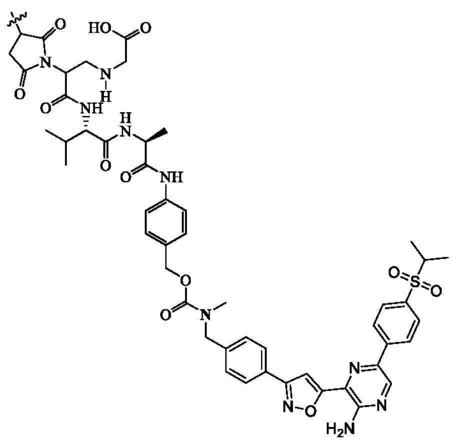 |
| R03-1 | 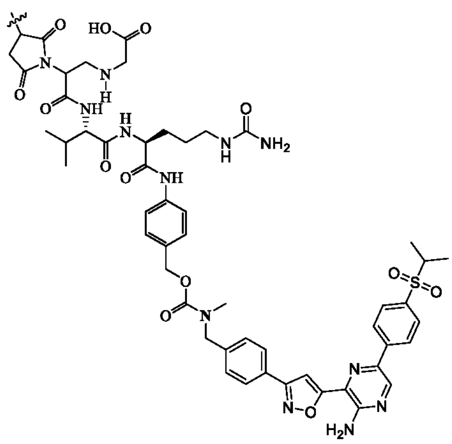 |

(continued)

| | |
|---|---|
| AT01 | 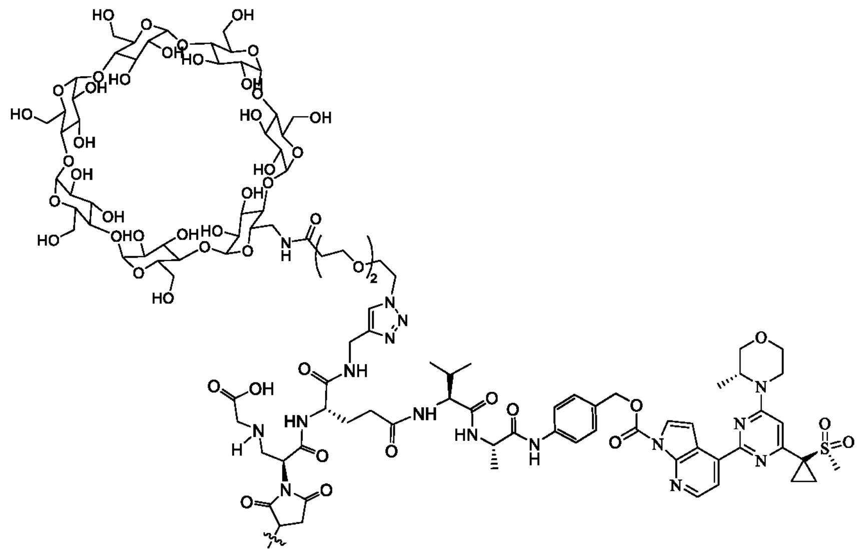 |
| AT02 | 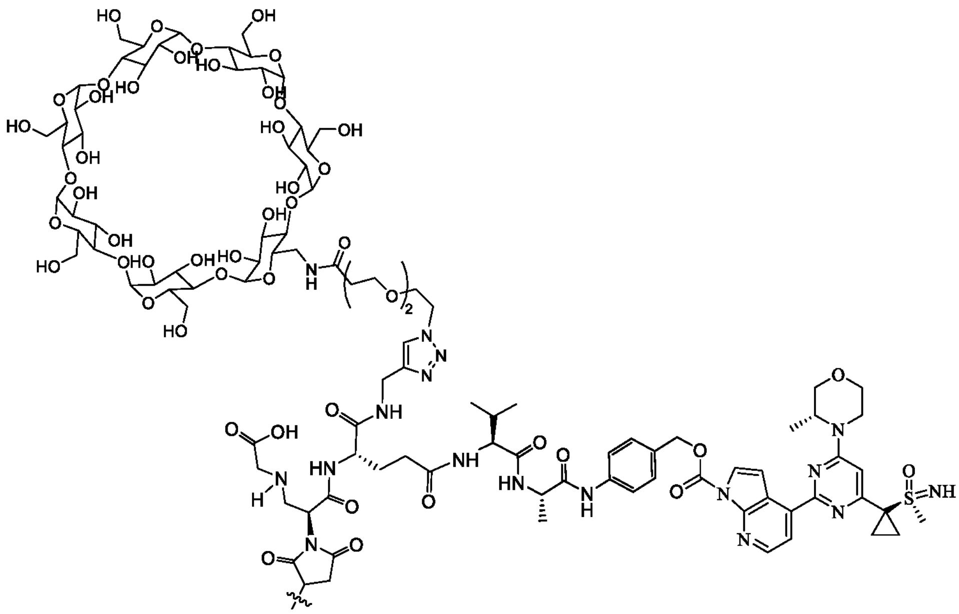 |

(continued)

| | |
|---|---|
| AT03 | 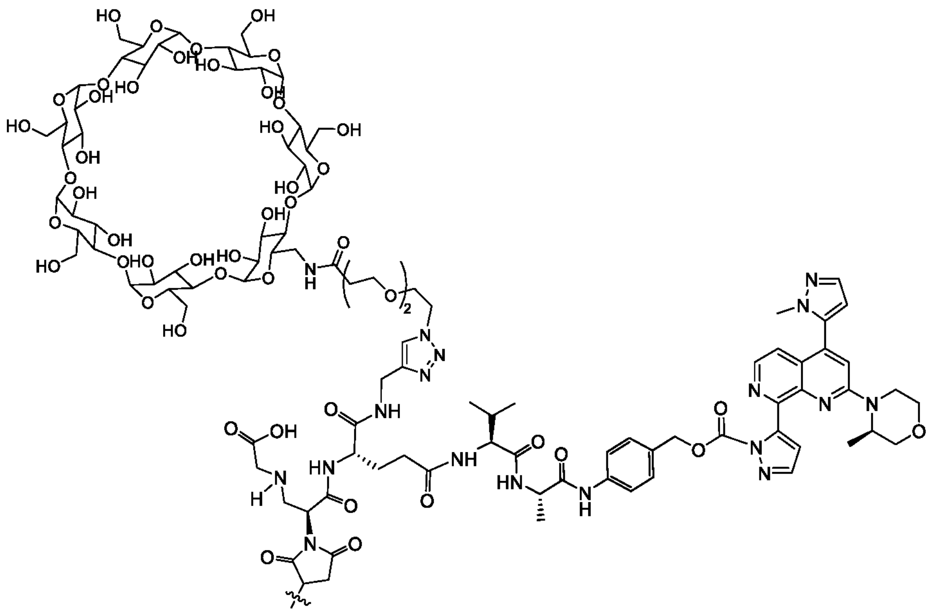 |
| AT04 | 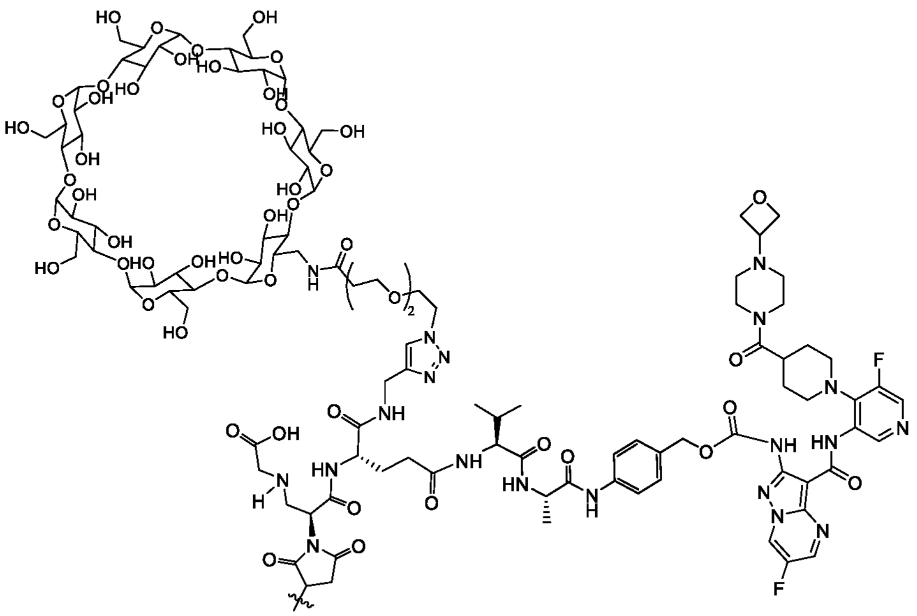 |

(continued)

| | |
|---|---|
| TA01 | 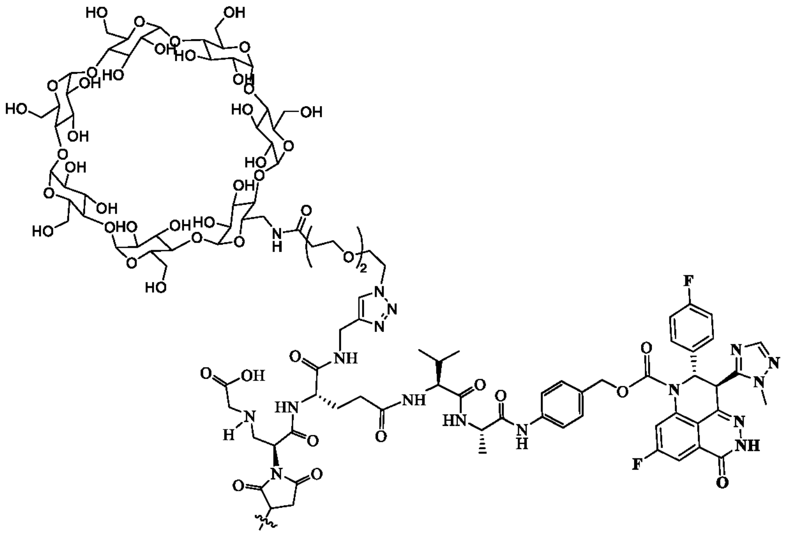 |
| VE01 | 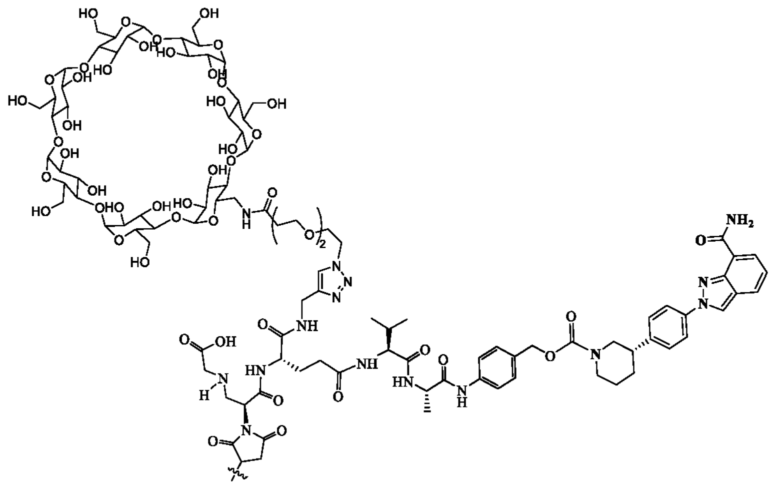 |
| E01 | 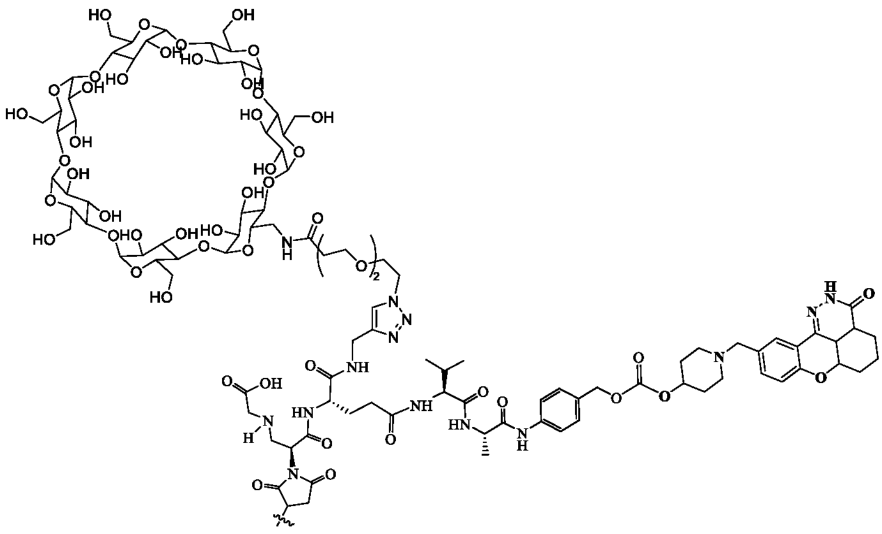 |

(continued)

| | |
|---|---|
| RU01 | 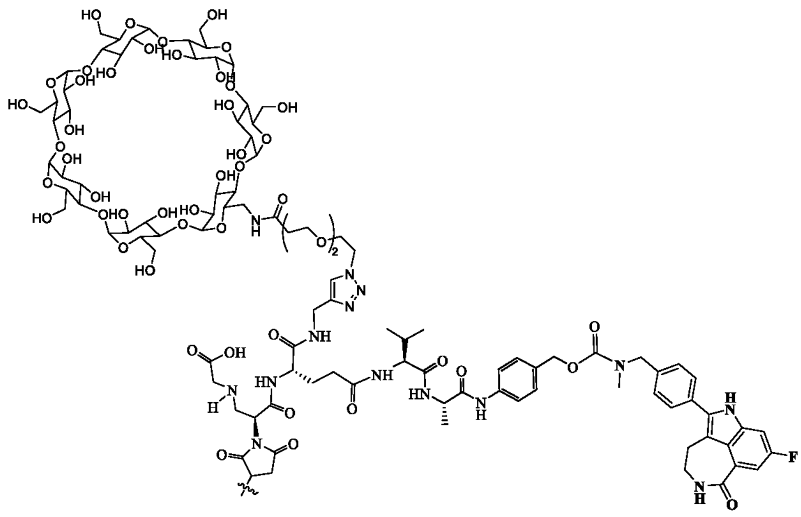 |
| PA01 | 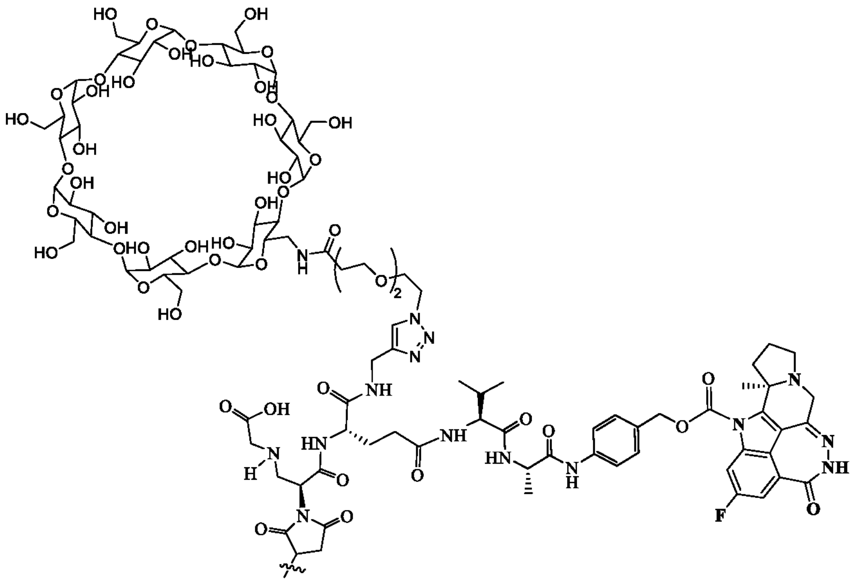 |
| CEP01 | 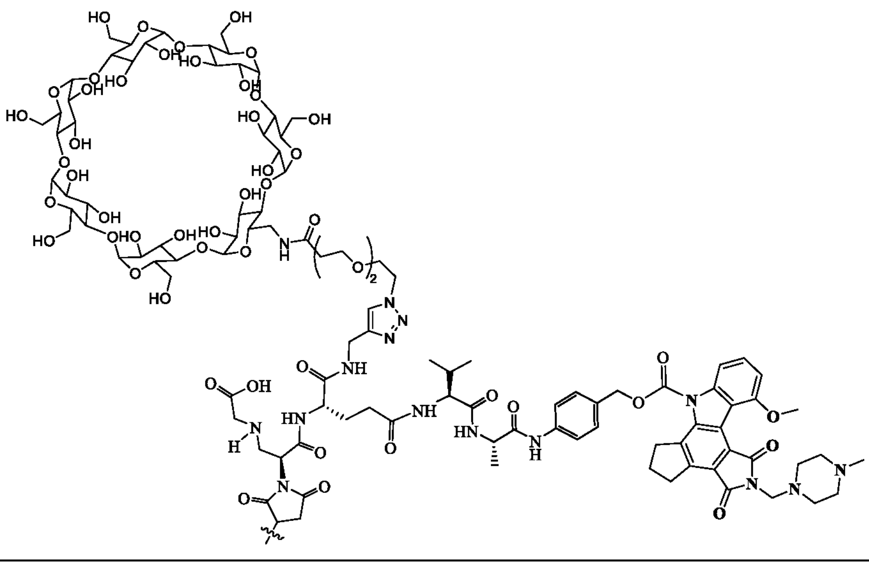 |

(continued)

| | |
|---|---|
| TM01 | 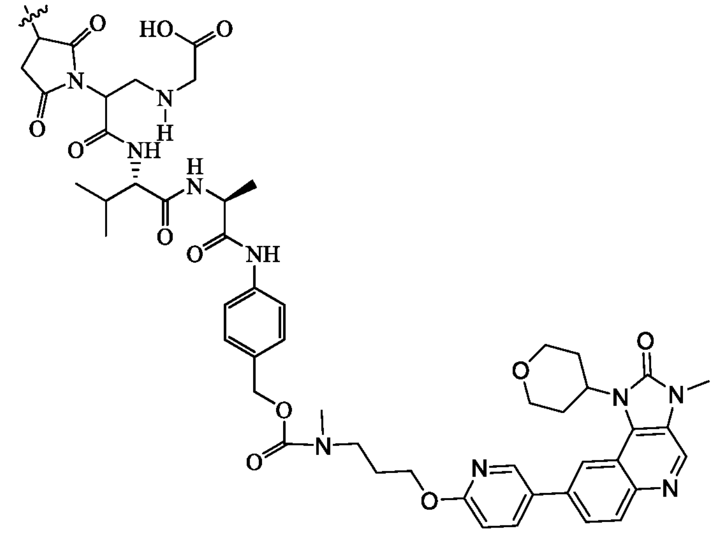 |
| TM02 | 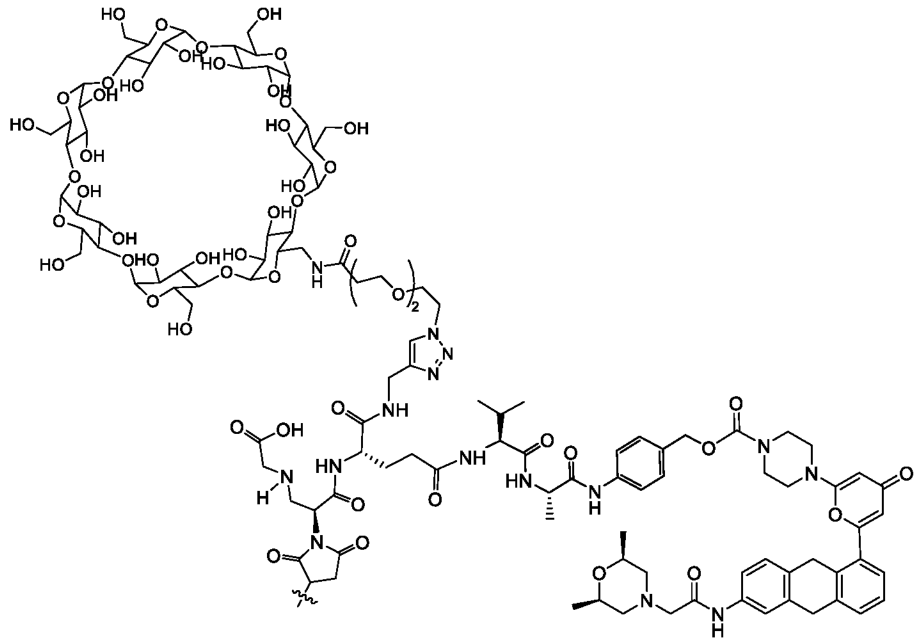 |

(continued)

| | |
|---|---|
| WE01 | 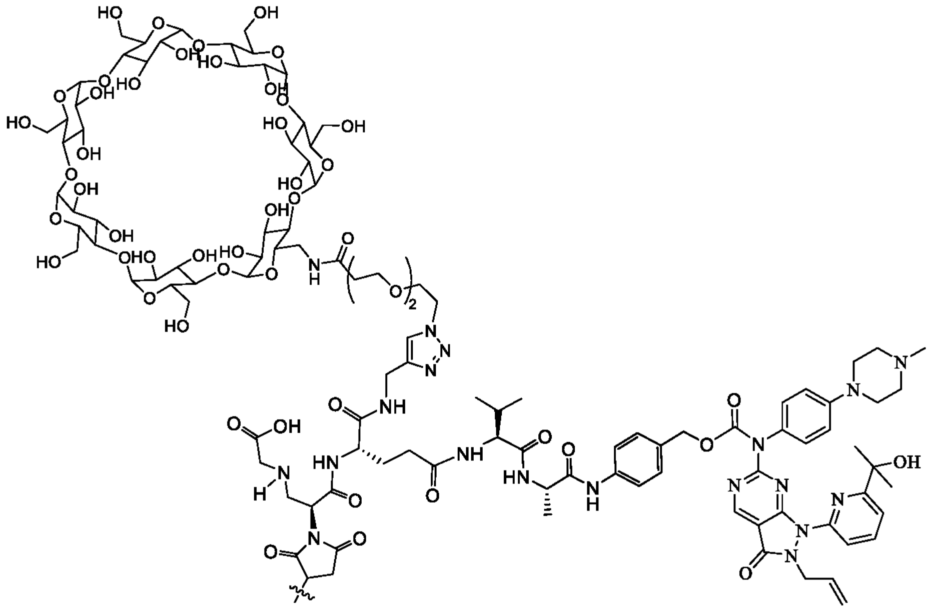 |
| CH01 | 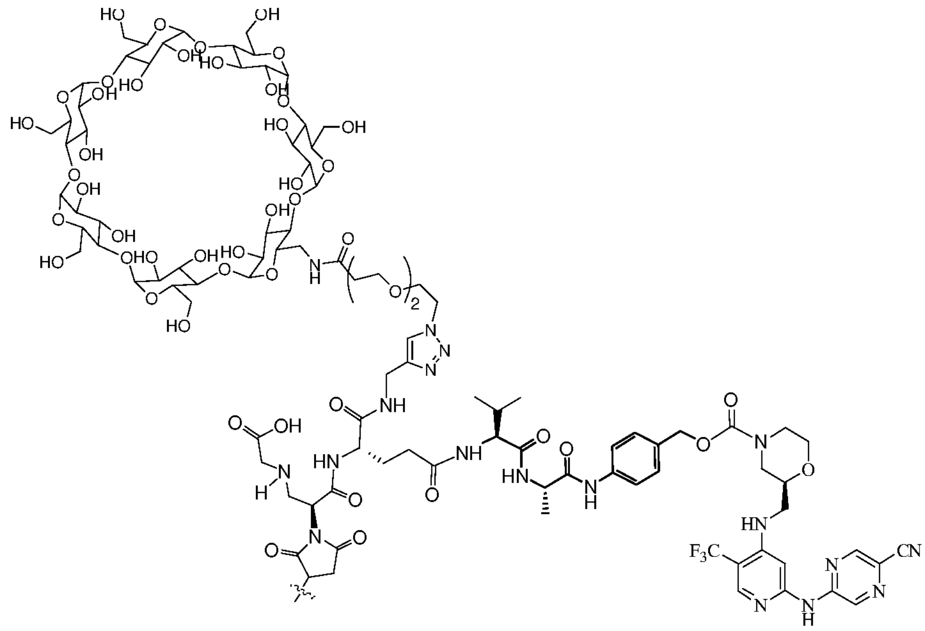 |

(continued)

| | |
|---|---|
| CH02 | 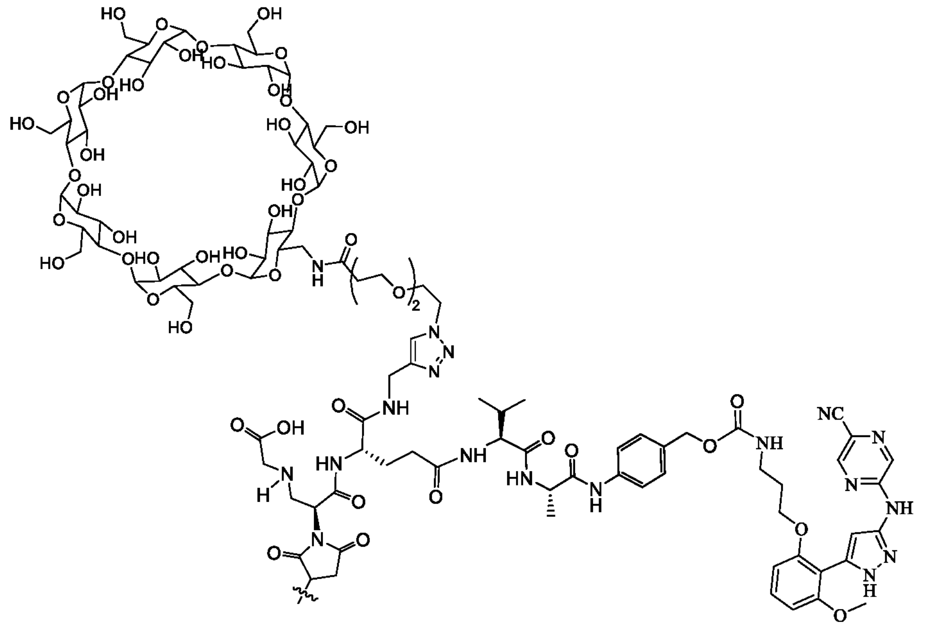 |
| CH03 | 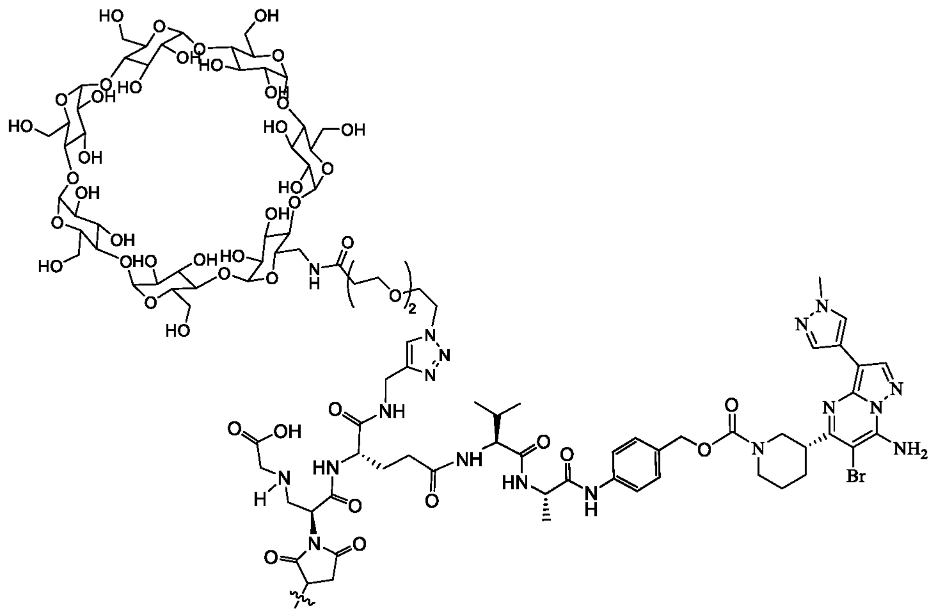 |

(continued)

| | |
|---|---|
| CH04 | 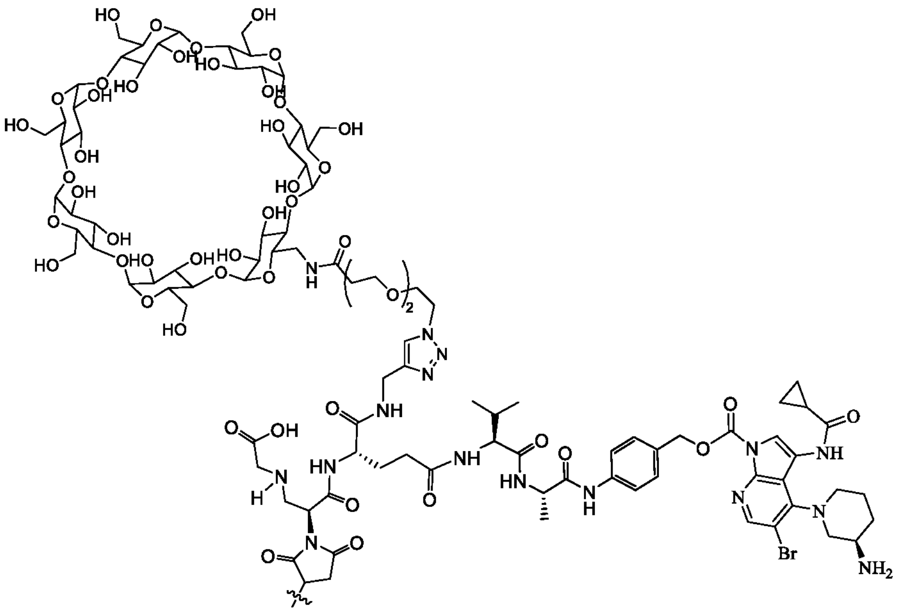 |
| DP01 | 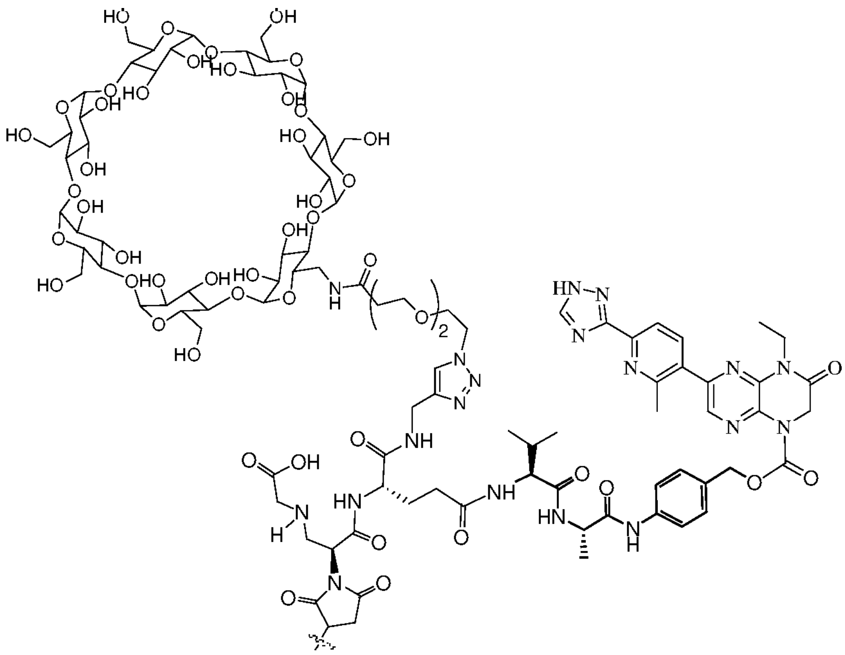 |

(continued)

| | |
|---|---|
| DP02 | 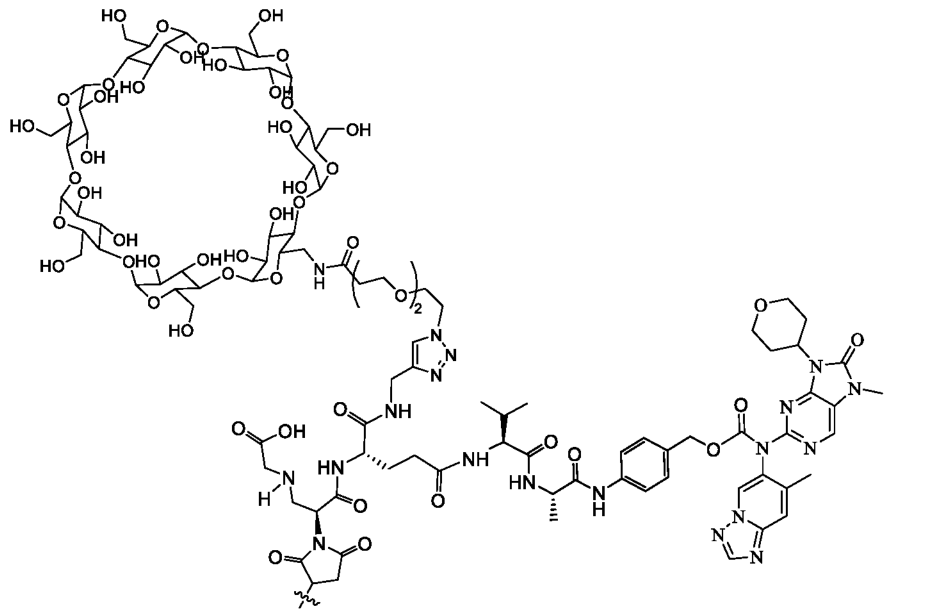 |
| DP03 | 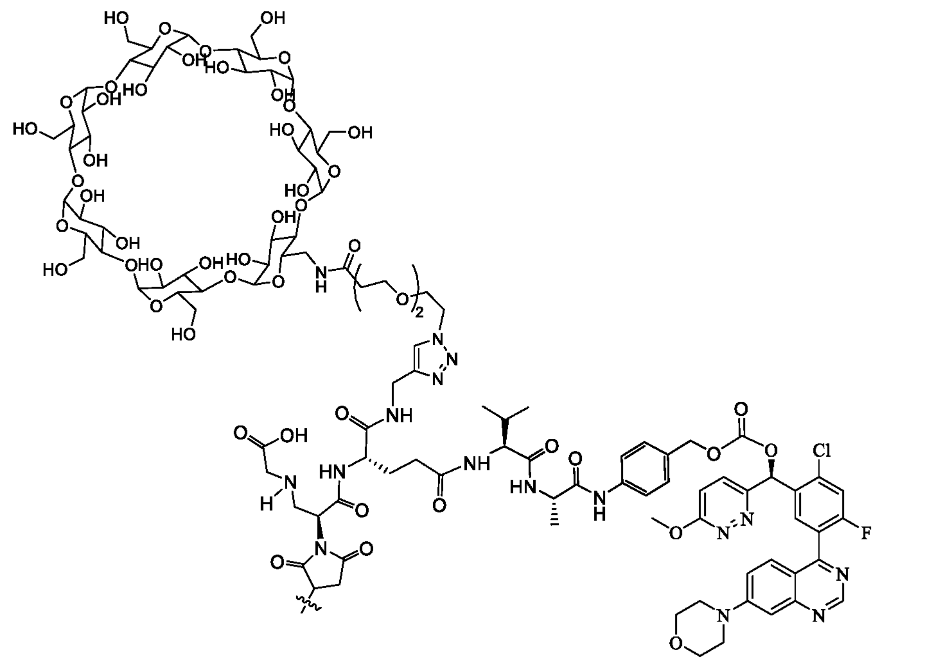 |

In certain embodiments, the first drug is unrestrictedly selected from the group consisting of a tubulin binding agent, a DNA damage agent, an enzyme inhibitor, an immunomodulator, a peptide and a nucleotide.

In certain embodiments, the first drug is selected from the group consisting of:

a) alkylating agents, PBD and its derivatives, chlorpheniramate, chlorpromazine, cyclophosphamide, dacarbazine, estramustine, ifosfamide, mechlorethamine, dimethoxyamine hydrochloride, mechlorethamine oxide, amlodipine hydrochloride, mycophenolic acid, dulcitol, pipobroman, novoembichin, Fenesterin, prednimustine, thiotepa, trofosfamide, uracil; CC-1065; docamycin; benzodiazepine dimer; nitrosourea; alkyl sulfonate; triazene; platinum-containing compounds; aziridines, such as chromanone, quinolone, meturedepa, and madopar; ethyleneimine and methyl melamine including hexamethylmelamine, triethylenetriamine, triethylphosphoramide, triethylenethiophosphoramide and trimethylolmethylamine and derivatives and deuterides thereof;

b) plant alkaloids, vinca alkaloids, taxol and analogs thereof, maytansinoids and analogs thereof, cryptophycin, epothilone, eleutherobin, discodermolide, bryostatin, aplysiatoxin, auristatins, tubulysin, cephalostatin, pancratistatin, sarcodictyin, spongistatin, Monomethyl auristatin E (MMAE), Monomethyl auristatin F (MMAF), Maytansinoid

(e.g., Maytansine DM1, Maytansine DM4), amatoxin and derivatives and deuterides thereof;

c) DNA topoisomerase inhibitors, etoposide, teniposide, 9-aminocamptothecin, camptothecin, crisnatol, daunomycin, etoposide phosphate, irinotecan, mitoxantrone, novantrone, retinoic acid, teniposide, topotecan, 9-nitrocamptothecin, SN38, mitomycin and derivatives and deuterides thereof;

d) antimetabolites, antifolates, DHFR inhibitors; MP dehydrogenase inhibitors; ribonucleotide reductase inhibitors; pyrimidine analogs, uracil analogs; cytosine analogs; purine analog; folic acid supplements;

e) hormone therapy agents, receptor antagonists, antiestrogens, LHRH agonists; antiandrogens; retinoids, vitamin D3 analogs; photodynamic therapy agents; cytokines, TNF containing human proteins and derivatives and deuterides thereof;

f) kinase inhibitors, BIBW2992, imatinib, gefitinib, pegaptanib, sorafenib, dasatinib, sunitinib, erlotinib, nilotinib, lapatinib, axitinib, pazopanib, vandetanib, E7080, mubritinib, ponatinib, bafetinib, bosutinib, cabozantinib, vismodegib, iniparib, ruxolitinib, CYT387, axitinib, tivozanib, sorafenib, bevacizumab, cetuximab, trastuzumab, ranibizumab, panitumumab, ispinesib and derivatives and deuterides thereof;

g) antibiotics, enediyne antibiotics, aclacinomysins, anthramycin, amrinomycin, azaserine, bleomycin, cetocycline, kalamycin, carminomycin, carcinophylin, doxorubicin, morpholino doxorubicin, 2-pyrroline doxorubicin and deoxydaunorubicin, epirubicin, aclarubicin, idarubicin, marcomycin, mycin, mycophenolic acid, lopimycin, pelomycin, puromycin, triferricdoxorubicin, streptozotocin, streptozotocin, tubercidin, ubenimex, zinostatin, zorubicin, PNU-159682 and derivatives and deuterides thereof;

h) polyketides, in particular bullatacin and bullatacinone; gemcitabine, epoxomicin, bortezomib, thalidomide, lenalidomide, pomal idomide, tosedostat, zybrestat, PLX4032, STA-9090, Stimuvax, allovectin-7, Xegeva, Provenge, Yervoy, prenylation inhibitors, dopaminergic neurotoxins, actinomycin, bleomycin, anthracycline antibiotics, doxorubicin, idarubicin, epirubicin, larrubicin, zorubicin, mitoxantrone, MDR inhibitors, Ca2+ ATPase inhibitors, histone deacetylase inhibitors, celecoxib, glitazones, epigallocatechin gallate, disulfiram, Salinosporamide A; anti-adrenal agents, such as aminoglutethimide, mitotane, trilostane, aceglatone, aldphosphoramide, aminolevulinic acid, amsacrine, arabinoside, bestrAbuci, bisantrene, edatraxate, defofamine, Meikexin, diaziquone, eflornithine, elfomithine, celiptium, ethylgluconic acid, gallium nitrate, cytosine, hydroxyurea, ibandronate, lentinan, lonidamine, mitoguazone, mitoxantrone, mopidamol, nitracrine, pentostatin, phenamet, pirarubicin, podophyllic acid, 2-ethyl hydrazine, procarbazine; PSK®; razoxane; rhizomycin; sizzo; spirogermanium; tenuazonic acid, triaziquone; trichlorotriethylamine; trichothecene, polyurethanes, siRNA and antisense drugs and derivatives and deuterides thereof.

In certain embodiments, the first drug D1 is a camptothecin-based compound or a deuteride thereof.

In certain embodiments, the camptothecin-based compound has a structure represented by formula B,

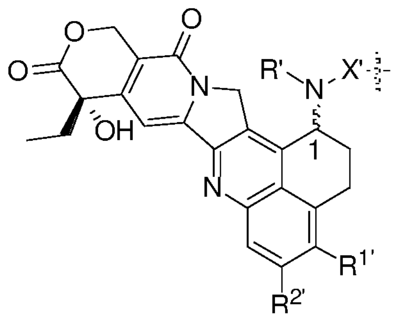

formula B

wherein:

the chiral carbon atom No. 1 linked to the N atom has absolute chirality in either R or S configuration;

R' is selected from the group consisting of H, D, halogen, alkyl, substituted alkyl, deuterated alkyl, cycloalkylalkyl, alkoxyalkyl, aryl, substituted aryl and heteroaryl;

$R^{1'}$ is selected from the group consisting of H, D, halogen, alkyl, substituted alkyl, deuterated alkyl, cycloalkylalkyl, alkoxyalkyl, carboxyl, heterocyclyl, aryl, substituted aryl and heteroaryl;

$R^{2'}$ is selected from the group consisting of H, D, halogen, alkyl, substituted alkyl, deuterated alkyl, cycloalkylalkyl, alkoxyalkyl, carboxyl, heterocyclyl, aryl, substituted aryl and heteroaryl;

X' is selected from the group consisting of $-C(O)-(CR^{3'}R^{4'})t-CR^{a'}R^{b'}-O-$, $-C(O)-(CR^{3'}R^{4'})t-CR^{a'}R^{b'}-NH-$ and $-C(O)-(CR^{3'}R^{4'})t-CR^{a'}R^{b'}-S-$, preferably, its carbonyl terminal is linked to N atom;

$R^{a'}$ is selected from the group consisting of H, D, halogen, alkyl, deuterated alkyl, haloalkyl, cycloalkyl, cycloalkylalkyl, alkoxyalkyl, heterocyclyl, aryl, substituted aryl and heteroaryl;

$R^{b'}$ is selected from the group consisting of H, D, halogen, alkyl, deuterated alkyl, haloalkyl, cycloalkyl, cycloalkylalkyl, alkoxyalkyl, heterocyclyl, aryl, substituted aryl and heteroaryl;

or, $R^{a'}$, $R^{b'}$ and the carbon atom they are linked to form C3-C6 cycloalkyl, cycloalkylalkyl or heterocyclyl;

$R^{3'}$, $R^{4'}$, the same or different, each are independently selected from the group consisting of H, D, halogen, alkyl, haloalkyl, deuterated alkyl, alkoxy, hydroxy, amino, cyano, nitro, hydroxyalkyl, cycloalkyl and heterocyclyl;

or, $R^{3'}$, $R^{4'}$ and the carbon atom they are linked to form C3-C6 cycloalkyl, cycloalkylalkyl or heterocyclyl;

the right wavy line linked to X' is linked to $L_1$;

t is an integer selected from 0 to 4 (e.g., 0, 1, 2, 3 or 4).

In certain embodiments, R' is selected from the group consisting of H, and C1-C6 alkyl.

In certain embodiments, R' is H.

In certain embodiments, $R^{1'}$ is selected from the group consisting of H, and C1-C6 alkyl.

In certain embodiments, $R^{1'}$ is selected from C1-C6 alkyl.

In certain embodiments, $R^{1'}$ is methyl.

In certain embodiments, $R^{2'}$ is selected from halogen.

In certain embodiments, $R^{2'}$ is F.

In certain embodiments, X' is selected from -C(O)-$(CR^{3'}R^{4})$t-$CR^{a'}R^{b'}$-O-, preferably, its carbonyl terminal is linked to N atom.

In certain embodiments, X' is selected from -C(O)-$CR^{a'}R^{b'}$-O-, preferably, its carbonyl terminal is linked to N atom.

In certain embodiments, $R^{a'}$ is selected from the group consisting of H, and C1-C6 alkyl.

In certain embodiments, $R^{a'}$ is H.

In certain embodiments, $R^{b'}$ is selected from the group consisting of H, D, C1-C6 alkyl, C3-C6 cycloalkyl, and C1-C6 haloalkyl.

In certain embodiments, $R^{b'}$ is selected from the group consisting of H, D, methyl, cyclopropyl, and trifluoromethyl.

In certain embodiments, t is 0.

In certain embodiments, X' is unrestrictedly selected from the group consisting of the following structures:

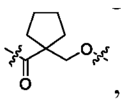, 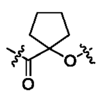, 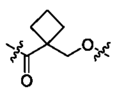, 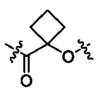, 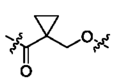, 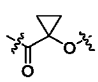,

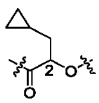, 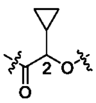, 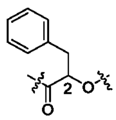, 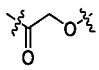, 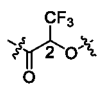, 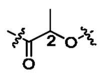 or 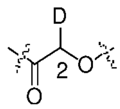;

wherein:

the chiral carbon atom No. 2 has absolute chirality in either R or S configuration;

the left wavy line is linked to N of the compound represented by formula B, and the right wavy line is linked to $L_1$.

In certain embodiments, the compound represented by formula B is selected from the group consisting of

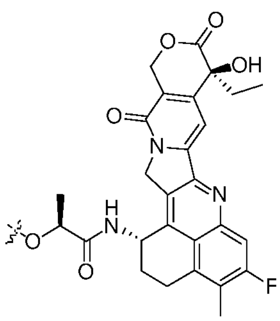, 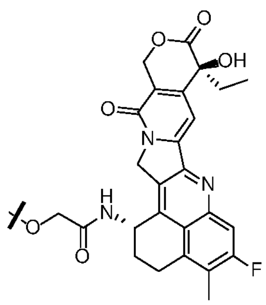, 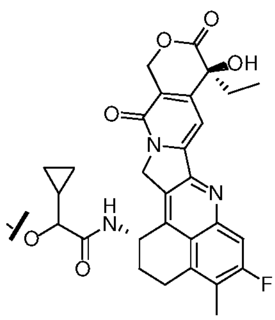,

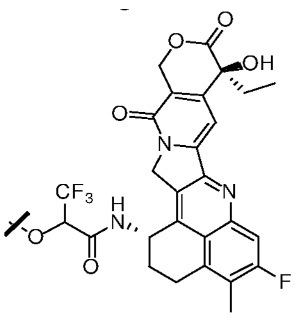, and 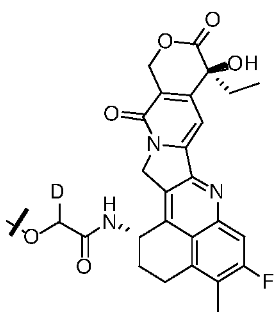.

In certain embodiments, the first drug is selected from the group consisting of

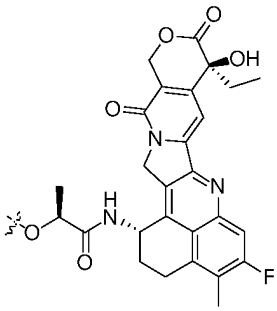, 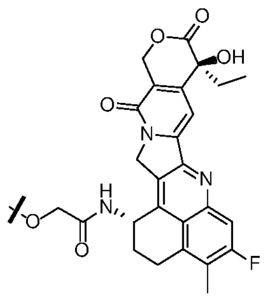, 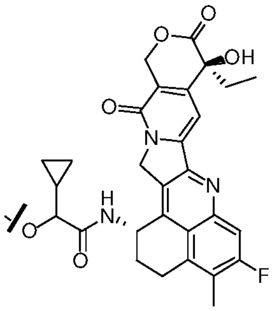, 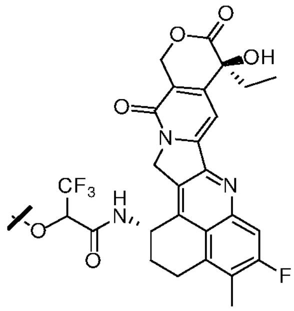,

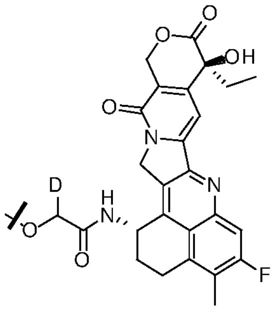, 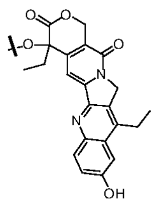, 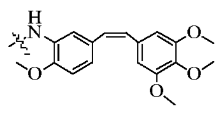, 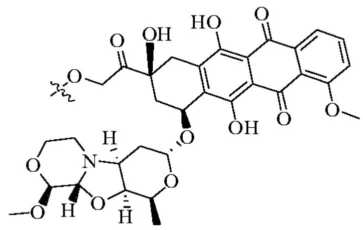,

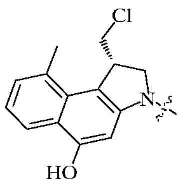, 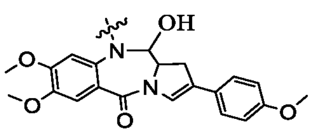, 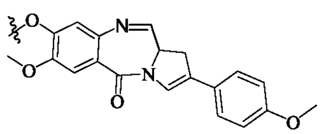,

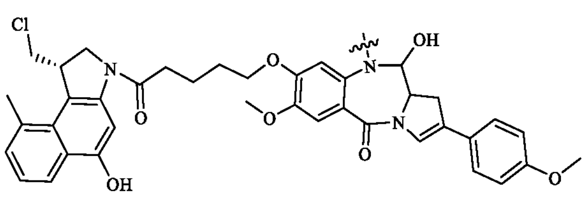, 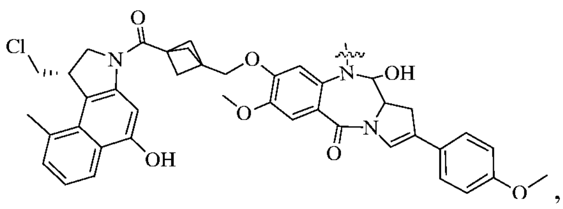, and

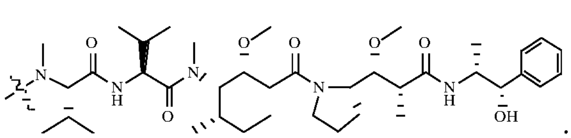.

In certain embodiments, the first linking unit is a breakable or non-breakable linking unit.

In certain embodiments, $L_1$ is $L_{11}$-$L_{12}$-$L_{13}$-$L_{14}$, and $L_{14}$ is linked to $D_1$, $L_{11}$ is linked to Ab (preferably, $L_{11}$ is coupled

to Ab via thiol group),

$L_{11}$ is selected from the group consisting of

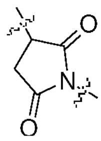,

and

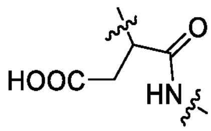,

with its N terminal linked to $L_{12}$,
$L_{12}$ is selected from the group consisting of:

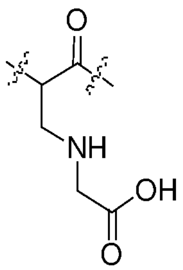,

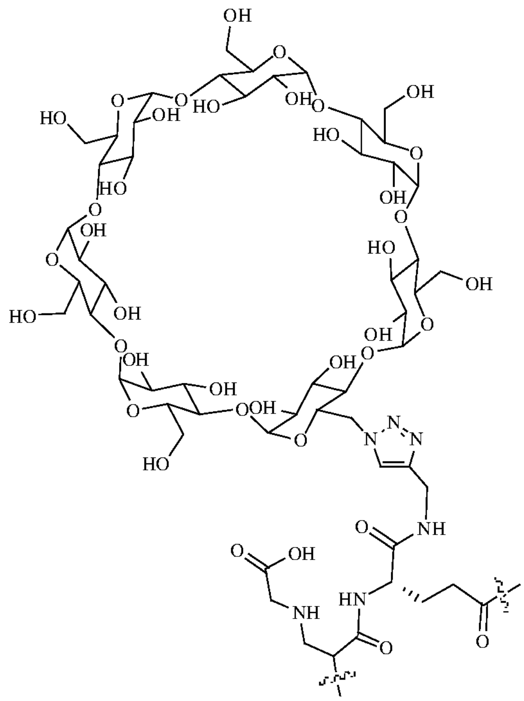,

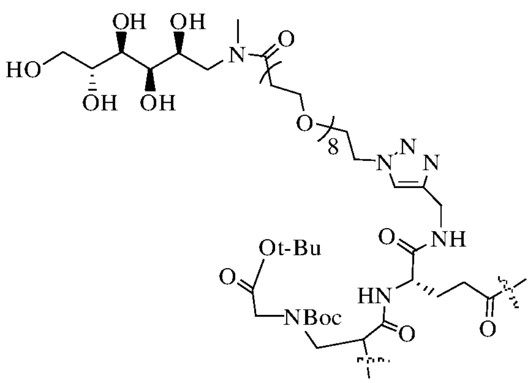,

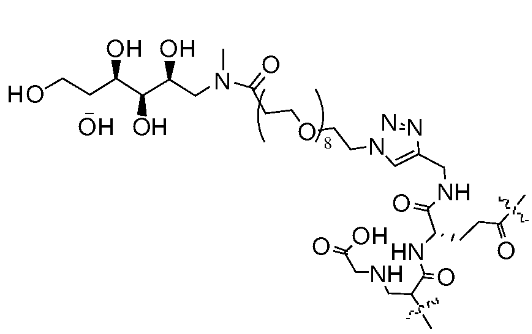,

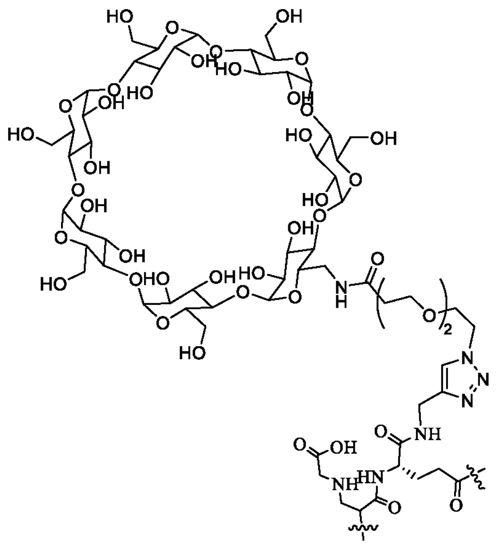 ,

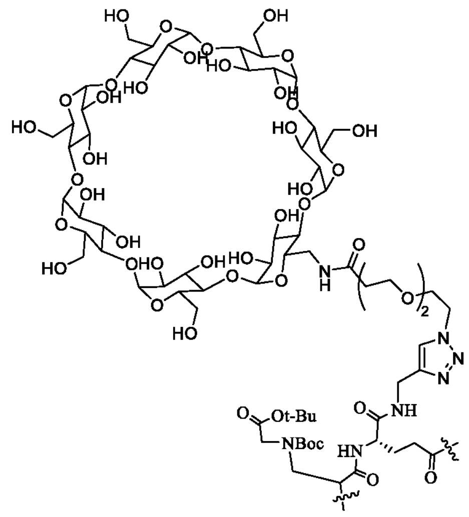 ,

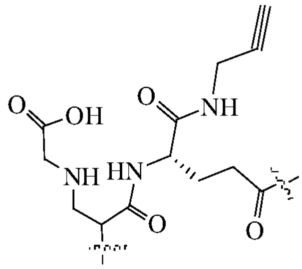 ,

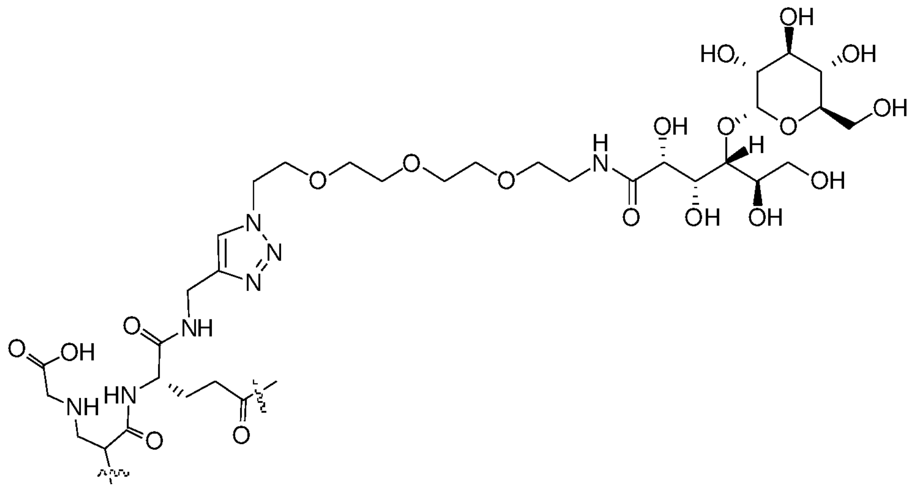 , 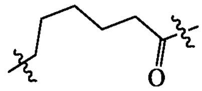 ,

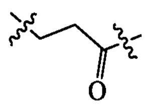 , 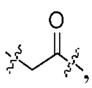 ,

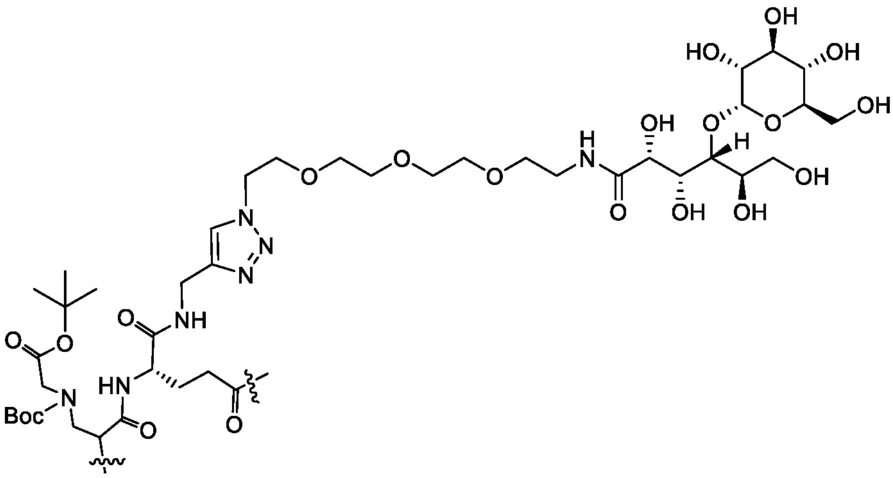

,

and

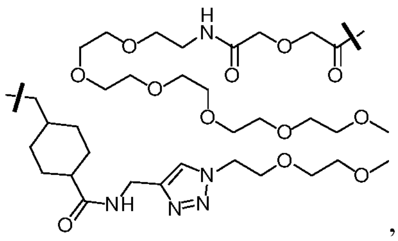

,

with its carbonyl terminal linked to $L_{13}$ or $L_{14}$;

$L_{13}$ is selected from a direct bond, an amino acid residue or a peptide residue consisting of 2-10 amino acid residues, and when $L_{13}$ is selected from an amino acid residue or a peptide residue consisting of 2-10 amino acid residues, its carbonyl terminal is linked to $L_{14}$;

$L_{14}$ is selected from the group consisting of

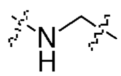

,

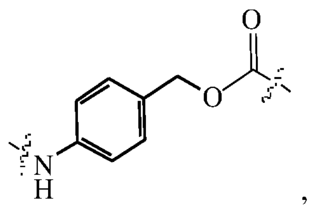

,

and

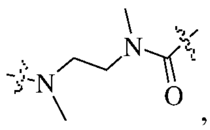

,

with its amino terminal linked to $L_{13}$ or $L_{12}$, and its carbonyl terminal or carbon terminal linked to $D_1$.

In certain embodiments, $L_{13}$ is selected from a direct bond, an amino acid residue, or a peptide residue consisting of 2-4 amino acid residues, and when $L_{13}$ is selected from an amino acid residue, or a peptide residue consisting of 2-4 amino acid residues, its carbonyl terminal is linked to $L_{14}$, preferably, the amino acid is selected from the group consisting of valine, alanine, phenylalanine, glycine, lysine, citrulline, serine, glutamic acid, and aspartic acid, more preferably, the amino acid is selected from the group consisting of valine, alanine, phenylalanine, glycine, citrulline, and lysine.

In certain embodiments, $L_{13}$ is selected from the group consisting of a direct bond, lysine, glycine-glycine-phenylalanine-glycine (Gly-Gly-Phe-Gly), valine-alanine (Val-Ala), and valine-citrulline (Val-Cit), and when $L_{13}$ is selected from the group consisting of lysine, glycine-glycine-phenylalanine-glycine (Gly-Gly-Phe-Gly), valine-alanine (Val-Ala),

and valine-citrulline (Val-Cit), its carbonyl terminal is linked to $L_{14}$.

In certain embodiments, $L_{13}$ is selected from the group consisting of a direct bond,

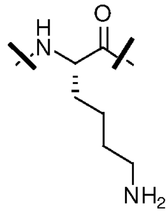, 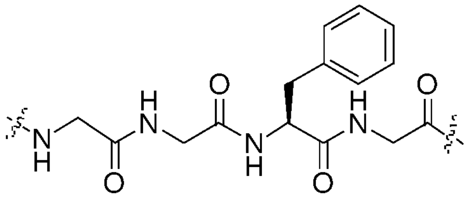, 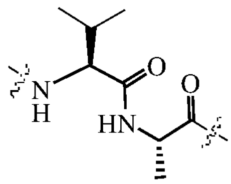, and 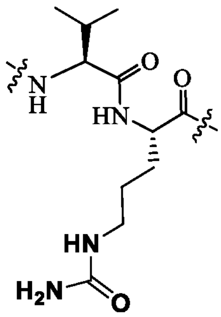,

and when $L_{13}$ is selected from the group consisting of

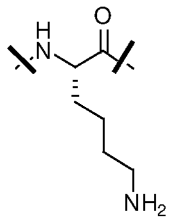,

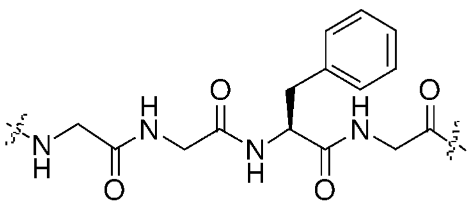, 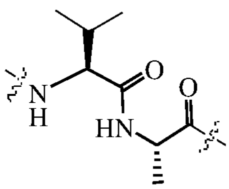, and 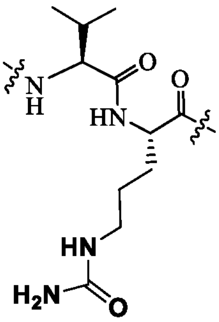,

its carbonyl terminal is linked to $L_{14}$.

In certain embodiments, $L_1$ is selected from the group consisting of:

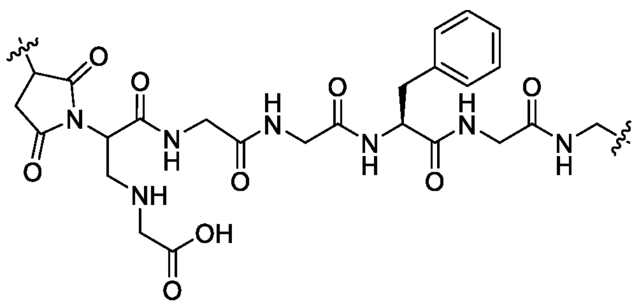,

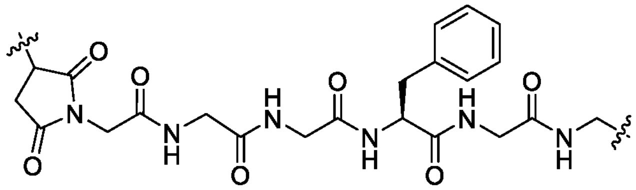,

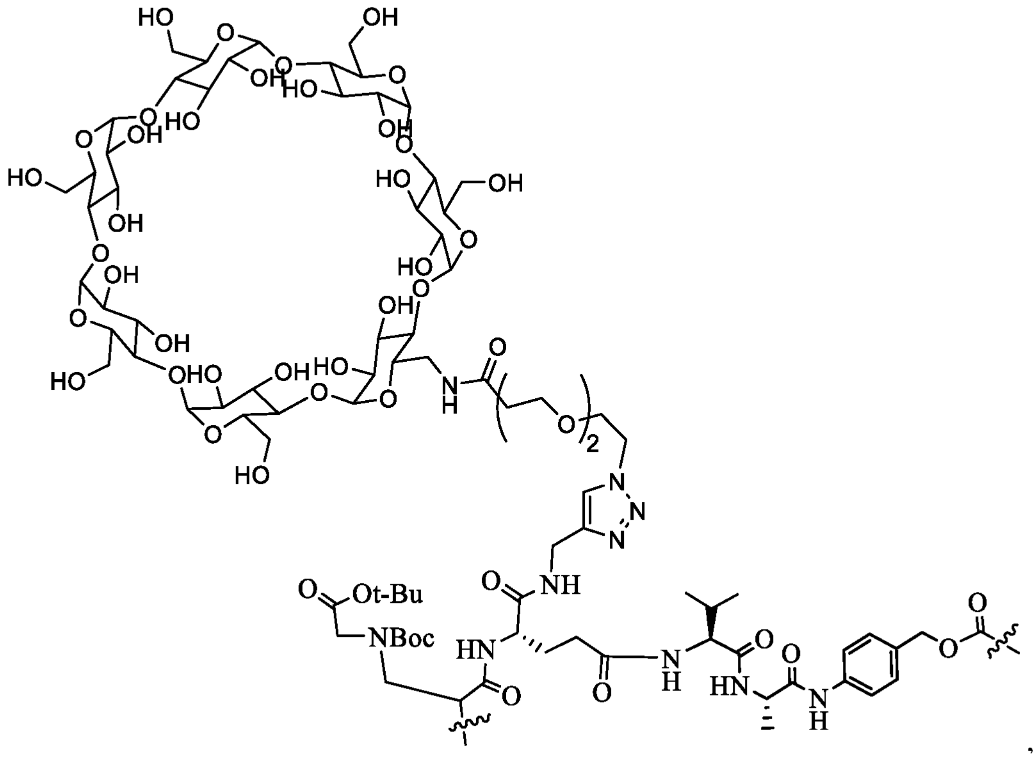

,

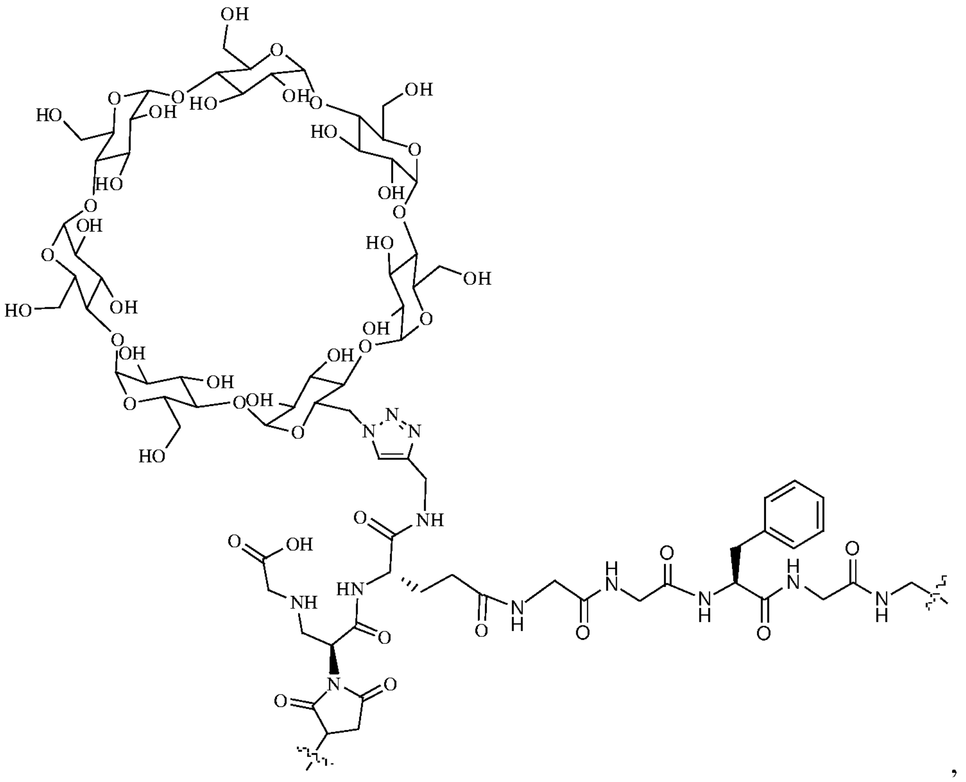

,

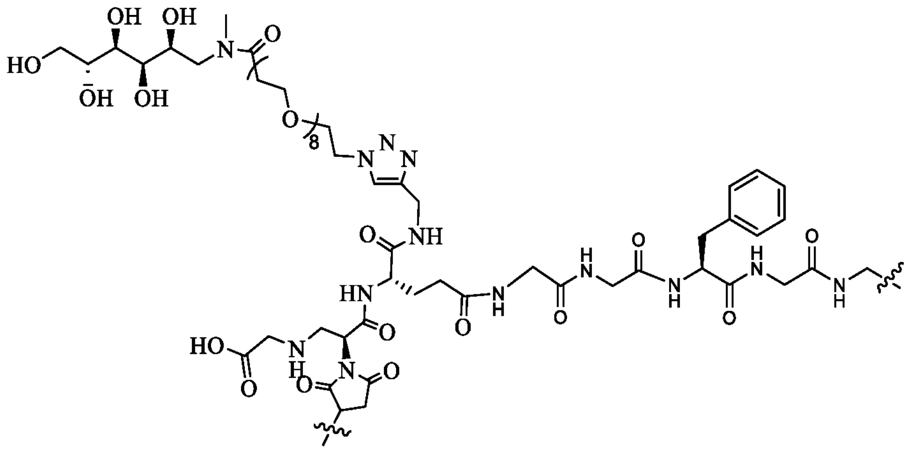

,

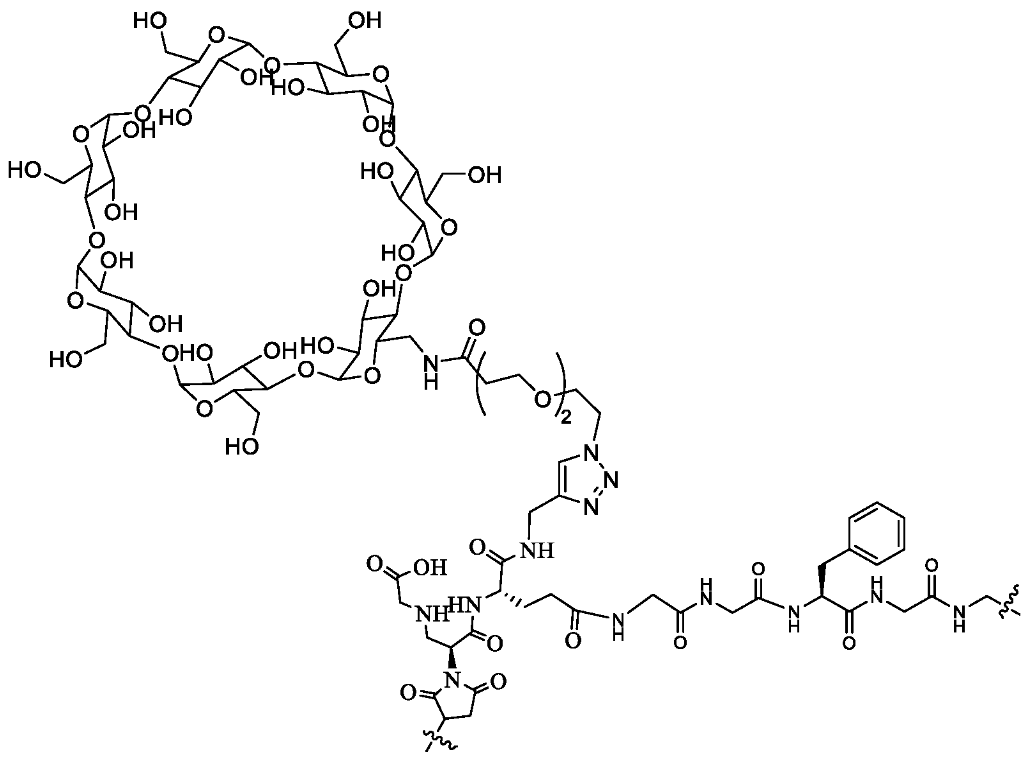

,

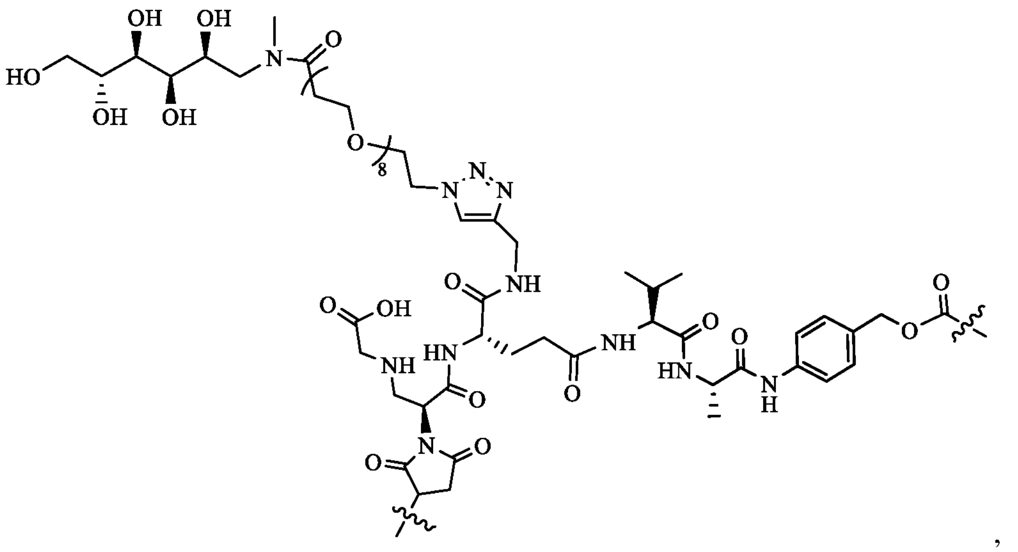

,

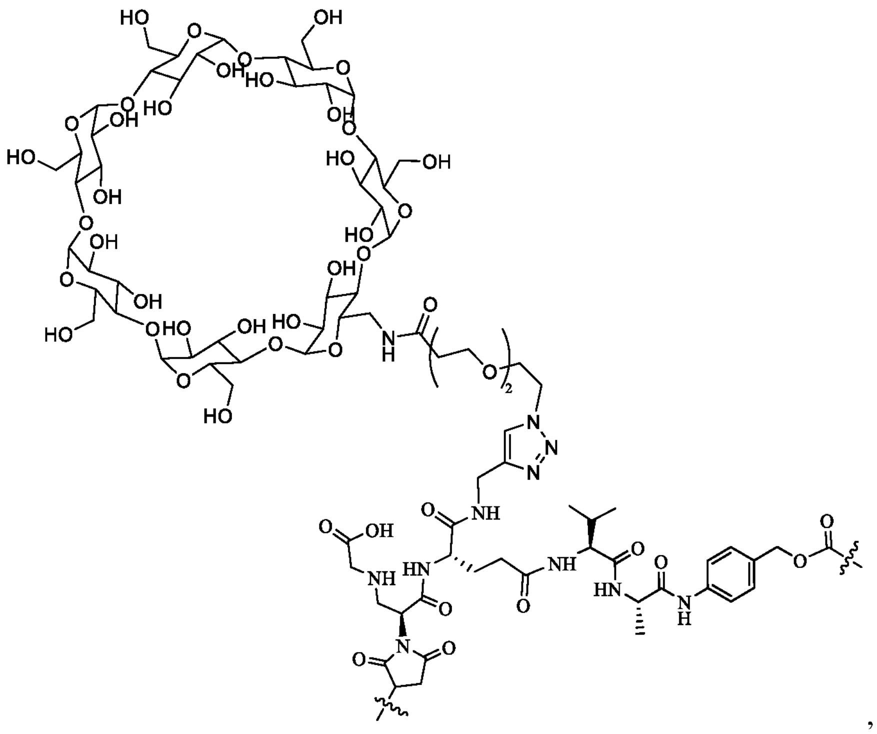

,

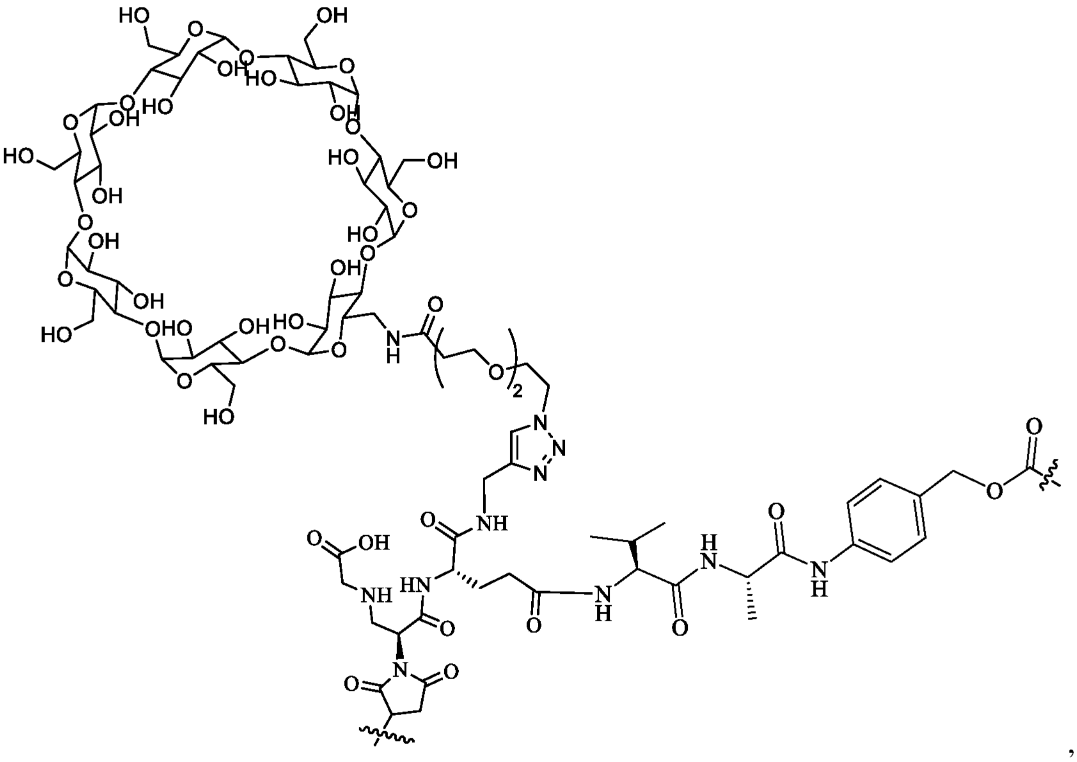

,

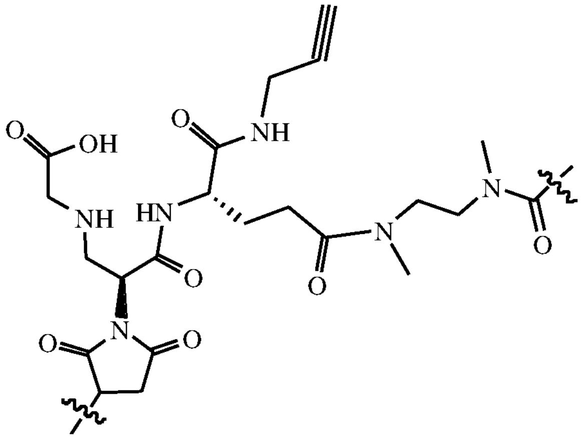

,

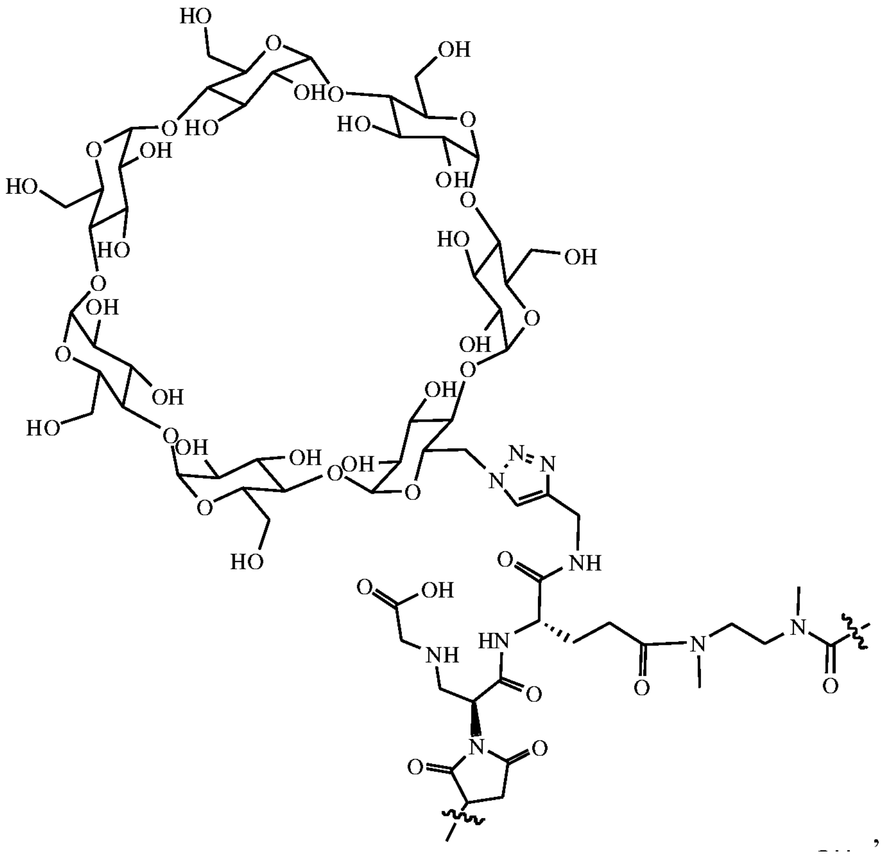

,

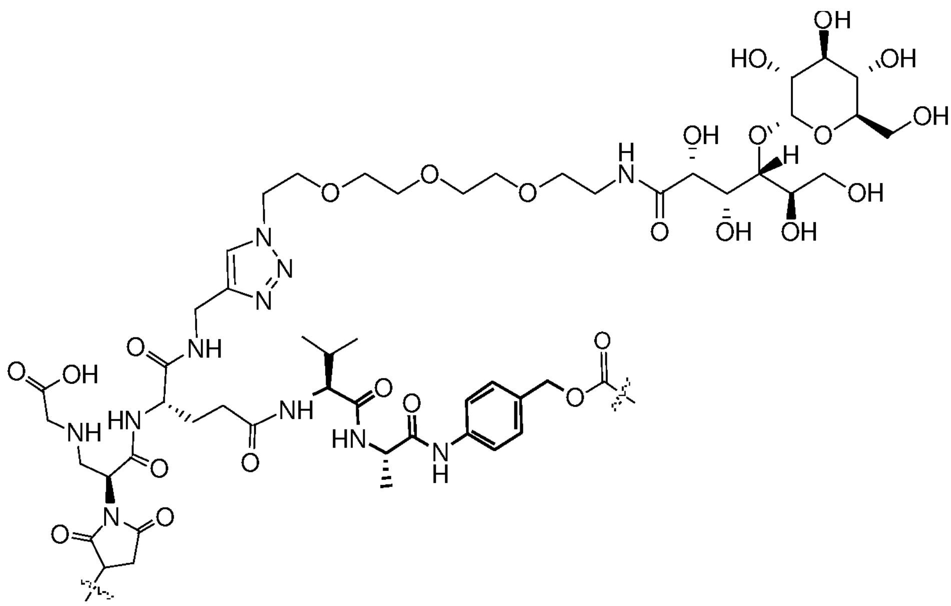

,

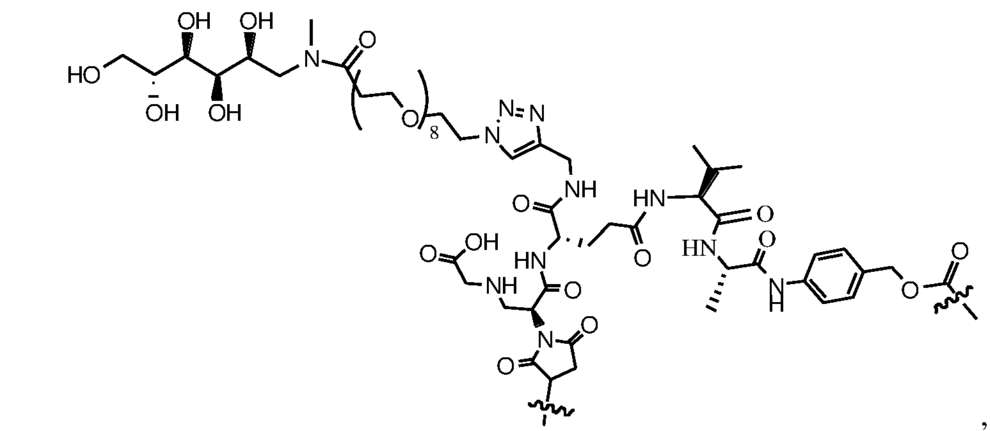

,

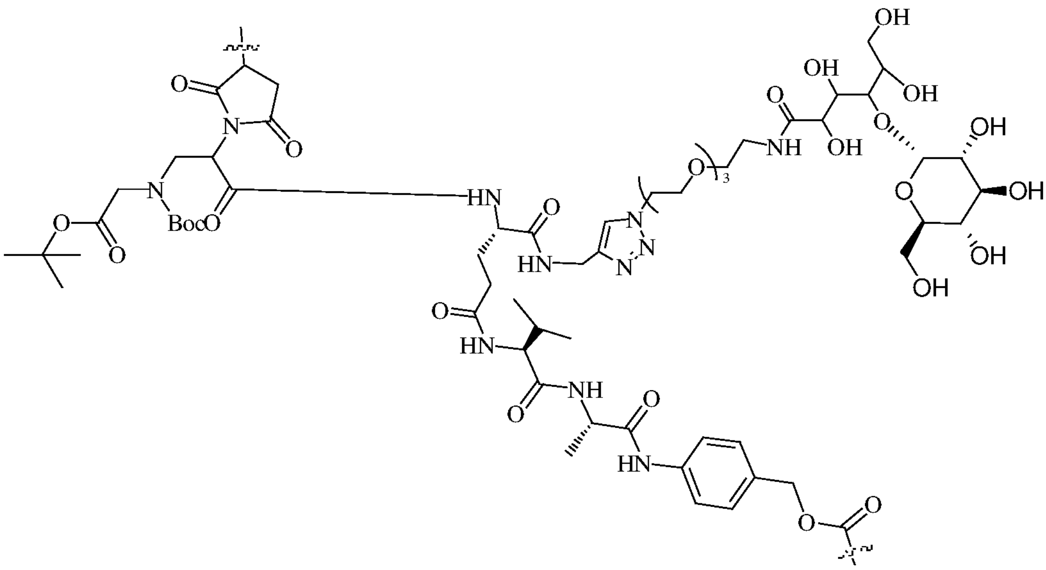

,

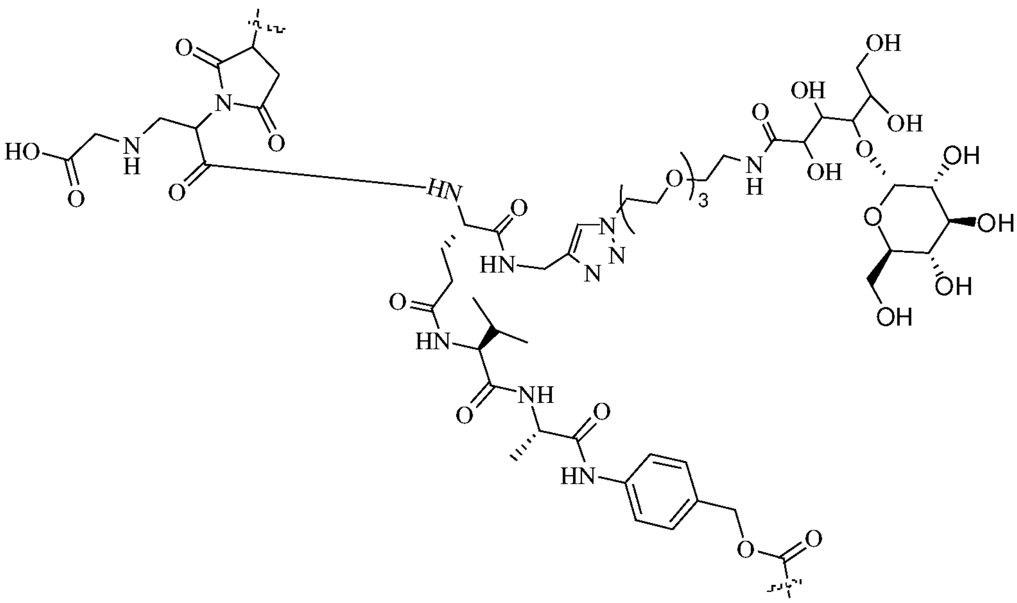

,

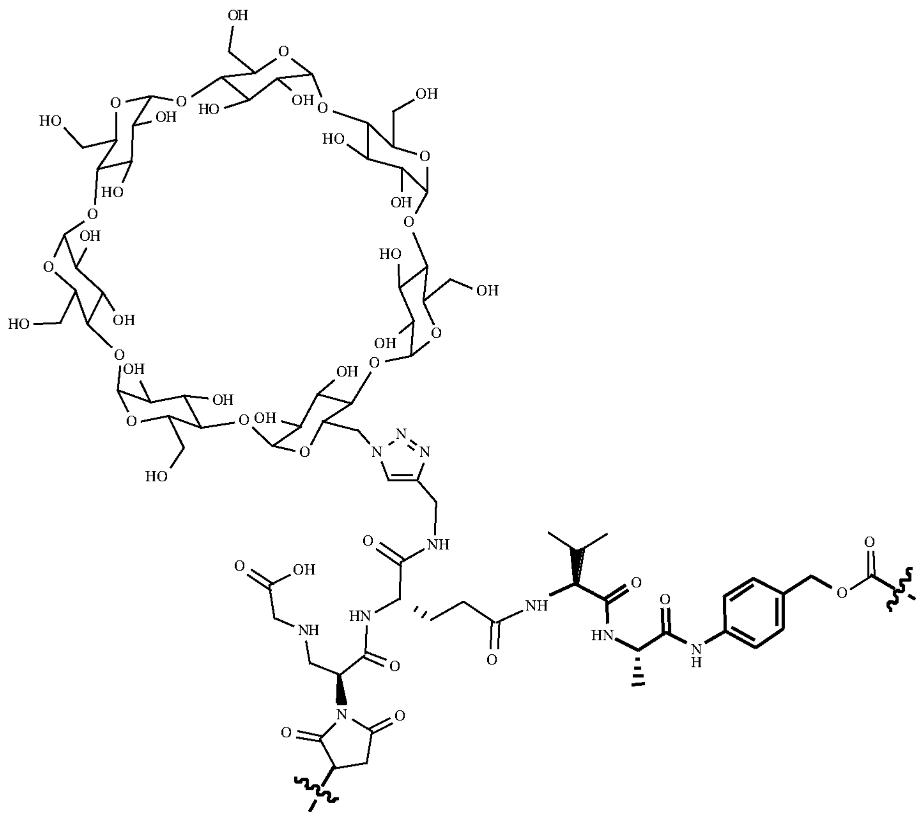

,

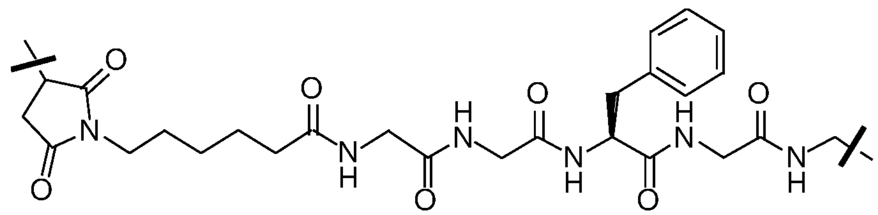

and

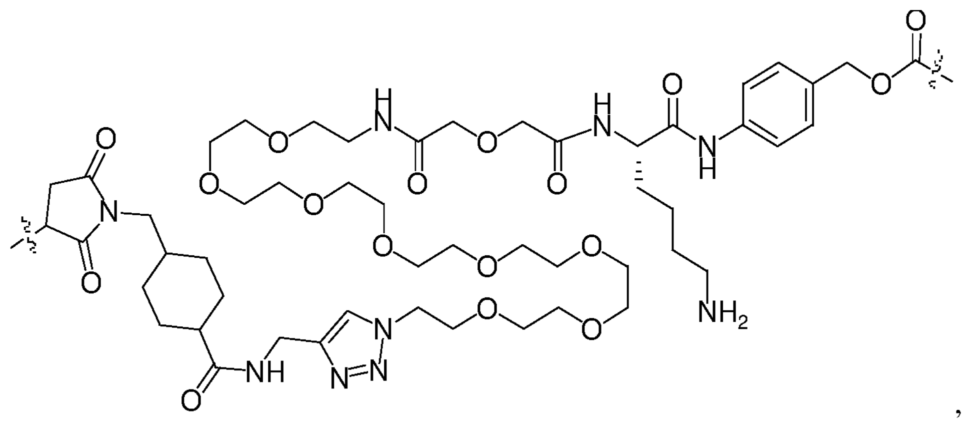

,

its carbonyl terminal or carbon terminal of the end containing

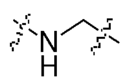

is linked to $D_1$.

In certain embodiments, $D_1$-$L_1$- is selected from the group consisting of:

| | |
|---|---|
| Ex01 | 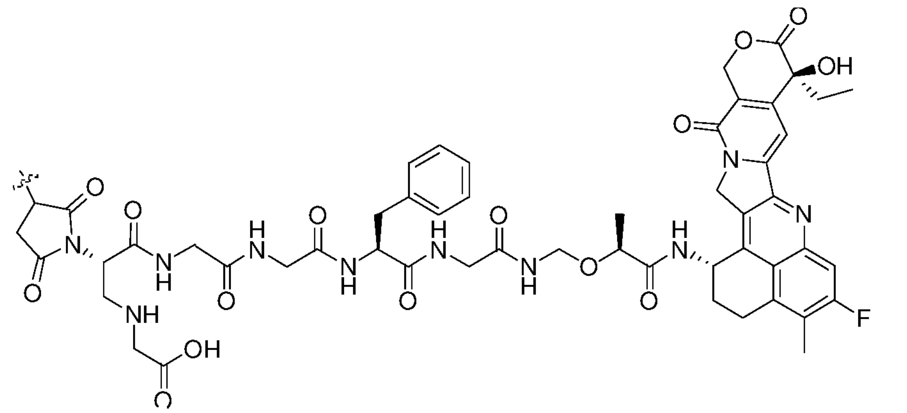 |
| Ex02 | 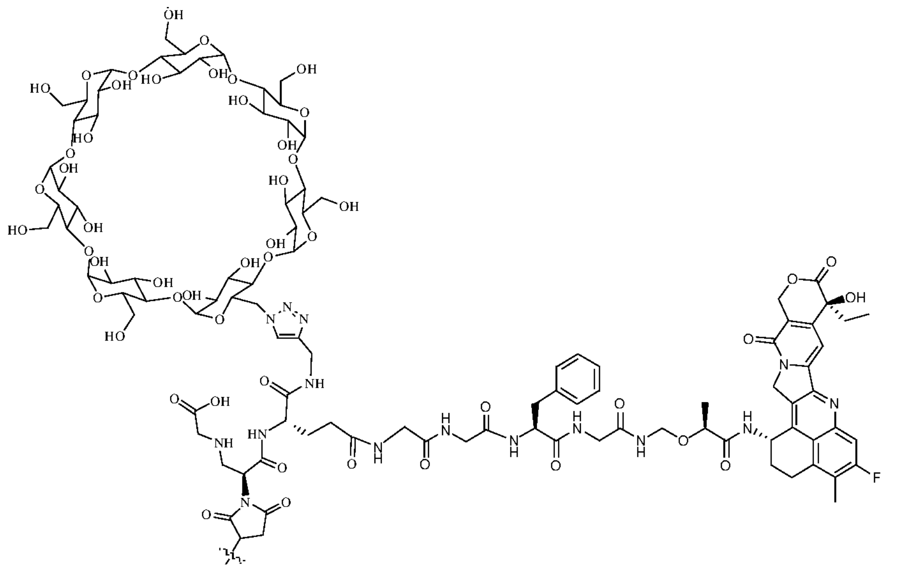 |
| Ex03 | 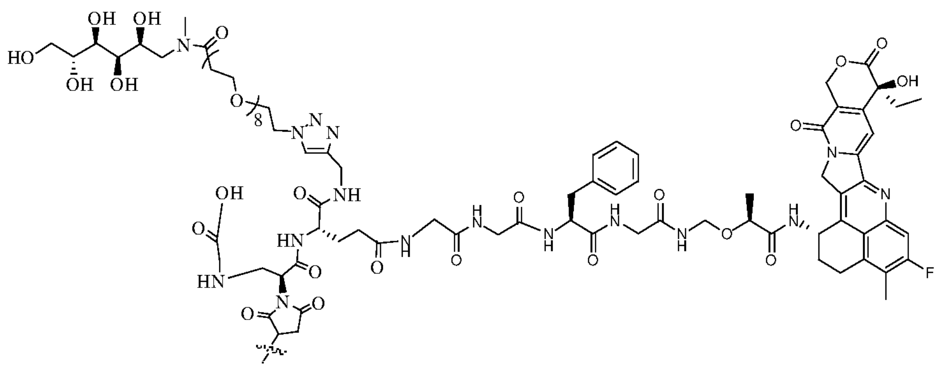 |

(continued)

| | |
|---|---|
| Ex04 | 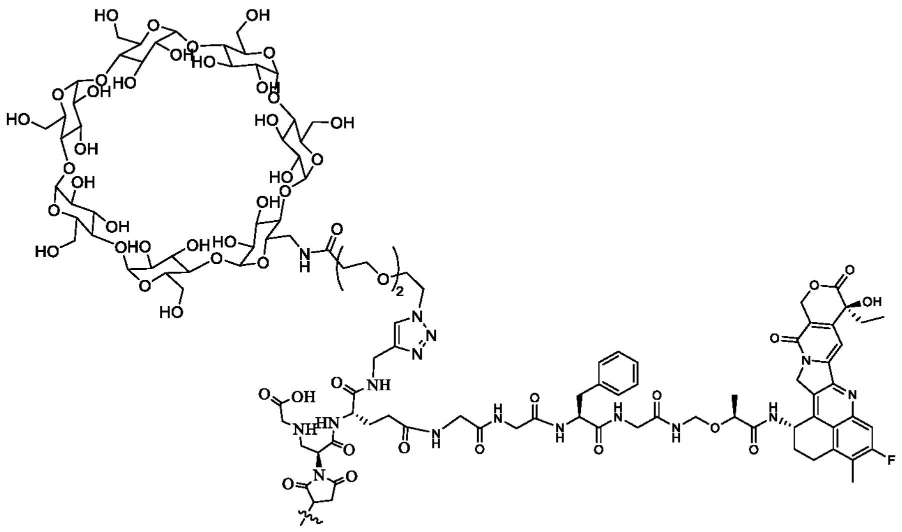 |
| Ex05 | 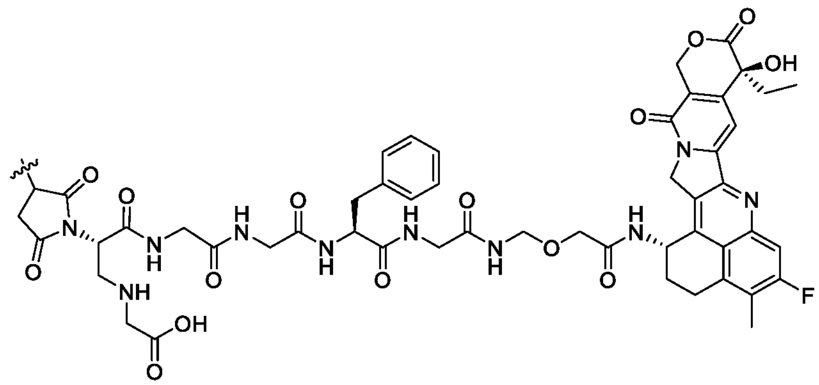 |
| Ex06 | 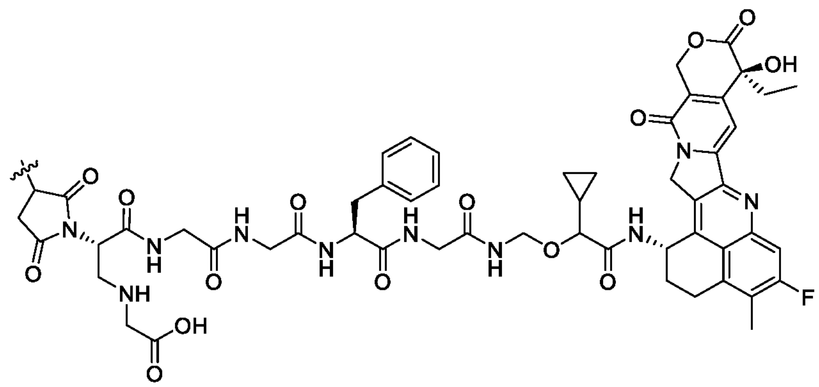 |
| Ex07 | 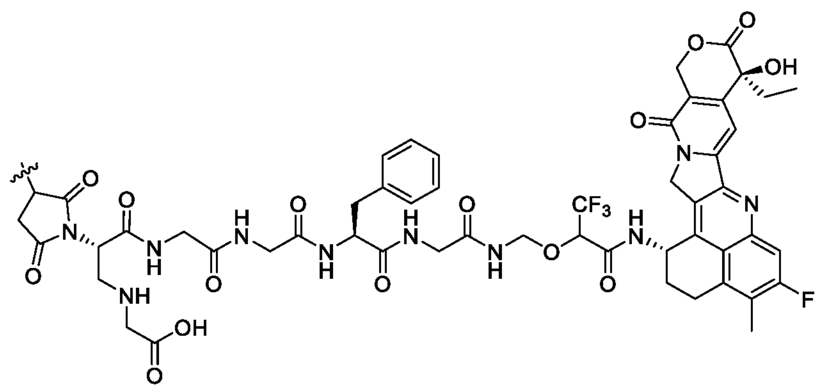 |

(continued)

| | |
|---|---|
| Ex08 | 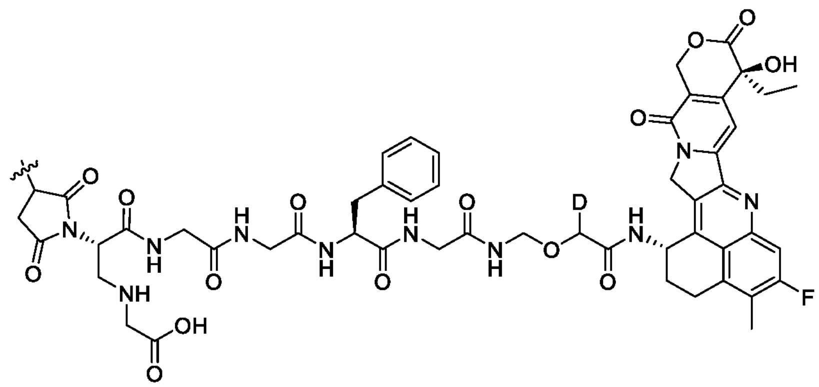 |
| Ex09 | 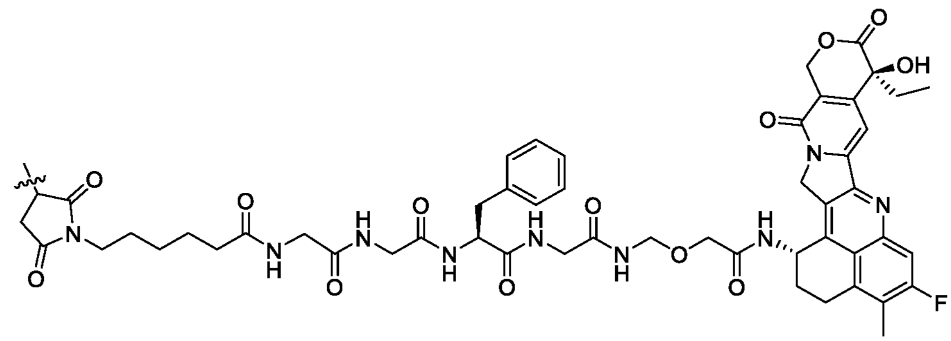 |
| SN01 | 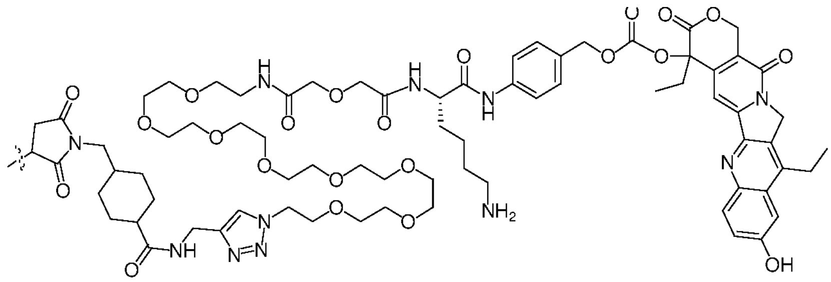 |
| PB01 | 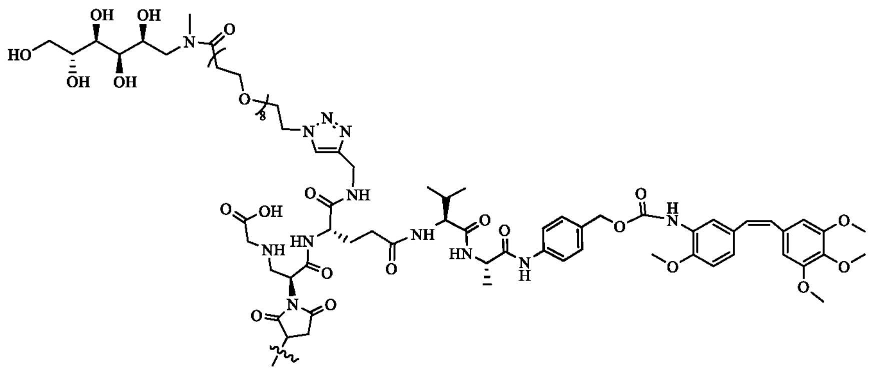 |

(continued)

| | |
|---|---|
| PB02 | 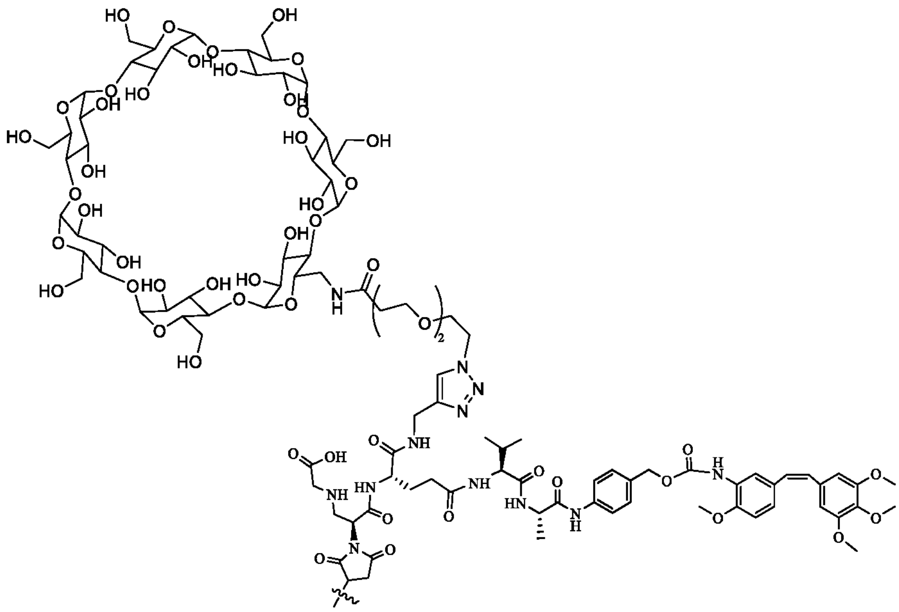 |
| PNU01 | 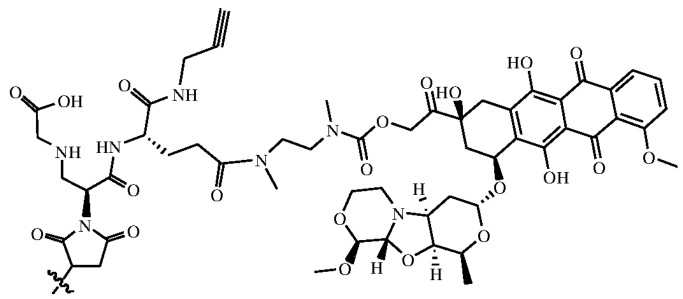 |
| PNU02 | 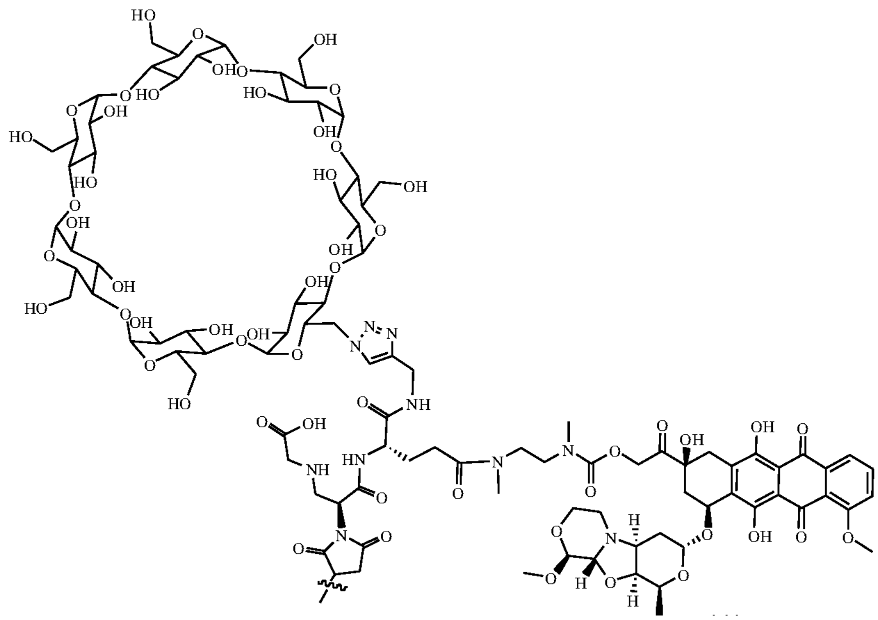 |

(continued)

| | |
|---|---|
| CBI01 | 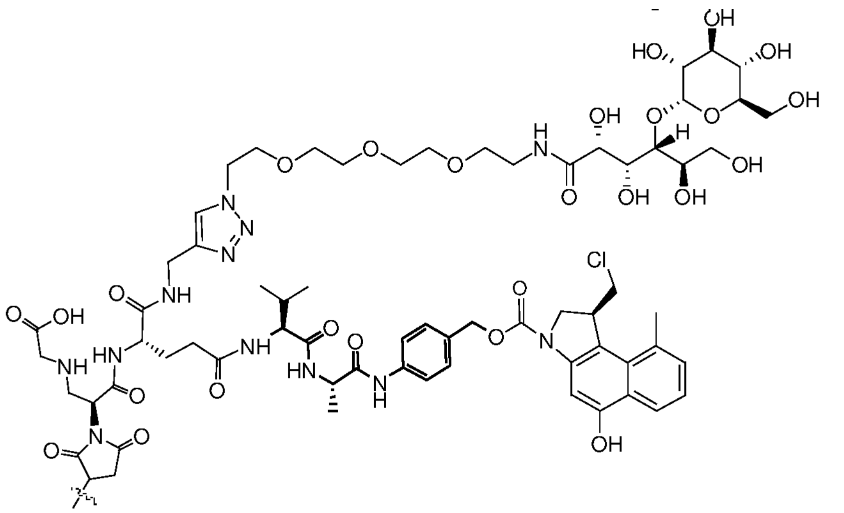 |
| CBI02 | 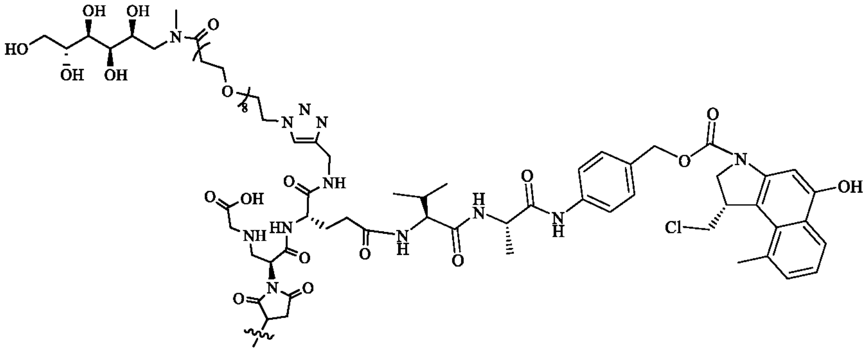 |
| CBI03 | 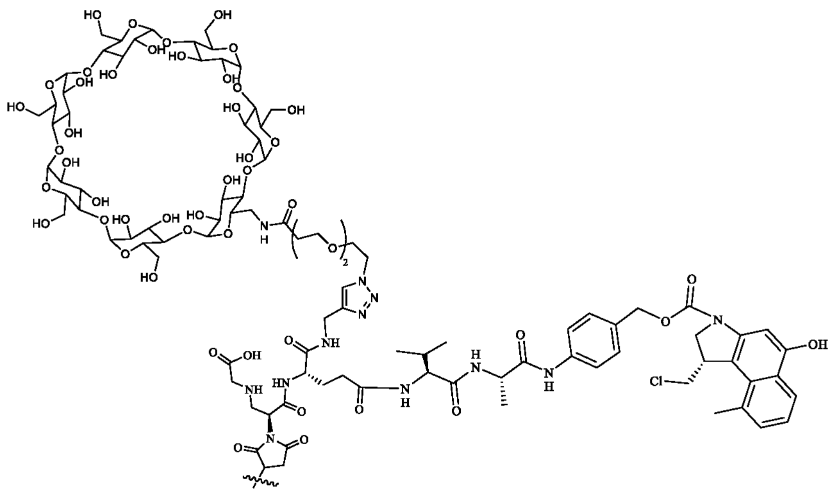 |

(continued)

| | |
|---|---|
| PBD01 | 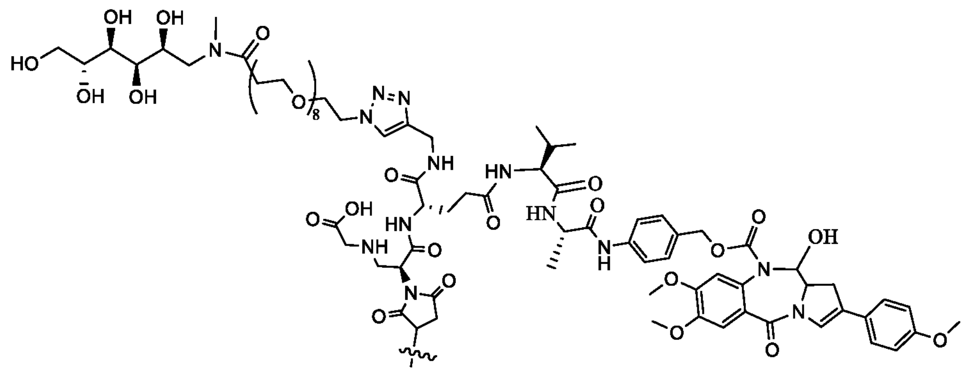 |
| PBD02 | 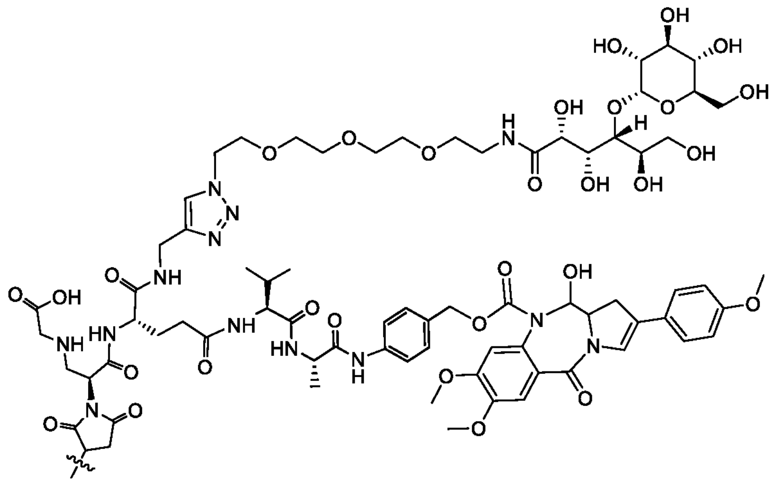 |
| PBD-CBI01 | 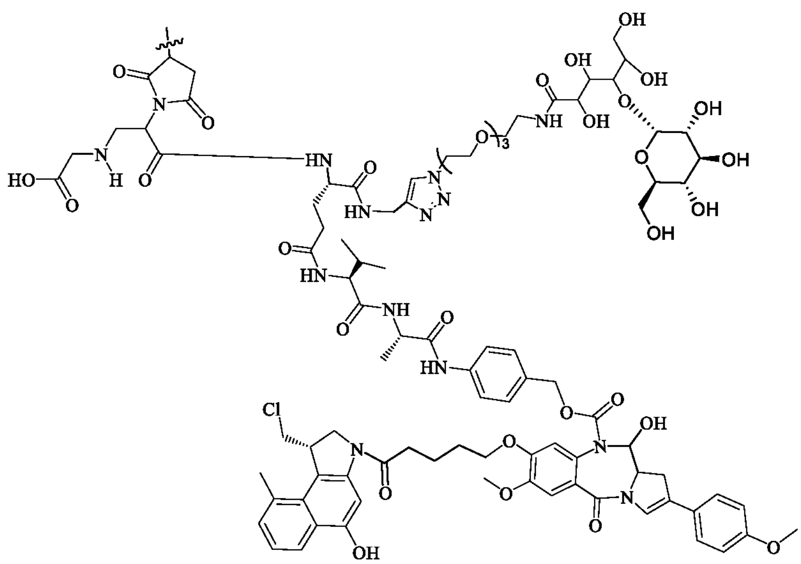 |

(continued)

| | |
|---|---|
| PBD-CBI02 | 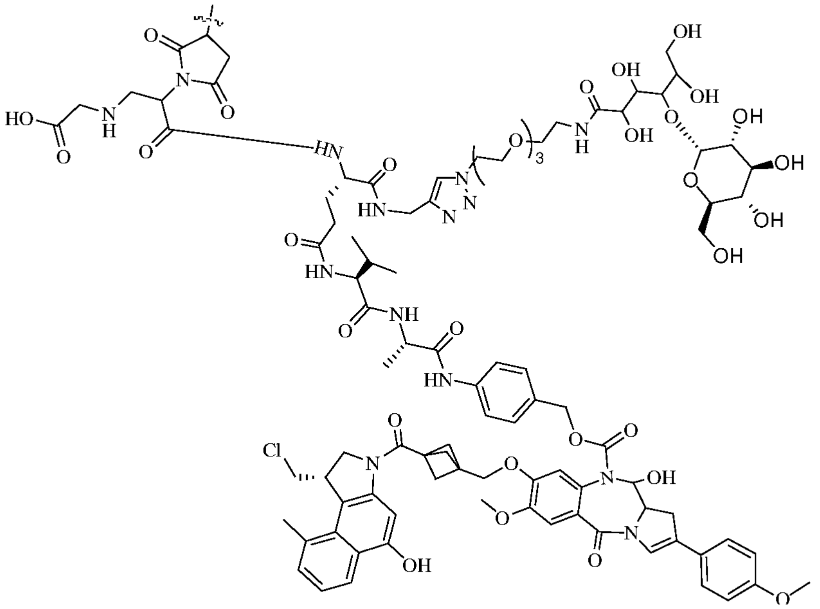 |
| M01 | 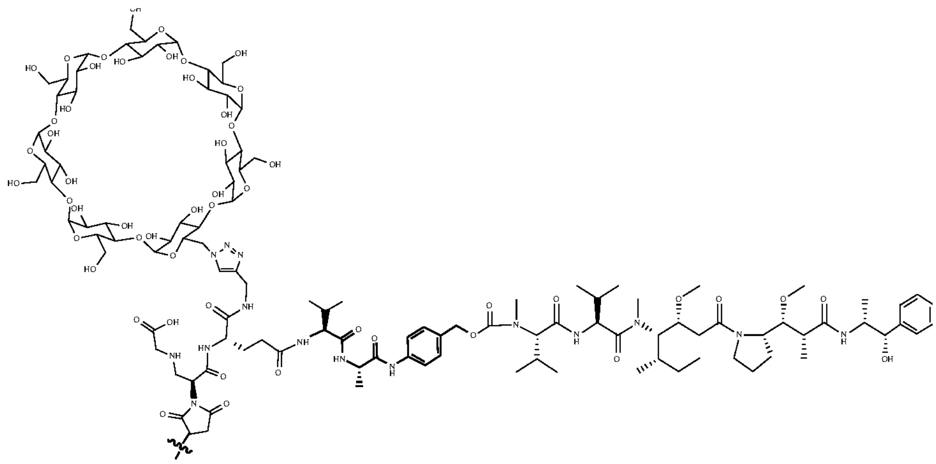 |
| M02 | 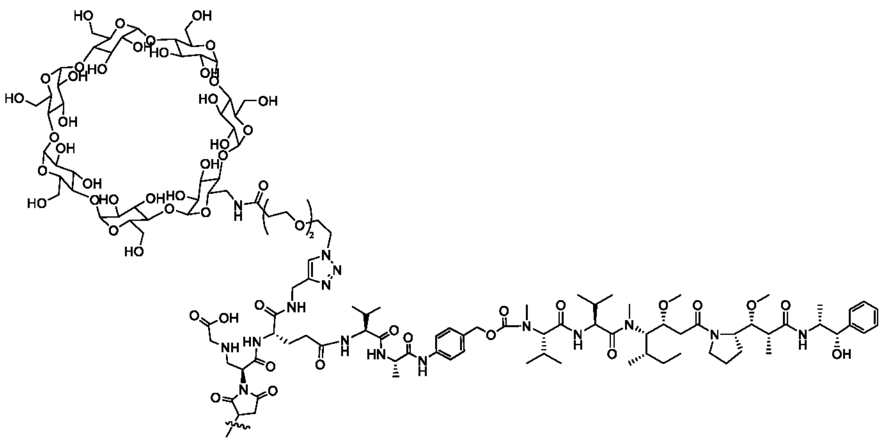 |

(continued)

| | |
|---|---|
| M03 | 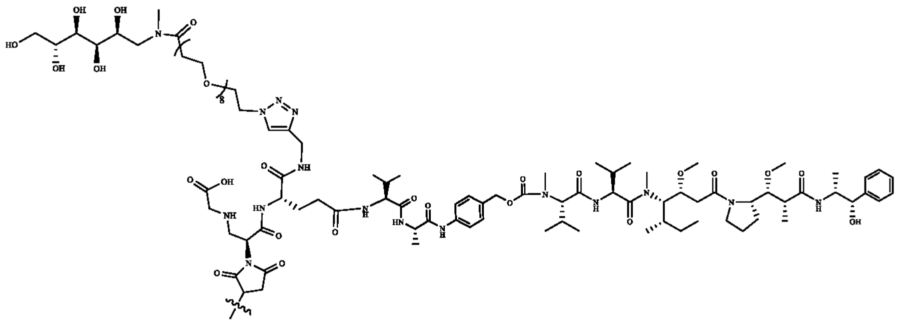 |

In certain embodiments, m is an integer selected from 1 to 10.
In certain embodiments, m is an integer selected from 2 to 8.
In certain embodiments, m is selected from the group consisting of 2, 4, and 8.
In certain embodiments, n is an integer selected from 1 to 10.
In certain embodiments, n is an integer selected from 2 to 8.
In certain embodiments, n is selected from the group consisting of 2, 4, and 8.
In certain embodiments, Ab is selected from the group consisting of murine antibodies, chimeric antibodies, humanized antibodies, fully humanized antibodies, antibody fragments, bispecific antibodies, and multispecific antibodies.
In certain embodiments, Ab forms a linking bond with the linking unit via its cysteine thiol group (-SH).
In certain embodiments, Ab is an antibody, which is a monoclonal antibody selected from the group consisting of: anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-cladin 18.2 antibody, anti-Mesothelin antibody, anti-HER2(ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody and anti-CD123 antibody.
In certain embodiments, Ab is an antibody selected from the group consisting of anti-CD33 antibody ANTI-CD33, anti-Trop2 antibody ANTI-Trop2-1, anti-Trop2 antibody ANTI-Trop2-2, anti-HER2 antibody Trastuzumab (Tras), and anti-HER2 antibody Tras-016, and the related sequences are as follows:

the sequence of anti-CD33 antibody ANTI-CD33

>light chain-LC

DIQLTQSPSTLSASVGDRVTITCRASESLDNYGIRFLTWFQQKPGKAPKLLMYAASNQGS
GVPSRFSGSGSGTEFTLTISSLQPDDFATYYCQQTKEVPWSFGQGTKVEVKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVCTKSFNRGEC (SEQ ID NO: 1)

>heavy chain-HC

EVQLVQSGAEVKKPGSSVKVSCKASGYTITDSNIHWVRQAPGQSLEWIGYIYPYNGGTD
YNQKFKNRATLTVDNPTNTAYMELSSLRSEDTAFYYCVNGNPWLAYWGQGTLVTVSS
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDK
SRWQQGNVFSCSVMHEALHNHYTQKSLSLSPG (SEQ ID NO: 2)

the sequence of anti-Trop2 antibody ANTI-Trop2-1

>light chain-LC

DIQMTQSPSSLSASVGDRVTITCRASQDINKYLAWYQQKPGKVPKLLIYSTSTLQSGVPS
RFSGSGSGTDFTLTISSLQPEDVATYYCLQYDDLFTFGQGTKLEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC (SEQ ID NO: 3)

>heavy chain-HC

QVQLVQSGAEVKKPGASVKLSCKASGYTFTSFDINWVRQAPEQRLEWMGWIFPGDGNT
KYSQKFQGRATITRDTSASTAYMELSSLRSEDTAVYYCVRGEALYYFDYWGQGTLVTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYSKLTV
DKSRWQQGNVFSCSVMHEALHNHYTQKSLSLSPG (SEQ ID NO: 4)

the sequence of anti-Trop2 antibody ANTI-Trop2-2

>light chain-LC

DIQMTQSPSSLSASVGDRVTITCRASQDINKYLAWYQQKPGKVPKLLIYSTSTLQSGVPS
RFSGSGSGTDFTLTISSLQPEDVATYYCLQYDDLFTFGQGTKLEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVCTKSFNRGEC (SEQ ID NO: 5)

>heavy chain-HC

QVQLVQSGAEVKKPGASVKLSCKASGYTFTSFDINWVRQAPEQRLEWMGWIFPGDGNT
KYSQKFQGRATITRDTSASTAYMELSSLRSEDTAVYYCVRGEALYYFDYWGQGTLVTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYSKLTV

DKSRWQQGNVFSCSVMHEALHNHYTQKSLSLSPG (SEQ ID NO: 6)

the sequence of anti-HER2 antibody Trastuzumab (Tras)

>light chain-LC

DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 7)

>heavy chain-HC

EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO: 8)

the sequence of anti-HER2 antibody Tras-016

>light chain-LC

DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVCTKSFNRGEC (SEQ ID NO: 9)

>heavy chain-HC

EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO: 10)

In certain embodiments, $D_1$-$L_1$- and $D_2$-$L_2$- both are coupled to Ab via thiol group.

In certain embodiments, the source of the thiol group is selected from the group consisting of: the thiol group on a cysteine residue formed after reduction of an existing interchain disulfide bond in the targeting moiety, the thiol group on a cysteine residue formed by site-directed mutagenesis of certain original amino acids in the targeting moiety, the thiol group on a cysteine residue formed by site-directed insertion of a cysteine into the targeting moiety, and the thiol group on a cysteine residue formed by site-directed insertion of a cysteine-containing peptide into the targeting moiety.

In certain embodiments, $D_1$-$L_1$- and $D_2$-$L_2$- are respectively coupled to Ab via thiol groups from different sources.

In certain embodiments, any one of $D_1$-$L_1$- and $D_2$-$L_2$- is coupled to Ab via the thiol group on a cysteine residue formed after reduction of an existing interchain disulfide bond in the targeting moiety, and the other is coupled to Ab via the thiol group on a cysteine residue formed by site-directed mutagenesis of certain original amino acids in the targeting moiety, the thiol group on a cysteine residue formed by site-directed insertion of a cysteine into the targeting moiety, or the thiol group on a cysteine residue formed by site-directed insertion of a cysteine-containing peptide into the targeting moiety.

In certain embodiments, the ligand-drug conjugate is selected from the group consisting of:

Ab-R01 (2) -Ex01 (8),
Ab-R01 (8) -Ex01 (2),
Ab-R01 (4) -Ex01 (4),
Ab-R01 (4) -Ex01 (8),

Ab-R01 (8) -Ex01 (4),
Ab-R01 (2) -Ex02 (8),
Ab-R01 (8) -Ex02 (2),
Ab-R01 (4) -Ex02 (4),
Ab-R01 (2) -Ex03 (8),
Ab-R01 (8) -Ex03 (2),
Ab-R01 (4) -Ex03 (4),
Ab-R01 (2) -Ex04 (8),
Ab-R01 (8) -Ex04 (2),
Ab-R01 (4) -Ex04 (4),
Ab-R01 (2) -Ex05 (8),
Ab-R01 (8) -Ex05 (2),
Ab-R01 (4) -Ex05 (4),
Ab-R01 (2) -Ex06 (8),
Ab-R01 (8) -Ex06 (2),
Ab-R01 (4) -Ex06 (4),
Ab-R01-1 (2) -Ex01 (8),
Ab-R01-1 (8) -Ex01 (2),
Ab-R01-1 (4) -Ex01 (4),
Ab-R01-1 (8) -Ex01 (4),
Ab-R01-1 (4) -Ex01 (8),
Ab-R01-1 (2) -Ex02 (8),
Ab-R01-1 (8) -Ex02 (2),
Ab-R01-1 (4) -Ex02 (4),
Ab-R01-1 (2) -Ex03 (8),
Ab-R01-1 (8) -Ex03 (2),
Ab-R01-1 (4) -Ex03 (4),
Ab-R01-1 (2) -Ex04 (8),
Ab-R01-1 (8) -Ex04 (2),
Ab-R01-1 (4) -Ex04 (4),
Ab-R01-1 (2) -Ex05 (8),
Ab-R01-1 (8) -Ex05 (2),
Ab-R01-1 (4) -Ex05 (4),
Ab-R01-1 (2) -Ex06 (8),
Ab-R01-1 (8) -Ex06 (2),
Ab-R01-1 (4) -Ex06 (4),
Ab-R01-1 (2) -PB01 (8),
Ab-R01-1 (2) -PB02 (8),
Ab-R01-1 (8) -PNU01 (2),
Ab-R01-1 (8) -PNU02 (2),
Ab-R01-1 (2) - CBI01 (8),
Ab-R01-1 (2) - CBI02 (8),
Ab-R01-1 (2) - CBI03 (8),
Ab-R01-1 (2) -PBD01 (8),
Ab-R01-1 (2) -PBD02 (8),
Ab-R01-1 (2) -PBD-CBI01 (8),
Ab-R01-1 (2) -PBD-CBI02 (8),
Ab-R01-1 (8) -M01 (2),
Ab-R01-1 (8) -M02 (2),
Ab-R01-1 (8) -M03 (2),
Ab-AT01 (2) -Ex01 (8),
Ab-AT02 (2) -Ex01 (8),
Ab-AT03 (2) -Ex01 (8),
Ab-AT04 (2) -Ex01 (8),
Ab-TA01 (2) -Ex01 (8),
Ab-VE01 (2) -Ex01 (8),
Ab-E01 (2) -Ex01 (8),
Ab-RU01 (2) -Ex01 (8),

Ab-PA01 (2) -Ex01 (8),
Ab-CEP01 (2) -Ex01 (8),
Ab-TM01 (2) -Ex01 (8),
Ab-TM02 (2) -Ex01 (8),
Ab-WE01 (2) -Ex01 (8),
Ab-CH01 (2) -Ex01 (8),
Ab-CH02 (2) -Ex01 (8),
Ab-CH03 (2) -Ex01 (8),
Ab-CH04 (2) -Ex01 (8),
Ab-DP01 (2) -Ex01 (8),
Ab-DP02 (2) -Ex01 (8), and
Ab-DP03 (2) -Ex01 (8).

With regard to the meanings of the structural formulas of the above ligand-drug conjugates, such as Ab-R01 (2) -Ex01 (8), it means that, in the ligand-drug conjugate represented by formula I

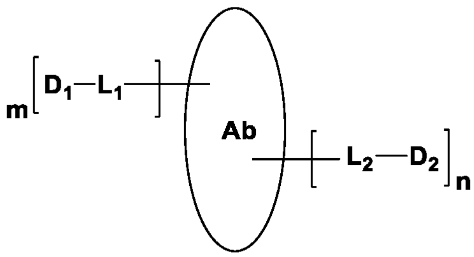

,

$D_2$-$L_2$- is R01, that is,

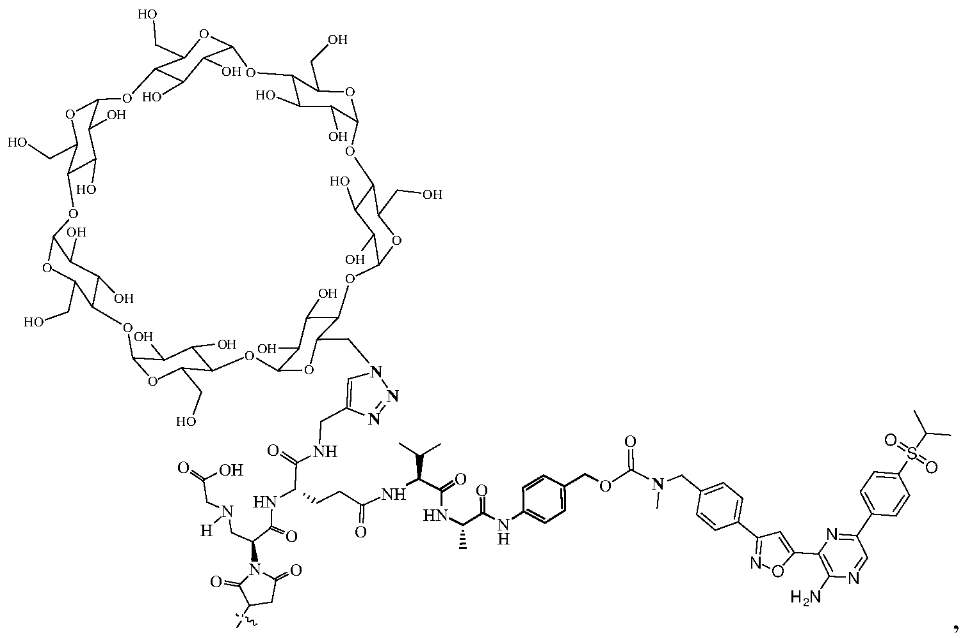

,

$D_1$-$L_1$- is Ex01, that is,

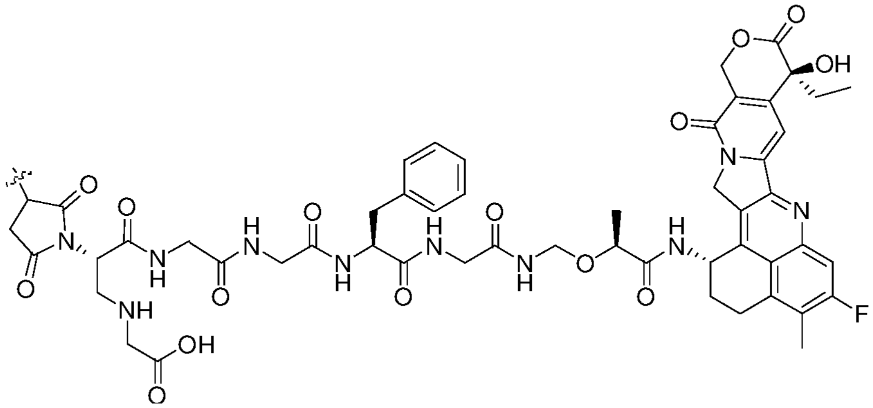

,

and the number of $D_1$-$L_1$-coupled to Ab is 8, that is, m is 8, the number of $D_2$-$L_2$- coupled to Ab is 2, that is, n is 2. The meanings of other structural formulas of the ligand-drug conjugates could be understood with a reference to the foregoing.

In the second aspect, the present invention provides a drug-linking unit conjugate, or a stereoisomer thereof, a prodrug thereof, a pharmaceutically acceptable salt thereof or a pharmaceutically acceptable solvate thereof, wherein, the drug-linking unit conjugate is selected from the group consisting of:

| | |
|---|---|
| R01' | 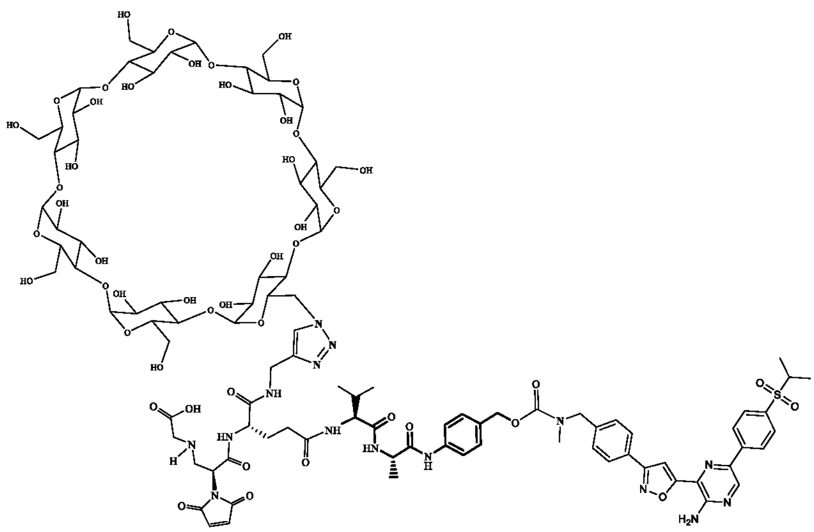 |
| R01-1' | 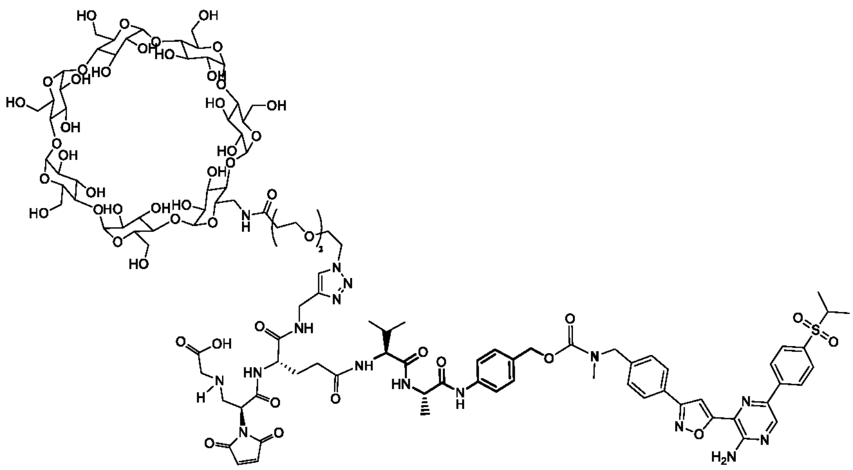 |

(continued)

| | |
|---|---|
| R01-2' | 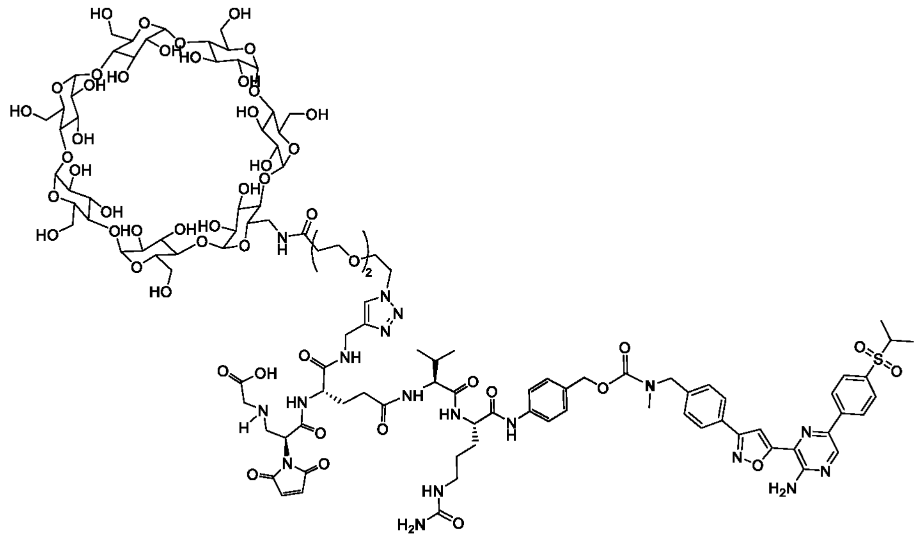 |
| R01-4' | 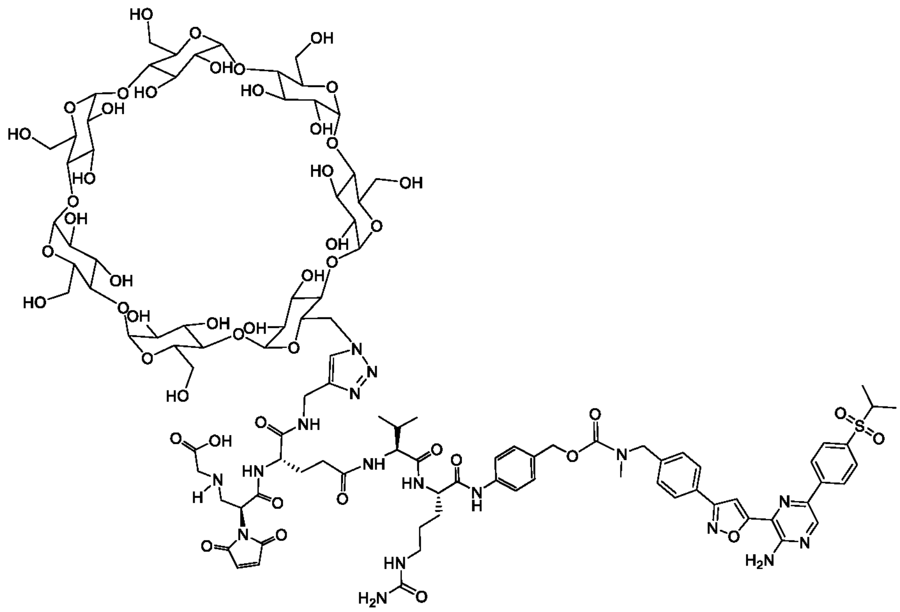 |
| R02' | 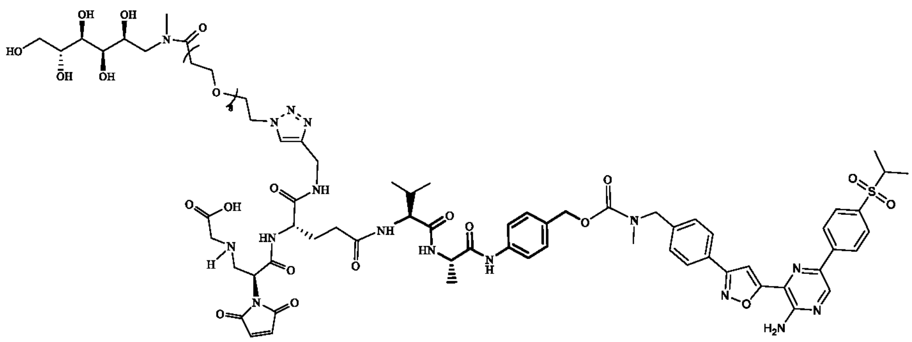 |

(continued)

| | |
|---|---|
| R02-1' | 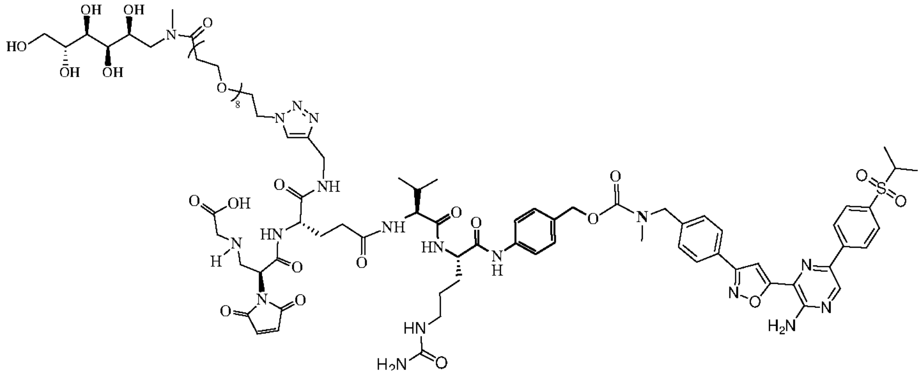 |
| R03' | 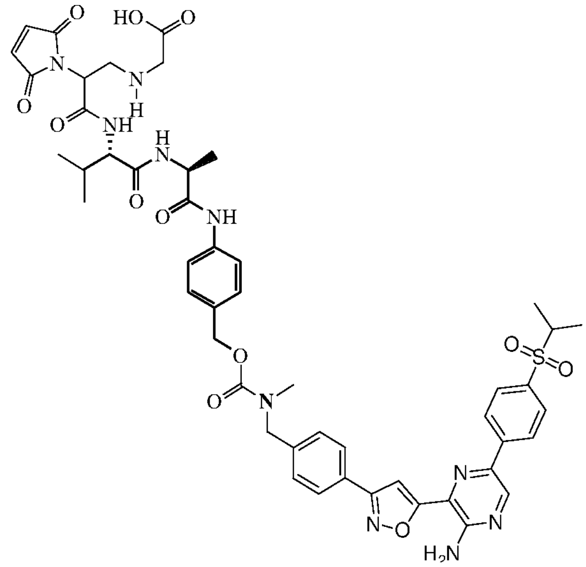 |
| R03-1' | 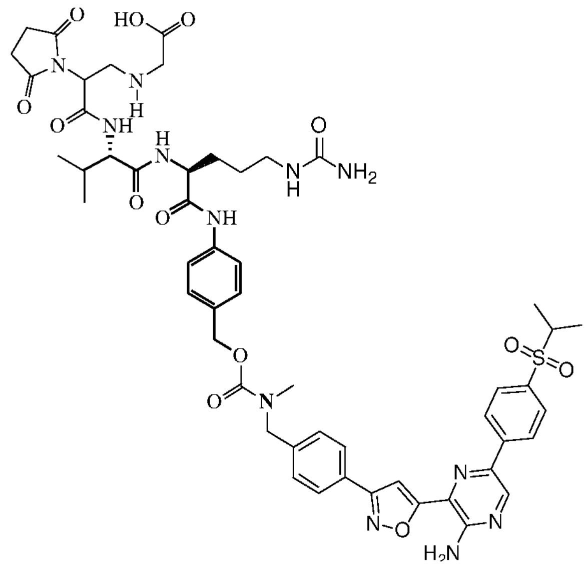 |

(continued)

| | |
|---|---|
| AT01' | 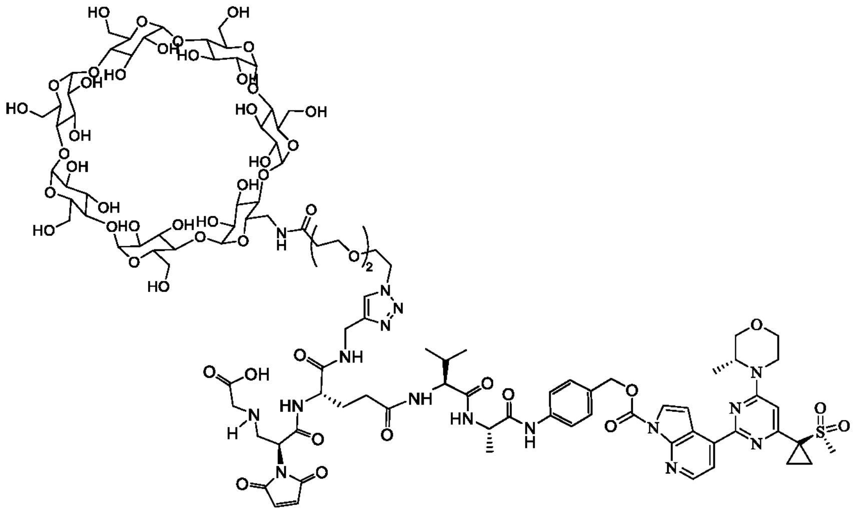 |
| AT02' | 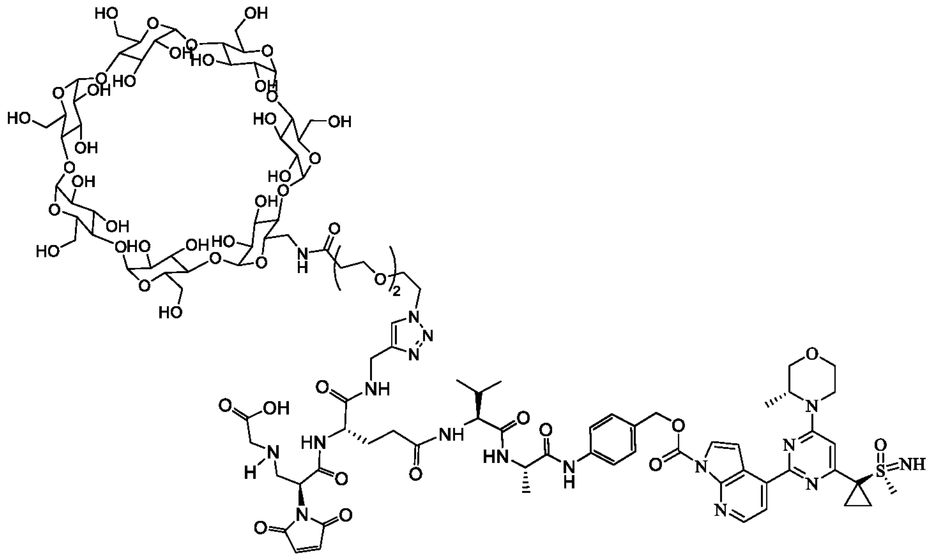 |
| AT03' | 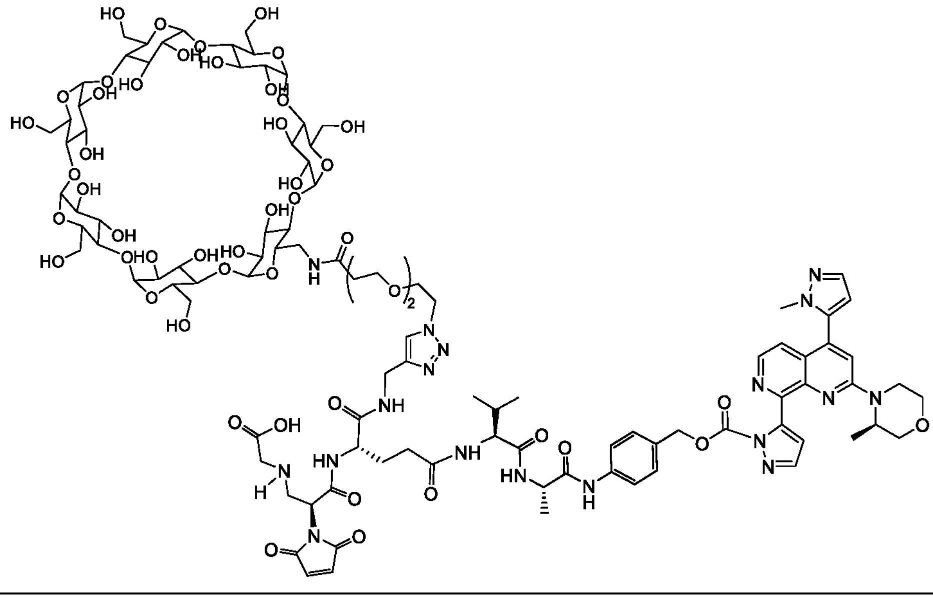 |

(continued)

| | |
|---|---|
| AT04' | 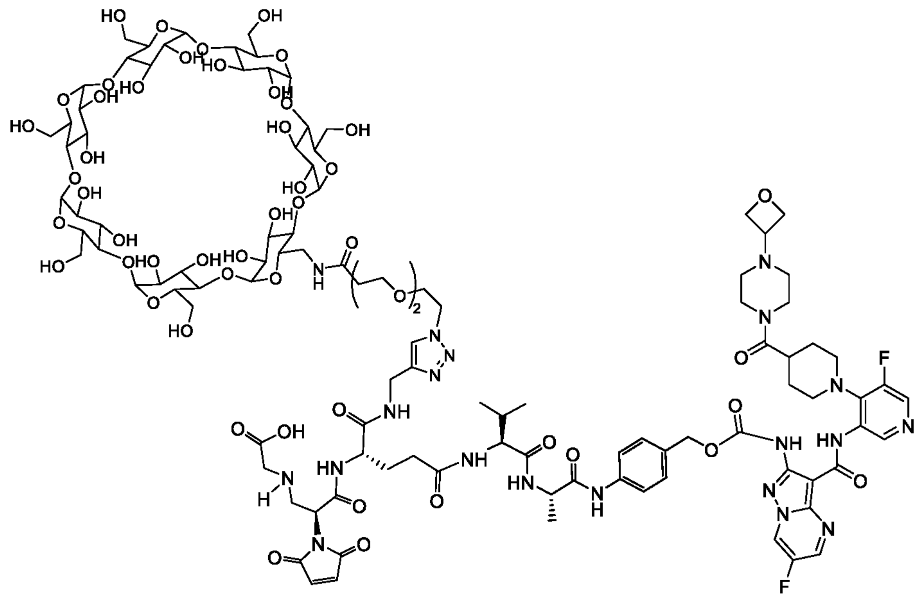 |
| TA01' | 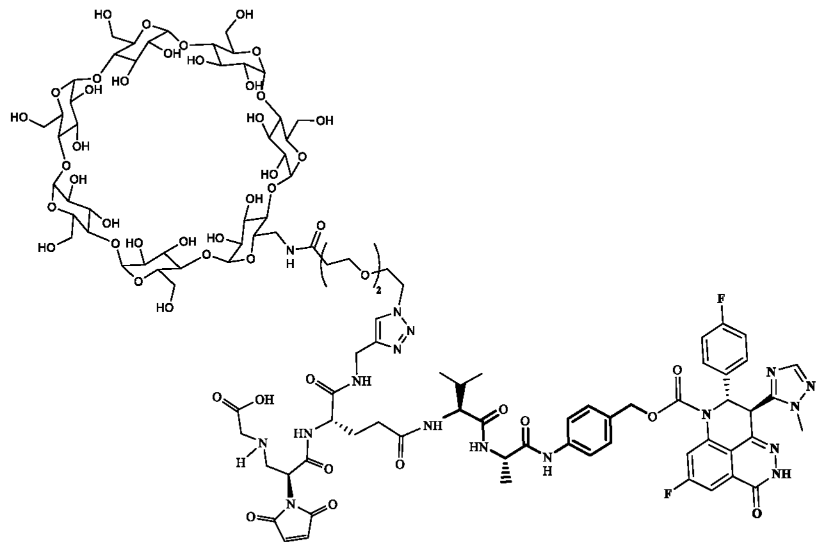 |
| VE01' | 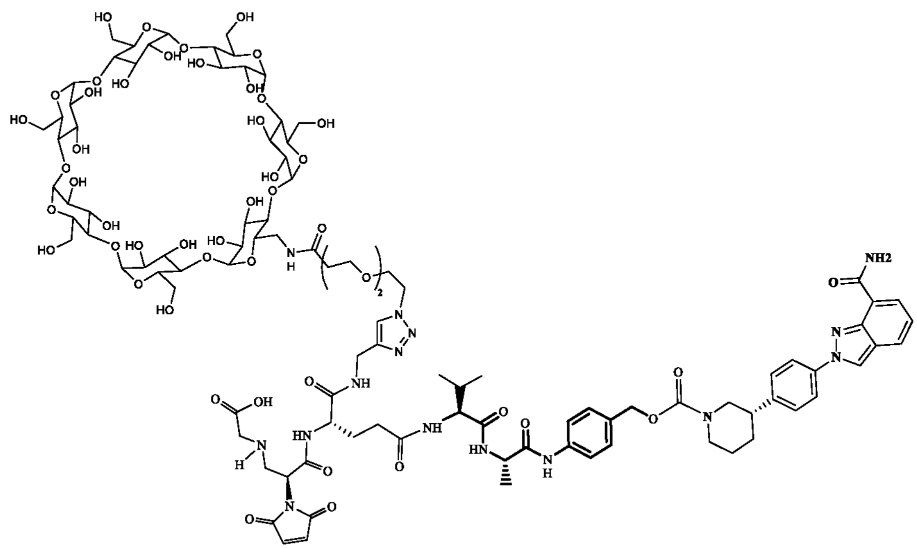 |

(continued)

| | |
|---|---|
| E01' | 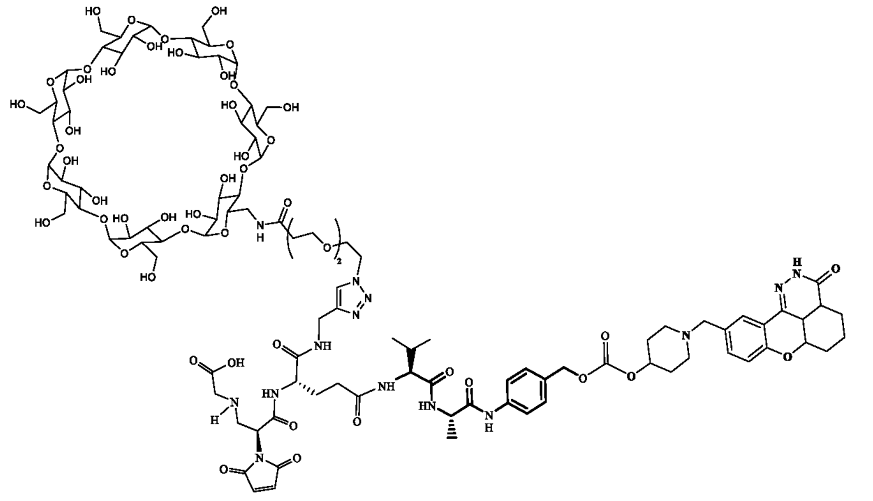 |
| RU01' | 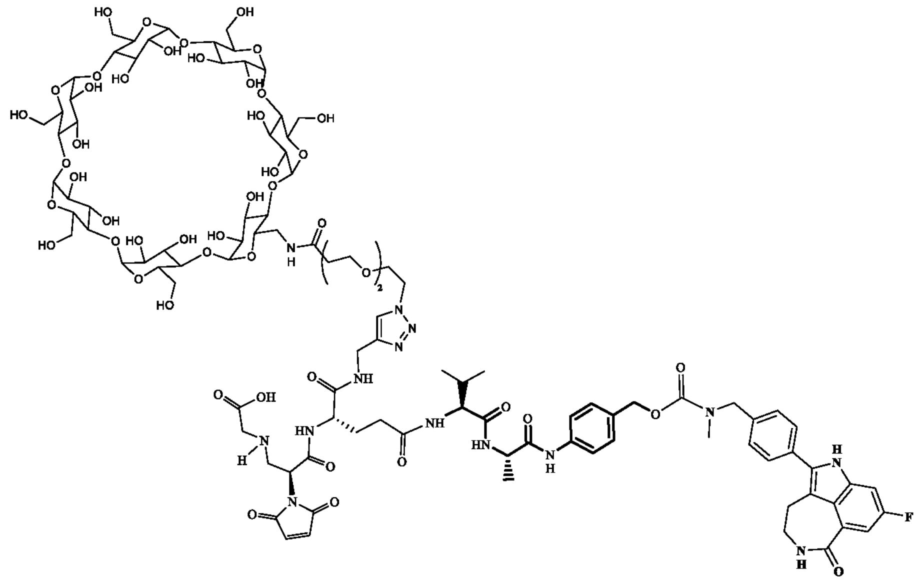 |
| PA01' | 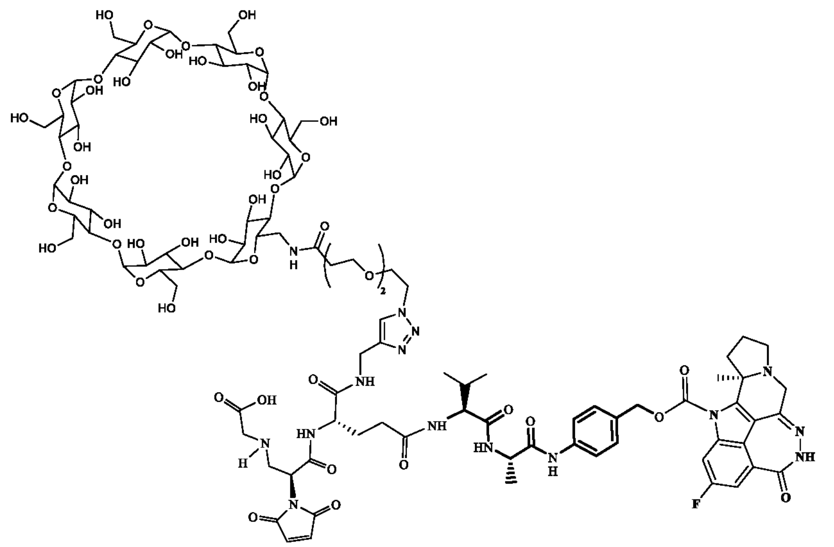 |

(continued)

| | |
|---|---|
| CEP01' | 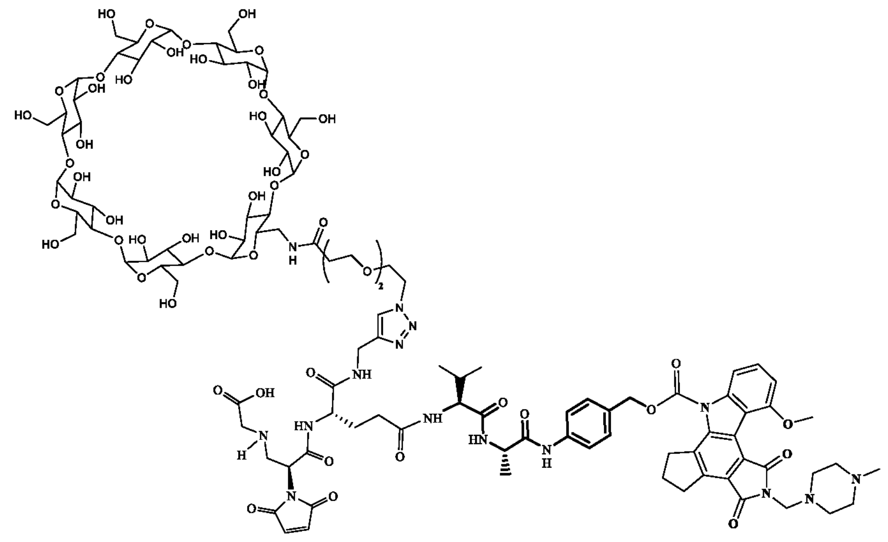 |
| TM01' | 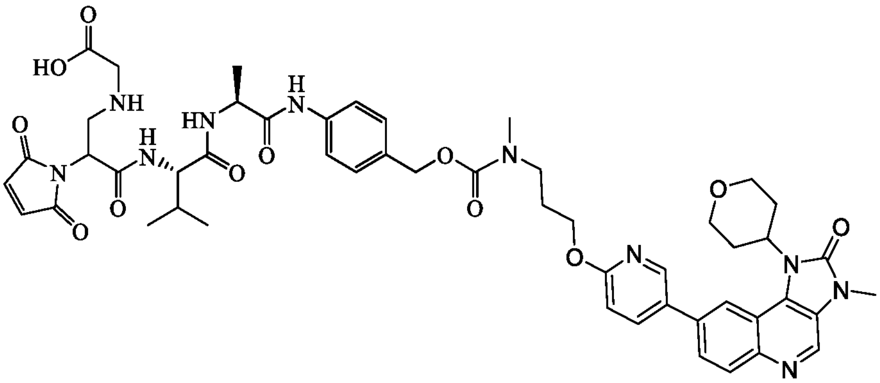 |
| TM02' | 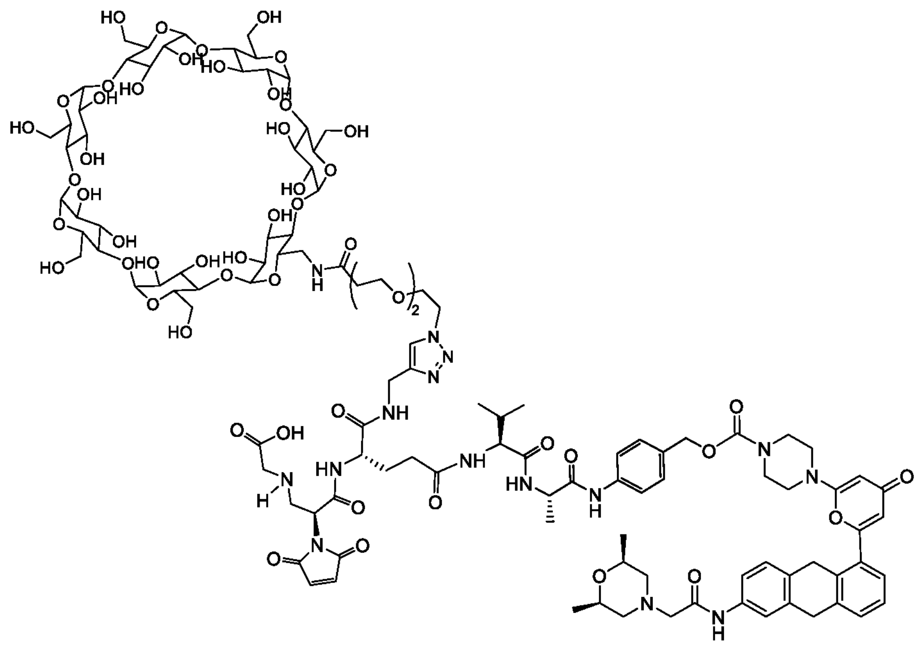 |

(continued)

| | |
|---|---|
| WE01' | 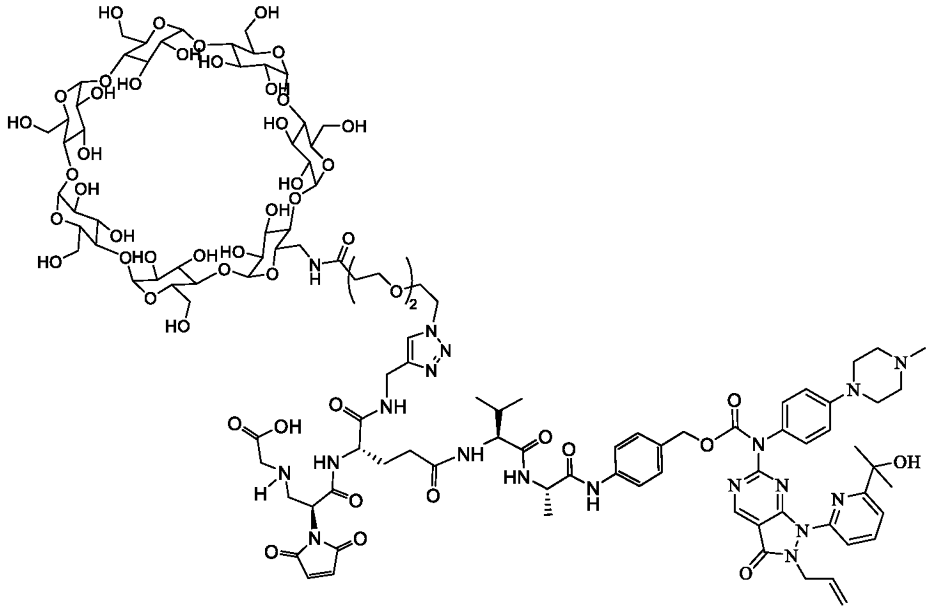 |
| CH01' | 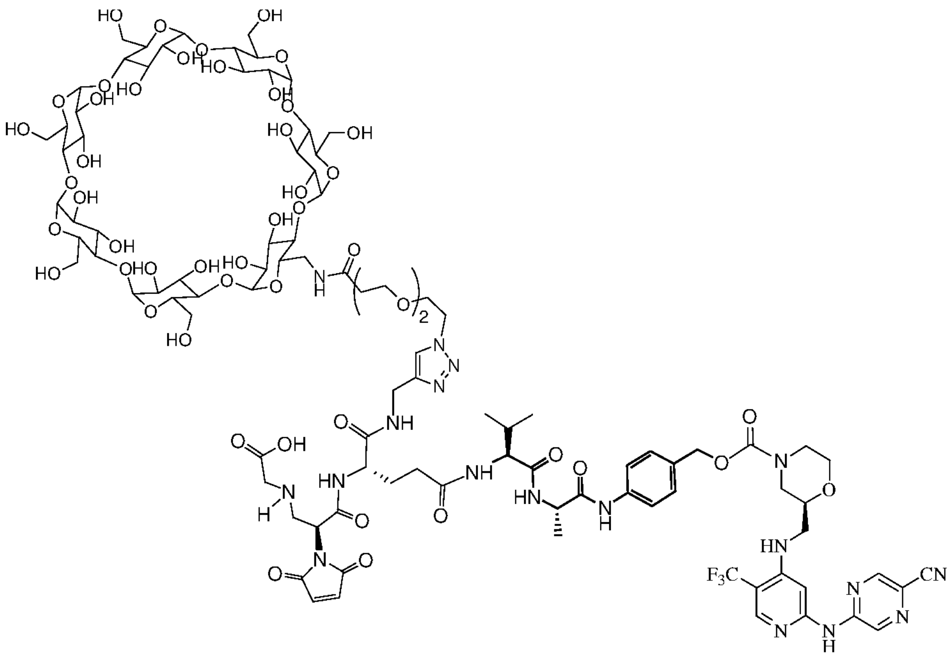 |

(continued)

| | |
|---|---|
| CH02' | 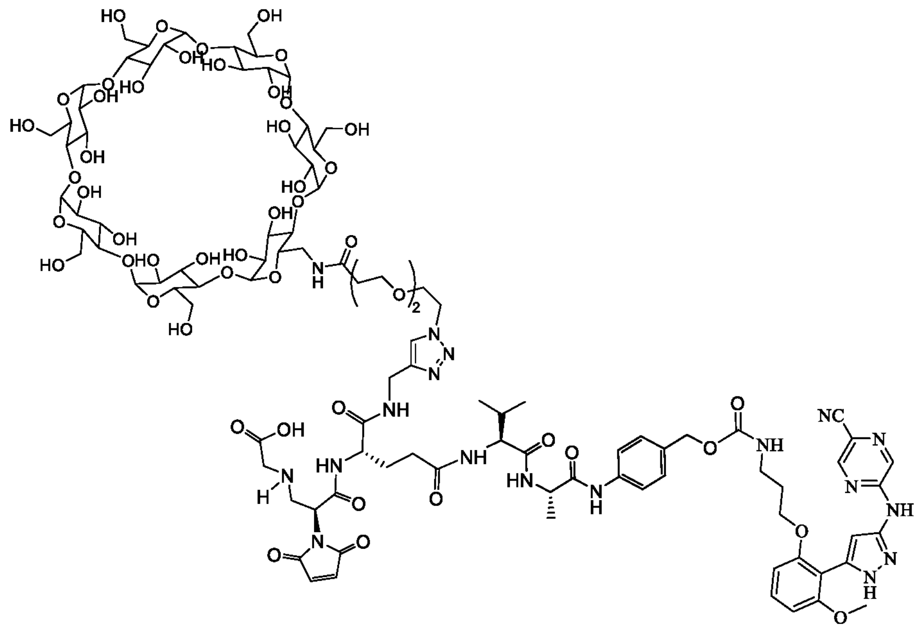 |
| CH03' | 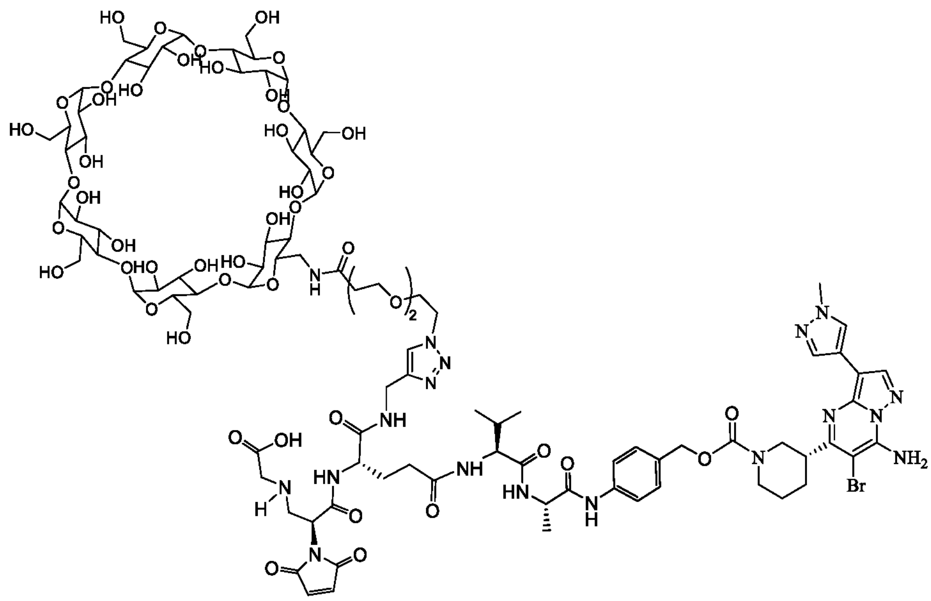 |

(continued)

| | |
|---|---|
| CH04' | 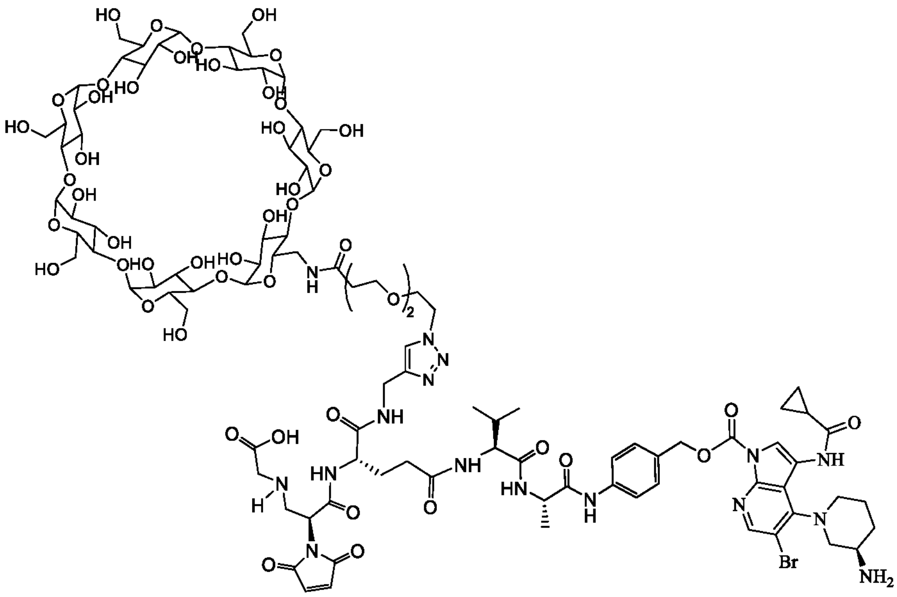 |
| DP01' | 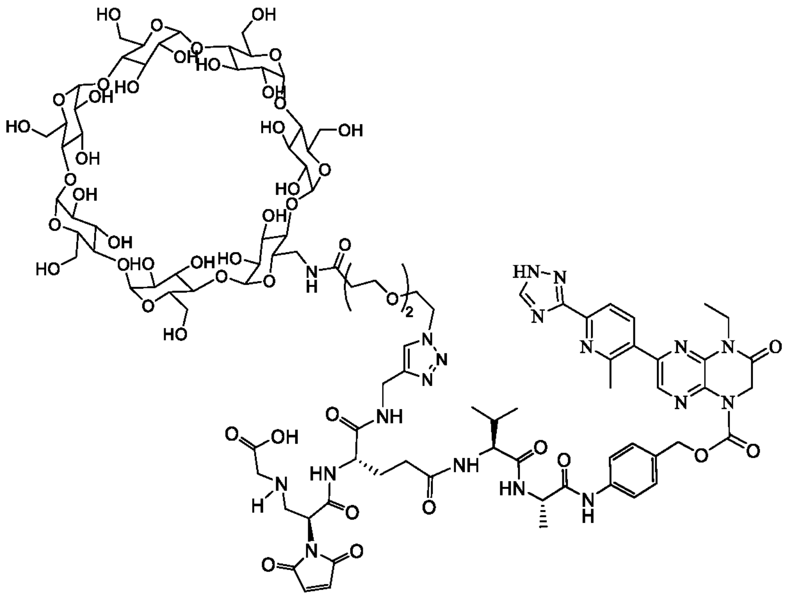 |

(continued)

| | |
|---|---|
| DP02' | 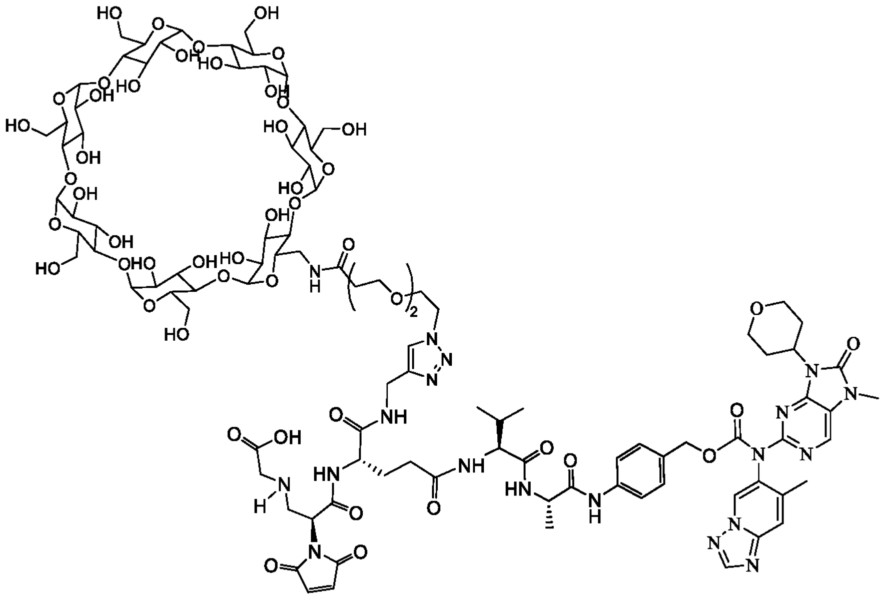 |
| DP03' | 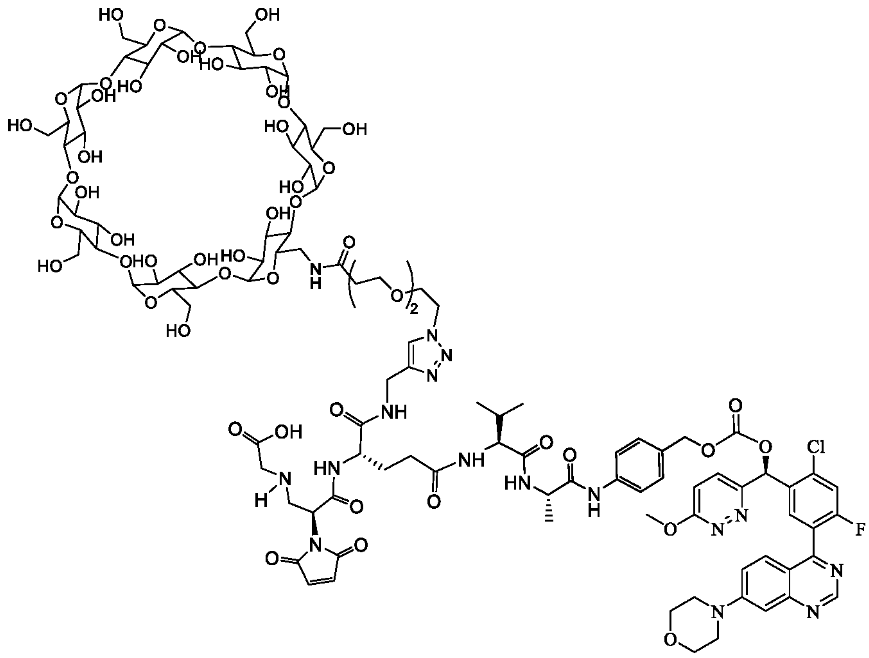 |
| Ex01' | 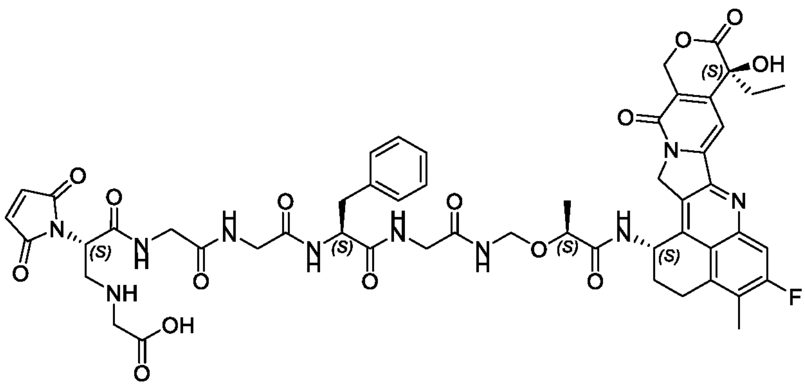 |

(continued)

| | |
|---|---|
| Ex02' | 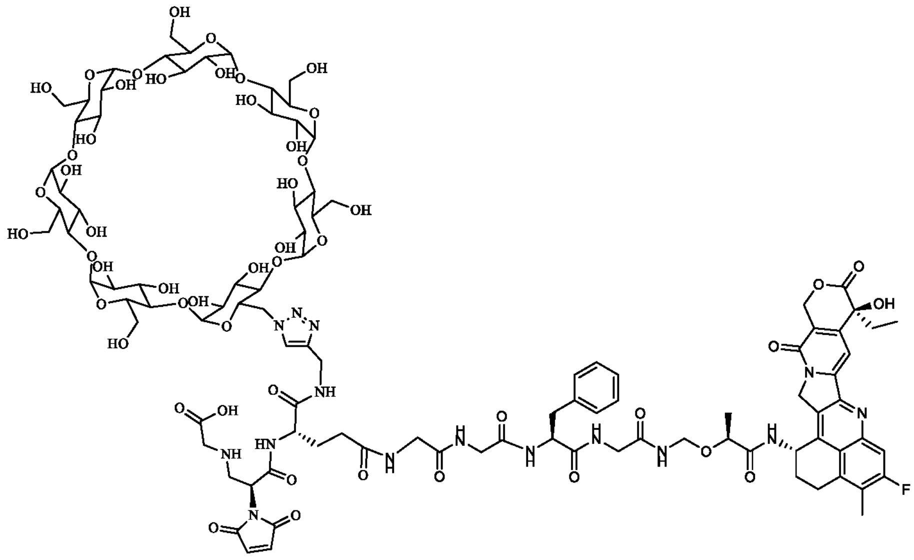 |
| Ex03' | 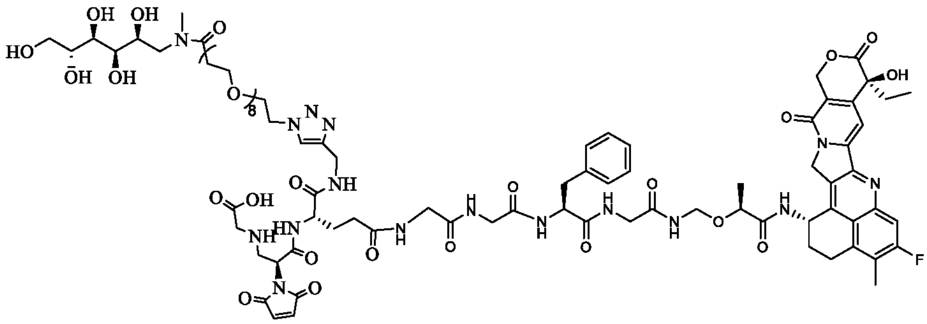 |
| Ex04' | 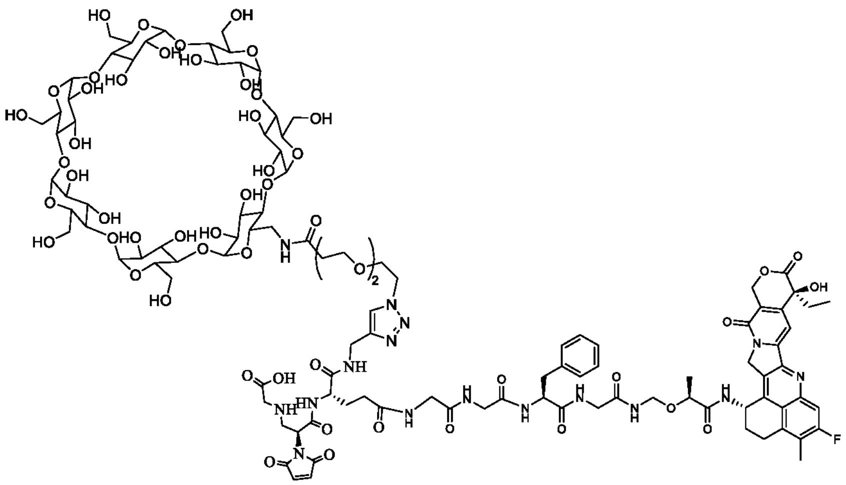 |

(continued)

| | |
|---|---|
| Ex05' | 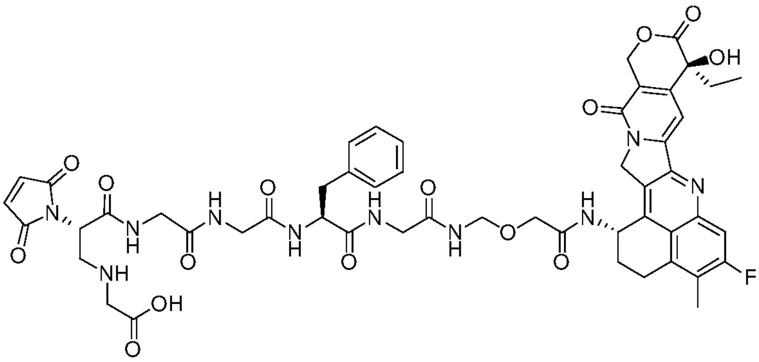 |
| Ex06' | 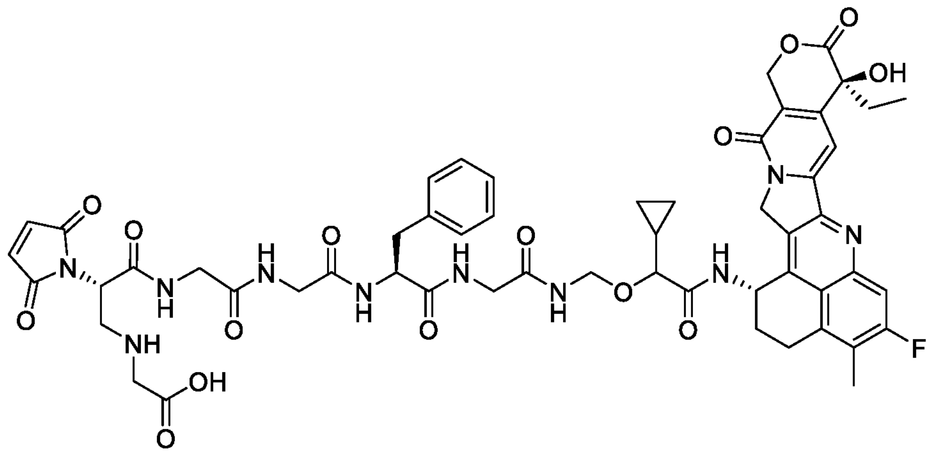 |
| Ex07' | 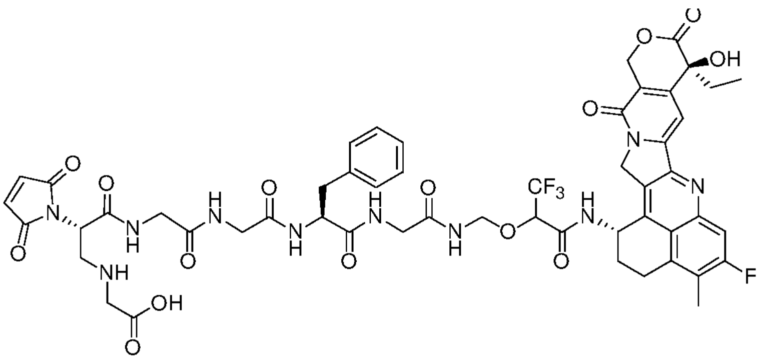 |
| Ex08' | 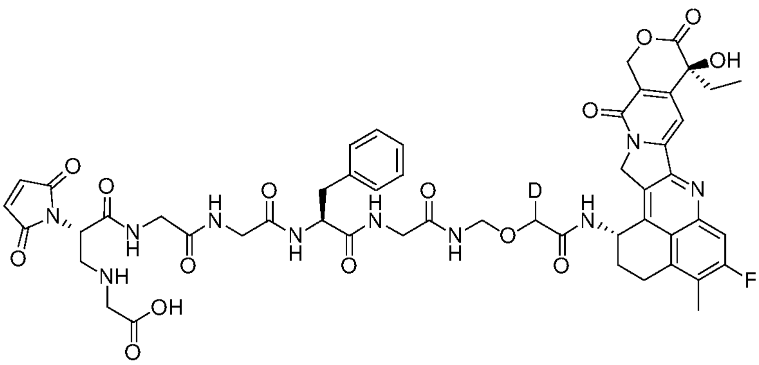 |

(continued)

| | |
|---|---|
| Ex09' | 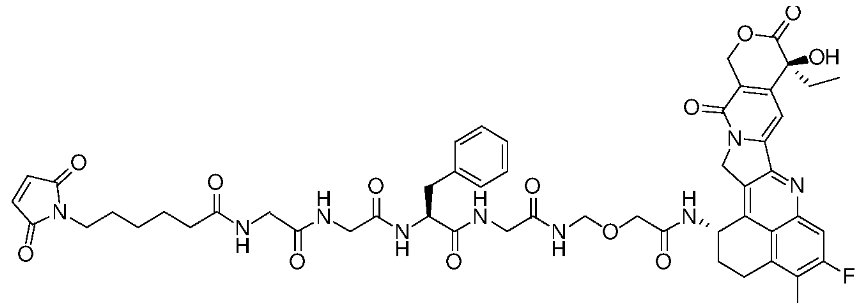 |
| SN01' | 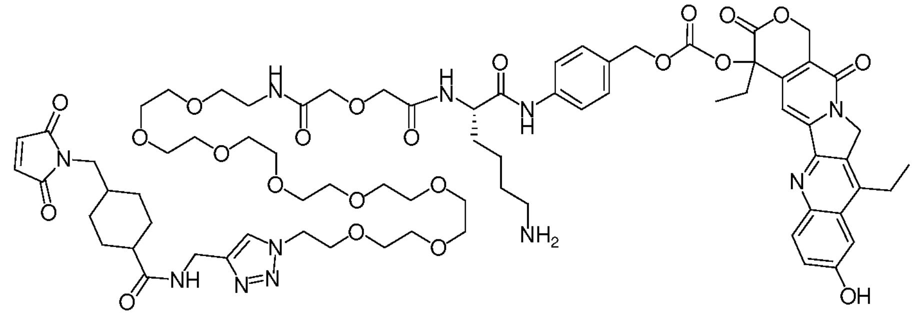 |
| PB01' | 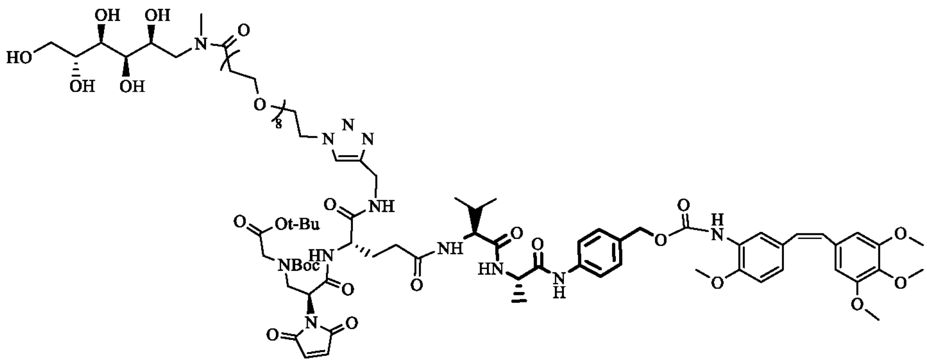 |
| PB02' | 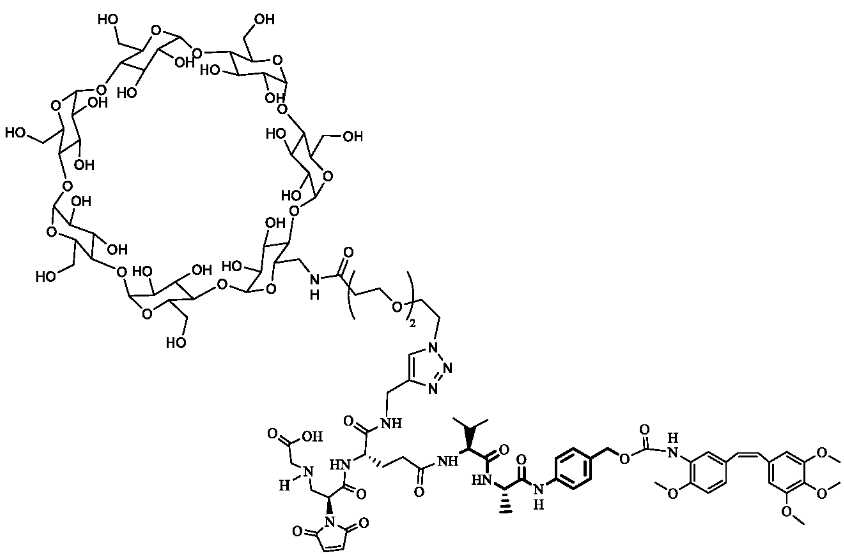 |

(continued)

| | |
|---|---|
| PNU01' | 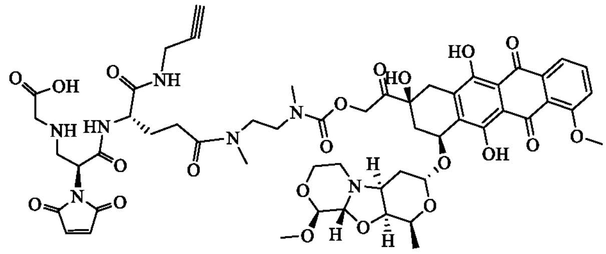 |
| PNU02' | 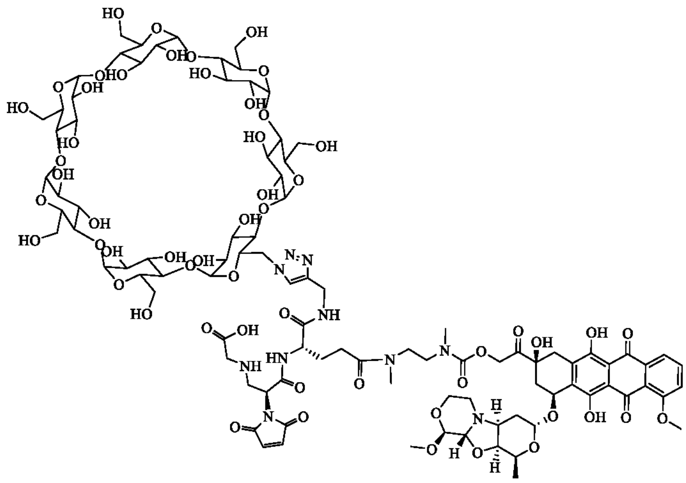 |
| CBI01' | 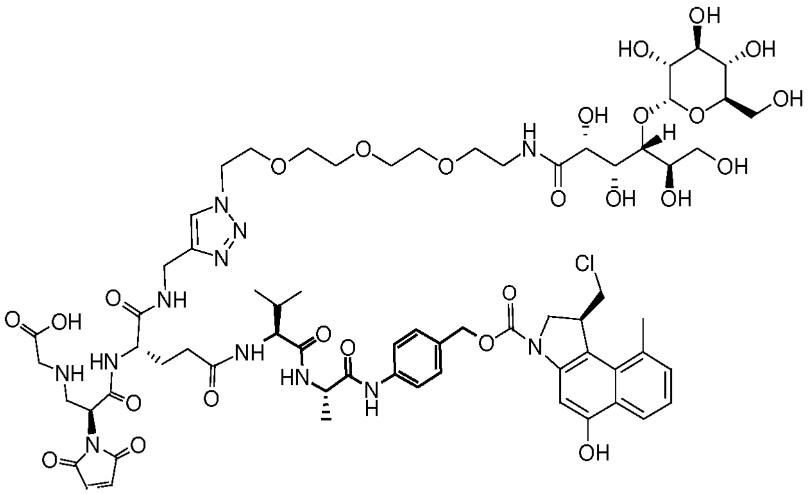 |
| CBI02' | 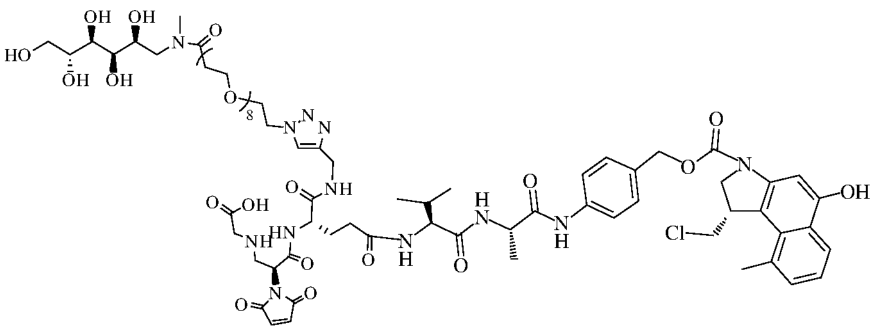 |

(continued)

| | |
|---|---|
| CBI03' | 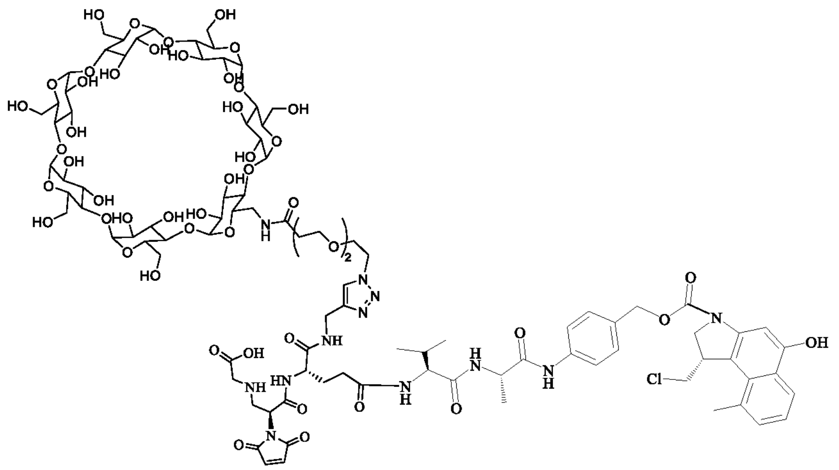 |
| PBD01' | 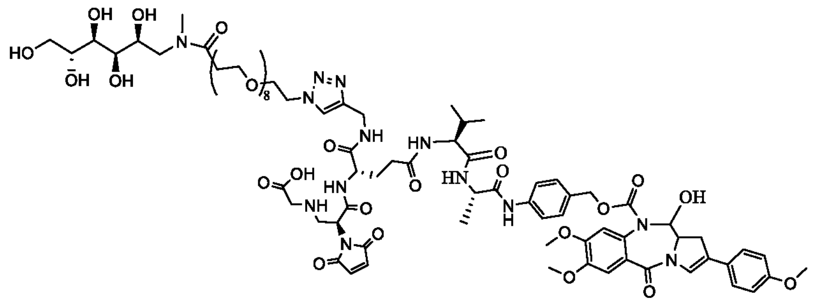 |
| PBD02' | 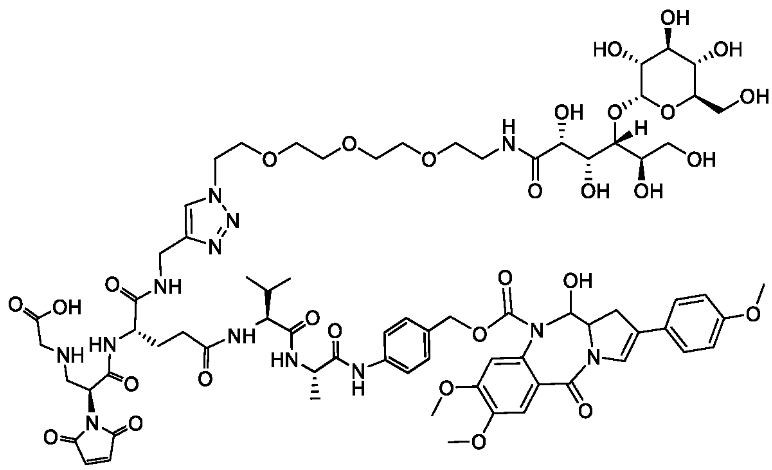 |
| PBD-CBI01' | 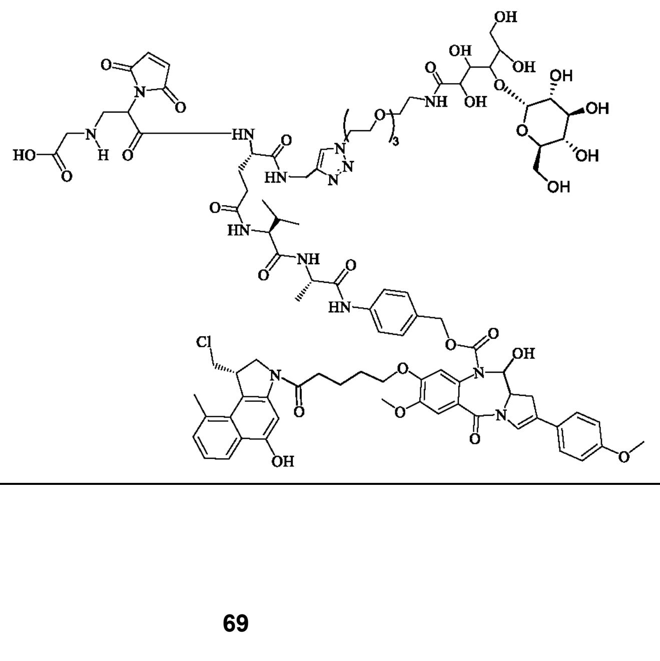 |

(continued)

| | |
|---|---|
| PBD-CBI02' | 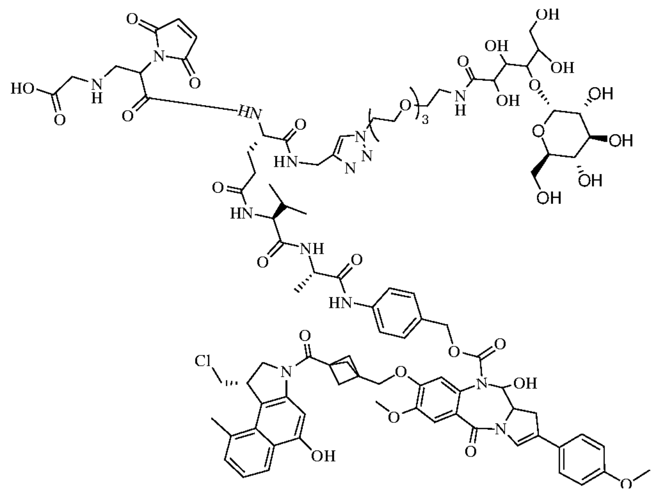 |
| M01' | 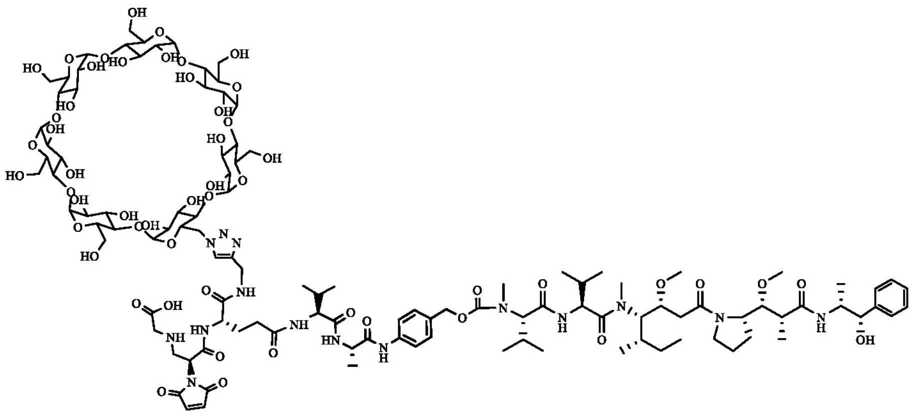 |
| M02' | 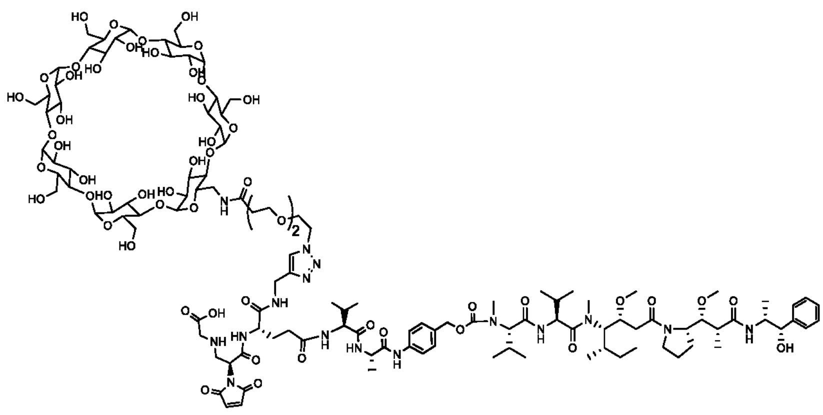 |
| M03' | 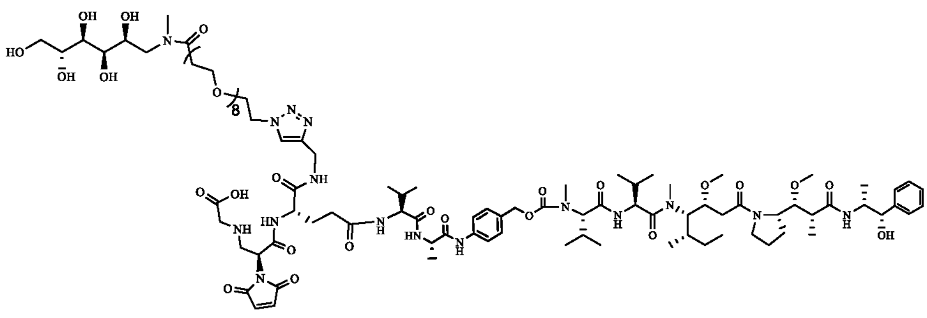 |

In the third aspect, the present invention provides a pharmaceutical composition, comprising the ligand-drug

conjugate, or a stereoisomer thereof, a prodrug thereof, a pharmaceutically acceptable salt thereof or a pharmaceutically acceptable solvate thereof as described above, or the drug-linking unit conjugate or a stereoisomer thereof, a prodrug thereof, a pharmaceutically acceptable salt thereof or a pharmaceutically acceptable solvate thereof as described above, and optionally, one or more pharmaceutical adjuvants.

In the fourth aspect, the present invention provides use of the ligand-drug conjugate, or a stereoisomer thereof, a prodrug thereof, a pharmaceutically acceptable salt thereof or a pharmaceutically acceptable solvate thereof as described above, or the drug-linking unit conjugate or a stereoisomer thereof, a prodrug thereof, a pharmaceutically acceptable salt thereof or a pharmaceutically acceptable solvate thereof as described above, or the pharmaceutical composition as described above in the preparation of a medicament for the treatment and/or prevention of a disease, and the disease is cancer.

In certain embodiments, the cancer is selected from the group consisting of skin cancer, lymphoma, esophageal cancer (e.g., esophageal adenocarcinoma and esophageal squamous cell carcinoma), brain tumor, lung cancer (e.g., small-cell lung cancer and non-small cell lung cancer), squamous cell cancer, bladder cancer, gastric cancer, ovarian cancer, peritoneal cancer, pancreatic cancer, breast cancer, head and neck cancer, cervical cancer, endometrial cancer, colon cancer, rectal cancer, colorectal cancer, liver cancer, kidney cancer, solid tumors, non-hodgkin's lymphoma, central nervous system tumors (e.g., glioma, glioblastoma multiforme, neuroblastoma, glioma or sarcoma), melanoma, prostate cancer, thyroid cancer, bone cancer, urinary tract cancer, salivary gland cancer, leukemia (e.g., acute myeloid leukemia), and other solid tumors and hematological tumors.

In certain embodiments, the cancer is selected from the group consisting of lung cancer (e.g., small-cell lung cancer and non-small cell lung cancer), squamous cell cancer, bladder cancer, gastric cancer, ovarian cancer, breast cancer, colon cancer, rectal cancer, colorectal cancer, liver cancer, and kidney cancer.

In the fifth aspect, the present invention provides the ligand-drug conjugate, or a stereoisomer thereof, a prodrug thereof, a pharmaceutically acceptable salt thereof or a pharmaceutically acceptable solvate thereof as described above, or the drug-linking unit conjugate or a stereoisomer thereof, a prodrug thereof, a pharmaceutically acceptable salt thereof or a pharmaceutically acceptable solvate thereof as described above, or the pharmaceutical composition as described above, for use in the treatment and/or prevention of a disease, and the disease is cancer.

In certain embodiments, the cancer is selected from the group consisting of skin cancer, lymphoma, esophageal cancer (e.g., esophageal adenocarcinoma and esophageal squamous cell carcinoma), brain tumor, lung cancer (e.g., small-cell lung cancer and non-small cell lung cancer), squamous cell cancer, bladder cancer, gastric cancer, ovarian cancer, peritoneal cancer, pancreatic cancer, breast cancer, head and neck cancer, cervical cancer, endometrial cancer, colon cancer, rectal cancer, colorectal cancer, liver cancer, kidney cancer, solid tumors, non-hodgkin's lymphoma, central nervous system tumors (e.g., glioma, glioblastoma multiforme, neuroblastoma, glioma or sarcoma), melanoma, prostate cancer, thyroid cancer, bone cancer, urinary tract cancer, salivary gland cancer, leukemia (e.g., acute myeloid leukemia), and other solid tumors and hematological tumors.

In certain embodiments, the cancer is selected from the group consisting of lung cancer (e.g., small-cell lung cancer and non-small cell lung cancer), squamous cell cancer, bladder cancer, gastric cancer, ovarian cancer, breast cancer, colon cancer, rectal cancer, colorectal cancer, liver cancer, and kidney cancer.

In the sixth aspect, the present invention provides a method for the treatment and/or prevention of a disease, the disease being cancer, the method comprising: administering to a subject in need a prophylactically and/or therapeutically effective amount of the ligand-drug conjugate, or a stereoisomer thereof, a prodrug thereof, a pharmaceutically acceptable salt thereof or a pharmaceutically acceptable solvate thereof as described above, or the drug-linking unit conjugate or a stereoisomer thereof, a prodrug thereof, a pharmaceutically acceptable salt thereof or a pharmaceutically acceptable solvate thereof as described above, or the pharmaceutical composition as described above.

In certain embodiments, the cancer is selected from the group consisting of skin cancer, lymphoma, esophageal cancer (e.g., esophageal adenocarcinoma and esophageal squamous cell carcinoma), brain tumor, lung cancer (e.g., small-cell lung cancer and non-small cell lung cancer), squamous cell cancer, bladder cancer, gastric cancer, ovarian cancer, peritoneal cancer, pancreatic cancer, breast cancer, head and neck cancer, cervical cancer, endometrial cancer, colon cancer, rectal cancer, colorectal cancer, liver cancer, kidney cancer, solid tumors, non-hodgkin's lymphoma, central nervous system tumors (e.g., glioma, glioblastoma multiforme, neuroblastoma, glioma or sarcoma), melanoma, prostate cancer, thyroid cancer, bone cancer, urinary tract cancer, salivary gland cancer, leukemia (e.g., acute myeloid leukemia), and other solid tumors and hematological tumors.

In certain embodiments, the cancer is selected from the group consisting of lung cancer (e.g., small-cell lung cancer and non-small cell lung cancer), squamous cell cancer, bladder cancer, gastric cancer, ovarian cancer, breast cancer, colon cancer, rectal cancer, colorectal cancer, liver cancer, and kidney cancer.

Definition of terms

In the present invention, unless otherwise indicated, the scientific and technical terms used herein have the

meaning as commonly understood by those skilled in the art. Also, cell culture, molecular genetics, nucleic acid chemistry, immunology laboratory procedures, as used herein, are all conventional procedures that are widely used in the relevant art. Meanwhile, in order to better understand the invention, definitions and explanations of the related terms are provided below.

In the present invention, examples of the term "pharmaceutically acceptable salt" are organic acid addition salts formed from organic acids that form pharmaceutically acceptable anions, including but not limited to formate, acetate, propionate, benzoate, maleate, fumarate, succinate, tartrate, citrate, ascorbate, $\alpha$-ketoglutarate, $\alpha$-glycerophosphate, alkylsulfonate or arylsulfonate; preferably, the alkyl sulfonate is methyl sulfonate or ethyl sulfonate; the aryl sulfonate is benzene sulfonate or p-toluene sulfonate. Suitable inorganic salts may also be formed, including but not limited to hydrochloride, hydrobromide, hydroiodide, nitrate, bicarbonate and carbonate, sulfate or phosphate and the like.

Pharmaceutically acceptable salts can be obtained using standard procedures well known in the art, for example, by reacting a sufficient amount of a basic compound with a suitable acid that provides a pharmaceutically acceptable anion.

In the present invention, the term "prodrug" refers to derivatives that can hydrolyze, oxidize, or otherwise react under biological conditions (*in vitro* or *in vivo*) to provide compounds of the present invention. Prodrugs only become active compounds through this reaction under biological conditions, or they do not have or only have low activity in their non-reactive form. Prodrugs can generally be prepared using well known methods, such as those described in Burger's Medicinal Chemistry and Drug Discovery (1995) 172-178, 949-982 (Manfred E. Wolff, 5th Edition).

Stereoisomers of the compounds described herein, when specifically specified by chemical name as (R)- or (S)-isomers, should be understood to have the predominant configuration as (R)-isomer or (S)-isomer, respectively. Any asymmetric carbon atom may be present in the (R)-, (S)- or (R, S)-configuration, preferably in the (R)- or (S)-configuration.

In the present invention, the term "solvated compound" or "solvate" may be used interchangeably and refers to a compound that is present in combination with a certain solvent molecule. The combination may include a stoichiometric amount of a certain solvent, such as a monohydrate or dihydrate, or may include any amount of water; as another example, methanol or ethanol may be used to form "alcoholate", which may also be stoichiometric or non-stoichiometric. The term "solvate" as used herein refers to a solid form, i.e., although a compound in a solution of a solvent may be solvated, it is not the solvate which the term used herein refers to.

In the present invention, the term "deuteride" refers to a substance obtained by replacing one or more hydrogen atoms in a compound with deuterium atoms.

In the present invention, the pharmaceutical adjuvants refer to excipients and additives used in the production of drugs and the formulation of prescriptions, and refer to substances that have been reasonably evaluated in terms of safety and are included in pharmaceutical preparations in addition to active ingredients. In addition to shaping, acting as vehicles, and improving stability, the pharmaceutical adjuvants have important functions of solubilization, dissolution assistance, sustained and controlled release and the like, and are important components that may affect the quality, safety, and efficacy of drugs. According to their sources, they can be divided into natural substances, semi synthetic substances, and fully synthetic substances. According to their roles and uses, they can be divided into: solvents, propellants, solubilizers, cosolvents, emulsifiers, colorants, binders, disintegrants, fillers, lubricants, wetting agents, osmotic regulators, stabilizers, glidants, flavoring agents, preservatives, suspending agents, coating materials, fragrances, anti-adherents, antioxidants, chelating agents, penetration enhancers, pH regulators, buffers, plasticizers, surfactants, foaming agents, defoaming agents, thickeners, encapsulating agents, humectants, absorbents, diluents, flocculants and deflocculants, filter aids, release retardants and the like. According to the administration route, they can be divided into oral, injection, mucosal, transdermal or local administration, nasal or oral inhalation administration, ocular administration and the like. The same pharmaceutical adjuvant can be used for different administration routes of pharmaceutical preparations, and have different roles and uses.

In the present invention, the pharmaceutical composition may be prepared into various suitable dosage forms according to the administration route. Fo example, tablets, capsules, granules, oral solutions, oral suspensions, oral emulsions, powders, tinctures, syrups, injections, suppositories, ointments, creams, pastes, ophthalmic preparations, pills, implants, aerosols, powder aerosols, sprays and the like. Wherein, the pharmaceutical composition or suitable dosage form may contain 0.01mg to 1000mg of a compound or a pharmaceutically acceptable salt or conjugate thereof as described herein, suitably 0.1mg to 800mg, preferably 0.5 to 500mg, preferably 0.5 to 350mg, particularly preferably 1 to 250 mg.

The pharmaceutical composition can be administered in the form of an injection, including an injection, a sterile powder for injection, and a concentrated solution for injection. Wherein, the useable vehicles and solvents include water, Ringer's solution and isotonic sodium chloride solution. In addition, sterilized non-volatile oils can also be used as solvents or suspension media, such as monoglycerides or diglycerides.

In the present invention, the term "treatment" generally refers to the acquisition of a required pharmacological and/or physiological effect. The effect may be prophylactic in terms of completely or partially preventing a disease or symptoms thereof; and/or may be therapeutic in terms of partially or completely stabilizing or curing a disease and/or side

effects due to the disease. As used herein, the term "treatment" encompasses any treatment of a disease in a patient, including: (a) preventing the disease or symptoms thereof in the patient who is susceptible to but has not yet been diagnosed as having the disease or symptoms thereof; (b) inhibiting the symptoms of the disease, i.e., arresting its development; or (c) alleviating the symptoms of the disease, i.e., causing regression of the disease or symptoms thereof.

In the present invention, the term "subject" includes a human or non-human animal. Exemplary human individuals include human individuals (referred to as patients) with a disease (e.g., a disease described herein) or normal human individuals. The term "non-human animal" used herein includes all vertebrates, such as non-mammals (e.g., birds, amphibians, reptiles) and mammals, such as non-human primates, livestocks and/or domesticated animals (e.g., sheep, dogs, cats, cows, pigs and the like).

In the present invention, the term "effective amount" refers to an amount of a compound that, when administered, will alleviate one or more symptoms of the condition being treated to some extent.

In the present invention, the term "targeting moiety" refers to a moiety in a conjugate that is capable of specifically binding to a target (or a portion of a target) on the surface of a cell. The conjugate can be delivered to a specific cell population by interaction of the targeting moiety with the target. Preferably, the targeting moiety is an antibody.

In the present invention, the term "antibody", interpreted in its broadest sense, includes intact monoclonal antibodies, polyclonal antibodies, and multispecific antibodies (e.g., bispecific antibodies) formed from at least two intact antibodies, so long as they possess the required biological activity. In the present invention, the terms "antibody" and "immunoglobulin" may be used interchangeably.

In the present invention, the term "monoclonal antibody" refers to an antibody from a population of substantially homogeneous antibodies, i.e., the antibodies that constitute the population are exactly the same, except for a small number of possible natural mutations. A monoclonal antibody has a high specificity for one determinant (epitope) of an antigen, while a polyclonal antibody in contrast thereto comprises different antibodies directed to different determinants (epitopes). In addition to specificity, a monoclonal antibody has the advantage that it can be synthesized without being contaminated by other antibodies. The modifier "monoclonal" used herein indicates that the antibody is characterized as being from a population of substantially homogeneous antibodies, and should not be understood that it has to be prepared by a special method.

In certain embodiments of the present invention, monoclonal antibodies also specifically include chimeric antibodies, i.e., a portion of the heavy and/or light chain is identical or homologous to an antibody of a certain type, class, or subclass, and the remainder is identical or homologous to an antibody of another type, class, or subclass, as long as they possess the required biological activity (see, e.g., US 4, 816, 567; and Morrison et al, 1984, PNAS, 81: 6851-6855). The useable chimeric antibodies include primatized antibodies comprising variable region antigen binding sequences from a non-human primate (e.g., an ancient monkey, orangutan and the like) and human constant region sequences.

In the present invention, the term "antibody fragment" refers to a portion of an antibody, preferably an antigen binding or variable region. Examples of antibody fragments include Fab, Fab', $F(ab')_2$, Fd, Fv, dAb, and complementarity determining region fragments, diabody, linear antibody, and single chain antibody molecules.

In the present invention, the term "bispecific antibody", also referred to as "bifunctional antibody conjugate", refers to a conjugate formed by a first antibody (fragment) and a second antibody (fragment) via a coupling arm, which retains the activity of the respective antibodies, and thus has bifunction and bispecificity.

In the present invention, the term "multispecific antibody" includes, for example, trispecific antibodies, which are antibodies having three different antigen-binding specificities, and tetraspecific antibodies, which are antibodies having four different antigen-binding specificities.

In the present invention, the term "intact antibody" refers to an antibody comprising an antigen binding variable region and a light chain constant region (CL), a heavy chain constant region (CH1, CH2, and CH3). The constant region may be a native sequence (e.g., a human native constant region sequence) or an amino acid sequence variant thereof. The intact antibody is preferably an intact antibody having one or more effector functions.

In the present invention, a "humanized" form of a non-human (e.g., murine) antibody refers to a chimeric antibody that contains minimal non-human immunoglobulin sequences. Most humanized antibodies are obtained by replacing hypervariable region residues of human recipient immunoglobulin with non-human (such as mice, rats, rabbits, or non-human primates) hypervariable region residues (donor antibodies) that possess the required specificity, affinity, and function. In certain embodiments, Framework Region (FR) residues of a human immunoglobulin are also replaced with non-human residues. Furthermore, a humanized antibody may also comprise residues that are not present in a recipient antibody or a donor antibody. These modifications are made to further optimize the performance of an antibody. A humanized antibody typically comprises at least one, and typically two, variable regions in which all or substantially all hypervariable loops correspond to a non-human immunoglobulin and FR is completely or substantially completely the sequences of a human immunoglobulin. A humanized antibody may also comprise at least a portion of an immunoglobulin constant region (Fc, typically a human immunoglobulin Fc). For details see, e.g., Jones et al, 1986, Nature, 321: 522-525; Riechmann et al, 1988, Nature, 332: 323-329; and Presta, 1992, Curr Op Struct Bwl 2: 593-596.

Monoclonal antibodies used herein may be prepared by a number of methods. For example, monoclonal

antibodies used herein may be obtained by hybridoma methods using a variety of species (including mouse, hamster, rat, and human cells) (see, e.g., Kohler et al, 1975, Nature, 256: 495), or prepared by recombinant DNA techniques (see, e.g., US 4, 816, 567), or isolated from phage antibody libraries (see, e.g., Clackson et al, 1991, Nature, 352: 624-628; and Marks et al, 1991, Journal of Molecular Biology, 222: 581-597).

In the present invention, unless otherwise expressly indicated, the expressions "each ... is independently selected from" and "... each are independently selected from" are interchangeable and should be understood in a broad sense, which mean that the specific options expressed by the same or different symbols in different groups do not affect each other, or that the specific options expressed by the same or different symbols in the same group do not affect each other.

In the present invention, in the structure represented by formula A

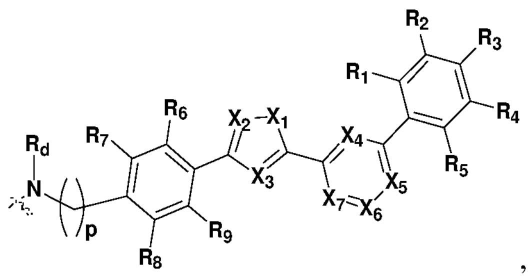

,

$X_7$ is selected from N, or $C(R_0)$, which means that, the structure represented by formula A is

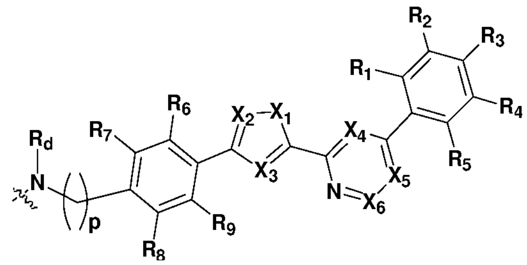

or

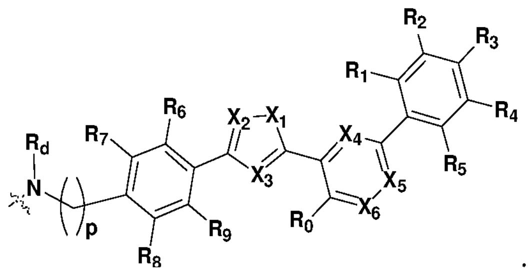

.

Other similar definitions could be understood with a reference to the foregoing.

In the present invention, the term "direct bond" means that the groups on both sides thereof are directly linked, for example, $L_1$ is $L_{11}$-$L_{12}$-$L_{13}$-$L_{14}$, and if $L_{13}$ is a direct bond, $L_1$ is $L_{11}$-$L_{12}$-$L_{14}$, that is, $L_{12}$ is directly linked to $L_{14}$. Other similar definitions could be understood with a reference to the foregoing.

In each part of the present invention, the substituents of the compound of the present invention are disclosed according to the group types or ranges. It is specifically indicated that the present invention includes each independent secondary combination of various members of these group types and ranges. For example, the term "C1-C6 alkyl" specifically refers to independently disclosed methyl, ethyl, C3 alkyl, C4 alkyl, C5 alkyl, and C6 alkyl.

In the present invention, the term "alkyl" is meant to include branched and straight saturated aliphatic hydrocarbon groups having the indicated number of carbon atoms. For example, the term "C1-C6 alkyl" refers to straight or branched alkyls containing 1-6 carbon atoms, including for example "C1-C3 alkyl" or "C1-C4 alkyl", methyl, ethyl and the like, specific examples including but not limited to, methyl, ethyl, n-propyl, isopropyl, n-butyl, isobutyl, sec-butyl, tert-butyl, pentyl, hexyl.

In the present invention, the term "C1-C4 alkyl" refers to straight or branched alkyls containing 1-4 carbon atoms,

including for example "C1-C3 alkyl", methyl, ethyl and the like, specific examples including but not limited to, methyl, ethyl, n-propyl, isopropyl, n-butyl, isobutyl, sec-butyl, tert-butyl.

In the present invention, the term "deuterated alkyl" refers to a group obtained by replacing one or more hydrogen atoms in alkyl with deuterium atom. Wherein, the alkyl can be C1-C6 alkyl, C1-C4 alkyl or C1-C3 alkyl. The alkyl, C1-C6 alkyl, C1-C4 alkyl or C1-C3 alkyl is as defined above.

In the present invention, the term "haloalkyl" refers to a group obtained by replacing one or more hydrogen atoms in alkyl with halogen. For example, it may be "C1-C6 haloalkyl", which refers to a group obtained by replacing one or more hydrogen atoms in C1-C6 alkyl (e.g., C1-C4 alkyl, C1-C3 alkyl and the like) with halogen (preferably fluorine, chlorine), for example, monofluoromethyl, difluoroethyl, trifluoromethyl and the like. The alkyl, C1-C6 alkyl, C1-C4 alkyl or C1-C3 alkyl is as defined above.

In the present invention, the term "hydroxyalkyl" refers to a group obtained by replacing one or more hydrogen atoms in alkyl with hydroxy. For example, it may be "C1-C6 hydroxyalkyl", which refers to a group obtained by replacing one or more hydrogen atoms in C1-C6 alkyl (e.g., C1-C4 alkyl, C1-C3 alkyl and the like) with hydroxy, for example, hydroxymethyl, 2-hydroxyethyl and the like. The alkyl, C1-C6 alkyl, C1-C4 alkyl or C1-C3 alkyl is as defined above.

In the present invention, the term "alkoxy" refers to any alkyl (e.g., C1-C6 alkyl, C1-C4 alkyl, C1-C3 alkyl and the like) described above attached to the rest of the molecule via an oxygen atom (-O-).

In the present invention, the term "cycloalkyl" refers to a hydrocarbon group having a monocyclic ring system of saturated carbon rings, and may be, for example, C3-C6 cycloalkyl, and specifically may be cyclopropyl, cyclobutyl, cyclopentyl, cyclohexyl and the like.

Halogen refers to fluorine, chlorine, bromine or iodine.

In the present invention, the term "aryl" refers to substituted and unsubstituted aromatic 6-membered monocyclic groups, 10-membered bicyclic groups, and 10- or more-membered tricyclic groups, all of which, with no ring heteroatom, contain ring carbon atoms. Specifically, the aryl may be phenyl or naphthyl.

In the present invention, the term "heteroaryl" refers to substituted and unsubstituted aromatic 5- or 6-membered monocyclic groups, 8-, 9- or 10-membered bicyclic groups, and 11- to 14-membered tricyclic groups having at least one heteroatom (N, O or S) in at least one ring, the heteroatom-containing ring optionally further having 1, 2 or 3 heteroatoms selected from N, O or S. Among them, substituted and unsubstituted aromatic 8-, 9- or 10-membered bicyclic groups and 11- to 14-membered tricyclic groups having at least one heteroatom (N, O or S) in at least one ring are "fused heteroaryl groups". As a bicyclic or tricyclic heteroaryl, a bicyclic or tricyclic structure overall is required to form an aromatic system. Heteroaryl may be attached at any available nitrogen or carbon atom of any ring. And those skilled in the art will understand that two adjacent atoms (preferably carbon atoms) are shared between each two rings in the fused ring.

Exemplary monocyclic heteroaryls include, but are not limited to, pyrrolyl, pyrazolyl, imidazolyl, oxazolyl, isoxazolyl, thiazolyl, thiadiazolyl, isothiazolyl, furanyl, thienyl, oxadiazolyl, pyridyl, pyrazinyl, pyrimidinyl, pyridazinyl, triazinyl, triazolyl and the like.

Exemplary bicyclic heteroaryls include, but are not limited to, indolyl, 5-azaindolyl, pyrrolo [2, 3-d]pyrimidinyl, 5, 6-diazaindolyl, 6-azaindolyl, 7-azaindolyl, pyrazolo[3, 4-b]pyridyl, pyrrolo [2, 3-c]pyridazinyl, thieno[2, 3-d]imidazolyl, pyrazolo[3, 4-c]pyridyl, benzothiazolyl, benzimidazolyl, benzoxazolyl, benzothienyl, quinolinyl, isoquinolinyl, benzofuranyl, indolizinyl, quinoxalinyl, indazolyl, pyrrolopyrimidinyl, furanopyridyl, isoindolyl and the like.

In the present invention, the terms "heterocycle", "heterocyclic" or "heterocyclyl" are used interchangeably and refer to substituted and unsubstituted 3- to 7-membered (preferably 4- to 7-membered, more preferably 5- to 6-membered) monocyclic groups, 7- to 11-membered bicyclic groups, and 10- to 15-membered tricyclic groups, which may contain one or more double bonds but do not constitute aromatic rings, wherein at least one ring has at least one heteroatom (N, O or S). Fused rings completing bicyclic and tricyclic groups may contain only carbon atoms and may be saturated or partially saturated, not constituting aromatic rings. Heterocyclic group may be attached at any available nitrogen or carbon atom.

Exemplary monocyclic heterocyclyls include azetidinyl, oxetanyl, pyrrolidinyl, imidazolinyl, oxazolidinyl, isoxazolinyl, thiazolidinyl, tetrahydrofuranyl, piperidinyl, piperazinyl, 2-oxopiperazinyl, 2-oxopiperidinyl, 2-oxopyrrolidinyl, 2-oxoazepinyl, 1-pyridonyl, 4-piperidinonyl, tetrahydropyranyl, morpholinyl, 1, 3-dioxolanyl and the like.

Wherein, "saturated heterocycle" means that the heterocycle defined above does not contain an unsaturated bond, e.g., it does not contain a double bond, for example, "nitrogen-containing saturated heterocyclyl" may be

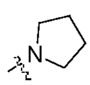 or 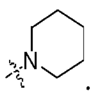.

In the present invention, the term "be substituted" or "substituted" or "substitution" is used interchangeably and refers to selective replacement of any one or more hydrogen atoms on a specified atom or group with a specified group, provided that it does not exceed the normal valence state of the specified atom. Wherein there may be one or more substituents, each substituent being independently selected from the group consisting of deuterium, halogen, alkyl,

cycloalkyl, alkoxy, aryl, heteroaryl, heterocyclyl, wherein halogen, alkyl, cycloalkyl, alkoxy, aryl, heteroaryl, heterocyclyl are as defined above.

From all the above descriptions, it is evident to those skilled in the art that any group whose name is a composite name, for example "cycloalkylalkyl," should be construed as derived conventionally from left to right from its parts, e.g., in the case of "cycloalkylalkyl" from "alkyl" substituted by "cycloalkyl," wherein cycloalkyl and alkyl are as defined above.

Description of figures

Fig. 1 shows the expression of CD33 target on the surface of A431 cell;
Fig. 2 shows the expression of CD33 target on the surface of HEL92.1.7 cell;
Fig. 3 shows the expression of CD33 target on the surface of CMK cell;
Fig. 4 shows the expression of CD33 target on the surface of U937 cell;
Fig. 5 shows the expression of CD33 target on the surface of HL-60 cell;
Figs. 6, 7, 8, 9 and 10 show the results of killing experiments using 4 AML cell lines and A431 as CD33 target cells, respectively;
Fig. 11 shows the expression of Trop2 target on the surface of N87 cell;
Fig. 12 shows the expression of Trop2 target on the surface of A431+SW620 heterogeneous tumor cell;
Figs. 13 and 14 show the results of killing experiments of 2 cells with different Trop2 target expression, respectively;
Figs. 15, 16, 17 and 18 show the results of killing experiments of 4 cells with different HER2 target expression, respectively;
Figs. 19 and 20 show the binding of anti-CD 33 antibody-drug conjugates (mono-drug ADC, dual-drug ADC) to recombinant human CD33 protein, respectively.

Advantageous effects

1. In the ADC drug of the present invention, the concept of synthetic lethality, which involves using drug to damage cancer cells while inhibiting their damage repair, leading to cancer cell death, requires a lower dosage of cell damage repair inhibitor compared to combination therapy, further reducing potential damage to normal cells.
2. The ADC with damage repair inhibitor of the present invention can increase sensitivity to moderately toxic drugs (or increase the availability or mode of administration of moderately toxic drugs).
3. In the ADC with damage repair inhibitor of the present invention, when the toxin drug is a highly toxic compound, the window can be enlarged.

Specific modes for carrying out the invention

The present invention is further illustrated, but is not intended to be limited, by the following description of specific embodiments. Various modifications or improvements may be made by those skilled in the art in light of the teachings of the present invention without departing from the basic spirit and scope thereof. The reagents or instruments used, for which the manufacturer is not indicated, are all conventional products commercially available.

The present application discloses an antibody-drug conjugate with a targeting protein simultaneously coupled with a DNA damage repair inhibitor and an additional drug. In particular, in certain embodiments, one of the DNA damage inhibitor and the additional drug is coupled to the original interchain disulfide cysteine (Cys) residue of the targeting protein, and the other is linked to a cysteine (Cys) residue of the targeting protein in a site-directed coupling manner, wherein the site-directed coupling mechanism is that some original amino acids on the targeting protein are subjected to site-directed mutagenesis to become cysteine, or that cysteine or a polypeptide containing cysteine is subjected to site-directed insertion.

In the present invention, the correspondence between the abbreviations of some drugs and their structural formulas is shown as follows:

XD-01:

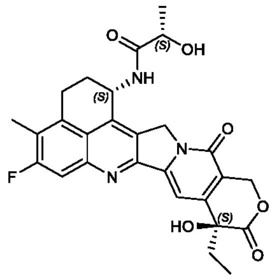

MMAE:

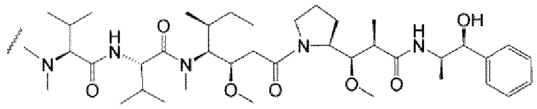

PNU159682:

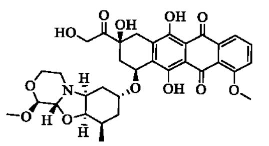

PBD:

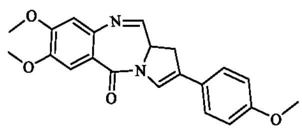

CBI:

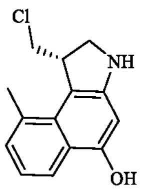

PBD-CBI:

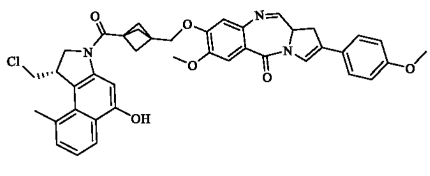

AMCA4:

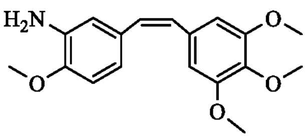

VE-822:

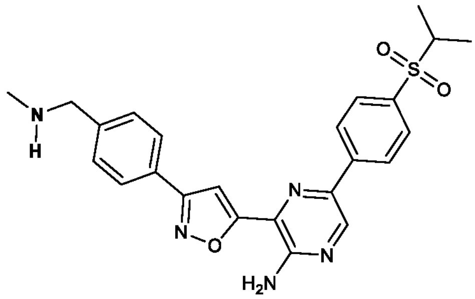

Talazoparib:

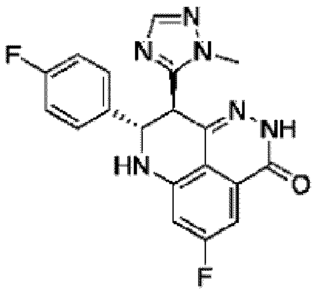

E7016:

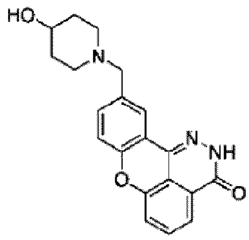

niraparib:

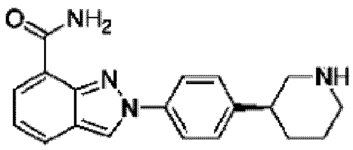

Rucaparib:

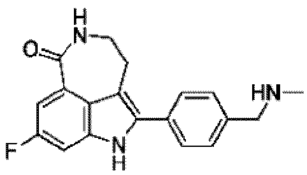

BGB-290:

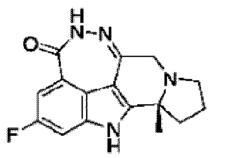

CEP-9722:

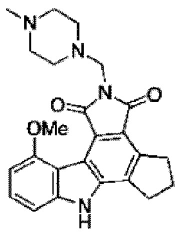

Ceralasertib (AZD6738):

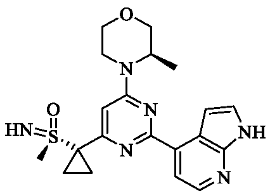

VX-803 (M4344):

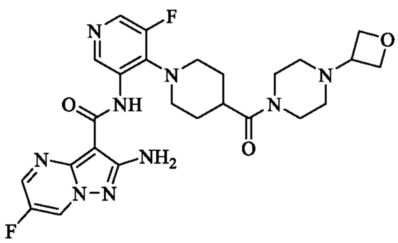

AZ20:

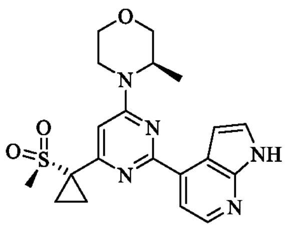

Elimusertib (BAY-1895344):

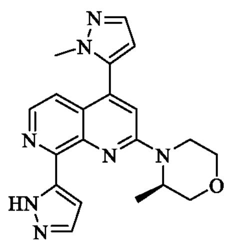

Adavosertib (MK-1775, AZD1775):

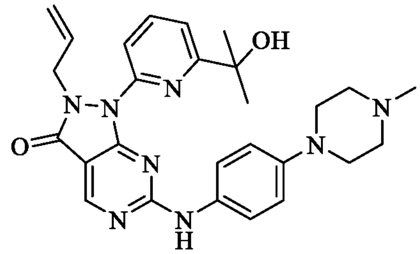

SRA737 (CCT245737, PNT-737):

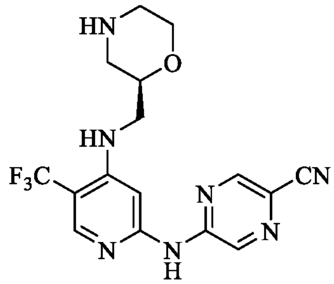

Prexasertib (LY2606368, ACR368):

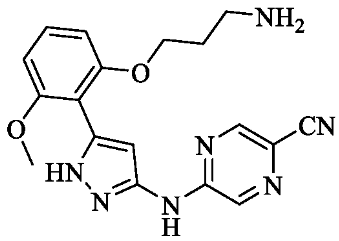

CHIR-124:

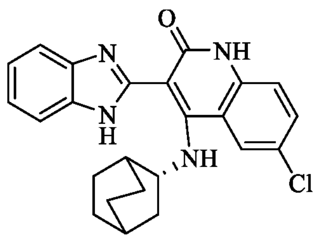

PF-477736 (PF-736):

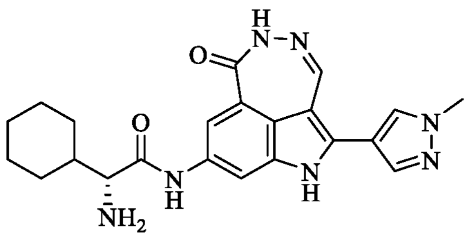

MK-8776 (SCH 900776):

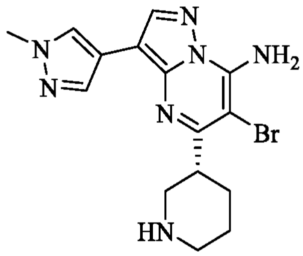

GDC-0575 (ARRY-575, RG7741):

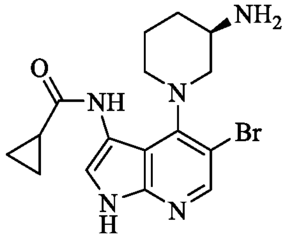

CC-115:

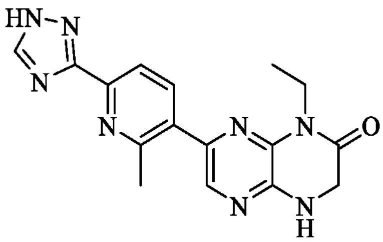

AZD7648:

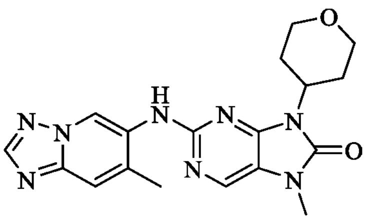

Nedisertib (M3814, Peposertib):

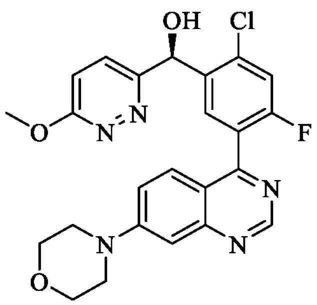

KU-0060648:

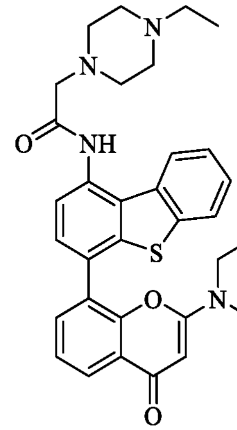

VX-984 (M9831):

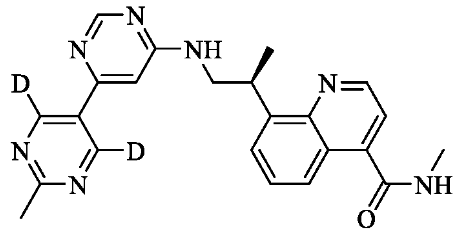

The present invention is further illustrated with a combination of the following examples in which, unless otherwise indicated, the starting materials are all commercially available.

**Example 1**

**Synthesis of compound A-4:**

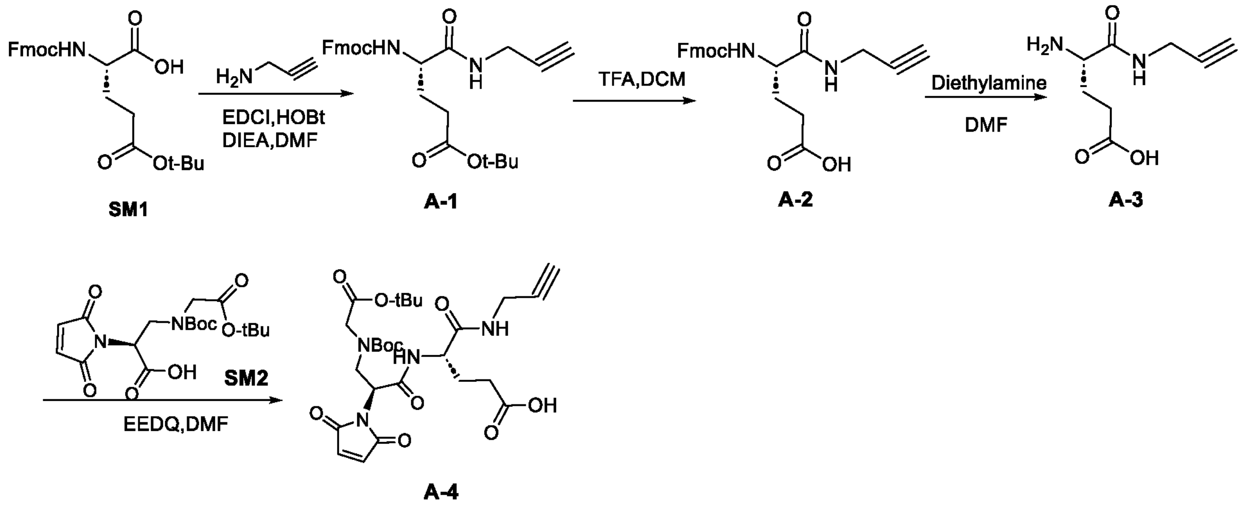


**Step 1:** Synthesis of compound **A-1**

To a 2L single-mouth bottle, Fmoc-L-glutamic acid 5-tert-butyl ester **(SM1,** 200g, 470mmol), propargylamine (28.5g, 517mmol) were added, dissolved with 400mL of DMF to obtain a clear solution, cooled with an ice-water bath to about 0°C, then EDCI (108.2g, 564mmol), HOBt (76.2g, 564mmol) and DIEA (117mL, 705mmol) were added, and reacted at room temperature for 2h, while the end point of the reaction being monitored by TLC. After completion of the reaction, the

reaction solution was poured into 2L of water, and extracted with ethyl acetate three times (500mL*3) The organic phases were combined, washed with saturated NaCl solution twice, and then dried over anhydrous sodium sulfate. After filtering to remove sodium sulfate, the filtrate was concentrated to obtain a crude product. The crude product was refined with petroleum ether/ethyl acetate (2/1), to obtain compound **A-1** (100.9g), yield 48%, LCMS: $[M+H]^+$=463.2.

**Step 2**: Synthesis of compound **A-2**

To a 1L single-mouth bottle, **A-1** (100.0 g) was added, dissolved with 500mL of dichloromethane, then 200mL of trifluoroacetic acid was added, and reacted at room temperature for 2h, while the end point of the reaction being monitored by TLC. After completion of the reaction, the reaction solution was concentrated to remove DCM and a part of trifluoroacetic acid, to obtain a crude product. Methyl tert-butyl ether was added to the crude product, to precipitate out a white solid, followed by filtering, drying, to obtain compound **A-2** (77.5g), yield 87%, LCMS: $[M-H]^-$=405.1.

**Step 3**: Synthesis of compound **A-3**

To a 2L single-mouth bottle, **A-2** (77.0g) was added, dissolved with 150mL of DMF, then 38mL of diethylamine was added, and reacted at room temperature for 2h, while the end point of the reaction being monitored by TLC. After completion of the reaction, 800mL of methyl tert-butyl ether was added to the reaction solution, to precipitate out a white solid, followed by filtering, drying, to obtain compound **A-3** (33.7g), yield 96.6%, LCMS: $[M-H]^-$=183.1.

**Step 4**: Synthesis of compound **A-4**

To a 100mL single-mouth bottle, compound **A-3** (10.02g, 54.4mmol), compound **SM2** (14.48g, 36.3 mmol) [synthesized according to the method in CN108452321] were added, dissolved with 100mL of DMF, then EEDQ (13.5g, 54.8mmol) was added, and reacted at room temperature for 2h, while the end point of the reaction being monitored by HPLC. After completion of the reaction, the reaction solution was purified by preparative liquid chromatography to obtain a product preparation solution, and then the preparation solution was lyophilized, to obtain compound **A-4** (9.94g), yield 48%, LCMS: $[M-H]^-$=563.2.

**Example 2**

**Synthesis of compound A-6:**

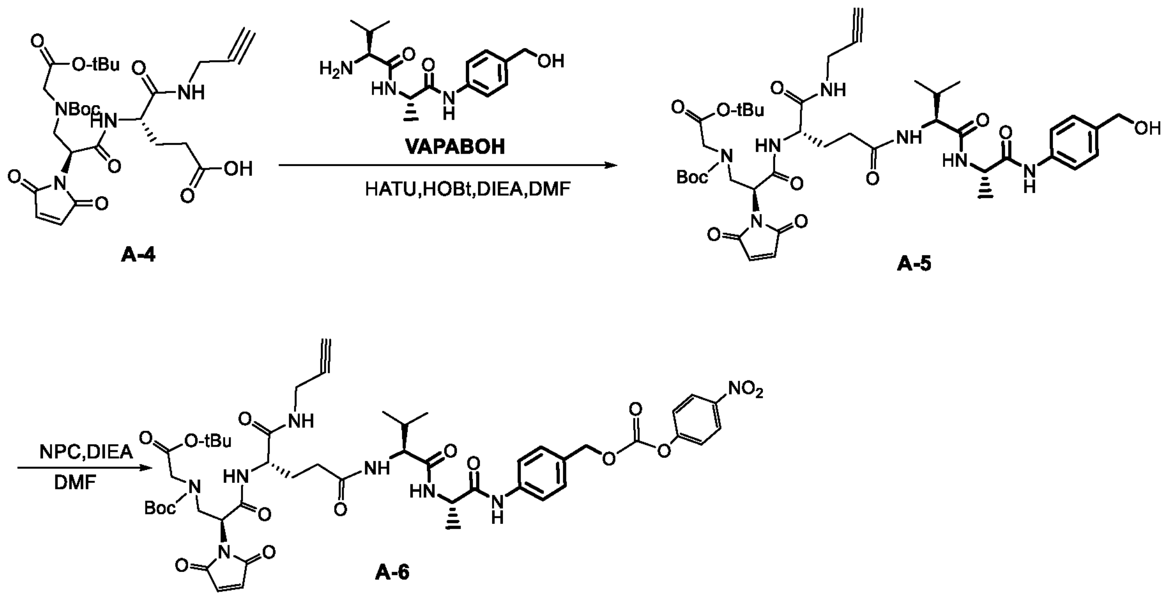


**A-4**

**A-5**

**A-6**

**Step 1:** Synthesis of compound **A-5**

To a 250mL single-mouth bottle, compound **A-4** (6.38g, 11.3mmol), compound VAPAB-OH (3.98g, 13.56 mmol)

were added, dissolved with 50mL of DMF, then EDCI (2.6g, 13.56mmol), HOBt (1.83g, 13.56mmol) and DIEA (2.8mL, 16.96mmol) were added, and reacted at room temperature for 2h, while the end point of the reaction being monitored by HPLC. After completion of the reaction, the reaction solution was purified by preparative liquid chromatography to obtain a product preparation solution, and then the preparation solution was lyophilized, to obtain a white solid compound **A-5** (5.07g), yield 53%, LCMS: $[M+H]^+$=840.4.

**Step 2**: Synthesis of compound **A-6**

To a 100mL single-mouth bottle, compound **A-5** (3.94g, 4.7mmol), compound di(p-nitrophenyl) carbonate (NPC, 8.56g, 28.14 mmol) were added, dissolved with 40mL of DMF, then DIEA (2.32mL, 14.07mmol) was added, and reacted at room temperature for 1.5h, while the end point of the reaction being monitored by TLC. After completion of the reaction, the reaction solution was purified by column chromatography, to obtain compound **A-6** (3.9g), yield 83%, LCMS: $[M+H]^+$=1005.4.

**Example 3**

**Synthesis of compound A-8:**

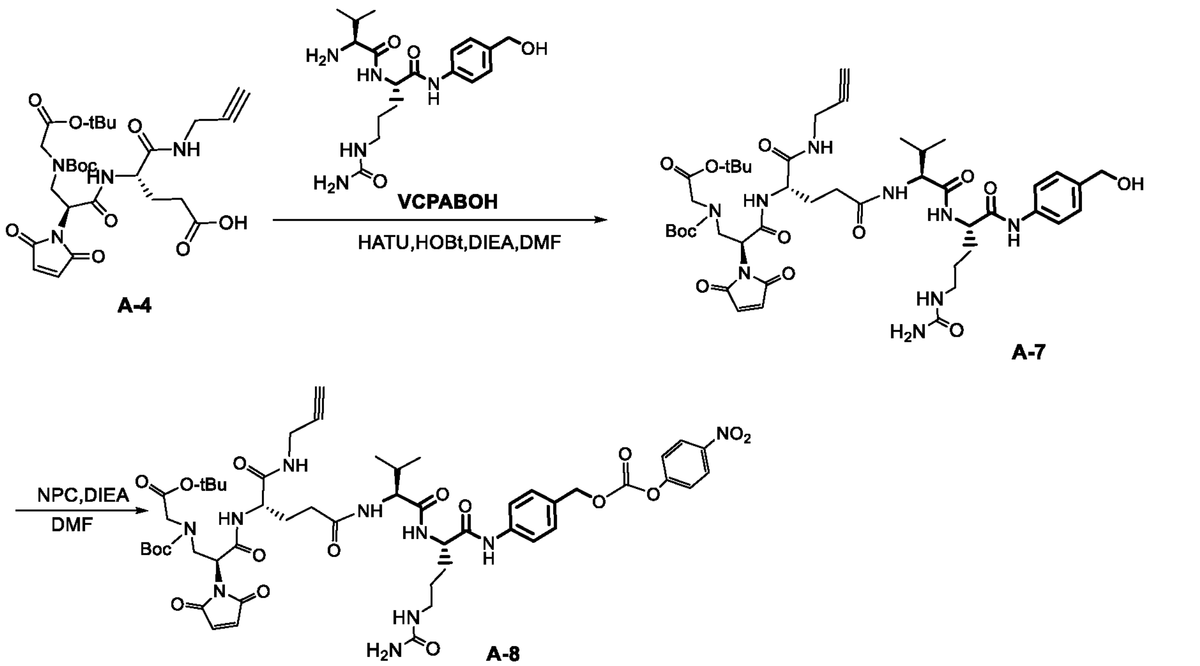


A-4 A-7 A-8

**Step 1**: Synthesis of compound **A-7**

To a 50mL single-mouth bottle, compound **A-4** (2.5 g, 4.4mmol), compound VCPAB-OH (2.0g, 5.28 mmol) were added, dissolved with 25mL of DMF, then EDCI (1.01g, 5.28mmol), HOBt (0.72g, 5.28mmol) and DIEA (1.1mL, 6.6mmol) were added, and reacted at room temperature for 2h, while the end point of the reaction being monitored by HPLC. After completion of the reaction, the reaction solution was purified by preparative liquid chromatography to obtain a product preparation solution, and then the preparation solution was lyophilized, to obtain a white solid compound **A-7** (1.96g), yield 48%, LCMS: $[M+H]^+$=926.4.

**Step 2**: Synthesis of compound **A-8**

To a 50mL single-mouth bottle, compound **A-7** (1.85g, 2.0mmol), compound di(p-nitrophenyl) carbonate (NPC, 3.65g, 12.0mmol) were added, dissolved with 15mL of DMF, then DIEA (1.05mL, 6.0mmol) was added, and reacted at room temperature for 1.5h, while the end point of the reaction being monitored by TLC. After completion of the reaction, the reaction solution was purified by column chromatography, to obtain compound **A-8** (1.77g), yield 81%, LCMS:

$[M+H]^+$=1091.5.

**Example 4**

**Synthesis of compound B-1:**

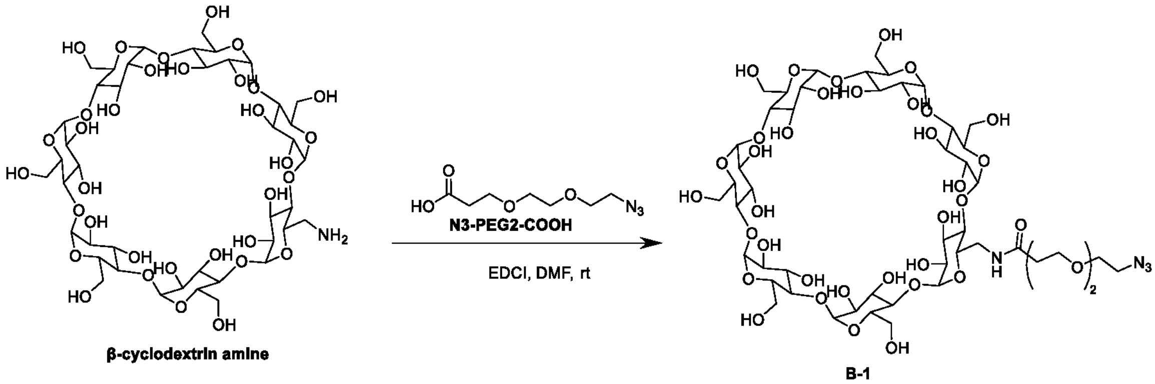


β-cyclodextrin amine

B-1

To a 25mL single-mouth bottle, **β-cyclodextrin amine** (mono(6-amino-6-deoxy)-β-cyclodextrin, purchased, 2.0 g, 1.76mmol), compound **N3-PEG2-COOH** (purchased, 428.8mg, 2.11 mmol) were added, dissolved with 7mL of DMF, then EDCI (405mg, 2.11mmol) was added, and reacted at room temperature for 2h, during which the system changed from turbid to clear, while the end point of the reaction being monitored by HPLC. After completion of the reaction, the reaction solution was purified by preparative liquid chromatography to obtain a product preparation solution, and then the preparation solution was lyophilized, to obtain a white solid compound **B-1** (1.68g), yield 72%, LCMS: $[M+H]^+$=1319.5.

**Example 5**

**Synthesis of compound B-2:**

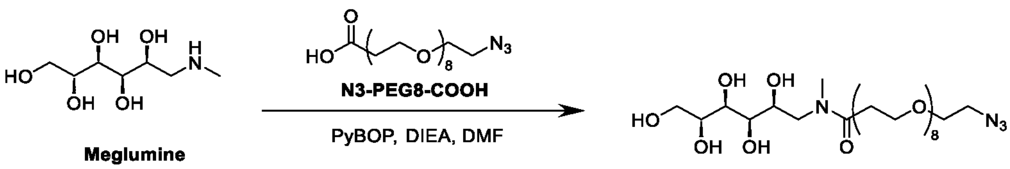


Meglumine

B-2

To a 100mL single-mouth bottle, **meglumine** (3.0 g, 6.4mmol), compound **N3-PEG8-COOH** (purchased, 1.5g, 7.68 mmol) were added, dissolved with 24mL of DMF, then PyBOP (4.0 g, 7.68mmol), DIEA (1.58mL, 9.6 mmol) were added, and reacted at room temperature for 2h, while the reaction being monitored by HPLC. The reaction was completed until there was no significant progress. The reaction solution was purified by preparative liquid chromatography to obtain a product preparation solution, and then the preparation solution was lyophilized, to obtain a light yellow oily substance **B-2** (3.27g), yield 32%, LCMS: $[M+H]^+$=645.3.

**Example 6**

**Synthesis of compound B-3:**

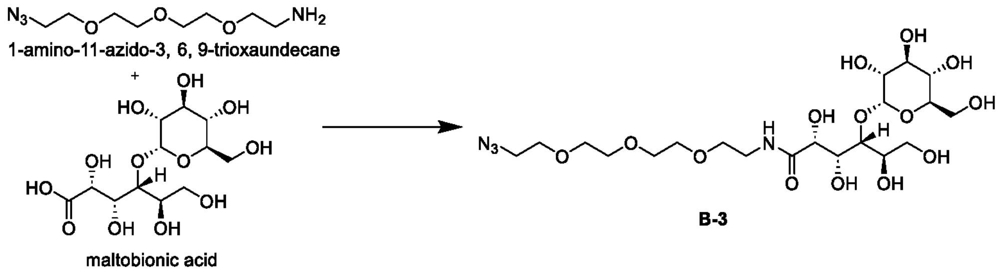

1-amino-11-azido-3, 6, 9-trioxaundecane

+

maltobionic acid

B-3

At room temperature, maltobionic acid (7.0 g, 19.5 mmol) was dissolved with 70 mL of anhydrous methanol, the atmosphere in the system was replaced with nitrogen three times, with the addition of 4Å molecular sieve, the reaction solution was heated to reflux overnight, after cooling to room temperature condition, 1-amino-11-azido-3, 6, 9-trioxaundecane was added, and stirred at room temperature for 24 h, and then the reaction solution was directly concentrated under reduced pressure. The obtained crude product was dissolved with 5 mL of methanol, then 30 mL of chloroform and 100 mL of tetrahydrofuran were added in sequence, followed by stirring and standing, to obtain a white solid. After filtering, the filter cake was rinsed with tetrahydrofuran twice. The obtained white solid was dissolved with 30 mL of water, and lyophilized to obtain 5.6 g of a white solid compound **B-3;** LC-MS m/z $(M+H)^+$: 559.3.

## Example 7

Synthesis of compound **R01**':

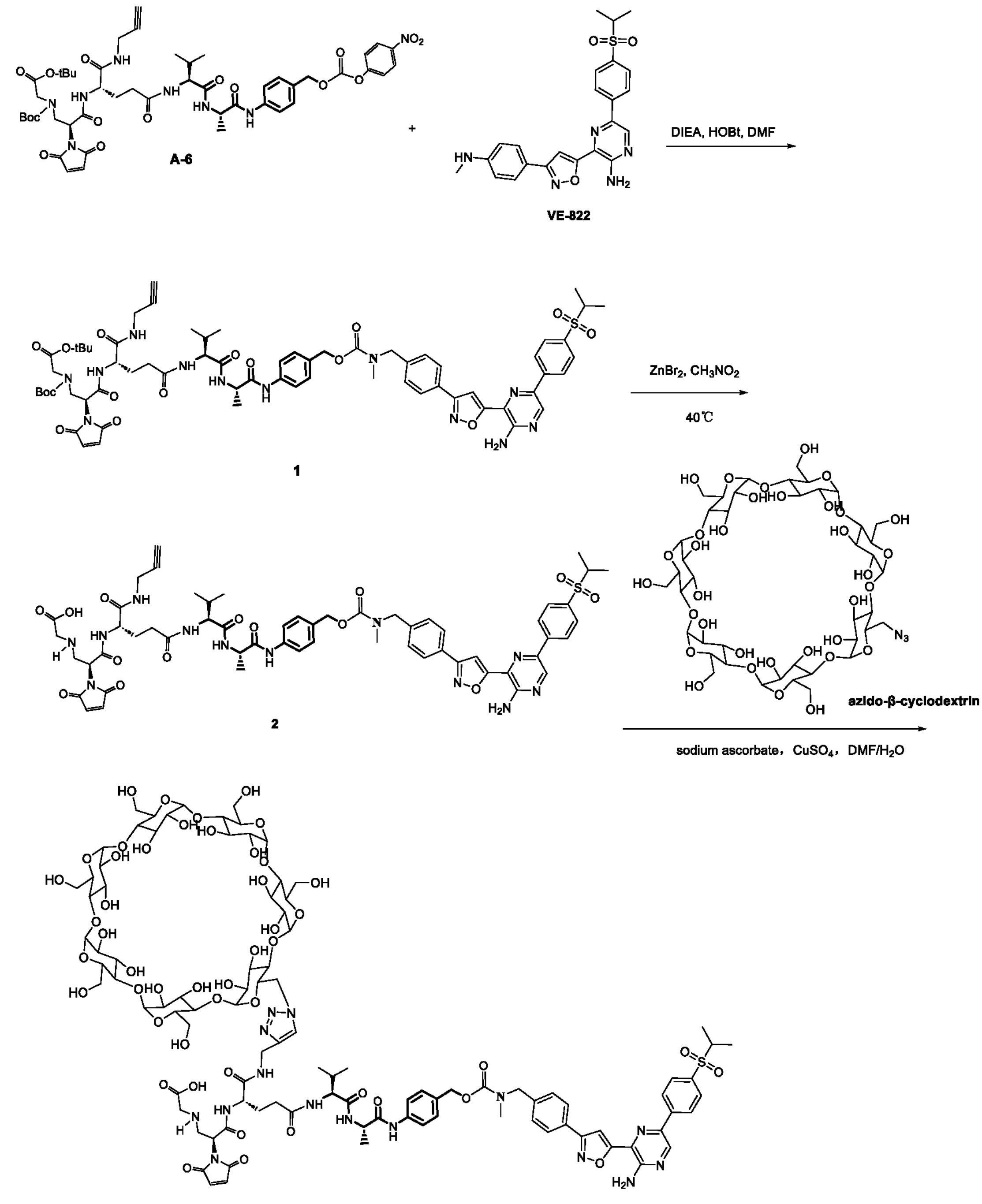


A-6

VE-822

1

2

R01'

**Step 1:** Synthesis of compound **1**

To a 25mL single-mouth bottle, compound **VE-822** (840.5mg, 1.81mmol), **A-6** (1.9231g, 1.90mmol), HOBt (369.0mg, 2.72mmol) were added, dissolved with 10mL of DMF, then DIEA (300uL, 1.81mmol) was added, and reacted at room temperature for 1h, while the reaction being monitored by TLC. After completion of the reaction, the reaction solution was purified by high-performance liquid preparative chromatography to obtain a product preparation solution, and then the preparation solution was lyophilized to obtain a yellow solid compound **1** (1.9270g), yield 80%, LCMS: $[M+H]^+$=1329.6.

**Step 2:** Synthesis of compound **2**

To a 100mL single-mouth bottle, compound **1** (604.2mg, 0.45mmol), zinc bromide (2017.6mg, 9.09mmol) were added, dissolved with 18mL of $CH_3NO_2$ to obtain a uniform yellow turbid solution, and reacted at 40°C for 0.5h, the solvent

was removed by concentration under reduced pressure at 45°C using a water pump, while the end point of the reaction being monitored by HPLC.

The concentrate was purified by high-performance liquid preparative chromatography to obtain a product preparation solution, and then the preparation solution was lyophilized to obtain compound **2** (225.6mg), yield 42%, LCMS: $[M+H]^+$= 1173.4.

**Step 3:** Synthesis of compound **R01'**

To a 10mL PE pipe, compound **2** (27.73mg, 0.02mmol), azido-β-cyclodextrin (purchased, 33.0mg, 0.026mmol) were added, dissolved with 2mL of DMF and 0.2mL of water, then $CuSO_4 \cdot 5H_2O$ (38.07mg, 0.075mmol), sodium ascorbate (21.99mg, 0.075mmol) were added, and reacted at room temperature for 1h, while the end point of the reaction being monitored by HPLC. After completion of the reaction, the reaction solution was purified by high-performance liquid preparative chromatography to obtain a product preparation solution, and then the preparation solution was lyophilized to obtain compound **R01'** (46.11mg), yield 83.6%, LC-MS: $[M+2H]^{2+}$=1167.4.

## Example 8

Synthesis of compound **R01-1'**:

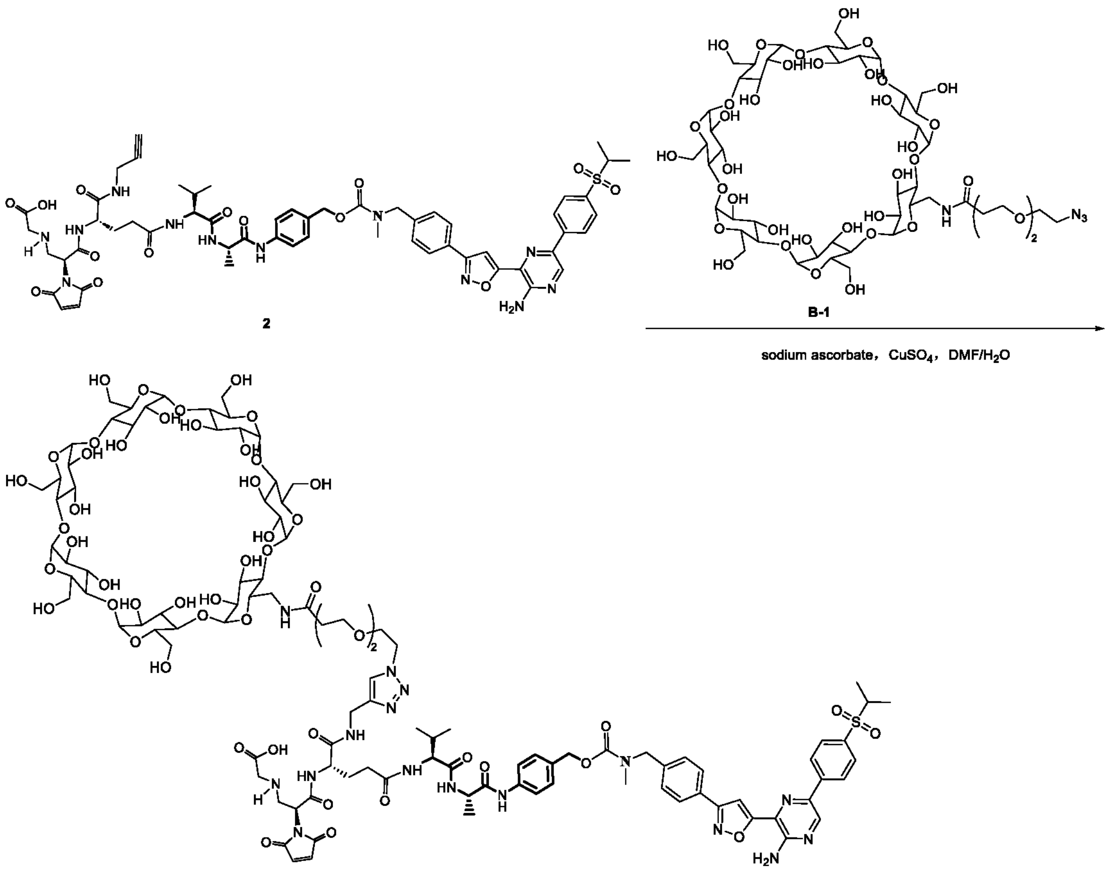


2

B-1

R01-1'

To a 50mL round bottom flask, compound 2 (379.8mg, 0.32mmol), **B-1** (480.65mg, 0.364mmol) were added, dissolved with 4mL of DMF and 0.4mL of water, then $CuSO_4 \cdot 5H_2O$ (262.8mg, 1.1mmol), sodium ascorbate (206.1mg, 1.04mmol) were added, and reacted at room temperature for 1h, while the end point of the reaction being monitored by HPLC. After completion of the reaction, the reaction solution was purified by high-performance liquid preparative chromatography to obtain a product preparation solution, and then the preparation solution was lyophilized to obtain compound **R01-1'** (386.4mg), yield 48%, LC-MS: $[M+2H]^{2+}$=1247.0.

**Example 9**

Synthesis of compound **R02':**

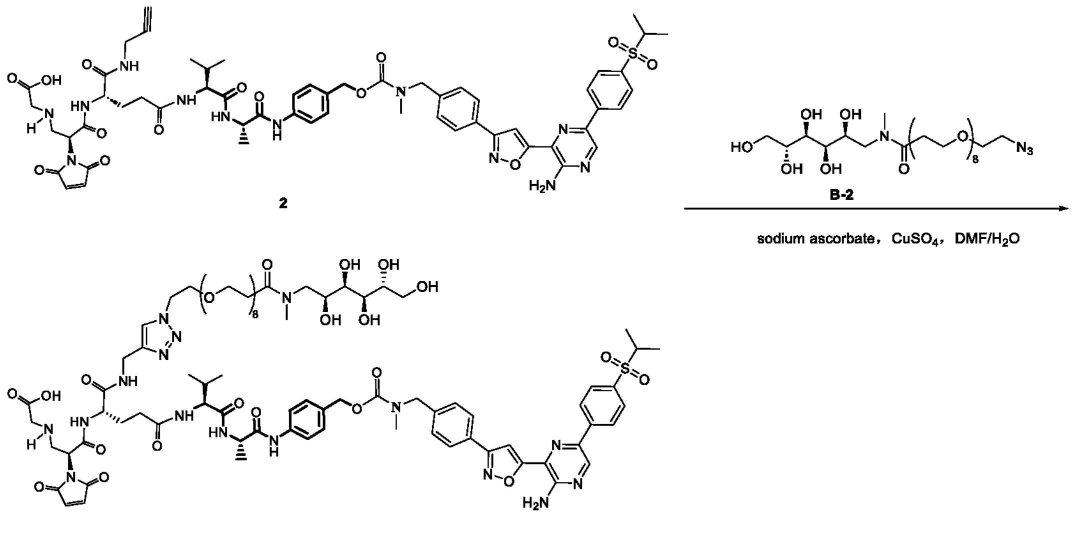


R02'

To a 50mL round bottom flask, compound 2 (105.2mg, 0.09mmol), **B-2** (72.3mg, 0.112mmol) were added, dissolved with 4mL of DMF and 0.8mL of water, then $CuSO_4 \cdot 5H_2O$ (69.9mg, 0.28mmol), sodium ascorbate (59.46mg, 0.30mmol) were added, and reacted at room temperature for 0.5h, while the end point of the reaction being monitored by HPLC. After completion of the reaction, the reaction solution was purified by high-performance liquid preparative chromatography to obtain a product preparation solution, and then the preparation solution was lyophilized to obtain compound **R02'** (120.8mg), yield 74%, LC-MS: $[M+2H]^{2+}$=909.4.

**Example 10**

Synthesis of compound **R01-4':**

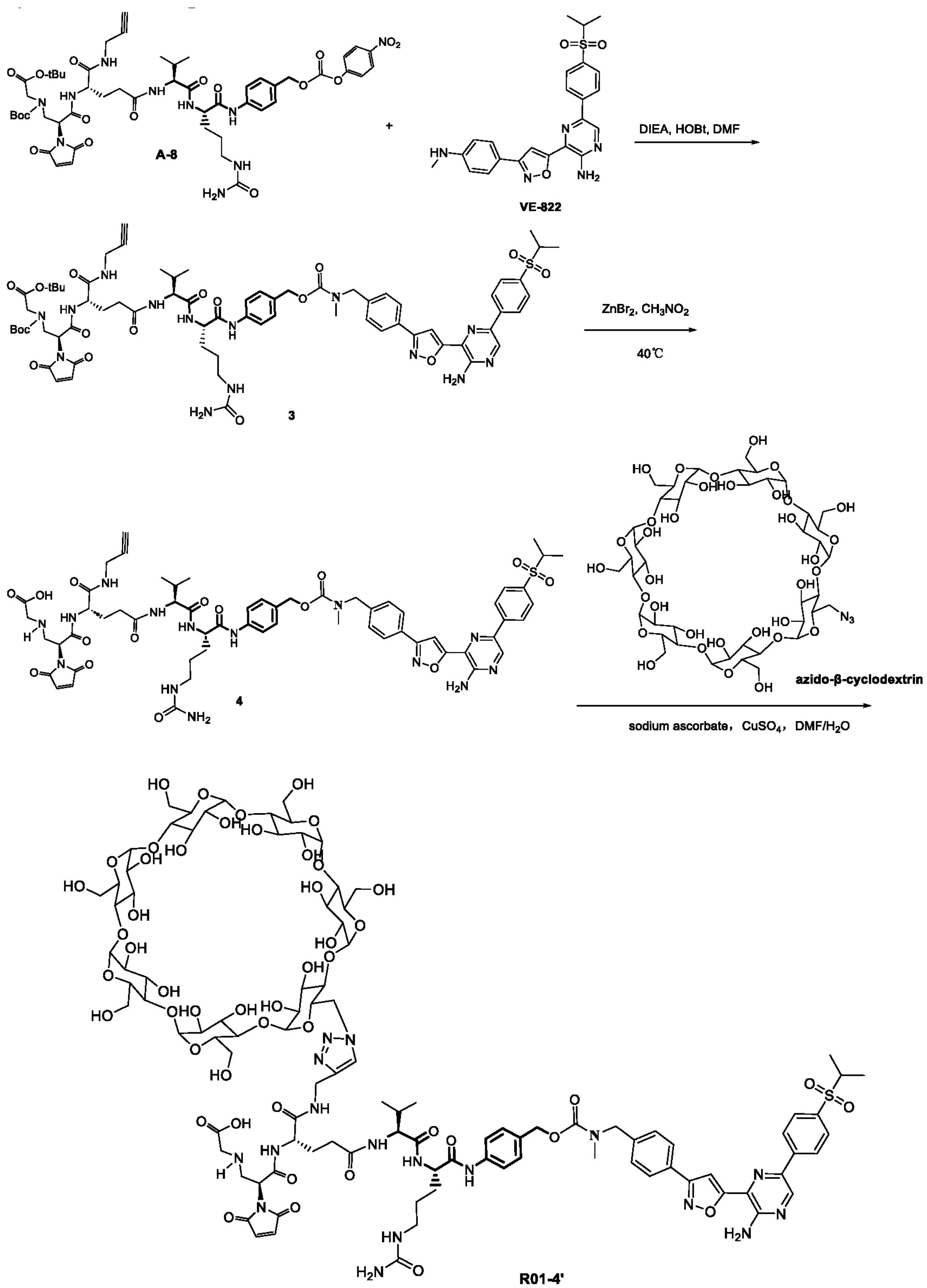


**Step 1:** Synthesis of compound **3**

To a 10mL PE pipe, compound **VE-822** (28.77mg, 0.062mmol), **A-8** (70.77g, 0.065mmol), HOBt (13.62mg, 0.10mmol) were added, dissolved with 1mL of DMF, then DIEA (10uL, 0.062mmol) was added, and reacted at room temperature for 1h, while the reaction being monitored by TLC. After completion of the reaction, the reaction solution was purified by high-performance liquid preparative chromatography to obtain a product preparation solution, and then the preparation solution was lyophilized to obtain a yellow solid compound **3** (75.44mg), yield 86%, LCMS: $[M+H]^+$=1415.6.

**Step 2**: Synthesis of compound **4**

To a 25mL round bottom flask, compound **3** (75.0mg, 0.053mmol), zinc bromide (238.95mg, 1.07mmol) were added, dissolved with 7.5mL of $CH_3NO_2$, and reacted at 40°C for 0.5h, the solvent was removed by concentration under reduced pressure at 45°C using a water pump, while the end point of the reaction being monitored by HPLC. The concentrate was purified by high-performance liquid preparative chromatography to obtain a product preparation solution, and then the preparation solution was lyophilized to obtain compound **4** (18.82mg), yield 28%, LCMS: $[M+H]^+$= 1259.5.

**Step 3**: Synthesis of compound **R01-4'**

To a 10mL PE pipe, compound **4** (18.5mg, 0.015mmol), azido-β-cyclodextrin (19.5mg, 0.017mmol) were added, dissolved with 1mL of DMF and 0.1mL of water, then $CuSO_4 \cdot 5H_2O$ (5.12mg, 0.02mmol), sodium ascorbate (4.02mg, 0.02mmol) were added, and reacted at room temperature for 1h, while the end point of the reaction being monitored by HPLC. After completion of the reaction, the reaction solution was purified by high-performance liquid preparative chromatography to obtain a product preparation solution, and then the preparation solution was lyophilized to obtain compound **R01-4'** (21.6mg), yield 60%, LC-MS: $[M+2H]^{2+}$=1210.3.

**Example 11**

Synthesis of compound **Ex01':**

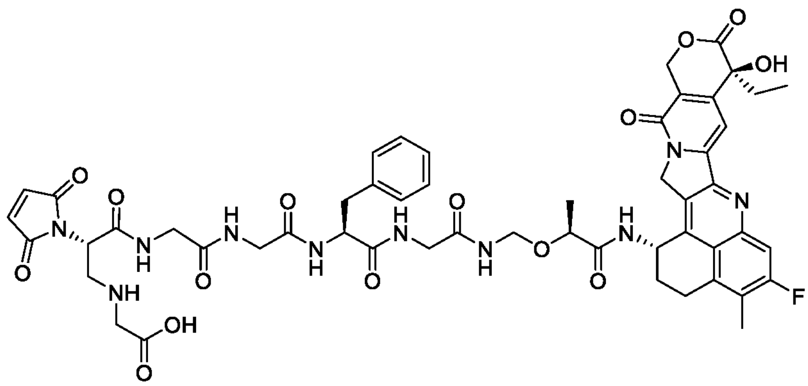

Compound **Ex01'** was synthesized with a reference to the patent CN113827736A of our company.

**Example 12**

Synthesis of compound **10:**

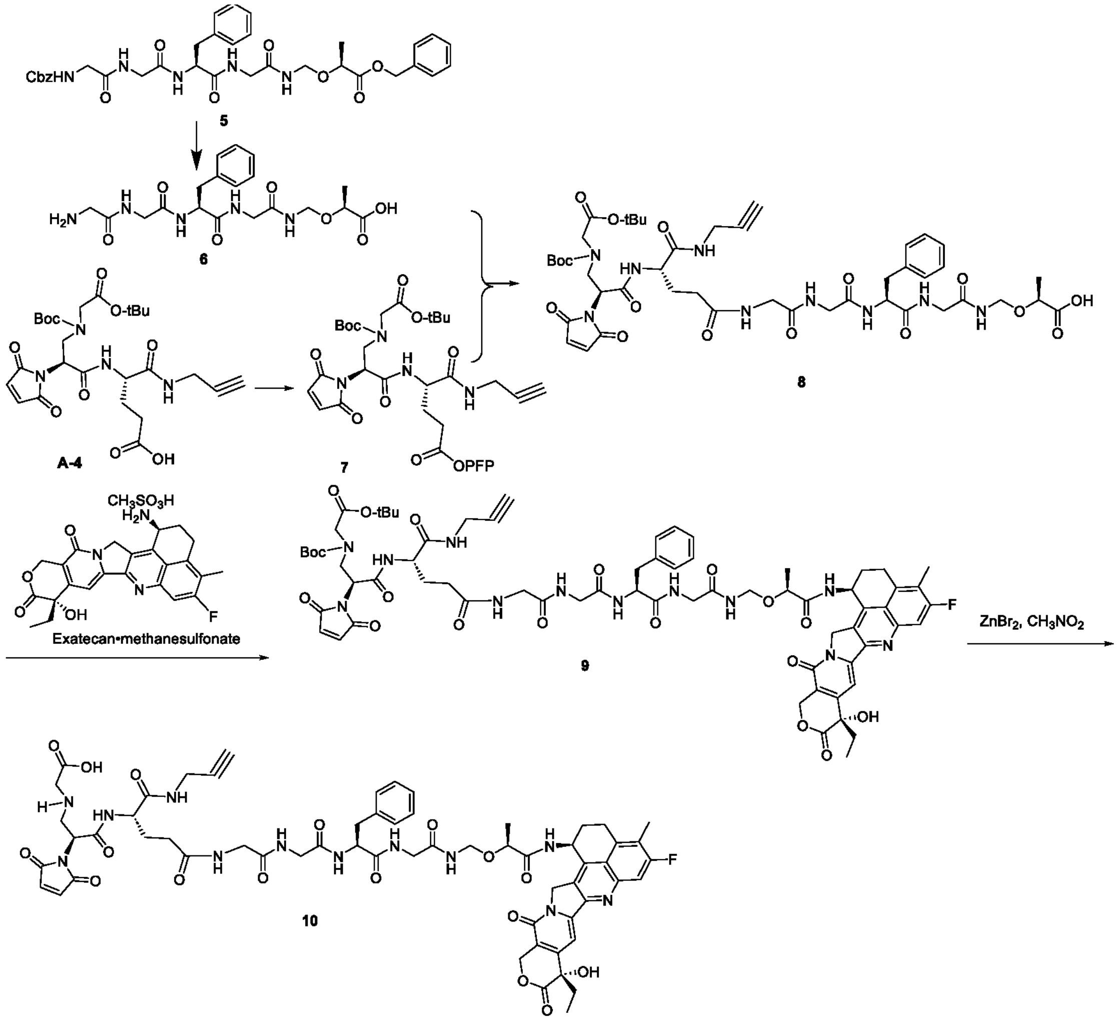


5 6 A-4 7 8 9 10

**Step 1:** Synthesis of compound **6**

To a 50 mL single-mouth bottle, compound **5** (1.0 g, 1.51mmol, synthesized according to the patent WO2020063676A1) was added, dissolved with 15 mL of DMF to obtain a clear solution, then 5%Pd/C(1.0 g) was added, to carry out hydrogenation at room temperature for 2 h, while the end point of the reaction being monitored by HPLC. The reaction solution was filtered to obtain a filtrate for later use, denoted as filtrate 1;

**Step 2**: Synthesis of compound **7**

To another 50 mL single-mouth bottle, compound **A-4** (0.85 g, 1.51 mmol) was added, dissolved with 15 mL of DMF to obtain a clear solution, then DCC (1.55 g, 7.56mmol), pentafluorophenol (0.31 g, 1.66 mmol) were added in an ice-water bath, and reacted at room temperature for 2 h, while the end point of the reaction being monitored by TLC. After completion of the reaction, the reaction solution was filtered to obtain a filtrate, denoted as filtrate 2;

**Step 3**: Synthesis of compound **8**

In an ice-water bath, the filtrate 1 was added to the filtrate 2, and then reacted at room temperature for 1 h, while the end point of the reaction being monitored by HPLC. After completion of the reaction, the reaction solution was purified by high-performance liquid preparative chromatography to obtain a product preparation solution, and then the preparation solution was lyophilized to obtain 805 mg of a white solid compound **8,** yield 54%, LC-MS m/z $(M+H)^+$: 985.2.

**Step 4**: Synthesis of compound **9**

To a 50 mL single-mouth bottle, compound **8** (500 mg, 0.51 mmol) was added, dissolved with 10 mL of DMF to

obtain a clear solution, PyBOP (396 mg, 0.76mmol), HOBt (103 mg, 0.76 mmol), Exatecan·methanesulfonate (270 mg, 0.51 mmol) were added in sequence in an ice-water bath, then DIEA (252 uL, 1.52 mmol) was added, and reacted at room temperature for 2 h, while the end point of the reaction being monitored by HPLC. After completion of the reaction, the reaction solution was purified by high-performance liquid preparative chromatography to obtain a product preparation solution, and then the preparation solution was lyophilized to obtain 500 mg of a white solid compound **9,** yield 70%, LC-MS m/z $(M+H)^+$: 1402.2.

**Step 5**: Synthesis of compound **10**

To a 100mL round bottom flask, compound **9** (450.1mg, 0.32mmol), zinc bromide (1448.2mg, 6.42mmol) were added, dissolved with 30mL of $CH_3NO_2$, and reacted at 45°C for 0.5h, the solvent was removed by concentration under reduced pressure at 45°C using a water pump, while the end point of the reaction being monitored by HPLC. The concentrate was purified by high-performance liquid preparative chromatography to obtain a product preparation solution, and then the preparation solution was lyophilized to obtain compound **10** (203.9mg), yield 51%, LCMS: $[M+H]^+$= 1245.6.

**Example 13**

Synthesis of compound **Ex02':**

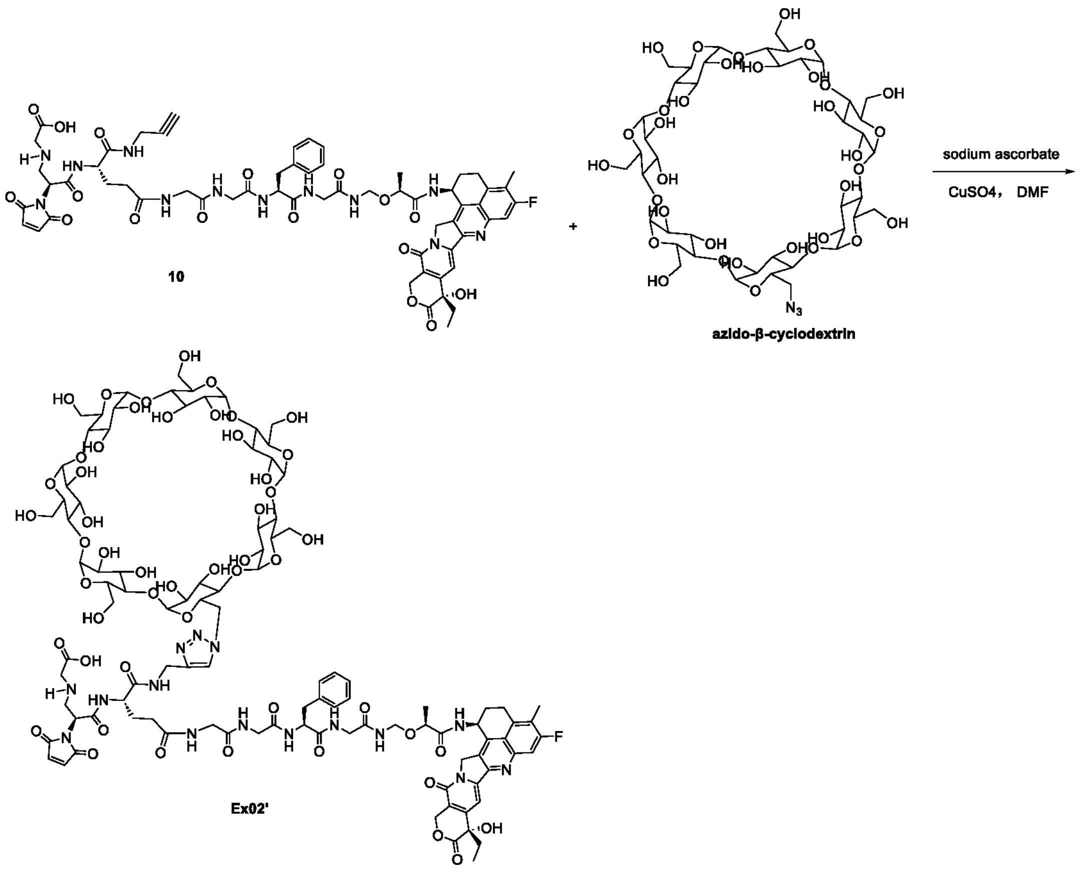


10

azido-β-cyclodextrin

Ex02'

To a 10mL PE pipe, compound **10** (100.1 mg, 0.081 mmol), azido-β-cyclodextrin (100.5 mg, 0.087 mmol) were added, dissolved with 2mL of DMF and 0.2mL of purified water to obtain a clear solution, then $CuSO_4 \cdot 5H_2O$ (20.1mg, 0.081mmol), sodium ascorbate (16.0mg, 0.081mmol) were added, and reacted at room temperature for 1h, while the end point of the reaction being monitored by HPLC. After completion of the reaction, the reaction solution was purified by high-performance liquid preparative chromatography to obtain a product preparation solution, and then the preparation solution was lyophilized to obtain compound **Ex02'** (91.6mg), yield 47%, LC-MS: $[M+2H]^{2+}$=1203.0.

**Example 14**

Synthesis of compound **Ex03'**

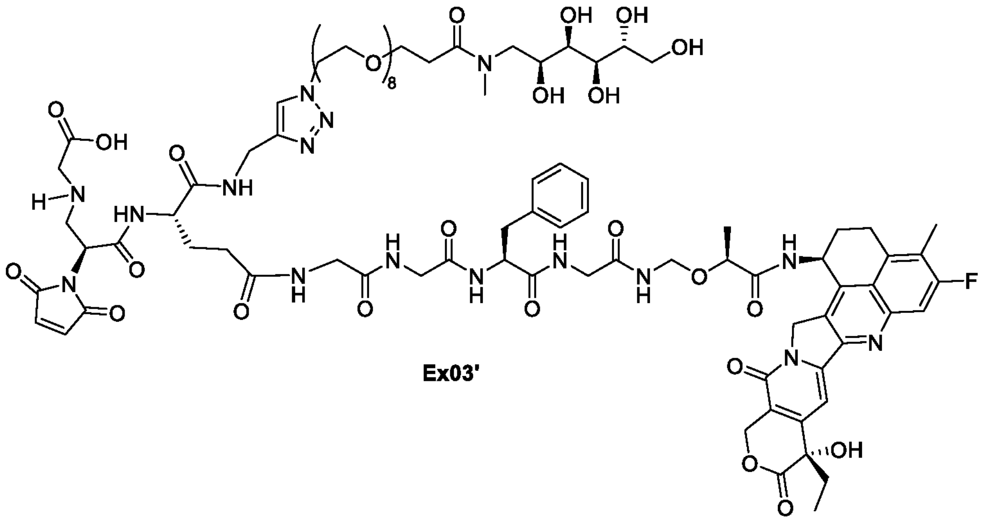

**Ex03'**

With a reference to the synthetic route of Example 9, compound **Ex03'** (57.8mg) was synthesized using compound **10** and compound **B-2;** LC-MS m/z$(M+2H)^{2+}$: 1023.9.

**Example 15**

Synthesis of compound **Ex04'**

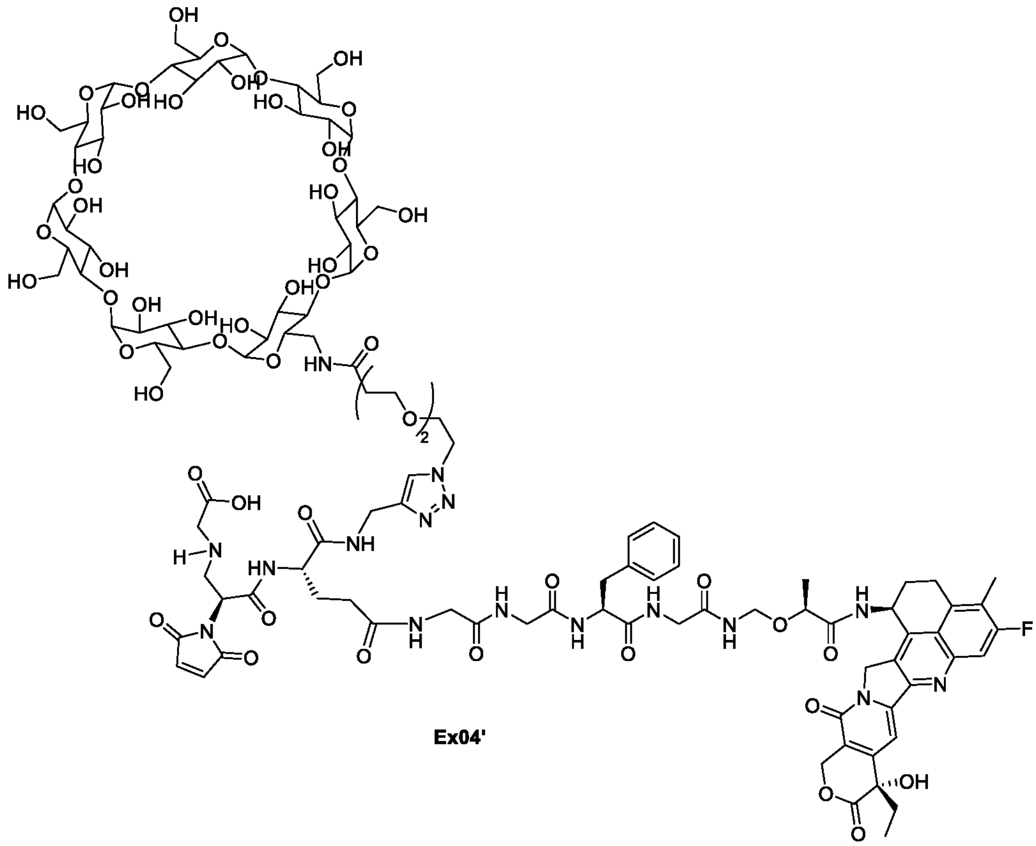

Ex04'

With a reference to the synthetic route of Example 8, compound **Ex04'** (125.2mg) was synthesized using compound **10** and compound **B-1;** LC-MS m/z$(M+2H)^{2+}$: 1282.8.

**Example 16**

Synthesis of compound **PB01':**

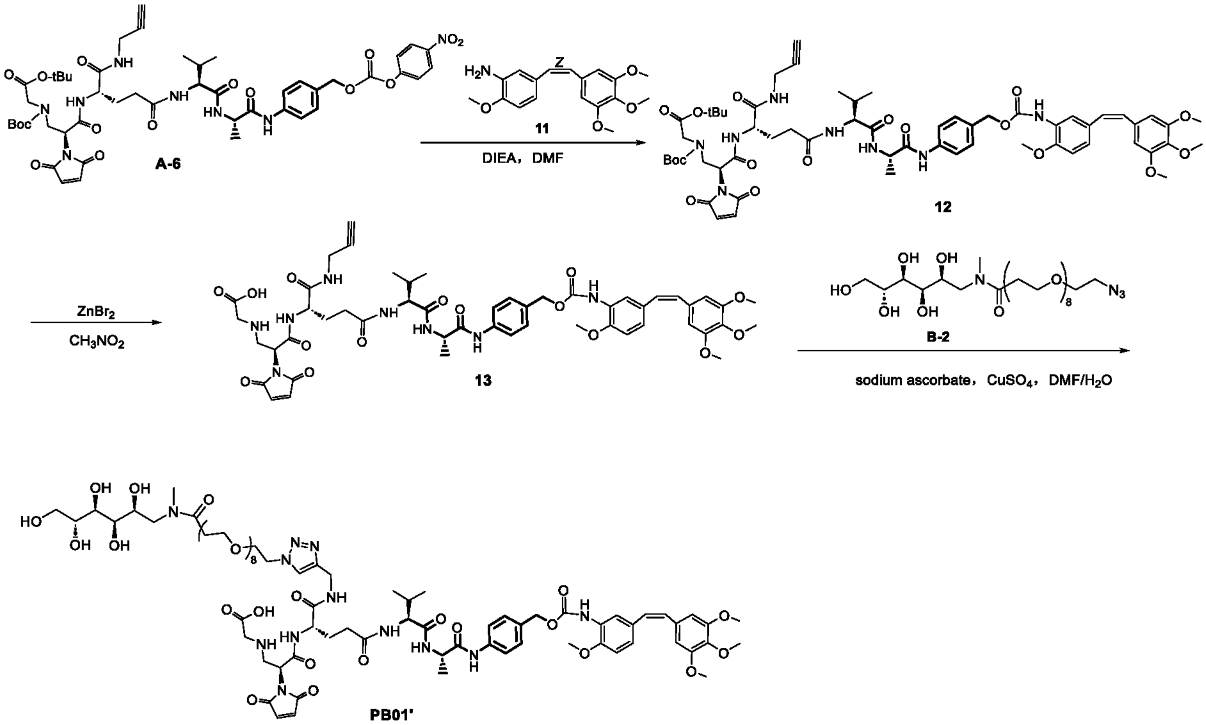

A-6 11 12 13 B-2 PB01'

**Step 1:** Synthesis of compound **12**

To a 25mL single-mouth bottle, compound **A-6** (100mg, 0.099mmol), compound **11** (purchased, 31.3mg, 0.099mmol) were added, dissolved with 5mL of DMF, then DIEA (32.8uL, 0.199mmol) was added, and reacted at room temperature for 2h, while the end point of the reaction being monitored by HPLC. After completion of the reaction, the reaction solution was purified by high-performance liquid preparative chromatography to obtain a product preparation solution, and then the preparation solution was lyophilized to obtain compound **12** (75.8mg), yield 64.5%, LCMS: $[M+H]^+$=1181.5.

**Step 2:** Synthesis of compound **13**

To a 25mL single-mouth bottle, compound **12** (75mg, 0.063mmol), zinc bromide (285.7mg, 1.27mmol) were added, dissolved with 10mL of $CH_3NO_2$, and reacted at room temperature for 2h, while the end point of the reaction being monitored by HPLC. After completion of the reaction, the solvent was removed by concentration under reduced pressure at 45°C using a water pump, to obtain a residue. The residue was purified by high-performance liquid preparative chromatography to obtain a product preparation solution, and then the preparation solution was lyophilized to obtain compound **13** (49.6mg), yield 76.3%, LCMS: $[M+H]^+$= 1025.4.

**Step 3:** Synthesis of compound **PB01'**

To a 25mL single-mouth bottle, compound **13** (25mg, 0.024mmol), compound **B-2** (15.8mg, 0.024mmol) were added, dissolved with 2mL of DMF and 1mL of water, then $CuSO_4 \cdot 5H_2O$ (6.7mg, 0.026mmol), sodium ascorbate (5.3mg, 0.026mmol) were added, and reacted at room temperature for 1h, while the end point of the reaction being monitored by HPLC. After completion of the reaction, the reaction solution was purified by high-performance liquid preparative chromatography to obtain a product preparation solution, and then the preparation solution was lyophilized to obtain compound **PB01'** (33.1mg), yield 82.5%, LC-MS: $[M+2H]^{2+}$ m/z =835.5.

**Example 17**

Synthesis of compound **PB02':**

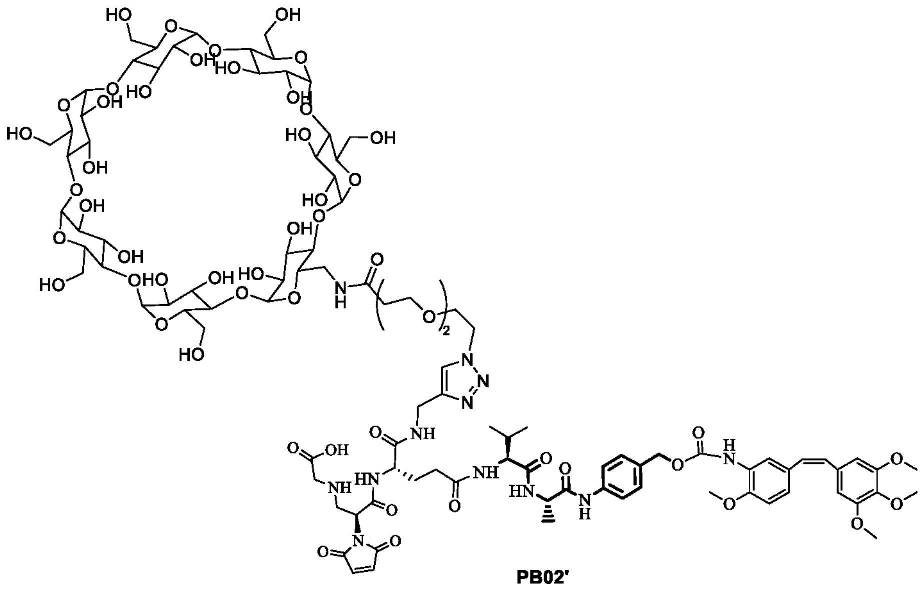

**PB02'**

With a reference to the synthetic route of Example 8, compound **PB02'** (43.6mg) was synthesized using compound 13 and compound **B-1,** yield 77.5%, LC-MS: $[M+2H]^{2+}$ m/z =1173.1.

**Example 18**

Synthesis of compound **CBI01':**

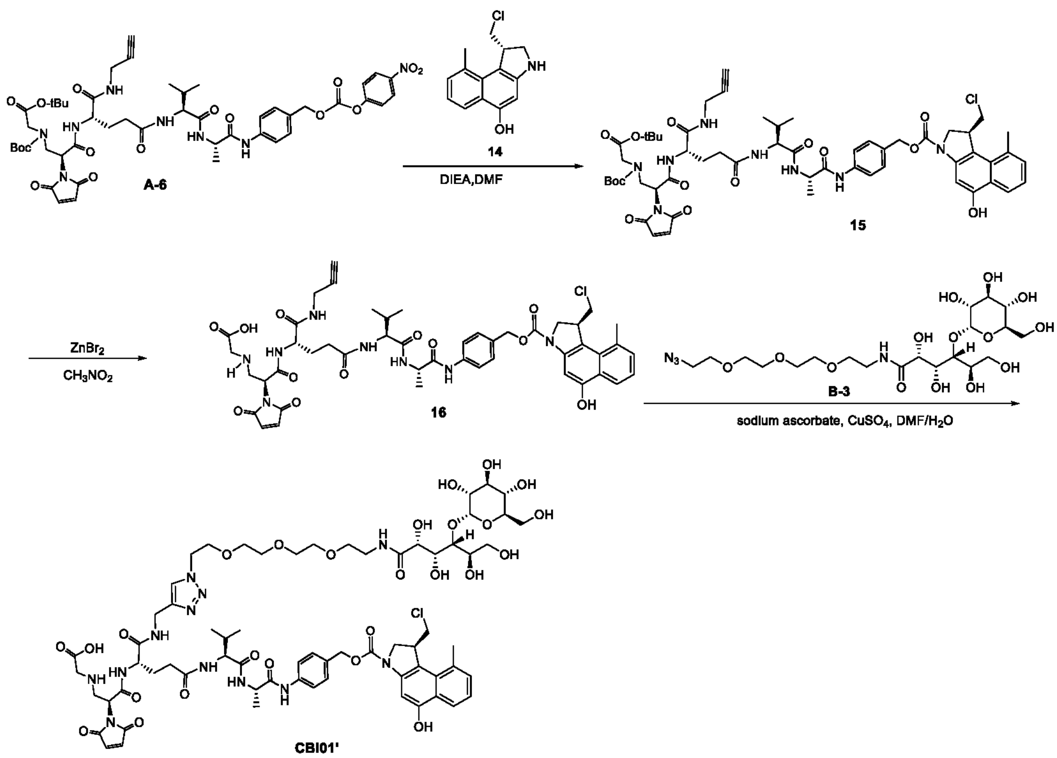


**Step 1:** Synthesis of compound **15**

To a 25mL single-mouth bottle, compound **A-6** (231.6mg, 0.23mmol), compound **14** (purchased, 50.0mg, 0.22mmol) were added, dissolved with 10 mL of DMF, then DIEA (42uL, 0.25mmol) was added, and reacted at room

temperature for 1h, while the end point of the reaction being monitored by HPLC. After completion of the reaction, the reaction solution was purified by high-performance liquid preparative chromatography to obtain a product preparation solution, and then the preparation solution was lyophilized to obtain compound **15** (190.9mg), yield 78%, LCMS: $[M+H]^+$=1113.5.

**Step 2:** Synthesis of compound **16**

To a 50mL single-mouth bottle, compound **15** (180.5mg, 0.163mmol), zinc bromide (740.8mg, 3.28mmol) were added, and dissolved with 10mL of $CH_3NO_2$, and reacted at room temperature for 0.5h, the solvent was removed by concentration under reduced pressure at 45°C using a water pump, to obtain a residue. The residue was purified by high-performance liquid preparative chromatography to obtain a product preparation solution, and then the preparation solution was lyophilized to obtain compound **16** (73.8mg), yield 48%, LCMS: $[M+H]^+$= 957.2.

**Step 3:** Synthesis of compound **CBI01'**

To a 25mL single-mouth bottle, compound **16** (30.5mg, 0.032mmol), compound **B-3** (18.0mg, 0.035mmol) were added, dissolved with 2mL of DMF and 0.5mL of water, then $CuSO_4 \cdot 5H_2O$ (8.0mg, 0.032mmol), sodium ascorbate (6.4mg, 0.032mmol) were added, and reacted at room temperature for 1h, while the end point of the reaction being monitored by HPLC. After completion of the reaction, the reaction solution was purified by high-performance liquid preparative chromatography to obtain a product preparation solution, and then the preparation solution was lyophilized to obtain compound **CBI01'** (29.5mg), yield 62.5%, LC-MS: $[M+H]^+$ m/z =1515.6.

**Example 19**

Synthesis of compound **CBI02'**

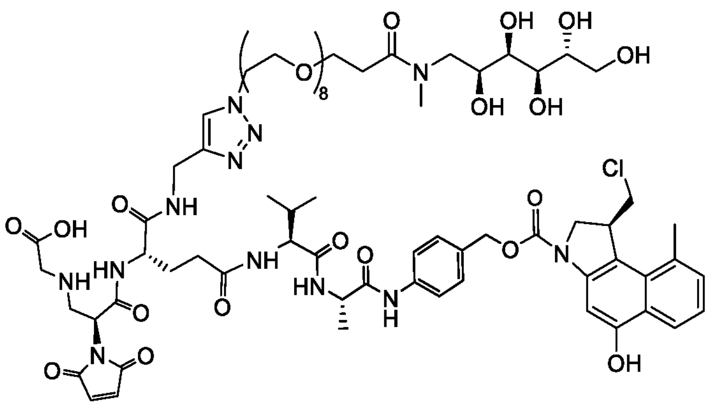

With a reference to the synthetic route of Example 9, compound **CBI02'** (17.6mg) was synthesized using compound **16** and compound **B-2;** LC-MS m/z$(M+2H)^{2+}$: 801.4.

**Example 20**

Synthesis of compound **CBI03'**

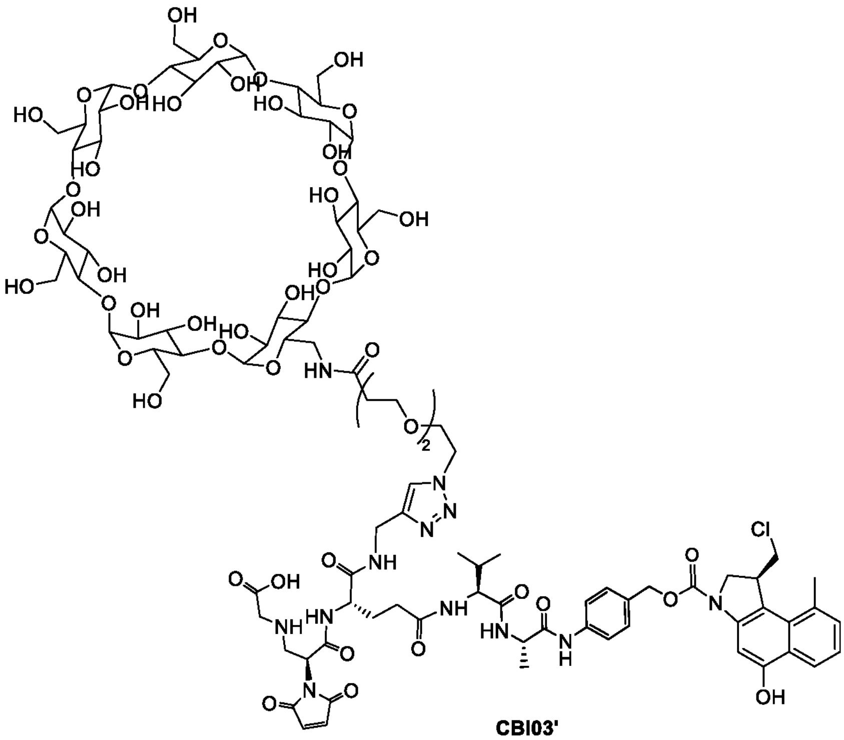

CBI03'

With a reference to the synthetic route of Example 8, compound **CBI03'** (12.5mg) was synthesized using compound **16** and compound **B-1;** LC-MS m/z$(M+2H)^{2+}$: 1138.9.

**Example 21**

Synthesis of compound **25:**

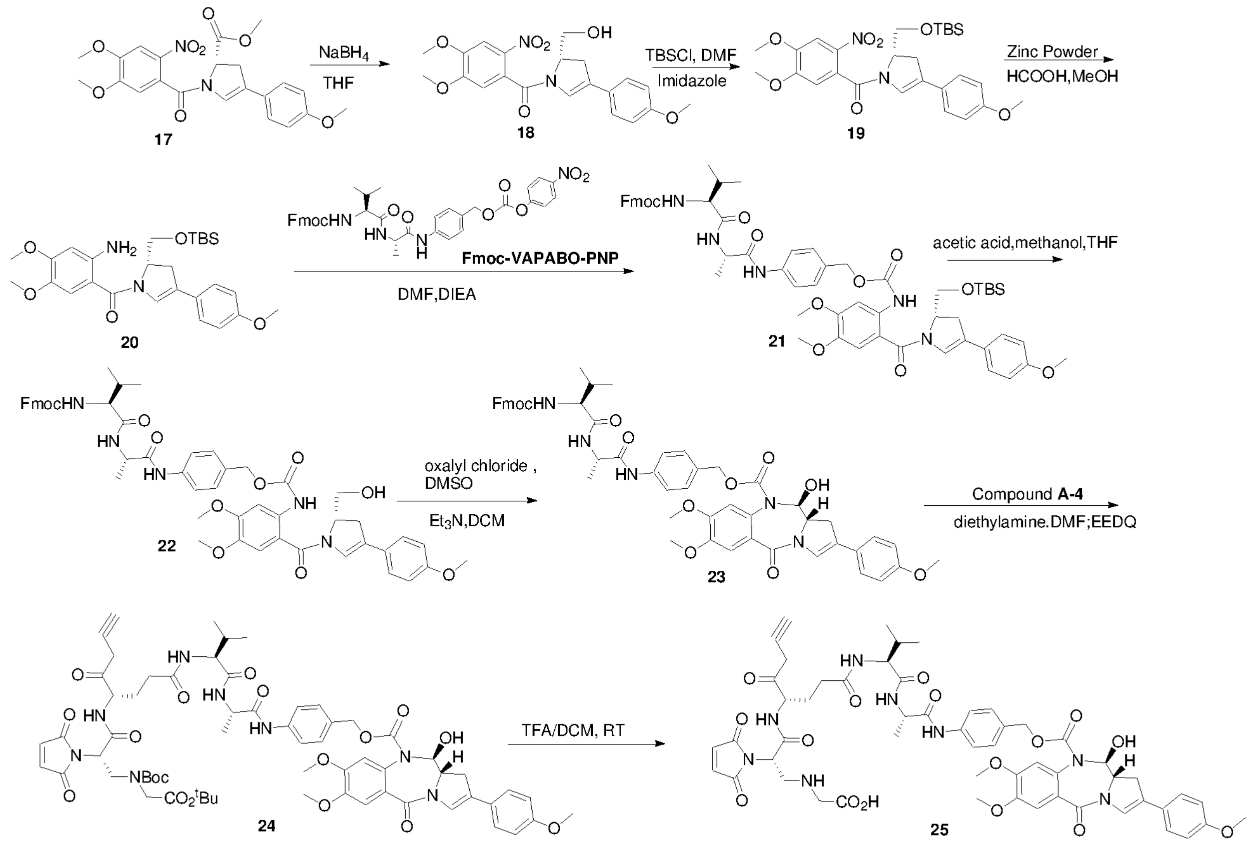


**Step 1:** Synthesis of compound **18**

With a reference to the synthesis of "compound **26**" in the patent CN102933236A, compound **18** was synthesized. To a 250mL three-mouth bottle, compound **17** (3 g, 6.7 mmol) was added, and dissolved with 50 mL of anhydrous THF. While maintaining the solution at 0°C under the protection of nitrogen in an ice-water bath, sodium borohydride solid (384 mg, 10.2 mmol) was added, reacted at 0°C for 30 min, and then reacted at room temperature for 2 h, while the end point of the reaction being monitored by TLC. After completion of the reaction, 50 mL of water was slowly added to the reaction solution for quenching the reaction in an ice-water bath, then 1N dilute HCl was added until no bubbles appeared, the reaction solution was extracted with ethyl acetate (60 mL*3), the organic phases were combined, washed with saturated NaCl solution twice, and dried over anhydrous sodium sulfate, followed by filtering, concentrating to obtain a crude product. The crude product was purified by column chromatography (DCM/MeOH=20: 1-5: 1), to obtain compound **18** (2.08g, yellow solid), yield 74.3%, LC-MS: $[M+H]^+$=415.1.

**Step 2:** Synthesis of compound **19**

To a 100mL single-mouth bottle, compound **18** (2 g, 4.8 mmol), TBSCl (1.32 g, 8.8 mmol), and imidazole (1.2 g, 17.6 mmol) were added, dissolved with 15 mL of anhydrous DMF, and reacted at room temperature for 3 h, while the end point of the reaction being monitored by TLC. After completion of the reaction, the reaction solution was poured into 30 mL of water, extracted with DCM (25 mL*3), the organic phases were combined, washed with water and brine, dried over anhydrous sodium sulfate and evaporated under reduced pressure, to obtain a crude product. The crude product was purified by column chromatography (PE/EA=5: 1-1: 2), to obtain compound **19** (2.28 g, yellow solid), yield 89.3%, LC-MS: $[M+H]^+$=529.2.

**Step 3:** Synthesis of compound **20**

To a 100 mL single-mouth bottle, compound **19** (2.2 g, 4.16 mmol) was added, dissolved with 30 mL of 5% formic acid/methanol solution, the solution was cooled to 5°C or lower in an ice-water bath, to which zinc powder (5.44 g, 83.2 mmol) was slowly added with stirring, and then the solution was allowed to react at room temperature for 1 h, while the end point of the reaction being monitored by TLC. After completion of the reaction, the reaction solution was filtered while it was hot, the filter cake was washed with a small amount of methanol, and the filtrate was adjusted to pH = 7 with a saturated

sodium bicarbonate solution, and then concentrated under reduced pressure at 45°C to remove the solvent, to obtain a brownish-yellow oily substance, to which 60 mL of DCM was added. The organic layer was separated, washed with saturated brine once, dried over anhydrous sodium sulfate, filtered, and then concentrated under reduced pressure at 45°C, to obtain a crude product. The crude product was purified by column chromatography (PE: EA=1: 1-1: 3), to obtain compound **20** (1.87 g, pale yellow solid), yield 90.1%, LC-MS: $[M+H]^+$=500.2.

**Step 4:** Synthesis of compound **21**

To a 50 mL single-mouth bottle, compound **20** (1.8 g, 3.6 mmol), Fmoc-VA-PABO-PNP (purchased, 2.45g, 3.6mmol) were added, dissolved with 15 mL of DMF to obtain a clear solution, then DIEA(893 uL, 5.4 mmol) was added, and reacted at room temperature for 2 h, while the end point of the reaction being monitored by HPLC. After completion of the reaction, the reaction solution was purified by high-performance liquid preparative chromatography to obtain a product preparation solution, and then the preparation solution was lyophilized, to obtain compound **21** (2.94 g, pale yellow solid), yield 78.3%, LC-MS: $[M+H]^+$=1040.5.

**Step 5:** Synthesis of compound **22**

To a 50 mL single-mouth bottle, compound **21** (2.9 g, 2.78 mmol) was added, dissolved with 8 mL of THF and 8 mL of water, then 20 mL of glacial acetic acid was added, and reacted at room temperature, while the end point of the reaction being monitored by HPLC. After completion of the reaction, the reaction solution was slowly added dropwise to 400 mL of saturated sodium bicarbonate solution, extracted with ethyl acetate (100 mL*3), the organic phases were combined, washed with water and brine, and dried over anhydrous sodium sulfate and evaporated under reduced pressure, to obtain a crude product. The crude product was purified by column chromatography (DCM/MeOH=10: 1-5: 1), to obtain compound **22** (1.94 g, pale yellow solid), yield 75%, LC-MS: $[M+H]^+$=926.3.

**Step 6:** Synthesis of compound **23**

To a 100mL three-mouth bottle, compound **22** (1.9 g, 2.0 mmol) was added, dissolved with 40 mL of anhydrous DCM, under the protection of nitrogen, Dess-Martin hypervalent iodine reagent (0.93 g, 2.2 mmol) was added, and reacted at room temperature for 4 h, while the end point of the reaction being monitored by TLC. After completion of the reaction, the reaction solution was filtered, the filtrate was washed with saturated sodium bicarbonate solution, water and saturated NaCl solution, dried over anhydrous sodium sulfate, and concentrated to obtain a crude product. The crude product was purified by high performance liquid chromatography, to obtain a product preparation solution, and then the preparation solution was lyophilized to obtain compound **23** (1.61 g, off-white solid), yield 85%, LC-MS: $[M+H]^+$=924.4.

**Step 7:** Synthesis of compound **24**

To a 50 mL single-mouth bottle, compound **23** (1.5 g, 1.62 mmol) was added, dissolved with 15 of mL DMF to obtain a clear solution, then 3 mL of diethylamine was added, and reacted at room temperature for 1 h, while the end point of the reaction being monitored by TLC. After completion of the reaction, the reaction solution was poured into 100 mL of methyl tert-butyl ether, to precipitate out a solid, and filtered, to obtain a deprotected product. The deprotected product was dissolved with 15 mL of DMF, then compound **A-4** (0.92 g, 1.62 mmol), EEDQ (0.48 g, 1.94 mmol) were added, and reacted at room temperature for 2 h, while the end point of the reaction being monitored by HPLC. After completion of the reaction, the reaction solution was purified by high-performance liquid preparative chromatography to obtain a product preparation solution, and then the preparation solution was lyophilized to obtain compound **24** (1.30 g, off-white solid), yield 64.2%, LC-MS: $[M+H]^+$=1248.5.

**Step 8:** Synthesis of compound **25**

To a 50 mL single-mouth bottle, compound **24** (500 mg, 0.4 mmol) was added, dissolved with 5 mL of DCM, then trifluoroacetic acid (5 mL) was added, and reacted at room temperature for 1 h, while the end point of the reaction being monitored by HPLC. After completion of the reaction, the solvent was removed by concentration under reduced pressure at 45°C, to obtain a residue. The residue was purified by high-performance liquid preparative chromatography to obtain a product preparation solution, and then the preparation solution was lyophilized to obtain compound **25** (294 mg), yield 67.4%, LC-MS: $[M+H]^+$=1092.4.

**Example 22**

Synthesis of compound **PBD01'**

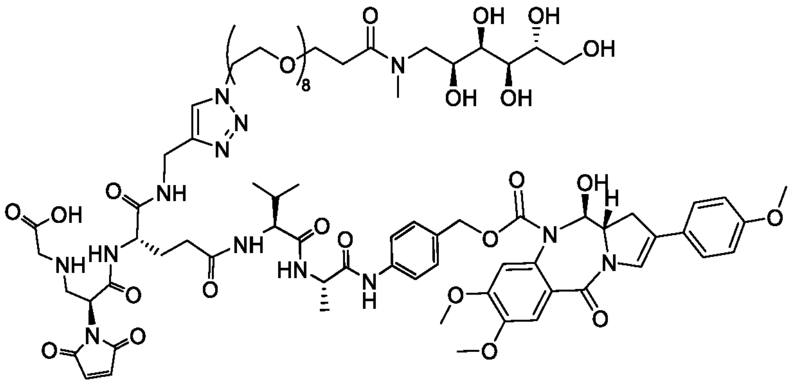

With a reference to the synthetic route of Example 9, compound **PBD01'** (45.2mg) was synthesized using compound **25** and compound **B-2;** LC-MS m/z(M+2H)$^{2+}$: 869.0.

**Example 23**

Synthesis of compound **PBD02'**

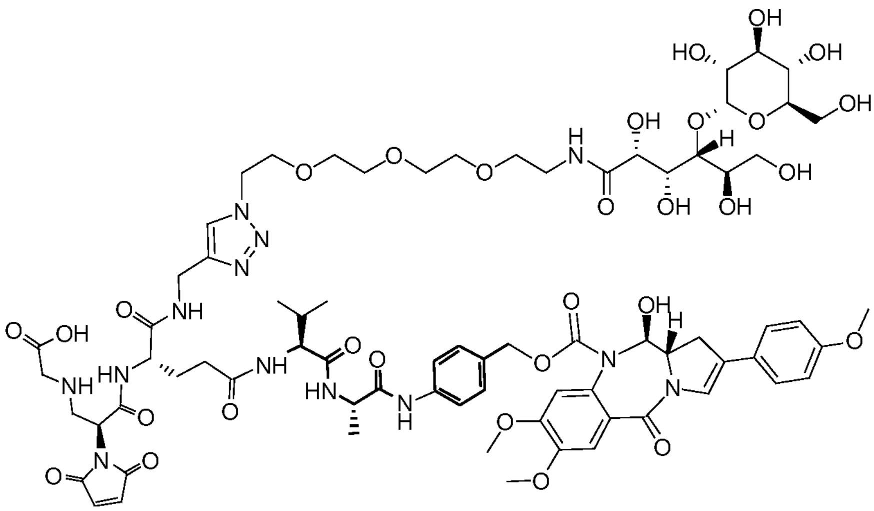

With a reference to the synthetic route of Example 18, compound **PBD02'** (31.6mg) was synthesized using compound **25** and compound **B-3;** LC-MS m/z(M+2H)$^{2+}$: 825.8.

**Example 24**

Synthesis of compound **27:**

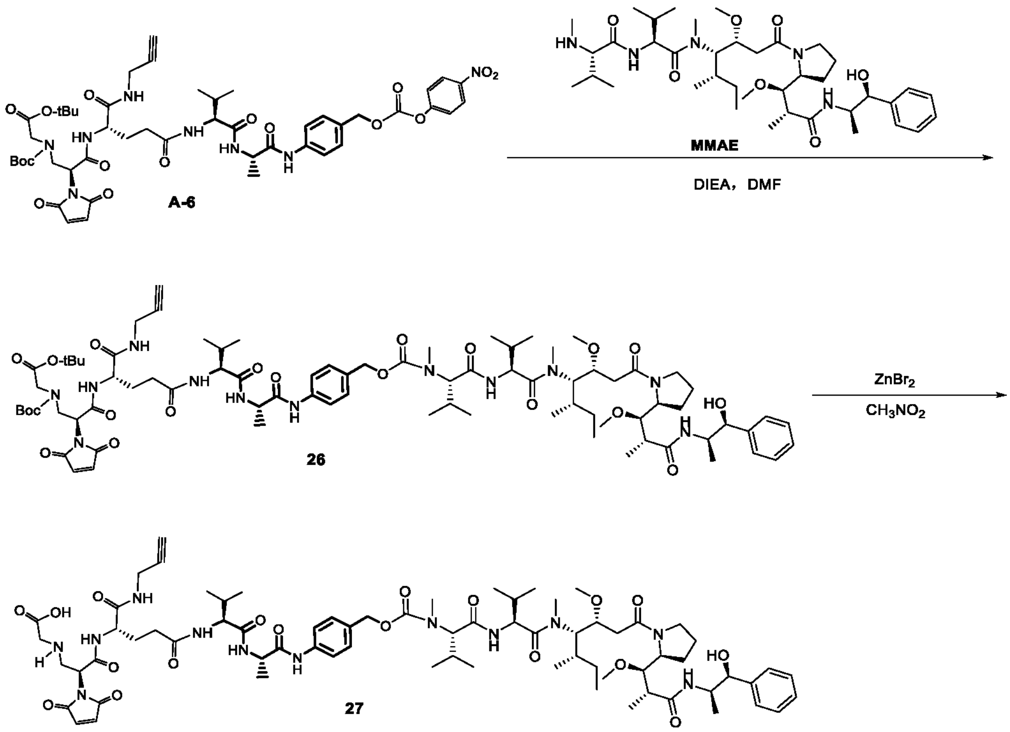


**Step 1:** Synthesis of compound **26**

To a 50mL single-mouth bottle, compound **A-6** (2.0012g, 1.99mmol), compound **MMAE** (purchased, 1.36g, 1.90mmol) were added, dissolved with 20 mL of DMF, then DIEA (0.48mL, 2.80mmol) was added, and reacted at room temperature for 2h, while the end point of the reaction being monitored by HPLC. After completion of the reaction, the reaction solution was purified by high-performance liquid preparative chromatography to obtain a product preparation solution, and then the preparation solution was lyophilized to obtain compound **26** (2.21g), yield 73.5%, LCMS: $[M+H]^+$=1584.0.

**Step 2:** Synthesis of compound **27**

To a 100mL single-mouth bottle, compound **26** (2.0 g, 1.26mmol), zinc bromide (5.69g, 25.25mmol) were added, dissolved with 40mL of $CH_3NO_2$, and reacted at 40°C in oil bath for 0.5h, the solvent was removed by concentration under reduced pressure at 45°C using a water pump, to obtain a residue. The residue was purified by high-performance liquid preparative chromatography to obtain a product preparation solution, and then the preparation solution was lyophilized to obtain compound **27** (773.5mg), yield 43%, LCMS: $[M+H]^+$=1427.8.

**Example 25**

Synthesis of compound **M01'**:

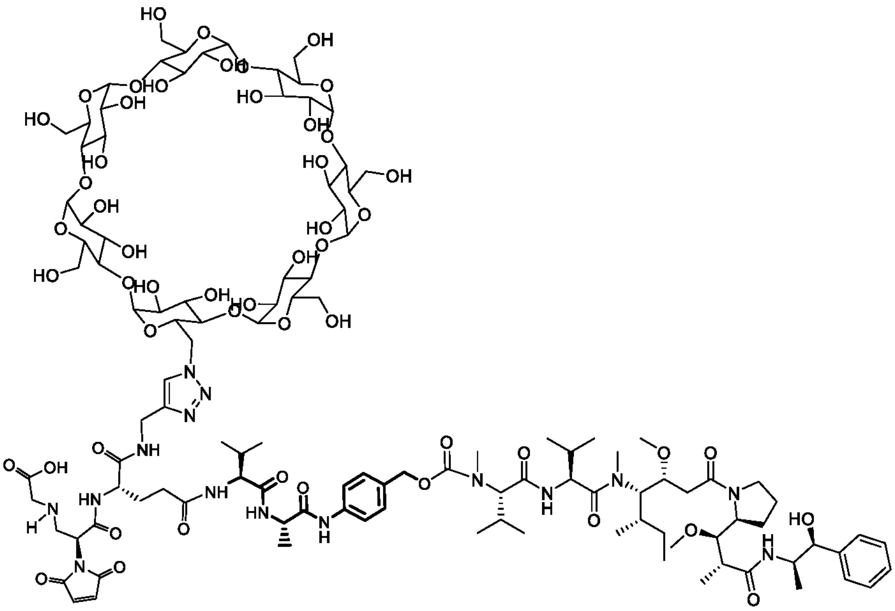

With a reference to the synthetic route of Example 13, compound **M01'** (85.5mg) was synthesized using compound **27** and azido-β-cyclodextrin as raw materials; LC-MS m/z(M+2H)$^{2+}$: 1294.5.

**Example 26**

Synthesis of compound **M02'**:

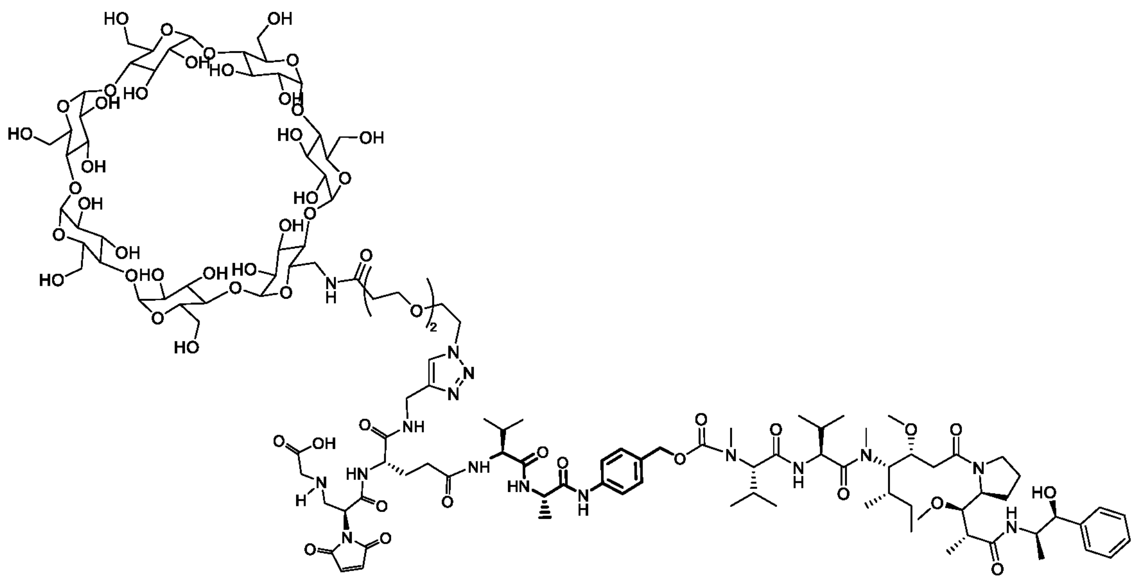

With a reference to the synthetic route of Example 8, compound **M02'** (293.2mg) was synthesized using compound **27** and compound **B-1;** LC-MS m/z(M+2H)$^{2+}$: 1374.0.

**Example 27**

Synthesis of compound **M03'**:

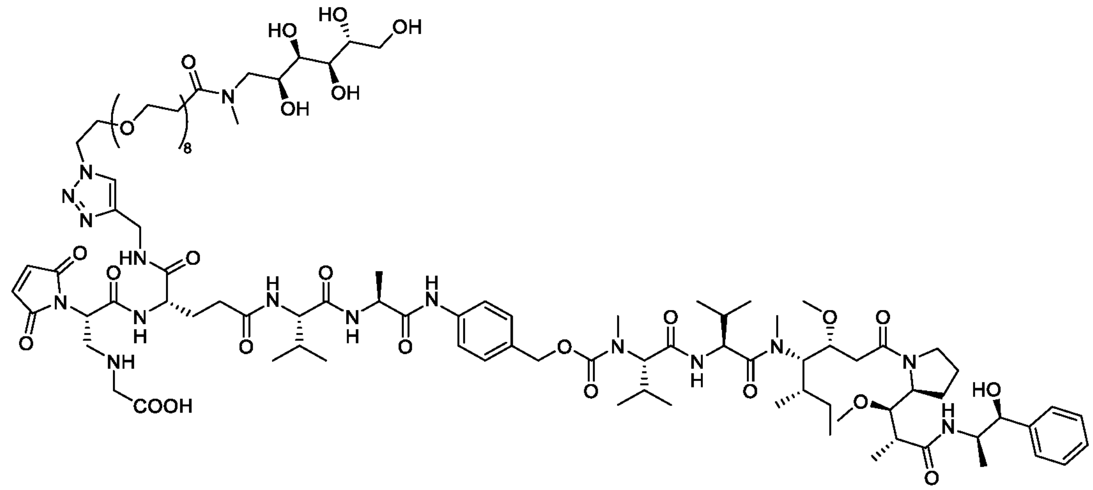

With a reference to the synthetic route of Example 9, compound **M03'** (98.2mg) was synthesized using compound **27** and compound **B-2;** LC-MS m/z(M+2H)$^{2+}$: 1037.1.

## Example 28

**Sequences of the antibodies used:**

the sequence of anti-CD33 antibody ANTI-CD33

>light chain-LC

DIQLTQSPSTLSASVGDRVTITCRASESLDNYGIRFLTWFQQKPGKAPKLLMYAASNQGS GVPSRFSGSGSGTEFTLTISSLQPDDFATYYCQQTKEVPWSFGQGTKVEVKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVCTKSFNRGEC

>heavy chain-HC

EVQLVQSGAEVKKPGSSVKVSCKASGYTITDSNIHWVRQAPGQSLEWIGYIYPYNGGTD YNQKFKNRATLTVDNPTNTAYMELSSLRSEDTAFYYCVNGNPWLAYWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSR DELTKNQVSLTCLVKGFYPSDIAVEWESNGQPENNYKTTPPVLDSDGSFFLYSKLTVDK SRWQQGNVFSCSVMHEALHNHYTQKSLSLSPG

the sequence of anti-Trop2 antibody ANTI-Trop2-1

>light chain-LC

DIQMTQSPSSLSASVGDRVTITCRASQDINKYLAWYQQKPGKVPKLLIYSTSTLQSGVPS RFSGSGSGTDFTLTISSLQPEDVATYYCLQYDDLFTFGQGTKLEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGEC

>heavy chain-HC

QVQLVQSGAEVKKPGASVKLSCKASGYTFTSFDINWVRQAPEQRLEWMGWIFPGDGNT
KYSQKFQGRATITRDTSASTAYMELSSLRSEDTAVYYCVRGEALYYFDYWGQGTLVTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYSKLTV
DKSRWQQGNVFSCSVMHEALHNHYTQKSLSLSPG

the sequence of anti-Trop2 antibody ANTI-Trop2-2

>light chain-LC

DIQMTQSPSSLSASVGDRVTITCRASQDINKYLAWYQQKPGKVPKLLIYSTSTLQSGVPS
RFSGSGSGTDFTLTISSLQPEDVATYYCLQYDDLFTFGQGTKLEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVCTKSFNRGEC

>heavy chain-HC

QVQLVQSGAEVKKPGASVKLSCKASGYTFTSFDINWVRQAPEQRLEWMGWIFPGDGNT
KYSQKFQGRATITRDTSASTAYMELSSLRSEDTAVYYCVRGEALYYFDYWGQGTLVTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYSKLTV
DKSRWQQGNVFSCSVMHEALHNHYTQKSLSLSPG

the sequence of anti-HER2 antibody Trastuzumab (Tras)

>light chain-LC

DIQMTQSPSSLSASVGDRVTITCRASQDVNTAVAWYQQKPGKAPKLLIYSASFLYSGVP
SRFSGSRSGTDFTLTISSLQPEDFATYYCQQHYTTPPTFGQGTKVEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC

>heavy chain-HC

EVQLVESGGGLVQPGGSLRLSCAASGFNIKDTYIHWVRQAPGKGLEWVARIYPTNGYT
RYADSVKGRFTISADTSKNTAYLQMNSLRAEDTAVYYCSRWGGDGFYAMDYWGQGT
LVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPG

the sequence of anti-HER2 antibody Tras-016

>light chain-LC

DIQMTQSPSSLSASVGDRVTITCRASQDVNTAVAWYQQKPGKAPKLLIYSASFLYSGVP
SRFSGSRSGTDFTLTISSLQPEDFATYYCQQHYTTPPTFGQGTKVEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVCTKSFNRGEC

>heavy chain-HC

EVQLVESGGGLVQPGGSLRLSCAASGFNIKDTYIHWVRQAPGKGLEWVARIYPTNGYT
RYADSVKGRFTISADTSKNTAYLQMNSLRAEDTAVYYCSRWGGDGFYAMDYWGQGT
LVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPG

**Example 29**

Preparation of mono-drug ADC (DAR = 8) (general method)

Antibody-drug conjugate sample was prepared by conjugating antibody A with L1-D1 or L2-D2:
After being expressed in cells and purified by Protein A affinity chromatography and molecular sieve chromatography, antibody A was exchanged into a buffer solution of 20 mM NaAc-HAc, pH6.0, and concentrated or diluted to a protein concentration of 5 mg/mL. L1-D1 or L2-D2 was dissolved in N,N-dimethylacetamide (DMA) to a concentration of 10 mg/mL for later use.

To break the interchain disulfide bond of antibody A, tris(2-carboxyethyl) phosphine (TCEP) was first added at a molar ratio of 15 to 25 times, and reacted at room temperature for 2 h. Then, L1-D1 or L2-D2 solution was added at a molar ratio of 15 to 25 times, and reacted at room temperature for 2 h. After completion of the reaction, a 30 KDa ultrafiltration spin column was used for buffer exchange, to remove unconjugated L1-D1 or L2-D2, thus obtaining an antibody A-D1 (DAR = 8) or antibody A-D2 (8) sample.

**Example 30**

Preparation of mono-drug ADC (DAR=10) and dual-drug ADC (DAR=2+8) (general method)

Antibody-drug conjugate sample B-D2 (2) -D1 (8), antibody B-D1 (2) -D2 (8), antibody B-D1 (10) or antibody B-D2 (10) was prepared by conjugating antibody B with L1-D1, L2-D2:
After being expressed in cells and purified by Protein A affinity chromatography and molecular sieve chromatography, antibody B was exchanged into a buffer solution of 20 mM NaAc-HAc, pH6.0, and concentrated or diluted to a protein concentration of 5 mg/mL. L1-D1 or L2-D2 was dissolved in DMA to a concentration of 10 mg/mL for later use.

TCEP was first added at a molar ratio of 15 to 25 times, and reacted at room temperature for 2-4 h, so as to reduce the interchain disulfide bond of antibody B or the cysteine side chain introduced on the antibody into thio group. After completion of the reaction, a 30 KDa ultrafiltration spin column was used for buffer exchange, to remove impurities in the reaction solution, thus obtaining an antibody B reduction sample.

Dehydroascorbic acid (dHA) solution was added at a molar ratio of 15 to 25 times to the antibody B reduction sample solution, and reacted at room temperature for 2-4 h so as to oxidize the reduced thio group on the antibody into an interchain disulfide bond. Then, L2-D2 or L1-D1 solution was added at a molar ratio of 5 to 15 times to react with the disulfide bond introduced on the antibody at room temperature for 2 h. After completion of the reaction, a 30 KDa ultrafiltration spin column was used for buffer exchange, to remove unconjugated L2-D2 or L1-D1, thus obtaining an antibody B-D2 (2) or antibody B-D1 (2) conjugate sample.

TCEP was first added at a molar ratio of 15 to 25 times to the antibody B-D2 (2) or the antibody B-D1 (2), and reacted at room temperature for 2 h, so as to break the interchain disulfide bond of the antibody on the antibody B-D2 (2) or the antibody B-D1 (2). Then, L1-D1 or L2-D2 solution was added at a molar ratio of 15 to 25 times to react with the interchain disulfide bond of the antibody at room temperature for 2 h. After completion of the reaction, a 30 KDa ultrafiltration spin column was used for buffer exchange, to remove unreacted L1-D1 or L2-D2, thus obtaining an antibody B-D2 (2) -D1 (8), antibody B-D1 (2) -D2 (8), antibody B-D1 (10) or antibody B-D2 (10) conjugate sample.

With a reference to the above method, the following ADCs were synthesized using antibodies ANTI-CD33, ANTI-Trop2-1, ANTI-Trop2-2, Tras, Tras-016, respectively:

| Sample name | DAR |
|---|---|
| ANTI-CD33-Ex01 | 10 |
| ANTI-CD33-R01-1-Ex01 | 2 (R01-1) +8 (Ex01) |
| ANTI-CD33-Ex01-R01-1 | 2 (Ex01) +8 (R01-1) |
| ANTI-Trop2-1-Ex01 | 8 |
| ANTI-Trop2-2-Ex01 | 10 |
| ANTI-Trop2-2-R01-1-Ex01 | 2 (R01-1) +8 (Ex01) |
| ANTI-Trop2-2-R02-Ex01 | 2 (R02) +8 (Ex01) |
| Tras-Ex01 | 8 |
| Tras-016-Ex01 | 10 |
| Tras-016-R01-1-Ex01 | 2 (R01-1) +8 (Ex01) |
| Tras-016-R02-Ex01 | 2 (R02) +8 (Ex01) |

**Test example 1:** Dual-drug ADC cell killing experiments using CD33, Trop2, HER2 as targets

1. Equipment, consumables and reagents

Reagents

| Category | Name | Manufacturer | Cargo No. |
|---|---|---|---|
| MTS test | CellTiter 96® One Solution Reagen | Promega | G3581 |
| | 0.25%Trypsin-EDTA (1×) | Gibco | 25200 |
| | 0.4% Trypan Blue | Gibco | 15250 |

Equipment

| Name of instrument | Model |
|---|---|
| Cell centrifuge | EQ4044 |
| HWS-24 electric heating constant temperature water bath | EQ4032 |
| Airstream class I A2 type biosafety cabinet | EQ4007/EQ4008 |
| $CO_2$ incubator | EQ4025 |
| Inverted microscope | EQ4055 |
| Count Star automatic cell counter | EQ4027 |
| Siemens refrigerator | EQ4020 |
| Cell incubator | INS-Q-070 |
| Microplate Reader | EQ4092 |

Cells

| Type | Cell line |
|---|---|
| Human acute promyelocytic leukemia cell | HL-60 |
| Human histiocytic lymphoma | U937 |
| Human acute megakaryocytic leukemia cell | CMK |
| Human erythroleukemia cell | HEL92.1.7 |
| Human skin squamous carcinoma cell | A431 |
| Human gastric cancer cell | N87 |
| Human colorectal adenocarcinoma cell | SW620 |
| Human lung adenocarcinoma cell | H1975 |
| Human breast cancer cell | MDA-MB-453 |
| Human breast adenocarcinoma cell | SK-BR-3 |
| Human breast cancer cell | MDA-MB-468 |

2. Sample information

| Sample name | DAR | Preservation conditions | Description of samples |
|---|---|---|---|
| ANTI-CD33 mAb | / | 4°C | Naked anti-CD33 antibody |
| ANTI-CD33-Ex01 | 10 | 4°C | Mono-antibody conjugated mono-drug ADC using CD33 as target |
| ANTI-CD33-R01-1-Ex01 | 2 (R01-1) +8 (Ex01) | 4°C | Mono-antibody conjugated dual-drug ADC using CD33 as target |
| ANTI-Trop2-1 mAb | / | 4°C | Naked anti-Trop2 antibody |
| ANTI-Trop2-2 mAb | / | 4°C | Naked anti-Trop2 antibody |
| ANTI-Trop2-1-Ex01 | 8 | 4°C | Mono-antibody conjugated mono-drug ADC using Trop2 as target |
| ANTI-Trop2-2-Ex01 | 10 | 4°C | Mono-antibody conjugated mono-drug ADC using Trop2 as target |
| ANTI-Trop2-2-R01-1-Ex01 | 2 (R01-1) +8 (Ex01) | 4°C | Mono-antibody conjugated dual-drug ADC using Trop2 as target |
| ANTI-Trop2-2-R02-Ex01 | 2 (R02) +8 (Ex01) | 4°C | Mono-antibody conjugated dual-drug ADC using Trop2 as target |
| Trastuzumab | / | 4°C | Naked anti-HER2 antibody |
| Tras-Ex01 | 8 | 4°C | Mono-antibody conjugated mono-drug ADC using HER2 as target |
| Tras-016-Ex01 | 10 | 4°C | Mono-antibody conjugated mono-drug ADC using HER2 as target |
| Tras-016-R01-1-Ex01 | 2 (R01-1) +8 (Ex01) | 4°C | Mono-antibody conjugated dual-drug ADC using HER2 as target |

(continued)

| | | | |
|---|---|---|---|
| Tras-016-R02-Ex01 | 2 (R02) +8 (Ex01) | 4°C | Mono-antibody conjugated dual-drug ADC using HER2 as target |

3. Principle and method

3.1 Principle of cell killing experiment by MTS method

Pharmacodynamic test at cellular level: cell plating was performed on a 96-well plate in a certain density. After the cells adhered to the wall (usually taking at least 4 h), a series of drugs in several folds of dilution were added to each well plate to act for a certain period of time, for killing the cells. At the end of action of the drugs, a certain amount of MTS was added to react at 37°C under 5% $CO_2$ for 1-4 h. After color development using the MTS reagent, the OD 490nm absorbance value was detected using a microplate reader, and then the $IC_{50}$ for cell killing was analyzed and calculated.

Therein MTS [3-(4,5-diethyl-thiazol-2-yl)-5-(3-carboxymethoxyphenyl)-2-(4-sulfophenyl) - 2H-tetrazolium, inner salt] is a new generation of blue tetrazolium compound, and the Formazan product formed by reduction of it has higher water solubility and stability, and faster color development. It is based on the same application principle as MTT, i.e., it is reduced by several dehydrogenases in mitochondria of living cell into various colored formazan products, the depth of the color being highly correlated with the number of living cells of some sensitive cell strains in a certain range.

3.2. Method

3.2.1. Sample preparation

Before addition, according to the concentration of each test sample, a drug (5× mother liquor concentration) was prepared using a preheated 1% FBS culture medium according to the initial concentration of 5.0 μM, and then diluted according to 10 folds of gradient concentration, totaling 8 detection points, followed by standing for later use. During the preparation, each concentration was uniformly mixed using an oscillator, specifically, oscillating for 2-3 sec at a time, with an interval of 1sec, and then oscillating again, repeating 2-3 times.

The final concentration of the antibody and the antibody conjugated drug was 0 (Vehicle), 0.6pM, 4.25pM, 29.75pM, 0.21nM, 1.46nM, 10.20nM, 71.43nM, 500 nM.

3.2.2. Experimental

1) Cell revival: after preheating in a 37°C water bath, 9 mL of the culture medium was taken and put in a centrifuge tube for later use; the cells were taken out from liquid nitrogen, immediately quickly shaked at 37°C to fully thaw, then transferred into the centrifuge tube, and centrifuged at 1000 rpm for 3 min at room temperature; the supernatant was discarded, and the precipitate was re-suspended with 1 mL of complete culture medium, where the process should be gentle, to prevent bubbles from being blown out, thereby reducing damage to the cells; the cells were transferred into a culture flask, and cultivated in a cell incubator at 37°C, 5% $CO_2$ and 95% relative humidity. After 24 h of revival, the cell status was observed, and the culture medium was replaced with fresh one in time for subculture and cell counting.
2) Cell plating: after all cell lines used for *in vitro* pharmacodynamic test were revived, each of them needed to undergo 3 generations of subculture. According to the verified cell plating density, the cell suspension was diluted using the cell culture medium containing 1% FBS, and gently added to a 96-well plate at 80 μL/well, finally, 200 μL of sterile PBS or sterile dd$H_2O$ was added to the 96-well plate without the cell suspension at the edge for edge sealing, and then the plate was placed in a $CO_2$ incubator for culture overnight.
3) Drugs were added to the well plate (e.g.: B2 to B10, C2 to C10, D2 to D10, E2 to E10, F2 to F10, or G2 to G10) at 5 times the expected concentration sequentially at 20 μL/well according to the principle of three replicates per concentration for each molecule to be tested, the initial well drug concentration was 1000nM, and two test drugs could be added to each 96-well plate. Typically, the three wells B11, C11, D11 containing cells, replenished only with 20 μL of 1% FBS culture medium, were used as negative controls. E11, F11, G11 were blank controls containing only 100 μL of 1% FBS culture medium without cells.
4) Typically, the treatment time with ADC drug was 5 days, during which the action of the drug was observed with an inverted microscope at 24h, 48h and 72h after addition.
5) CellTiter 96® One Solution Reagen MTS reagent was taken from -20°C environment, thawed at room temperature in the dark, thoroughly vortexed and mixed. Then, in a biosafety cabinet, 20 μL of the CellTiter 96® One Solution

Reagen MTS reagent was added per 100 μL cell culture volume along the side wall of the well. The MTS reagent was added in equal proportions to different cell culture volumes. The MTS solution was uniformly mixed by gently tapping the plate surface, and then placed in a cell incubator for incubation in the dark for 2-4 h.
6) After completion of the reaction, the 96-well plate was taken out, and the OD 490nm absorbance value was detected using a microplate reader, while performing data recording, sorting and storing.
7) The data were processed and analyzed according to the following formula to calculate the cell viability at each different drug concentration.

$$\text{Cell viability} = \frac{\text{OD 490nm (drug)} - \text{OD 490nm (blank control)}}{\text{OD 490nm (negative control)} - \text{OD 490nm (blank control)}} \text{ x } 100\%$$

OD 490nm (drug): OD 490nm value after treatment of cells with small molecular drug;
OD 490nm (negative control): OD 490nm value after treatment of cells with complete culture medium containing 0.1% DMSO;
OD 490nm (blank control): OD 490nm value of complete culture medium containing 0.1% DMSO.

8) A processing curve was fitted by adopting a Graphpad Prism 9.0 data processing software four-parameter logic fitting module to obtain $IC_{50}$.

4. Results and analysis

4.1 CD33 cell killing experiment:

Taking a CD33 high-expression acute myelogenous leukemia cell line HEL92.1.7, CD33 moderate-expression acute myelogenous leukemia cell lines CMK, U937 and HL-60, and a skin squamous carcinoma cell line A431 which did not express CD33 as target cells respectively, the CD33 expression level of the target cell was firstly determined by flow analysis, and then the cell killing activity of the CD33 dual-drug ADC was evaluated by MTS method. The results are shown in Figs. 1-10 as well as Table 1 and Table 2.

Table 1 CD33 expression in test cells

| Name of cell strain | CD33 Ratio | CD33 Status |
|---|---|---|
| HEL92.1.7 | 20.88 | High expression |
| CMK | 15.65 | Moderate expression |
| U937 | 8.79 | |
| HL-60 | 8.42 | |
| A431 | 0.98 | Negative |

Table 2 $IC_{50}$ of test drugs at cellular level

| Test drug $IC_{50}$ (nM) | | | |
|---|---|---|---|
| Cell | ANTI-CD33 mAb | ANTI-CD33-Ex01 (10) | ANTI-CD33-R01-1 (2) - Ex01 (8) |
| HEL92.1.7 | >500 | 10.089 | 2.136 |
| CMK | >500 | 11.462 | 2.525 |
| U937 | >500 | 31.642 | 6.627 |
| HL-60 | >500 | 5.387 | 2.22 |

(continued)

| Test drug $IC_{50}$ (nM) | | | |
|---|---|---|---|
| Cell | ANTI-CD33 mAb | ANTI-CD33-Ex01 (10) | ANTI-CD33-R01-1 (2) - Ex01 (8) |
| A431 | >500 | 87.217 | 14.671 |
| Conclusion:<br>Under the experimental conditions, in the killing experiments of 4 AML cell strains and 1 epithelial squamous carcinoma cell models,<br>1. Naked antibody of CD33 ANTI-CD33-mAb had no significant killing activity against the multiple AML cell strains;<br>2. In the CD33 high-expression, moderate-expression, and CD33 negative cell lines, the dual-drug ADC obtained by simultaneously coupling a low toxicity repair inhibitor and a high toxicity damage agent to anti-CD33 antibody, namely ANTI-CD33-R01-1 (2) - Ex01 (8), showed significantly better *in vitro* sensitivity to the multiple AML tumor cell strains than the mono-drug ADC obtained by coupling only a high toxicity damage agent, namely ANTI-CD33-Ex01 (10). | | | |

4.2 Trop2 cell killing experiment:

Taking a Trop2 high-expression cell line N87 and a heterogeneous tumor cancer cell A431+ SW620 as target cells respectively, the Trop2 expression level of the target cell was firstly determined by flow analysis, and then the cell killing activity of the Trop2 dual-drug ADC was evaluated by MTS method. The results are shown in Figs. 11-14 as well as Table 3 and Table 4.

Table 3 Trop2 expression in test cells

| Name of cell strain | Trop2 Ratio | Trop2 Status |
|---|---|---|
| N87 | 298.50 | High expression |
| A431+SW620 | 0.92 | Negative |

Table 4 $IC_{50}$ of test drugs at cellular level

| Cell | Test drug $IC_{50}$(nM) | | | | | |
|---|---|---|---|---|---|---|
| | ANTI-Trop2-1 mAb | ANTI-Trop2-2 mAb | ANTI-Trop2-1-Ex01(8) | ANTI-Trop2-2-Ex01(10) | ANTI-Trop2-2-R01-1(2)-Ex01(8) | ANTI-Trop2-2-R02(2)-Ex01(8) |
| N87 | >500 | >500 | 336.49 | 64.10 | 24.99 | 26.55 |
| A431+SW620 | >500 | >500 | >500 | 635.30 | 185.36 | 161.17 |
| Conclusion:<br>Under the experimental conditions, in the killing experiments of 2 cell models,<br>1. Naked antibodies of Trop2 ANTI-Trop2-1-mAb and ANTI-Trop2-2-mAb had no significant killing activity against the two cell lines;<br>2. In the Trop2 high-expression cell line and negative cell line, the dual-drug ADCs obtained by coupling a low toxicity and low activity repair inhibitor to anti-Trop2 antibody at the same time of coupling a high killing activity damage agent, namely ANTI-Trop2-2-R01-1(2)-Ex01(8) and ANTI-Trop2-2-R02(2)-Ex01(8), showed significantly better *in vitro* sensitivity to the tumor cells than the mono-drug ADC obtained by coupling only a high killing activity damage agent, namely ANTI-Trop2-1-Ex01(8), and significantly better *in vitro* sensitivity than the mono-drug ADC ANTI-Trop2-2-Ex01(10). | | | | | | |

4.3 HER2 cell killing experiment:

Taking cell lines SK-BR-3, H1975, MDA-MB-453, and MDA-MB-468 with varying degrees of HER2 expression as target cells respectively, the HER2 expression level of the target cell was firstly determined by flow analysis, and then the cell killing activity of the HER2 dual-drug ADC was evaluated by MTS method. The results are shown in Figs. 15-18 as well as Table 5 and Table 6.

Table 5 HER2 expression in test cells

| Name of cell strain | Number of HER2 antigens (HER2/Cell) | HER2 Status |
|---|---|---|
| SK-BR-3 | 822, 659 | High expression |
| MDA-MB-453 | 158, 428 | Moderate expression |
| H1975 | 6, 697 | Low expression |
| MDA-MB-468 | Undetectable | Negative |

Table 6 $IC_{50}$ of test drugs at cellular level

| | IC50 (nM) | | | |
|---|---|---|---|---|
| Cell | Tras-Ex01 (DAR8) | Tras-016-Ex01 (DAR10) | Tras-016-R01-1(2)-Ex01(8) | Tras-016-R02(2)-Ex01(8) |
| SK-BR-3 | >500 | >500 | 343.44 | 178.42 |
| MDA-MB-453 | >500 | >500 | 119.76 | 123.88 |
| H1975 | 365.85 | 162.92 | 36.46 | 21.72 |
| MDA-MB-468 | 99.20 | 22.06 | 5.11 | 3.92 |
| Conclusion:<br>Under the experimental conditions, in the killing experiments of 4 cell models,<br>1. The mono-drug ADCs, namely Tras-Ex01 (DAR8) and Tras-016-Ex01 (DAR10), obtained by coupling a high killing activity damage agent to anti-HER2 antibody, showed significant differences in their killing activity against the tumor cells, and the activity was significantly higher when the DAR is 10 compared to when it is 8;<br>2. In the cell lines with different HER2 expression, the dual-drug ADCs obtained by coupling a low toxicity and low activity repair inhibitor to anti-HER2 antibody at the same time of coupling a high killing activity damage agent, namely Tras-016-R01-1(2)-Ex01(8) and Tras-016-R02(2)-Ex01(8), showed significantly better *in vitro* sensitivity to the tumor cells than the mono-drug ADC obtained by coupling only a high killing activity damage agent, namely Tras-Ex01 (DAR8), and significantly better activity than the mono-drug ADC Tras-016-Ex01 (DAR10). | | | | |

**Test example 2**: Test of binding of anti-CD33 antibody-drug conjugate to recombinant human CD33 protein by ELISA:
The binding ability of antibody to antigen can be preliminarily judged by ELISA. Here, recombinant human CD33 protein was immobilized on an enzyme strip, and tested with the addition of a series of concentration gradients of CD33 antibody.

1. Coating: coating with CD33 diluted to 0.5 μg/mL using 1×PBS, at 100 μL per well, at 4°C overnight.
2. Plate washing: washing the plate with a plate wash buffer (1 xPBS +0.05% Tween 20) 3 times, at 200 μ L per well, while standing for 1min each time.
3. Sealing: after the 3rd patting-dry of the wash buffer, sealing with a sealing buffer (1×PBS +0.05% Tween20+1% BSA), at 200 μL per well, at 37°C for 1 h.
4. Plate washing: washing the plate with the plate wash buffer 3 times, at 200 μ L per well, while standing for 1min each time.
5. Primary antibody loading: diluting the antibody-drug conjugate (same as Test example 1) at a 3-fold gradient from 2 μg/mL using a sample diluent (1×PBS +0.05% Tween20+1% BSA); washing the plate with the plate wash buffer 3 times, at 200 μ L per well, while standing for 1min each time.
6. Secondary antibody loading: diluting Solarbio rabbit anti-human antibody at a ratio of 1:10000 with an assay buffer (1×PBS +0.05% Tween20+1% BSA) to 100 μ L per well, while standing at 37°C for 1 h.
7. Plate washing: washing the plate with the plate wash buffer 3 times, at 200 μ L per well, while standing for 1min each time.
8. Reading: after 7 min of color development with the addition of a TMB color development liquid (50 μL per well), adding 2M $H_2SO_4$ (50 μL per well) to terminate it. Plate reading using a microplate reader at 450 nm.

The results of the experiment are shown in Figs. 19 and 20.

Conclusion: The binding ability of anti-CD33 antibody-drug conjugates, mono-drug ADC: ANTI-CD33-Ex01 (10), dual-drug ADCs: ANTI-CD33-R01-1 (2) - Ex01 (8), and ANTI-CD33-Ex01 (2) - R01-1 (8), to recombinant human CD33 protein was basically the same as that of naked anti-CD33 antibody to recombinant human CD33 protein, with no

significant difference.

**Test example 3:** Plasma stability test of anti-CD33 antibody and antibody-drug conjugate

Mixtures of naked ANTI-CD33 antibody and ADC (ANTI-CD33-R01-1 (2) - Ex01 (8)) with IgG depleted human plasma were formulated respectively, with a final concentration of 0.6 mg/ml for the naked antibody and the ADC, and incubated in a water bath in a 37°C incubator for 0, 3 and 7 days, while setting a non-incubated and unextracted control and a non-incubated and extracted control. After incubation, the samples were tested by SEC-HPLC method. The results are shown in Table 7.

Table 7. Plasma stability data

| Name | | | Naked anti-CD33 antibody | ANTI-CD33-R01-1 (2) -Ex01 (8) |
|---|---|---|---|---|
| Plasma stability SEC | Non-incubated and extracted in plasma | Aggregation% | 5.82 | 7.88 |
| | | Monomer% | 94.19 | 92.13 |
| | | Degradation% | 0.00 | 0.00 |
| | Incubated in plasma for 0 day | Aggregation% | 8.48 | 9.11 |
| | | Monomer% | 91.52 | 90.89 |
| | | Degradation% | 0.00 | 0.00 |
| | Incubated in plasma for 3 days | Aggregation% | 14.65 | 11.04 |
| | | Monomer% | 85.35 | 88.96 |
| | | Degradation% | 0.00 | 0.00 |
| | Incubated in plasma for 7 days | Aggregation% | 15.18 | 19.00 |
| | | Monomer% | 84.82 | 81.00 |
| | | Degradation% | 0.00 | 0.00 |
| Conclusion: the ADCs disclosed in the present invention had excellent properties including good plasma stability, low degradation rate, low aggregation rate, and high monomer rate. | | | | |

While specific embodiments of the invention have been described in detail, those skilled in the art will understand that: various modifications and variations may be made to the details in light of the overall teachings of the invention, and such changes are all within the scope of the invention. The whole scope of the invention is given by the appended claims and any equivalents thereof.

## Claims

1. A ligand-drug conjugate represented by formula I, or a stereoisomer thereof, a prodrug thereof, a pharmaceutically acceptable salt thereof or a pharmaceutically acceptable solvate thereof

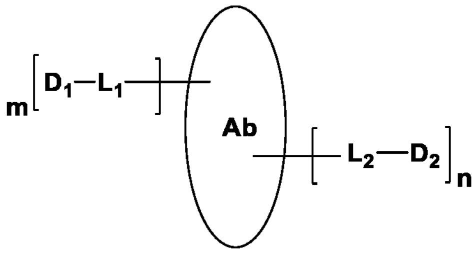

formula I

wherein:

Ab is a targeting moiety selected from the group consisting of an antibody, an antibody fragment, a targeting protein, and an Fc-fusion protein;

$D_1$ is a first drug linked to $L_1$;

$D_2$ is a second drug selected from DNA damage repair inhibitors, and is linked to $L_2$;

$L_1$ is a first linking unit linking the first drug to the targeting moiety;

$L_2$ is a second linking unit linking the second drug to the targeting moiety;

m is an integer selected from 0 to 20;

n is an integer selected from 1 to 20.

**2.** The ligand-drug conjugate, or a stereoisomer thereof, a prodrug thereof, a pharmaceutically acceptable salt thereof or a pharmaceutically acceptable solvate thereof according to claim 1, wherein, the second drug is selected from the group consisting of an ATR inhibitor, an ATM inhibitor, a PARP inhibitor, a WEE1 inhibitor, a CHK1 inhibitor, and a DNA-PK inhibitor;

preferably, the ATR inhibitor is selected from the group consisting of

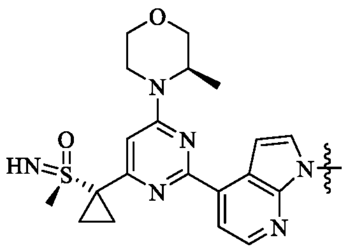
,
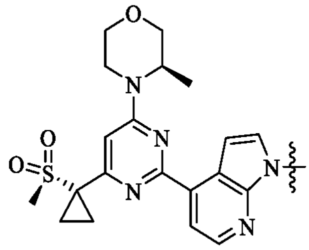
,
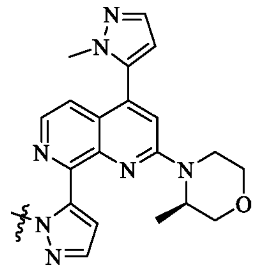
,
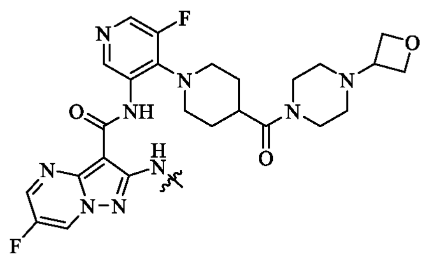
,

and a compound represented by formula A or a deuteride thereof:

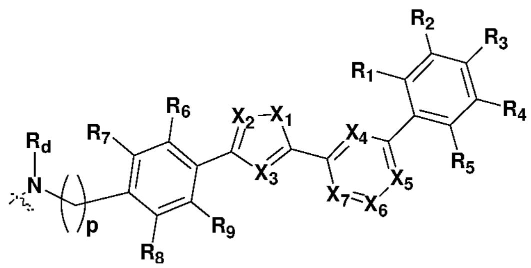

formula A

wherein,

$X_1$ is selected from the group consisting of O, S, NH, and $CH_2$;

preferably, $X_1$ is selected from the group consisting of O, and S;

more preferably, $X_1$ is O;

$X_2$, $X_3$ each are independently selected from the group consisting of N, and CH;

preferably, $X_2$ is N, $X_3$ is CH;

$X_4$, $X_5$, $X_6$ each are independently selected from the group consisting of N, and CH;

preferably, $X_4$ and $X_6$ are N, $X_5$ is CH;

$X_7$ is selected from the group consisting of N, and $C(R_0)$;

preferably, $X_7$ is $C(R_0)$;
$R_0$ is selected from the group consisting of H, C1-C6 alkyl, and $R_bR_aN$-;
preferably, $R_0$ is $R_bR_aN$-;
$R_a$, $R_b$ each are independently selected from the group consisting of H, and C1-C6 alkyl;
preferably, $R_a$, $R_b$ are H;
$R_1$, $R_2$, $R_3$, $R_4$, $R_5$ each are independently selected from the group consisting of H, C1-C6 alkyl, and

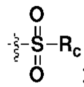
;

preferably, $R_1$, $R_2$, $R_3$, $R_4$, $R_5$ each are independently selected from H, and

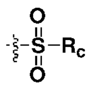
;

more preferably, any one of $R_1$, $R_2$, $R_3$, $R_4$, $R_5$ is

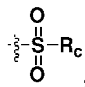
,

with the rest being H;
most preferably, $R_3$ is

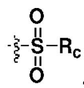
,

$R_1$, $R_2$, $R_4$, and $R_5$ are H;
$R_c$ is selected from the group consisting of H, and C1-C6 alkyl;
preferably, $R_c$ is selected from C1-C6 alkyl;
more preferably, $R_c$ is isopropyl;
$R_6$, $R_7$, $R_8$, $R_9$ each are independently selected from the group consisting of H, and C1-C6 alkyl;
preferably, $R_6$, $R_7$, $R_8$, and $R_9$ are H;
$R_d$ is selected from the group consisting of H, and C1-C6 alkyl;
preferably, $R_d$ is selected from C1-C6 alkyl;
more preferably, $R_d$ is methyl;
p is selected from the group consisting of 1, 2, 3, 4, 5, and 6;
preferably, p is selected from the group consisting of 1, 2, and 3;
more preferably, p is 1;
more preferably, the compound represented by formula A is

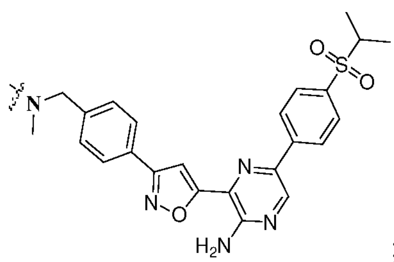
;

preferably, the PARP inhibitor is selected from the group consisting of

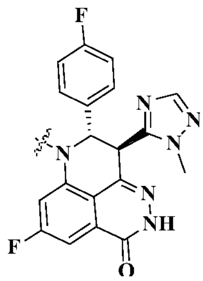,

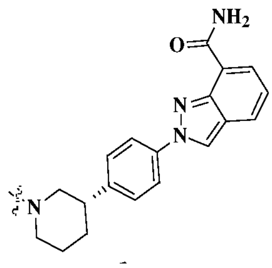, 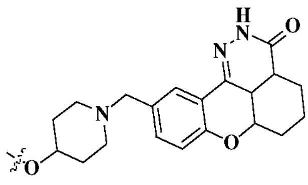, 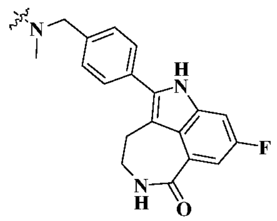, 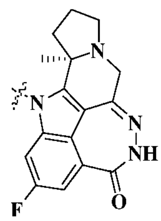,

and

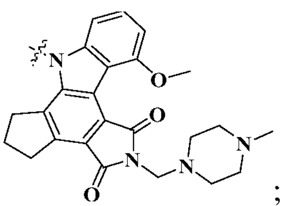;

preferably, the ATM inhibitor is selected from the group consisting of

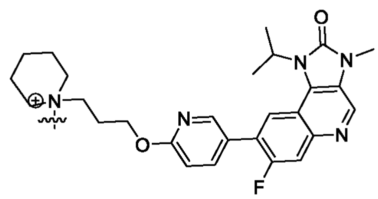, 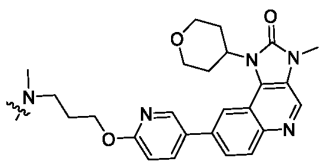, 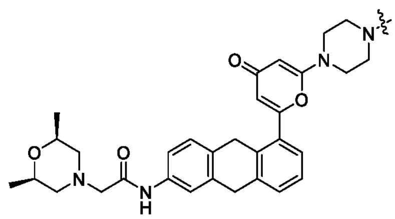,

and

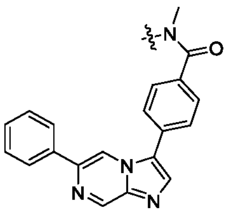;

preferably, the WEE1 inhibitor is

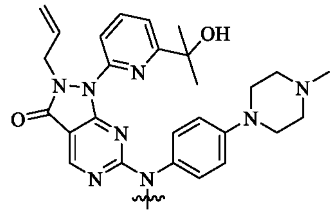

;

preferably, the CHK1 inhibitor is selected from the group consisting of

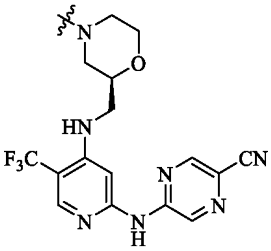

,

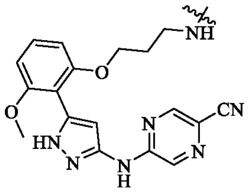

,

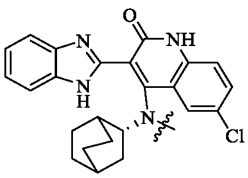

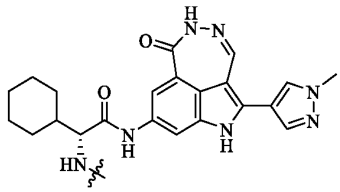

,

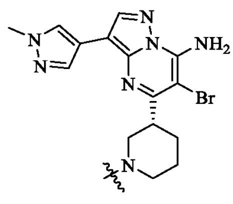

,

and

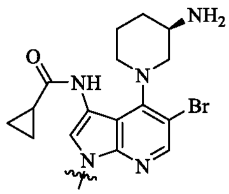

;

preferably, the DNA-PK inhibitor is selected from the group consisting of

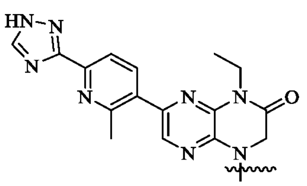

,

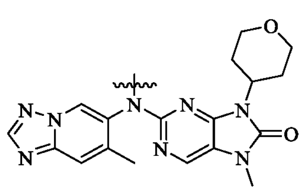

,

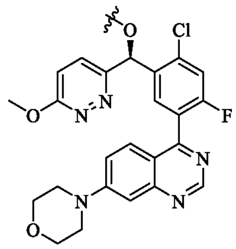

,

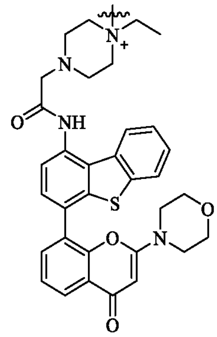

,

and

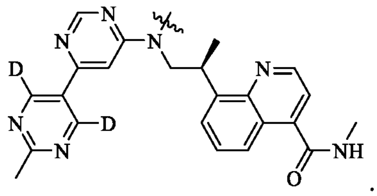

3. The ligand-drug conjugate, or a stereoisomer thereof, a prodrug thereof, a pharmaceutically acceptable salt thereof or a pharmaceutically acceptable solvate thereof according to any one of claims 1-2, wherein, the second linking unit is a breakable or non-breakable linking unit;

preferably, $L_2$ is $L_{21}$-$L_{22}$-$L_{23}$-$L_{24}$, and $L_{24}$ is linked to $D_2$, $L_{21}$ is linked to Ab (preferably, $L_{21}$ is coupled to Ab via thiol group),

$L_{21}$ is selected from the group consisting of

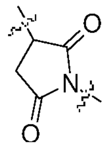

,

and 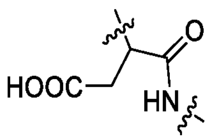

,

with its N terminal linked to $L_{22}$,

$L_{22}$ is selected from the group consisting of:

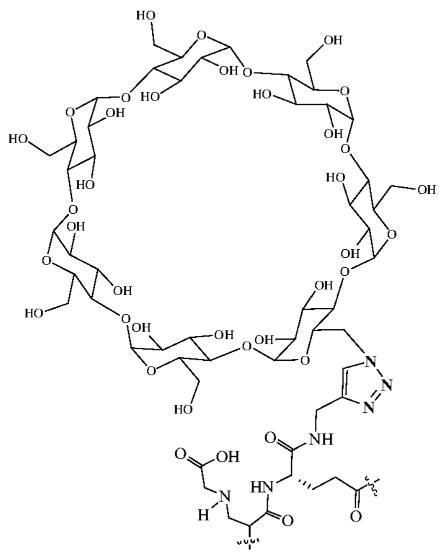

,

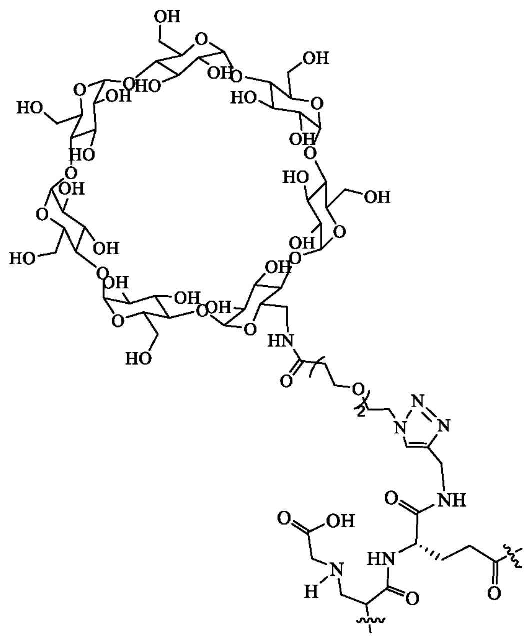

,

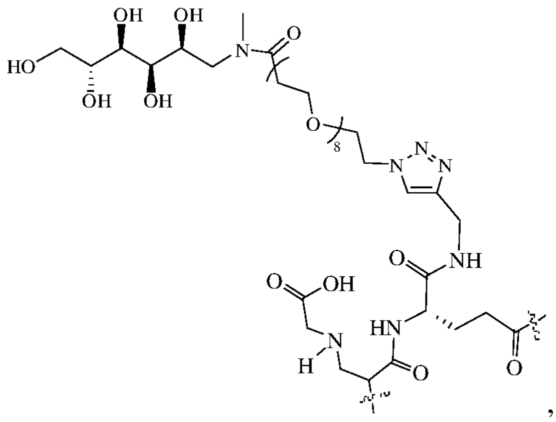

,

and

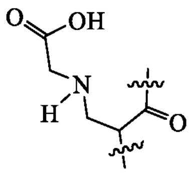

,

with its carbonyl terminal linked to $L_{23}$;

$L_{23}$ is selected from an amino acid residue or a peptide residue consisting of 2-10 amino acid residues, with its carbonyl terminal linked to $L_{24}$;

preferably, $L_{23}$ is a peptide residue consisting of 2-4 (preferably 2) amino acid residues, with its carbonyl terminal linked to $L_{24}$, preferably, the amino acid is selected from the group consisting of valine, alanine, phenylalanine, glycine, lysine, citrulline, serine, glutamic acid, and aspartic acid, more preferably, the amino acid is selected from the group consisting of valine, alanine, and citrulline;

more preferably, $L_{23}$ is selected from the group consisting of valine-alanine (Val-Ala), valine-citrulline (Val-Cit), with its carbonyl terminal linked to $L_{24}$;

most preferably, $L_{23}$ is selected from

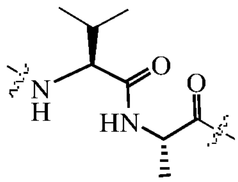

, 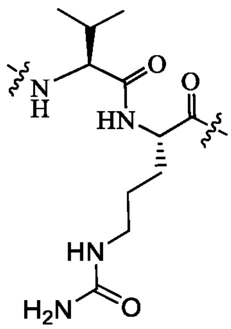

,

with its carbonyl terminal linked to $L_{24}$;

$L_{24}$ is

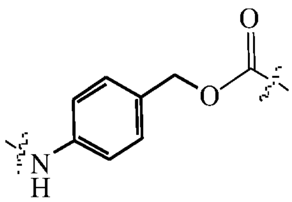

or a direct bond, and when $L_{24}$ is

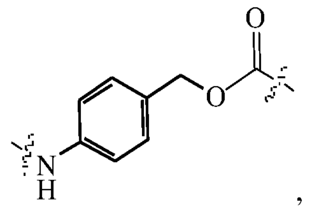

,

its carbonyl terminal is linked to $D_2$;

preferably, $L_2$ is selected from the group consisting of:

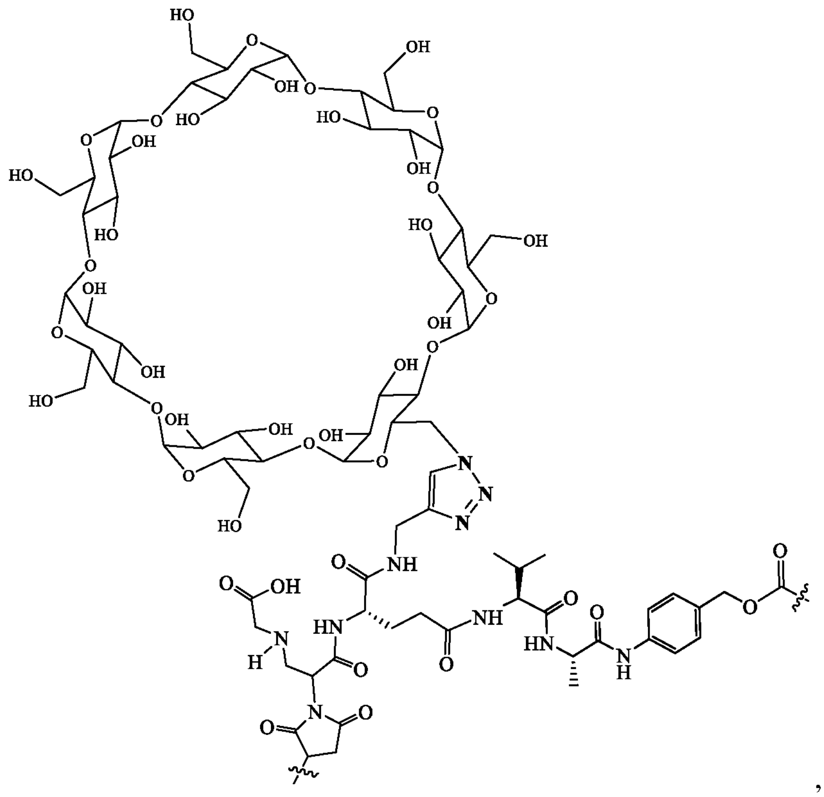

,

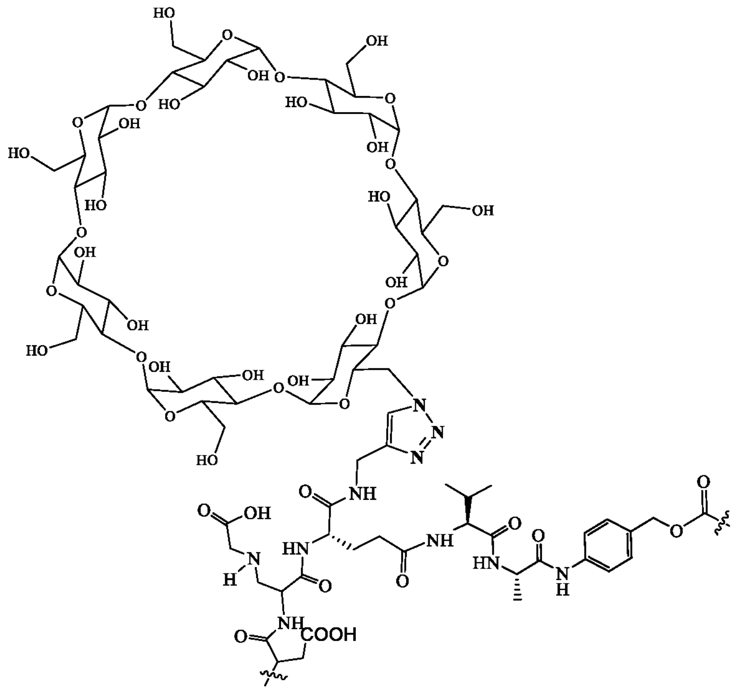

,

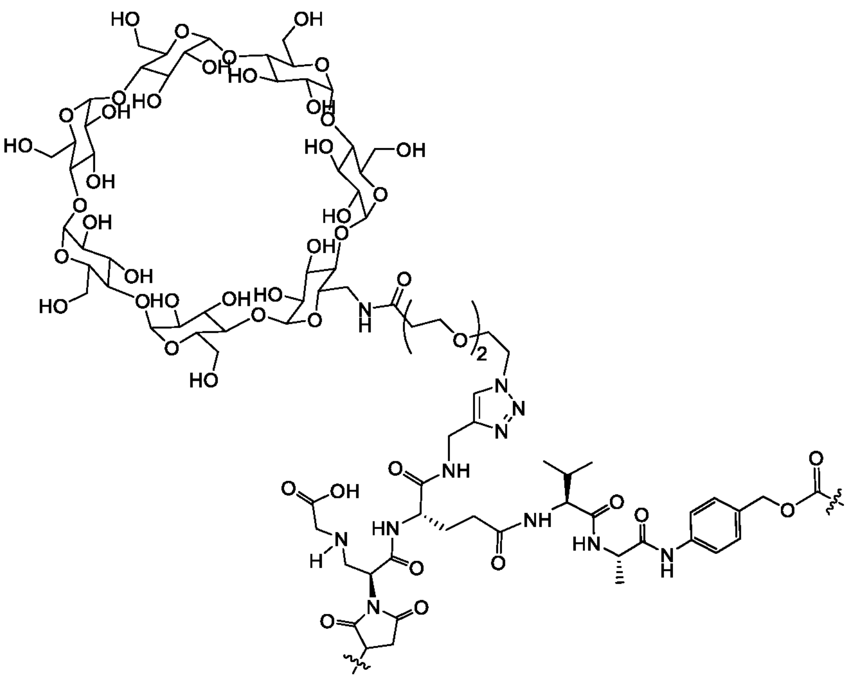

,

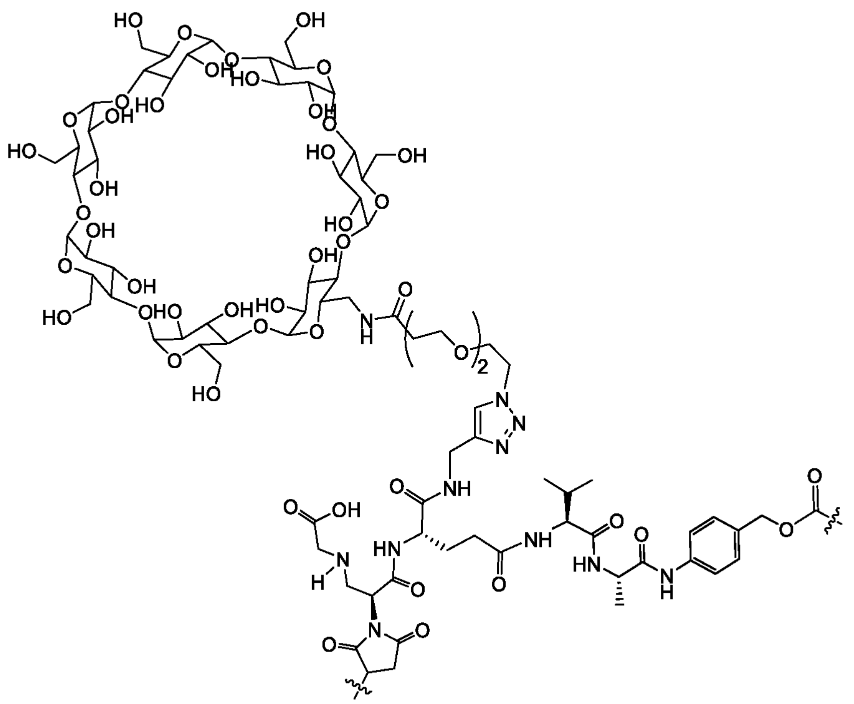

,

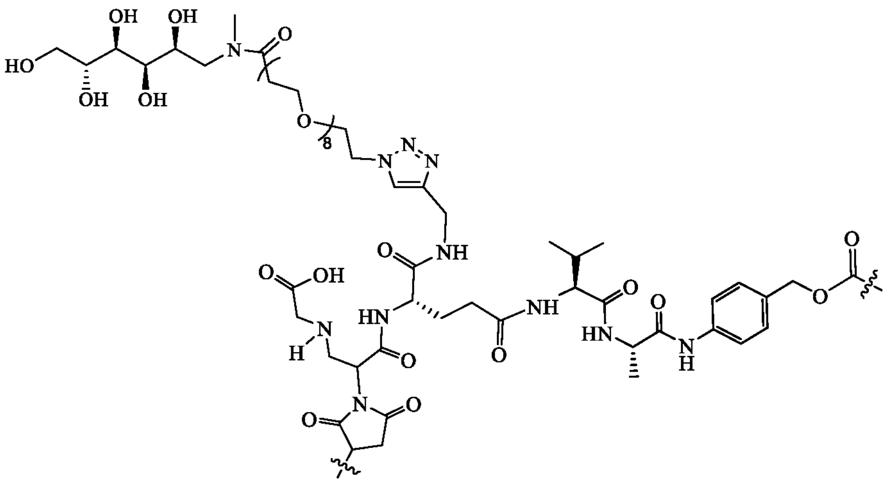

,

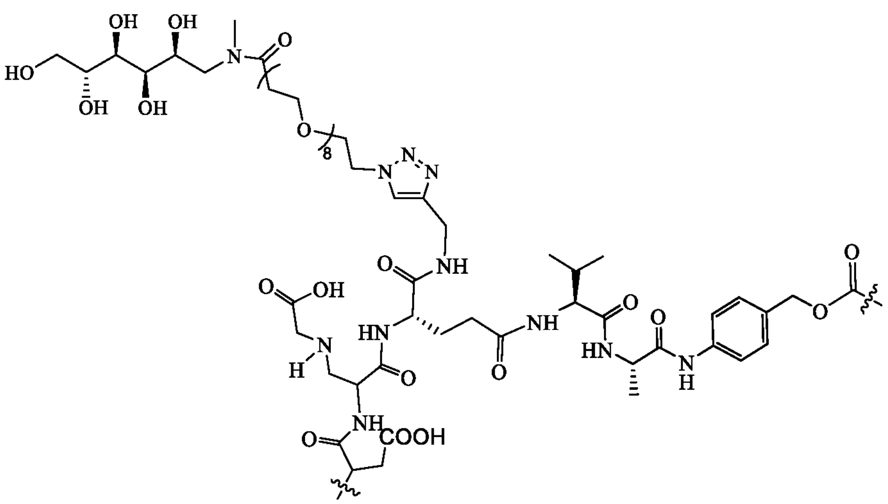

,

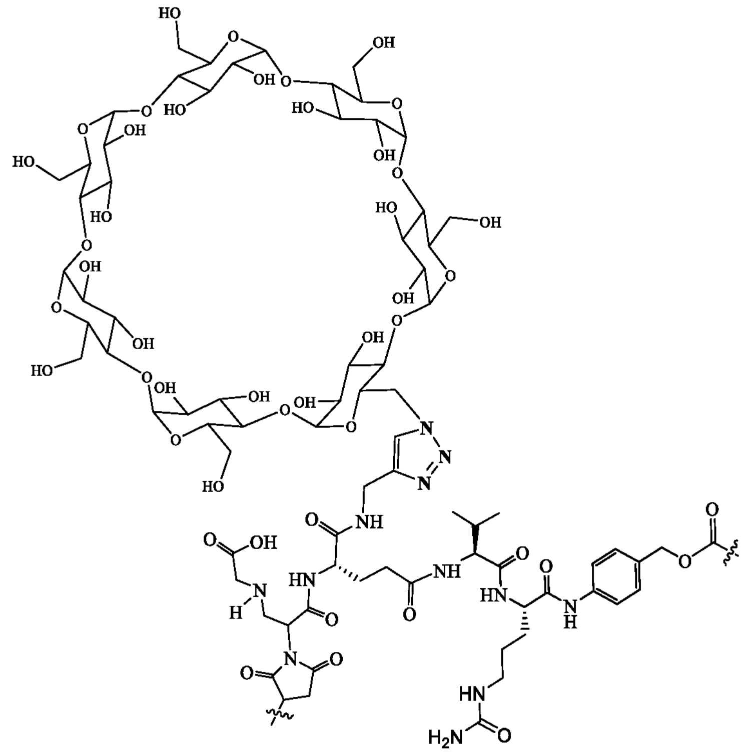

,

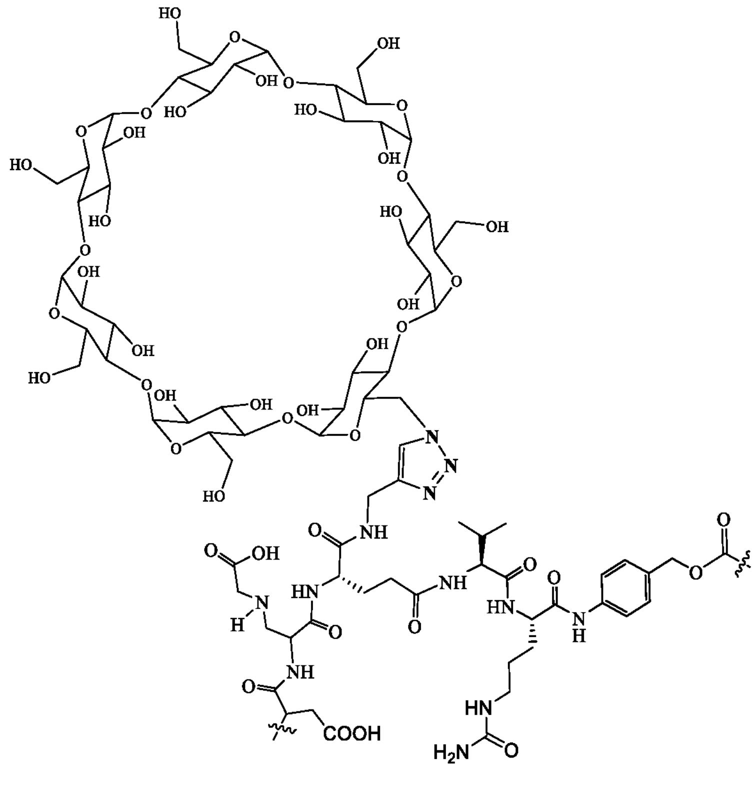

,

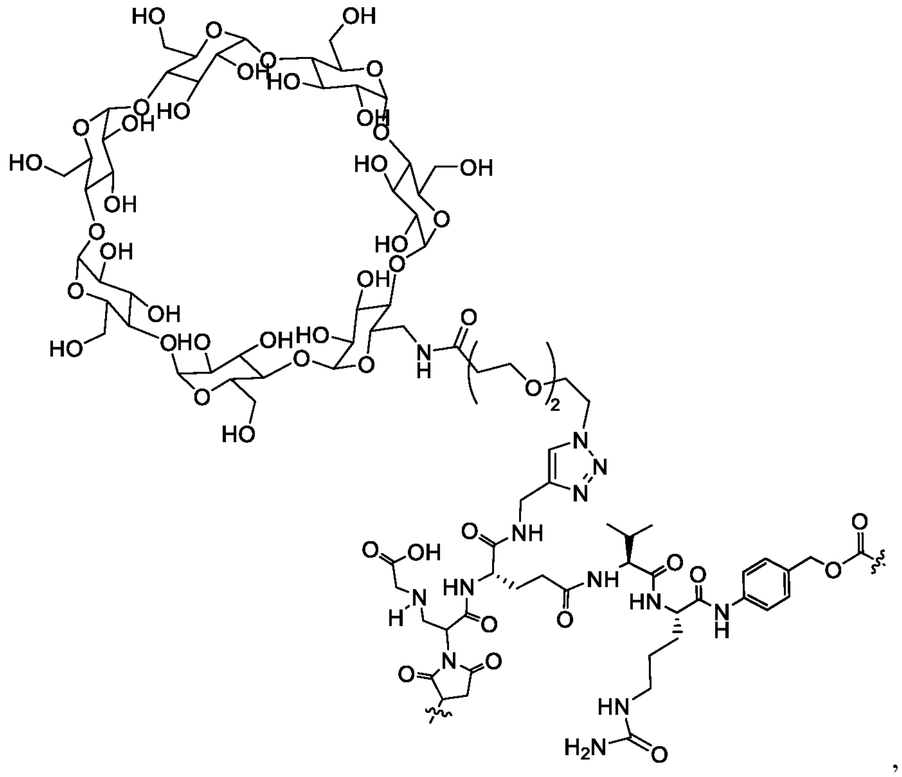

,

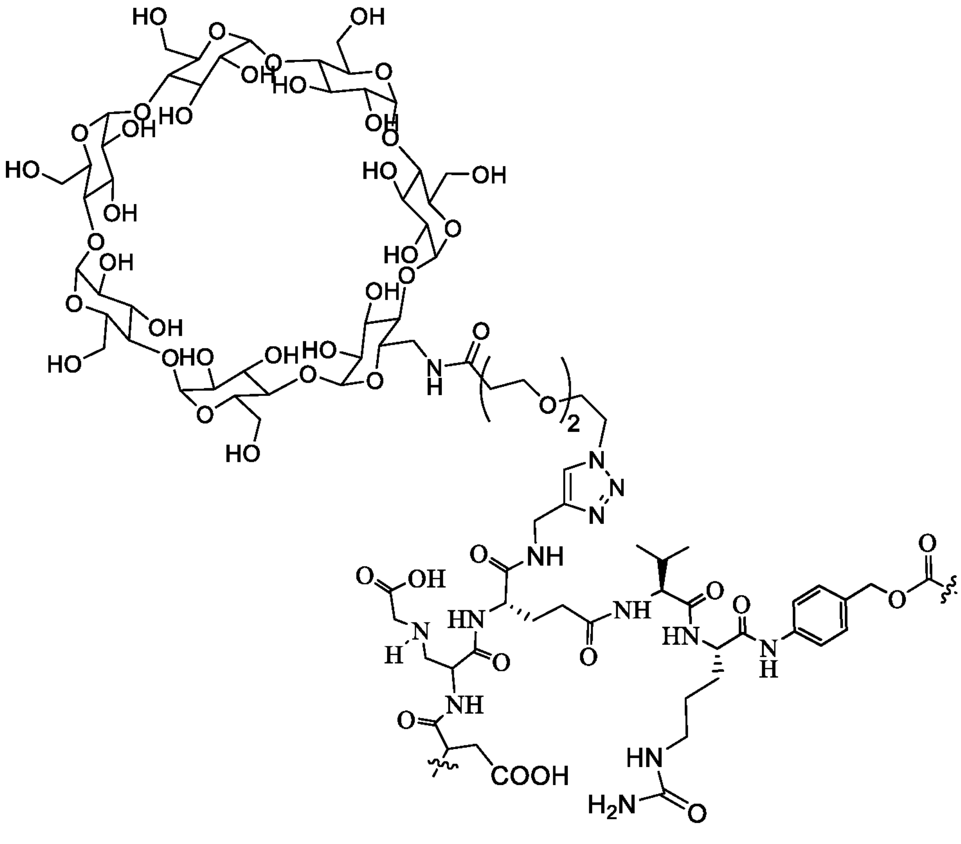

,

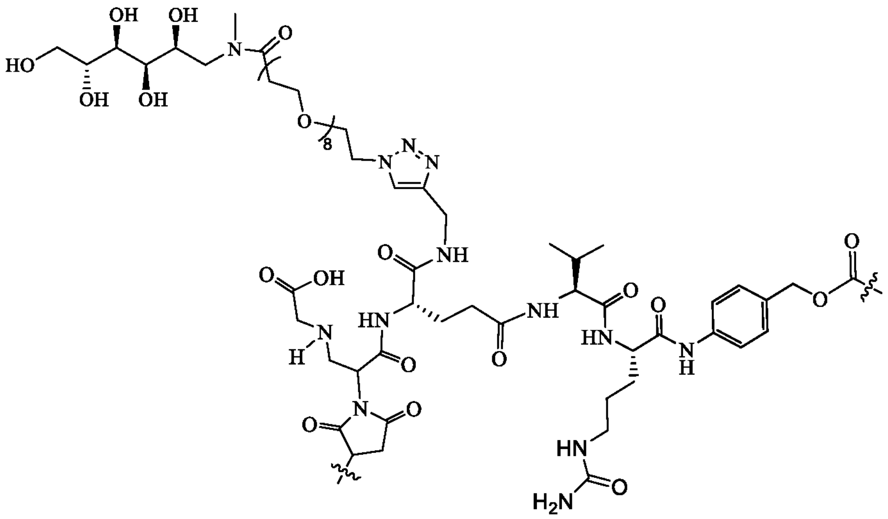

,

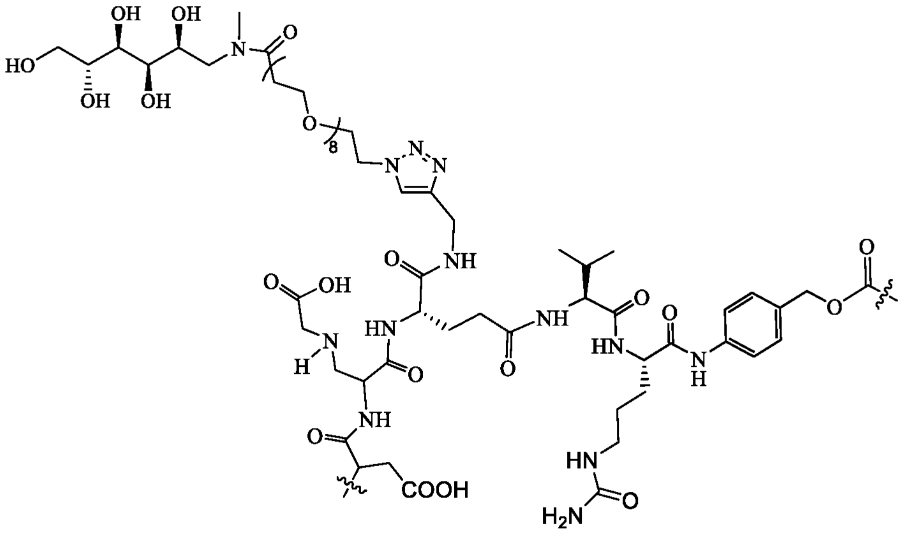

,

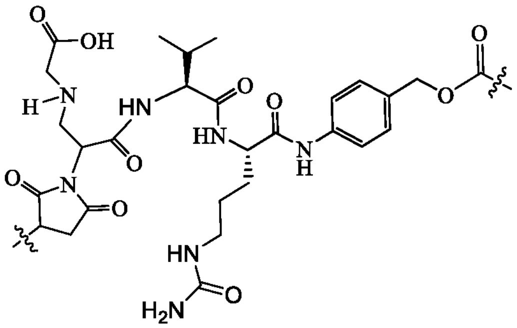

,

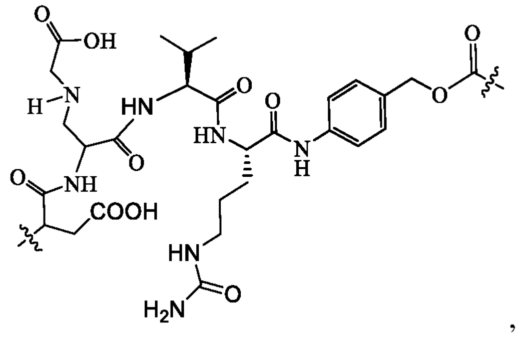

,

and

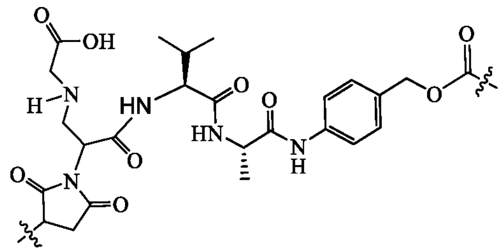

with its carbonyl terminal linked to $D_2$.

**4.** The ligand-drug conjugate, or a stereoisomer thereof, a prodrug thereof, a pharmaceutically acceptable salt thereof or a pharmaceutically acceptable solvate thereof according to any one of claims 1-3, wherein, $D_2$-$L_2$- is selected from the group consisting of:

| | |
|---|---|
| R01 | 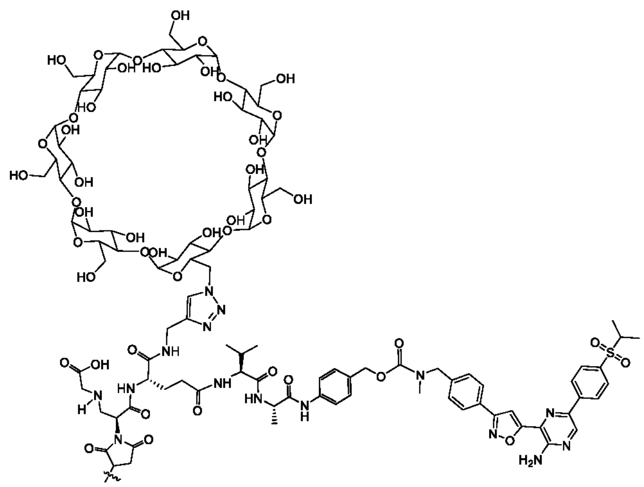 |

(continued)

| | |
|---|---|
| R01-1 | 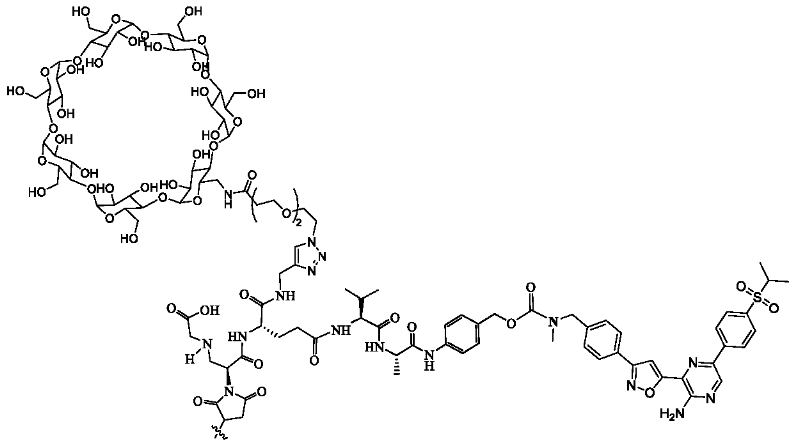 |
| R01-2 | 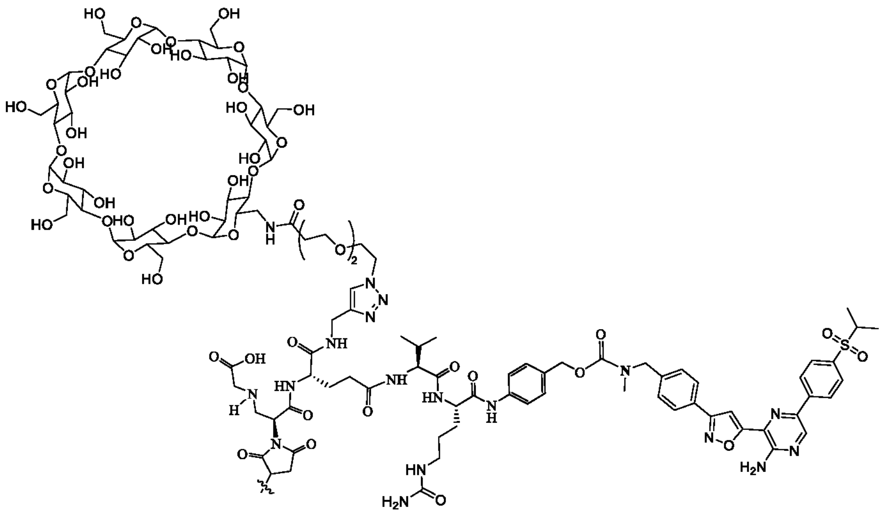 |
| R01-3 | 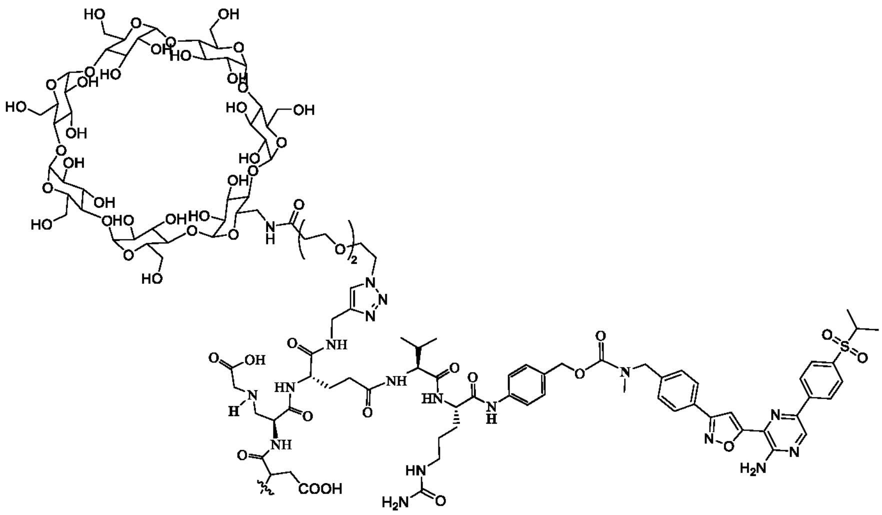 |

(continued)

| | |
|---|---|
| R01-4 | 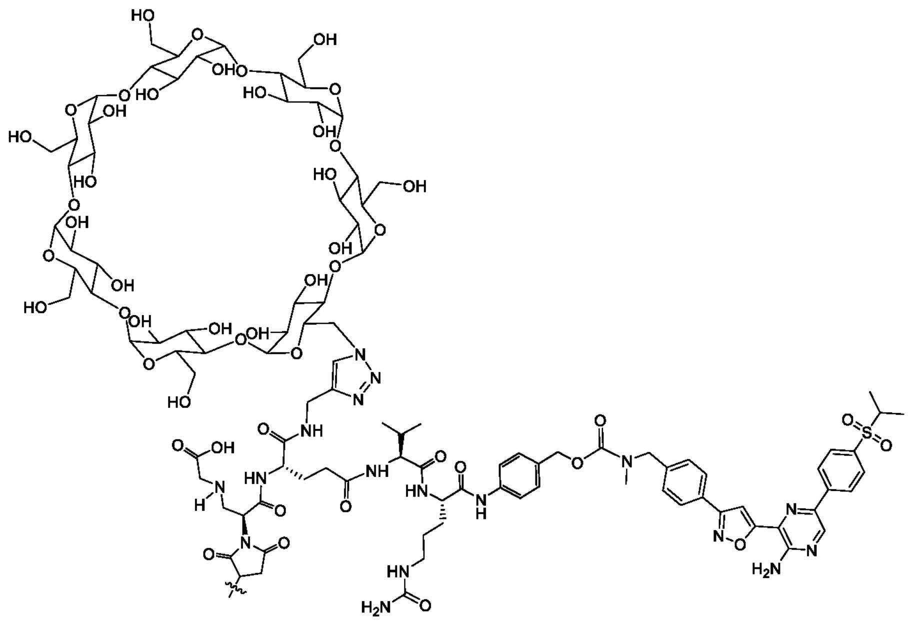 |
| R02 | 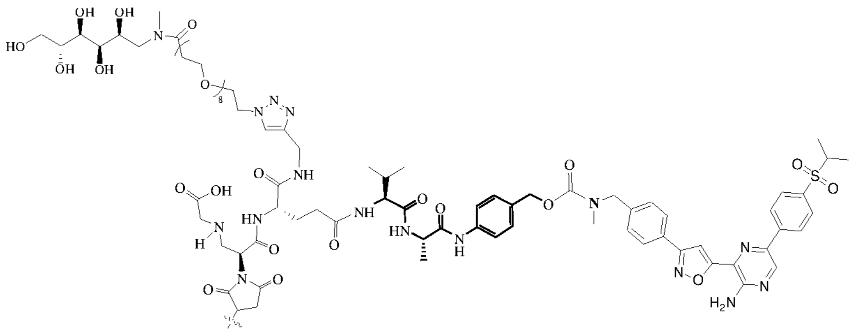 |
| R02-1 | 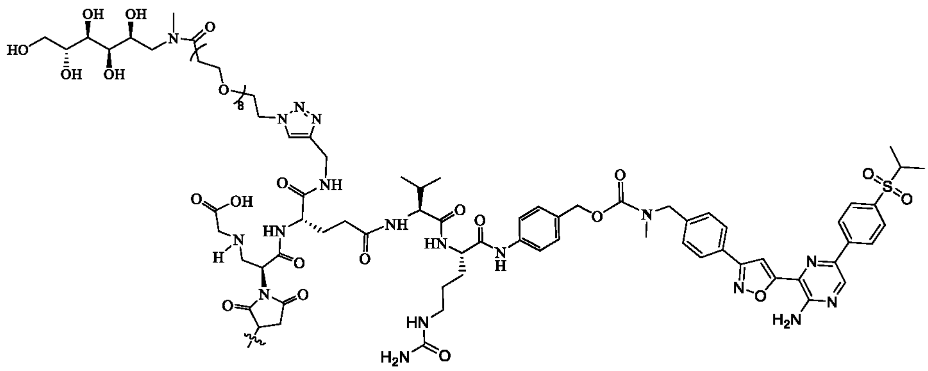 |

(continued)

| | |
|---|---|
| R03 | 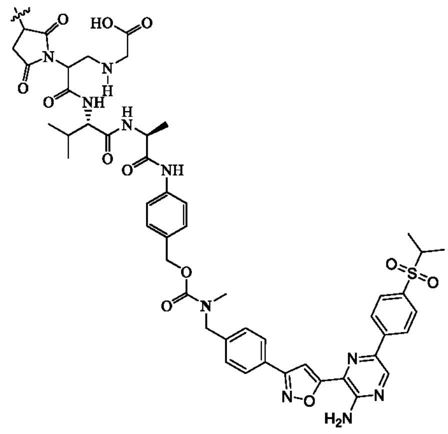 |
| R03-1 | 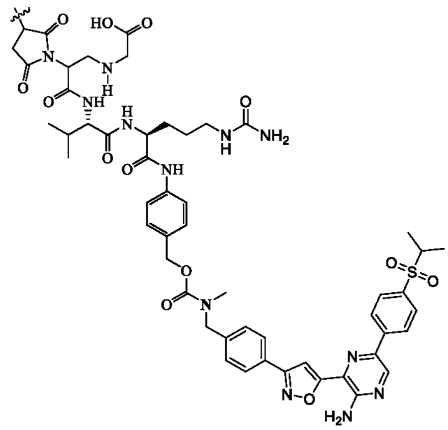 |
| AT01 | 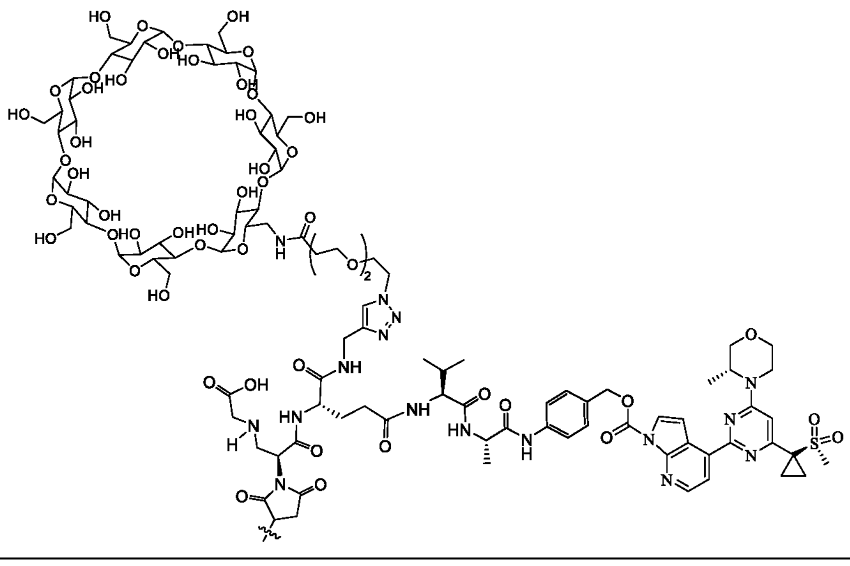 |

(continued)

| | |
|---|---|
| AT02 | 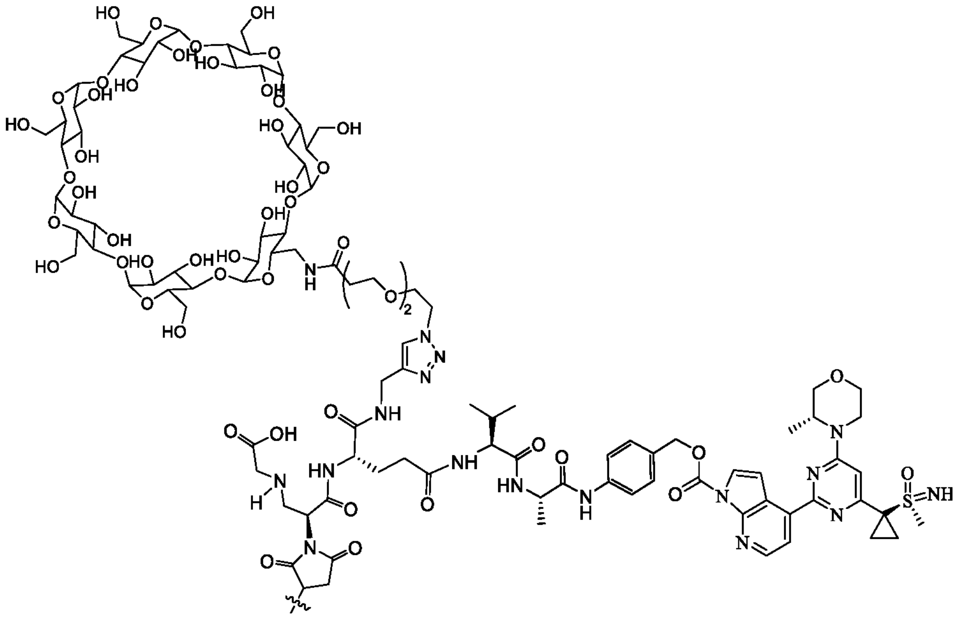 |
| AT03 | 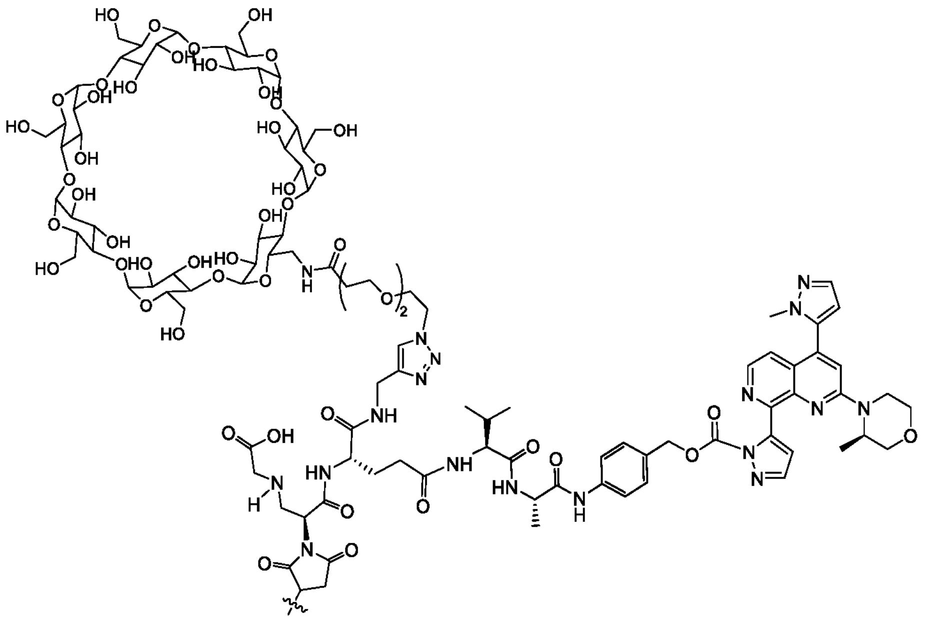 |

(continued)

| | |
|---|---|
| AT04 | 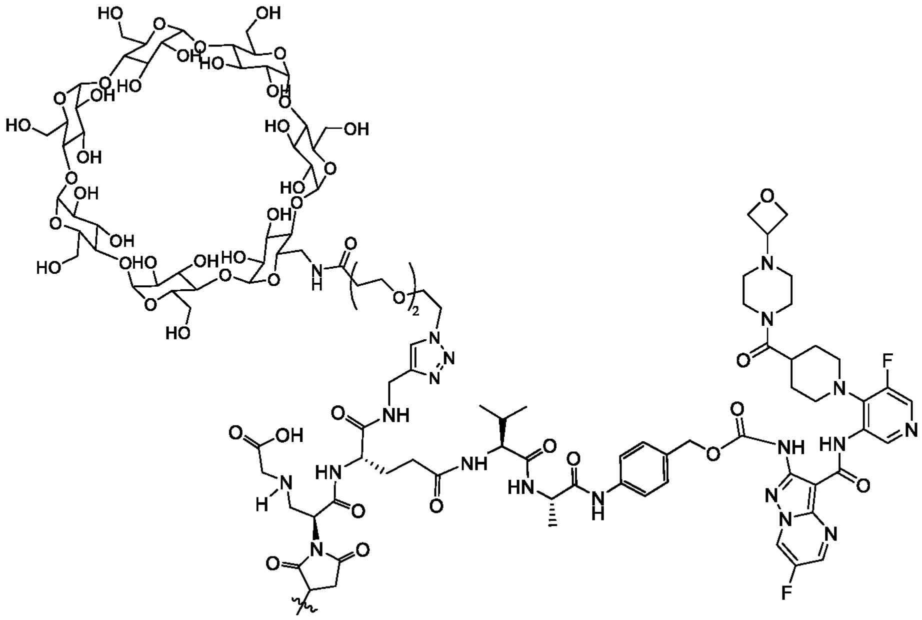 |
| TA01 | 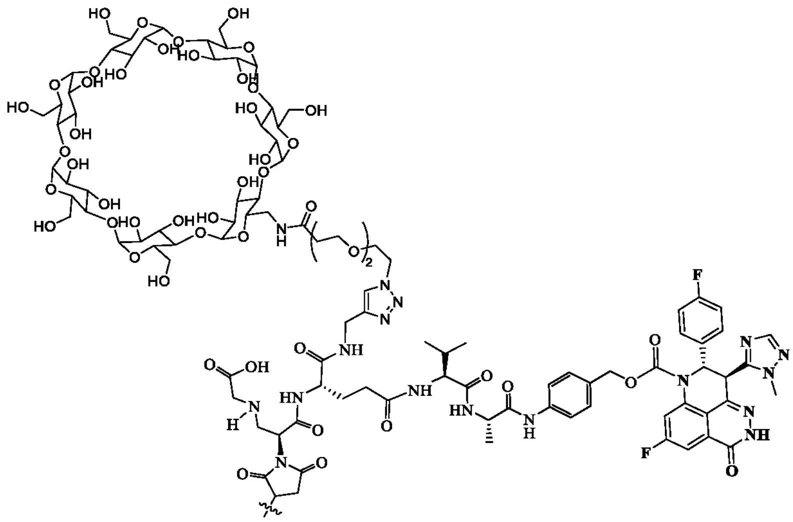 |
| VE01 | 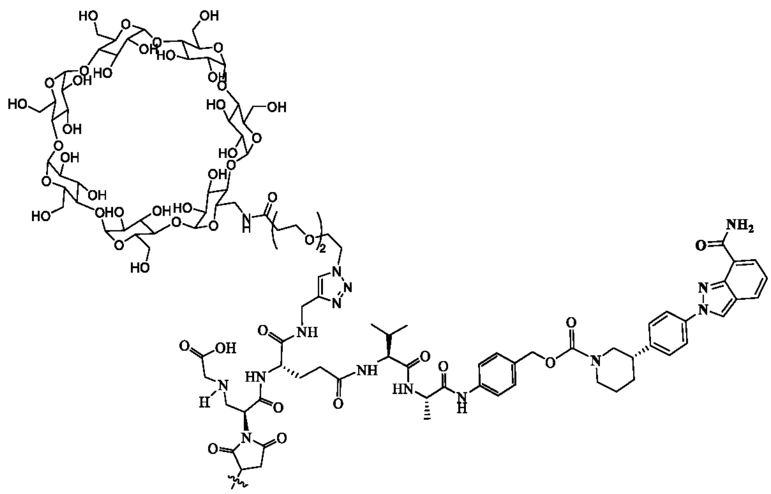 |

(continued)

| | |
|---|---|
| E01 | 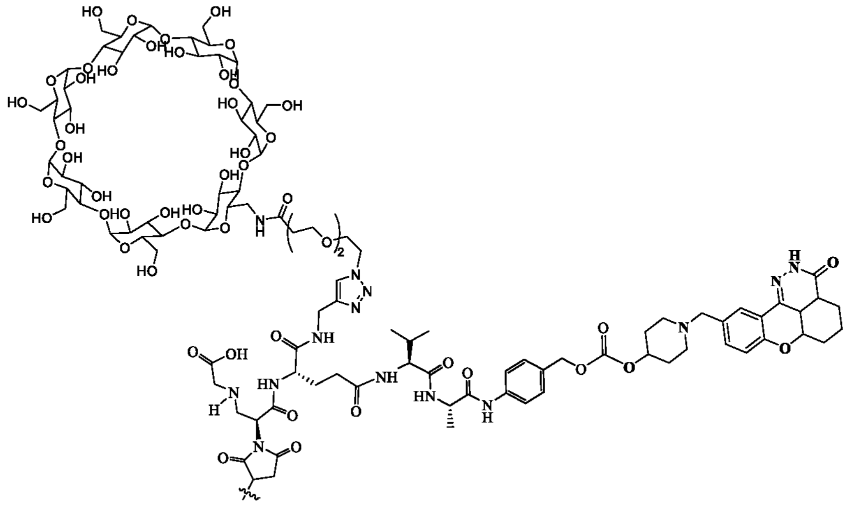 |
| RU01 | 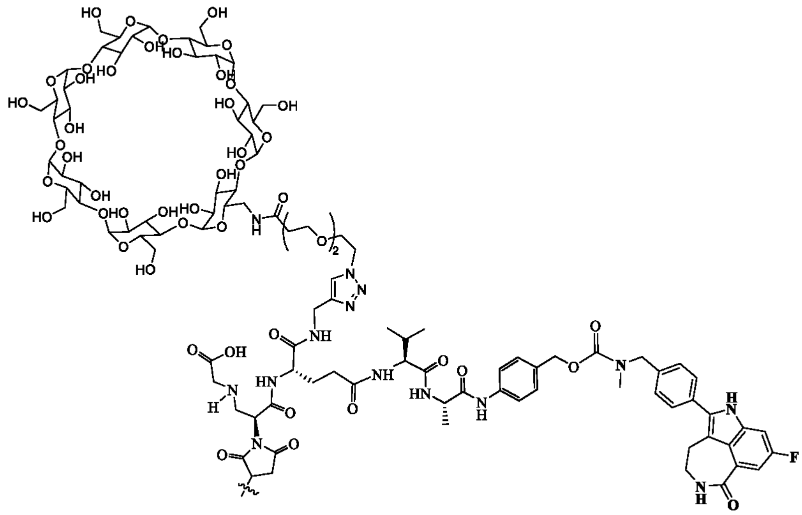 |
| PA01 | 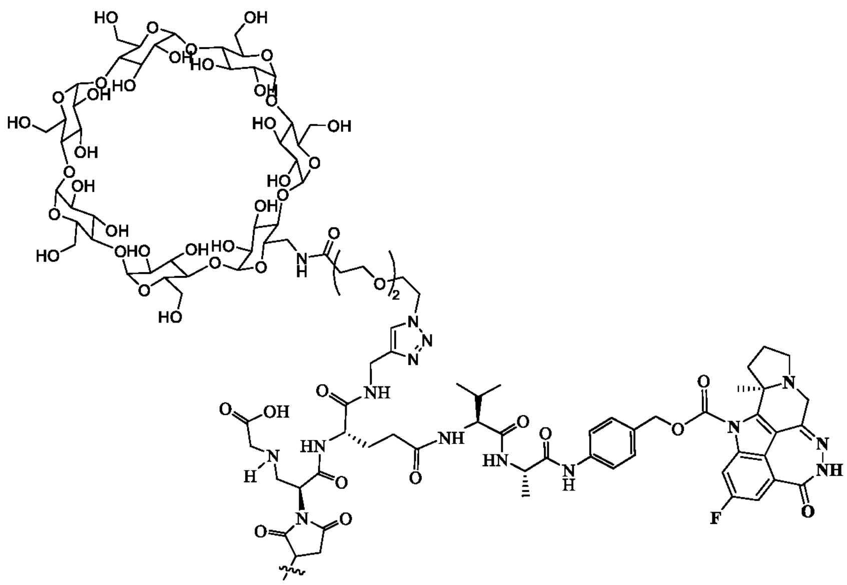 |

(continued)

| | |
|---|---|
| CEP01 | 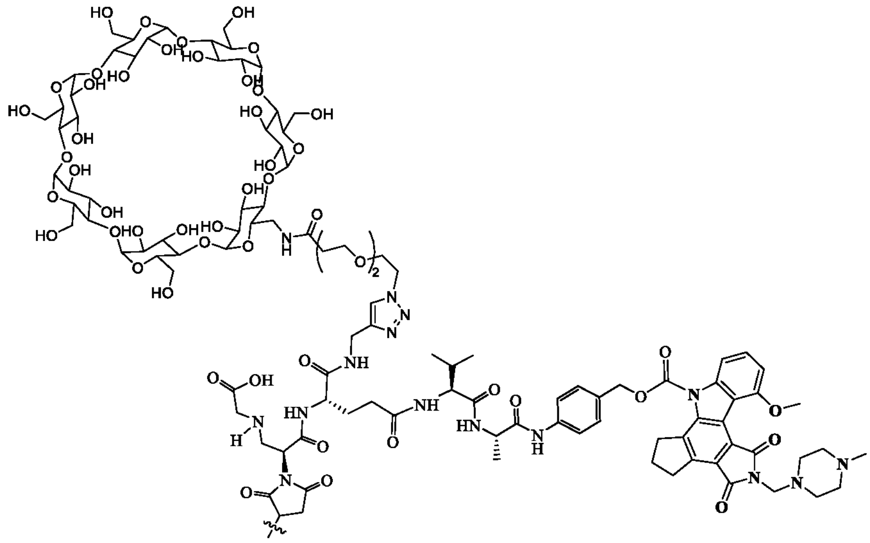 |
| TM01 | 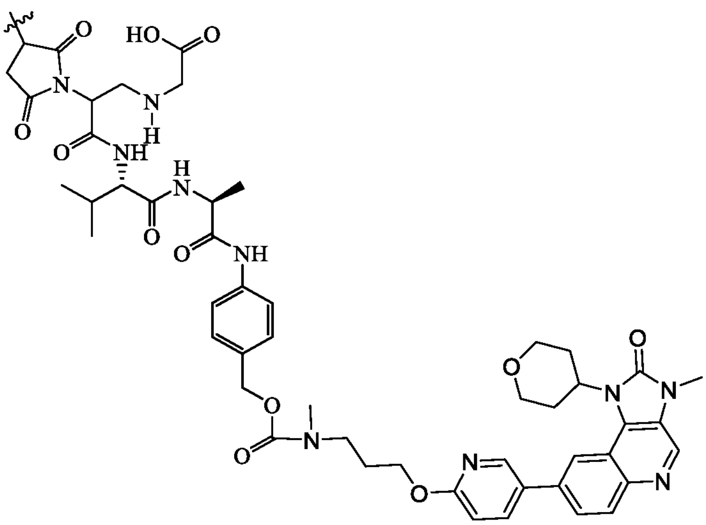 |
| TM02 | 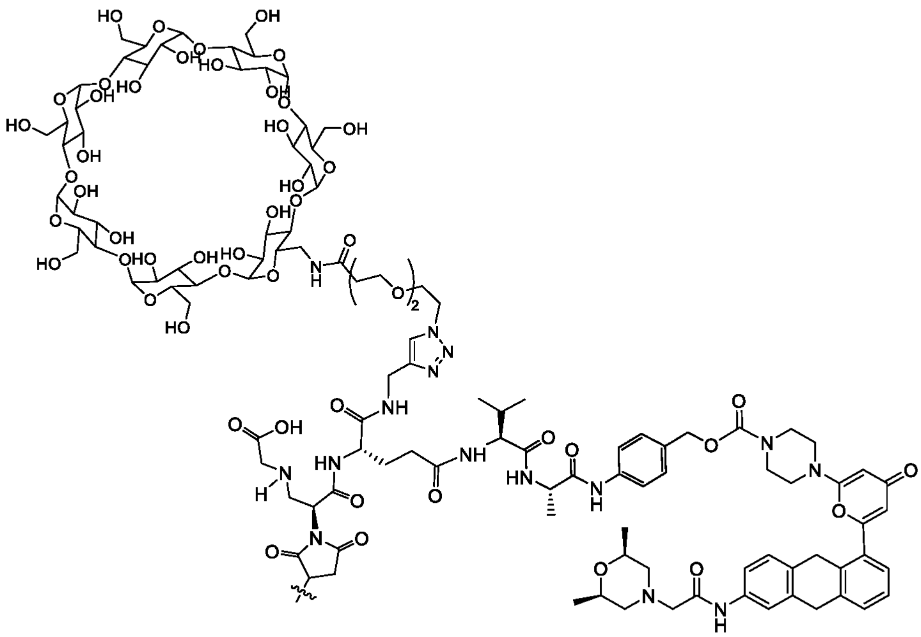 |

(continued)

| | |
|---|---|
| WE01 | 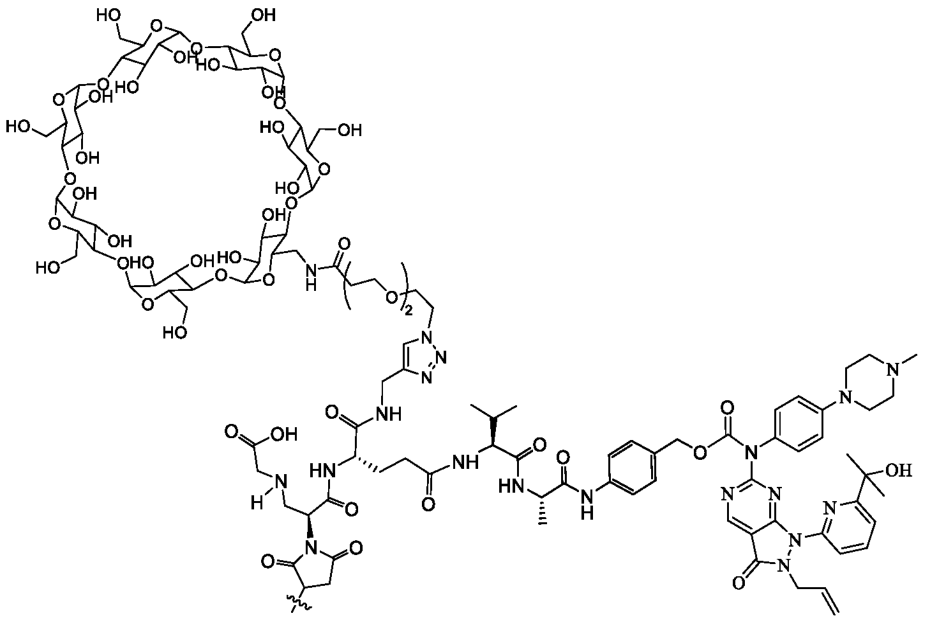 |
| CH01 | 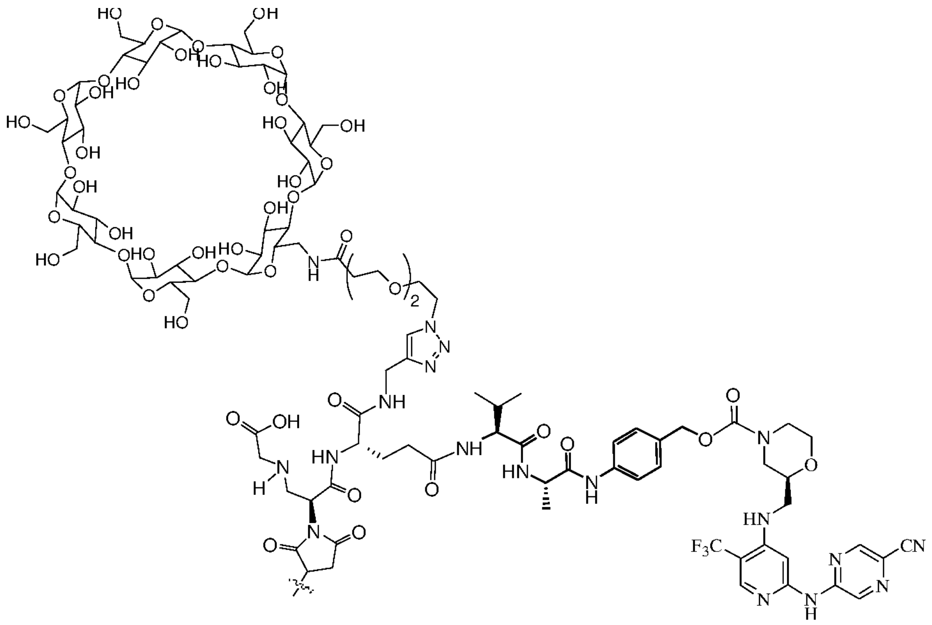 |

(continued)

| | |
|---|---|
| CH02 | 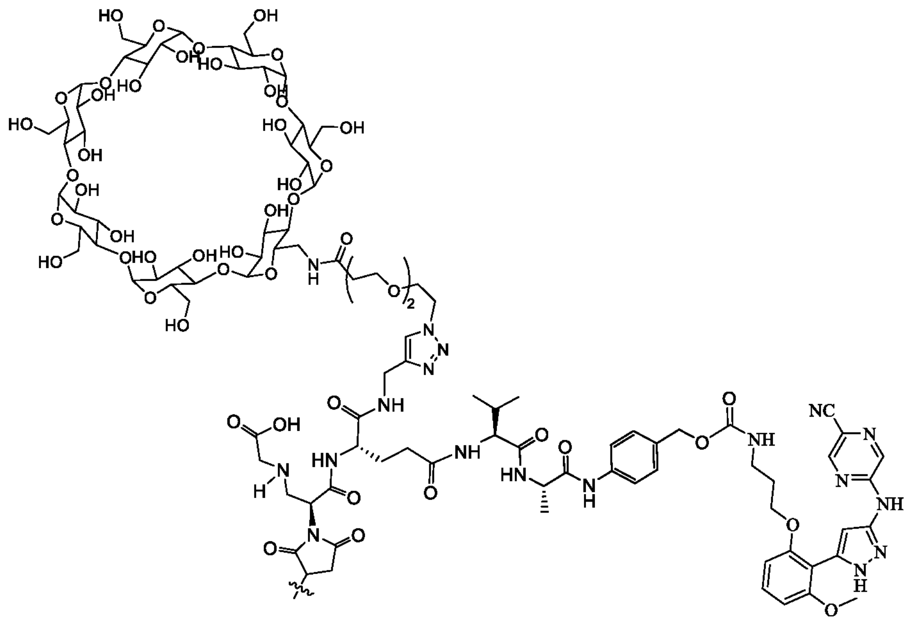 |
| CH03 | 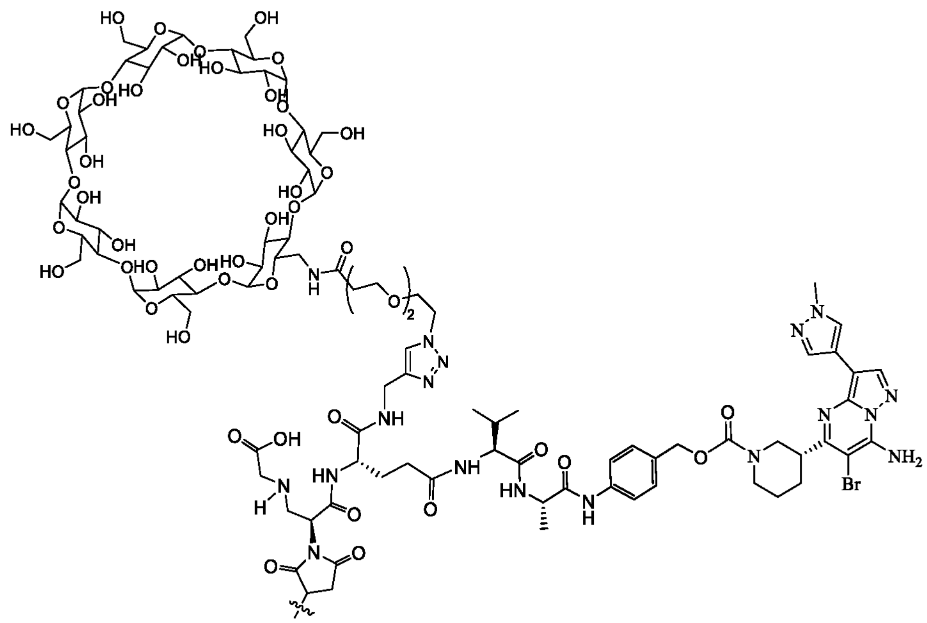 |

(continued)

| | |
|---|---|
| CH04 | 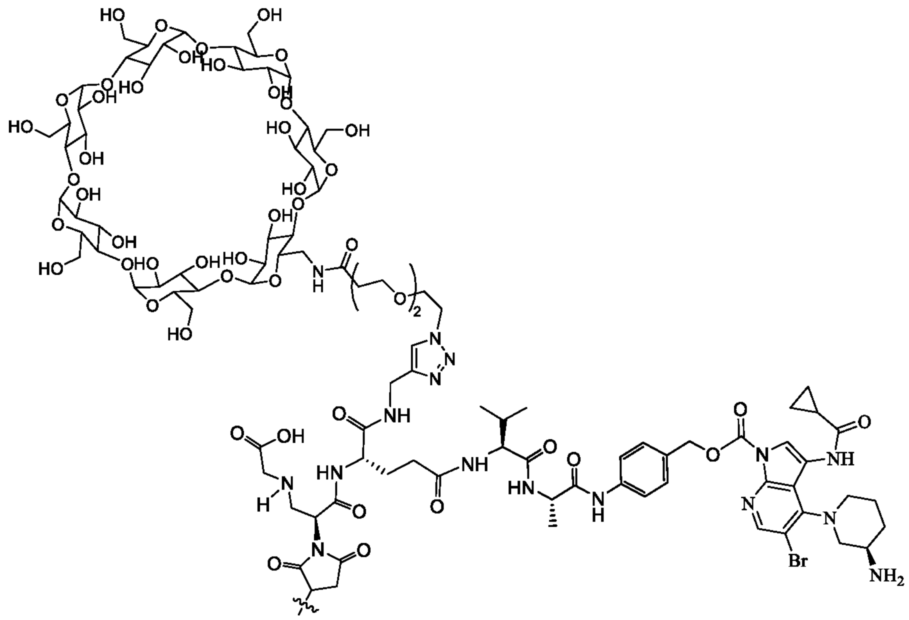 |
| DP01 | 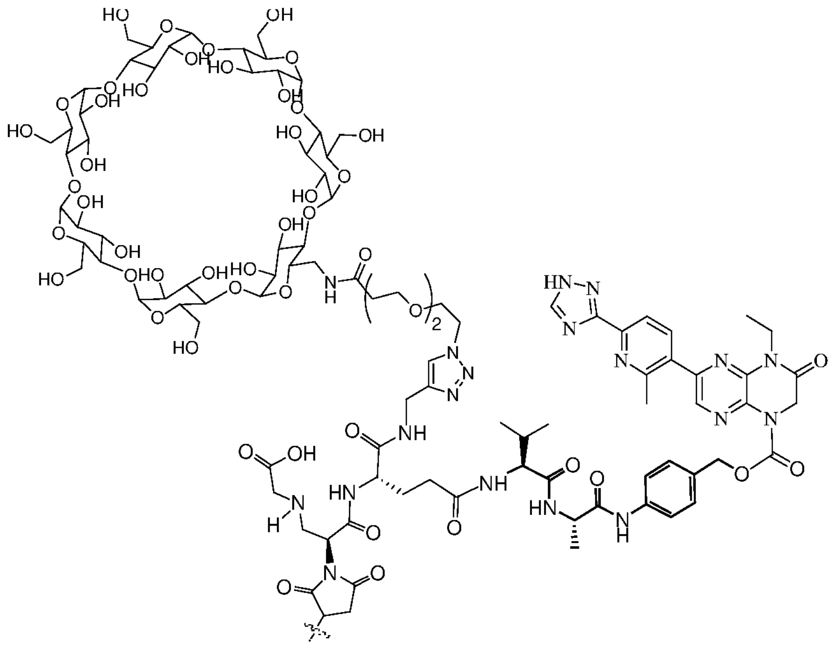 |

(continued)

| | |
|---|---|
| DP02 | 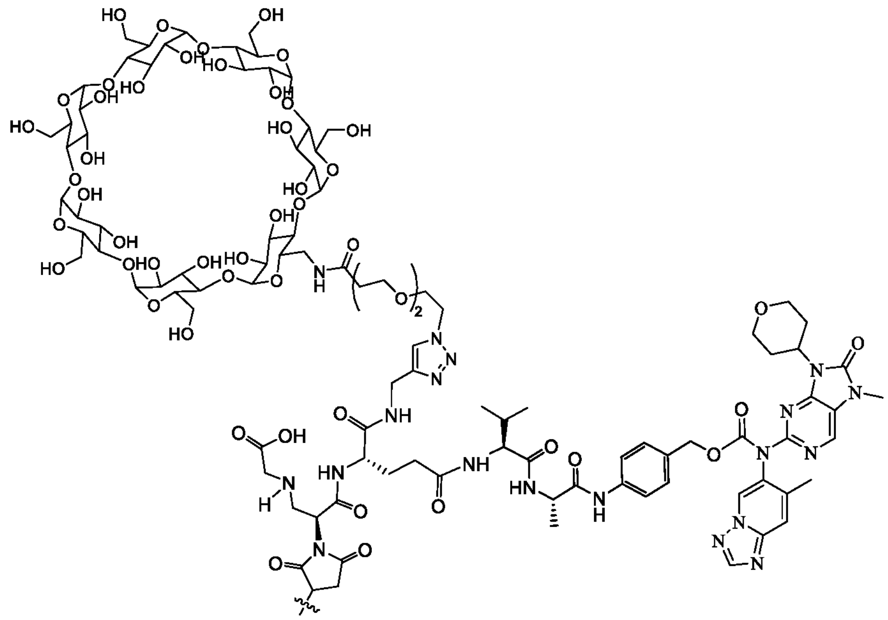 |
| DP03 | 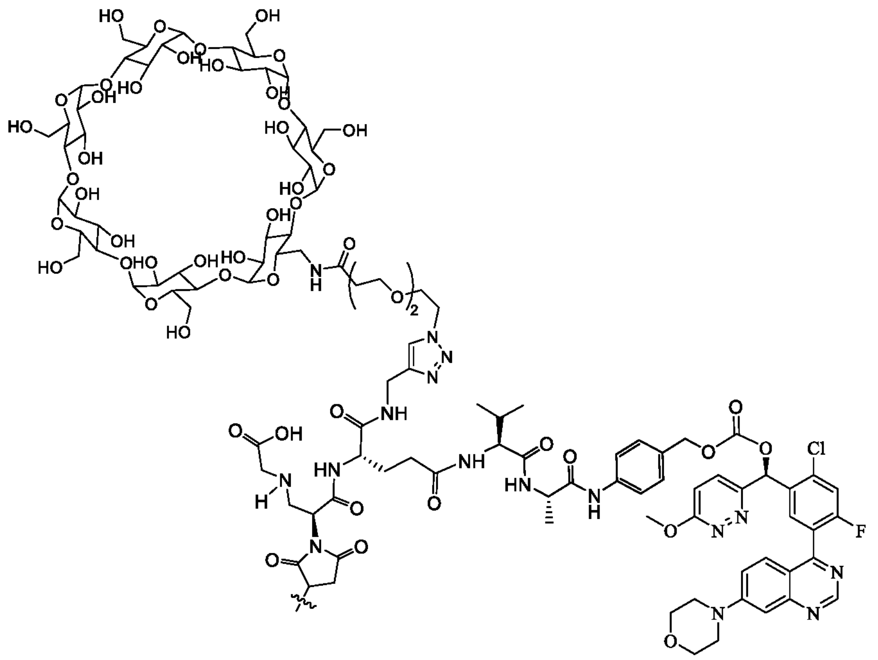 |

5. The ligand-drug conjugate, or a stereoisomer thereof, a prodrug thereof, a pharmaceutically acceptable salt thereof or a pharmaceutically acceptable solvate thereof according to any one of claims 1-4, wherein, the first drug is unrestrictedly selected from the group consisting of a tubulin binding agent, a DNA damage agent, an enzyme inhibitor, an immunomodulator, a peptide and a nucleotide;

preferably, the first drug is selected from the group consisting of:

a) alkylating agents, PBD and its derivatives, chlorpheniramate, chlorpromazine, cyclophosphamide, dacarbazine, estramustine, ifosfamide, mechlorethamine, dimethoxyamine hydrochloride, mechlorethamine oxide, amlodipine hydrochloride, mycophenolic acid, dulcitol, pipobroman, novoembichin, Fenesterin, prednimustine, thiotepa, trofosfamide, uracil; CC-1065; docamycin; benzodiazepine dimer; nitrosourea; alkyl sulfonate; triazene; platinum-containing compounds; aziridines, such as chromanone, quinolone, meturedepa, and madopar; ethyleneimine and methyl melamine including hexamethylmelamine, triethylenetriamine, triethylphosphoramide, triethylenethiophosphoramide and trimethylolmethylamine and derivatives and deuterides thereof;
b) plant alkaloids, vinca alkaloids, taxol and analogs thereof, maytansinoids and analogs thereof, cryptophycin, epothilone, eleutherobin, discodermolide, bryostatin, aplysiatoxin, auristatins, tubulysin, cephalostatin, pancratistatin, sarcodictyin, spongistatin, Monomethyl auristatin E (MMAE), Monomethyl auristatin F (MMAF), Maytansinoid (e.g., Maytansine DM1, Maytansine DM4), amatoxin and derivatives and deuterides thereof;
c) DNA topoisomerase inhibitors, etoposide, teniposide, 9-aminocamptothecin, camptothecin, crisnatol, daunomycin, etoposide phosphate, irinotecan, mitoxantrone, novantrone, retinoic acid, teniposide, topotecan, 9-nitrocamptothecin, SN38, mitomycin and derivatives and deuterides thereof;
d) antimetabolites, antifolates, DHFR inhibitors; MP dehydrogenase inhibitors; ribonucleotide reductase inhibitors; pyrimidine analogs, uracil analogs; cytosine analogs; purine analog; folic acid supplements;
e) hormone therapy agents, receptor antagonists, antiestrogens, LHRH agonists; antiandrogens; retinoids, vitamin D3 analogs; photodynamic therapy agents; cytokines, TNF containing human proteins and derivatives and deuterides thereof;
f) kinase inhibitors, BIBW2992, imatinib, gefitinib, pegaptanib, sorafenib, dasatinib, sunitinib, erlotinib, nilotinib, lapatinib, axitinib, pazopanib, vandetanib, E7080, mubritinib, ponatinib, bafetinib, bosutinib, cabozantinib, vismodegib, iniparib, ruxolitinib, CYT387, axitinib, tivozanib, sorafenib, bevacizumab, cetuximab, trastuzumab, ranibizumab, panitumumab, ispinesib and derivatives and deuterides thereof;
g) antibiotics, enediyne antibiotics, aclacinomysins, anthramycin, amrinomycin, azaserine, bleomycin, cetocycline, kalamycin, carminomycin, carcinophylin, doxorubicin, morpholino doxorubicin, 2-pyrroline doxorubicin and deoxydaunorubicin, epirubicin, aclarubicin, idarubicin, marcomycin, mycin, mycophenolic acid, lopimycin, pelomycin, puromycin, triferricdoxorubicin, streptozotocin, streptozotocin, tubercidin, ubenimex, zinostatin, zorubicin, PNU-159682 and derivatives and deuterides thereof;
h) polyketides, in particular bullatacin and bullatacinone; gemcitabine, epoxomicin, bortezomib, thalidomide, lenalidomide, pomal idomide, tosedostat, zybrestat, PLX4032, STA-9090, Stimuvax, allovectin-7, Xegeva, Provenge, Yervoy, prenylation inhibitors, dopaminergic neurotoxins, actinomycin, bleomycin, anthracycline antibiotics, doxorubicin, idarubicin, epirubicin, larrubicin, zorubicin, mitoxantrone, MDR inhibitors, Ca2+ ATPase inhibitors, histone deacetylase inhibitors, celecoxib, glitazones, epigallocatechin gallate, disulfiram, Salinosporamide A; anti-adrenal agents, such as aminoglutethimide, mitotane, trilostane, aceglatone, aldphosphoramide, aminolevulinic acid, amsacrine, arabinoside, bestrAbuci, bisantrene, edatraxate, defofamine, Meikexin, diaziquone, eflornithine, elfomithine, celiptium, ethylgluconic acid, gallium nitrate, cytosine, hydroxyurea, ibandronate, lentinan, lonidamine, mitoguazone, mitoxantrone, mopidamol, nitracrine, pentostatin, phenamet, pirarubicin, podophyllic acid, 2-ethyl hydrazine, procarbazine; PSK®; razoxane; rhizomycin; sizzo; spirogermanium; tenuazonic acid, triaziquone; trichlorotriethylamine; trichothecene, polyurethanes, siRNA and antisense drugs and derivatives and deuterides thereof;

more preferably, the first drug D1 is a camptothecin-based compound or a deuteride thereof;
preferably, the camptothecin-based compound has a structure represented by formula B,

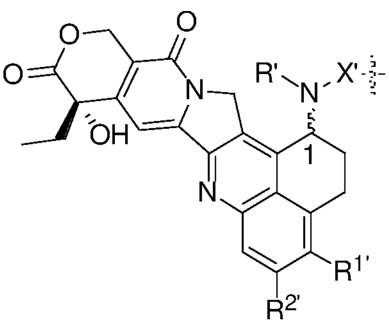

formula B

wherein:

the chiral carbon atom No. 1 linked to the N atom has absolute chirality in either R or S configuration;
R' is selected from the group consisting of H, D, halogen, alkyl, substituted alkyl, deuterated alkyl, cycloalkylalkyl, alkoxyalkyl, aryl, substituted aryl and heteroaryl;
preferably, R' is selected from the group consisting of H, and C1-C6 alkyl;

more preferably, R' is H;
$R^{1'}$ is selected from the group consisting of H, D, halogen, alkyl, substituted alkyl, deuterated alkyl, cycloalkylalkyl, alkoxyalkyl, carboxyl, heterocyclyl, aryl, substituted aryl and heteroaryl;
preferably, $R^{1'}$ is selected from the group consisting of H, and C1-C6 alkyl;
more preferably, $R^{1'}$ is selected from C1-C6 alkyl;
most preferably, $R^{1'}$ is methyl;
$R^{2'}$ is selected from the group consisting of H, D, halogen, alkyl, substituted alkyl, deuterated alkyl, cycloalkylalkyl, alkoxyalkyl, carboxyl, heterocyclyl, aryl, substituted aryl and heteroaryl;
preferably, $R^{2'}$ is selected from halogen;
more preferably, $R^{2'}$ is F;
X' is selected from the group consisting of -C(O)-($CR^{3'}R^{4'}$)t-$CR^{a'}R^{b'}$-O-, -C(O)-($CR^{3'}R^{4'}$)t-$CR^{a'}R^{b'}$-NH- and -C(O)-($CR^{3'}R^{4'}$)t-$CR^{a'}R^{b'}$-S-, preferably, its carbonyl terminal is linked to N atom;
preferably, X' is selected from -C(O)-($CR^{3'}R^{4'}$)t-$CR^{a'}R^{b'}$-O-, preferably, its carbonyl terminal is linked to N atom;
more preferably, X' is selected from -C(O)-$CR^{a'}R^{b'}$-O-, preferably, its carbonyl terminal is linked to N atom;
$R^{a'}$ is selected from the group consisting of H, D, halogen, alkyl, deuterated alkyl, haloalkyl, cycloalkyl, cycloalkylalkyl, alkoxyalkyl, heterocyclyl, aryl, substituted aryl and heteroaryl;
preferably, $R^{a'}$ is selected from the group consisting of H, and C1-C6 alkyl;
more preferably, $R^{a'}$ is H;
$R^{b'}$ is selected from the group consisting of H, D, halogen, alkyl, deuterated alkyl, haloalkyl, cycloalkyl, cycloalkylalkyl, alkoxyalkyl, heterocyclyl, aryl, substituted aryl and heteroaryl;
preferably, $R^{b'}$ is selected from the group consisting of H, D, C1-C6 alkyl, C3-C6 cycloalkyl, and C1-C6 haloalkyl;
more preferably, $R^{b'}$ is selected from the group consisting of H, D, methyl, cyclopropyl, and trifluoromethyl;
or, $R^{a'}$, $R^{b'}$ and the carbon atom they are linked to form C3-C6 cycloalkyl, cycloalkylalkyl or heterocyclyl;
$R^{3'}$, $R^{4'}$, the same or different, each are independently selected from the group consisting of H, D, halogen, alkyl, haloalkyl, deuterated alkyl, alkoxy, hydroxy, amino, cyano, nitro, hydroxyalkyl, cycloalkyl and heterocyclyl;
or, $R^{3'}$, $R^{4'}$ and the carbon atom they are linked to form C3-C6 cycloalkyl, cycloalkylalkyl or heterocyclyl;
the right wavy line linked to X' is linked to $L_1$;
t is an integer selected from 0 to 4;
preferably, t is 0;
preferably, X' is unrestrictedly selected from the group consisting of the following structures:

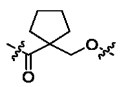, 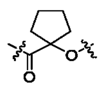, 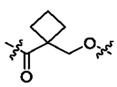, 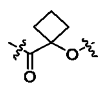, 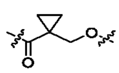, 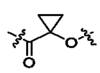,

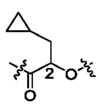, 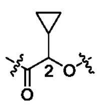, 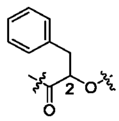, 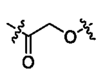, 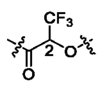, 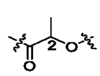 and 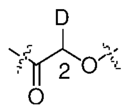;

wherein:

the chiral carbon atom No. 2 has absolute chirality in either R or S configuration;
the left wavy line is linked to N of the compound represented by formula B, and the right wavy line is linked to $L_1$;
preferably, the compound represented by formula B is selected from the group consisting of

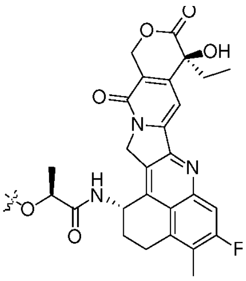, 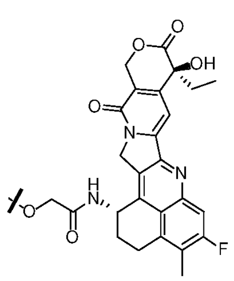, 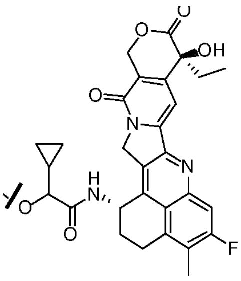, 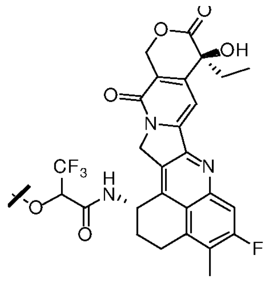,

and

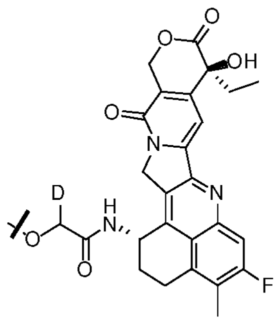;

preferably, the first drug is selected from the group consisting of

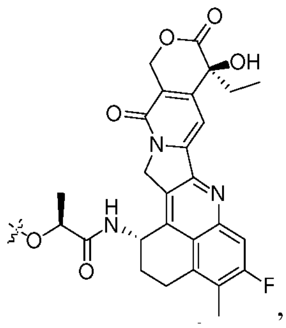,

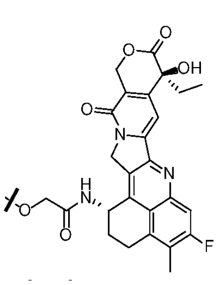, 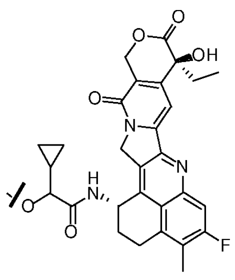, 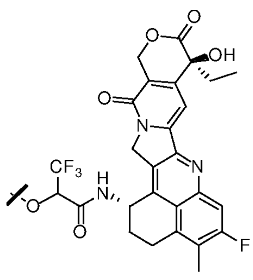, 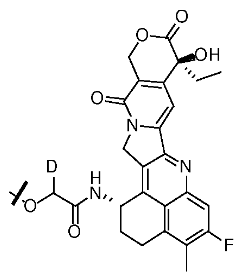,

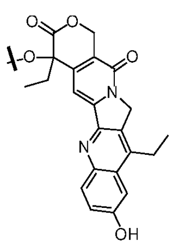, 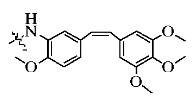, 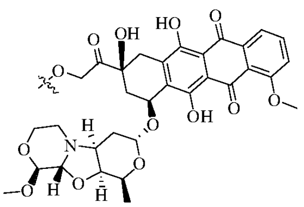, 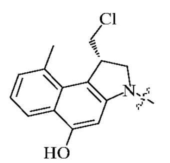,

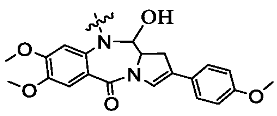,

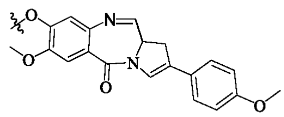,

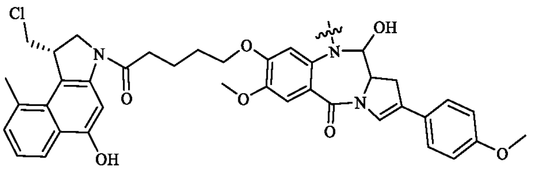,

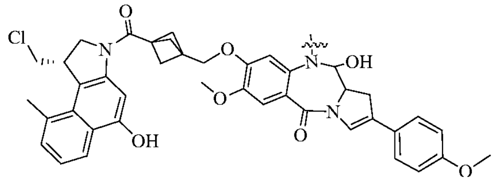,

and

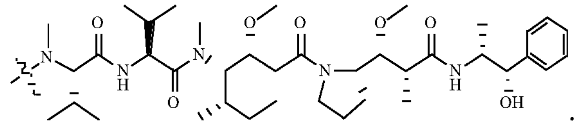.

**6.** The ligand-drug conjugate, or a stereoisomer thereof, a prodrug thereof, a pharmaceutically acceptable salt thereof or a pharmaceutically acceptable solvate thereof according to any one of claims 1-5, wherein, the first linking unit is a breakable or non-breakable linking unit;

preferably, $L_1$ is $L_{11}$-$L_{12}$-$L_{13}$-$L_{14}$, and $L_{14}$ is linked to $D_1$, $L_{11}$ is linked to Ab (preferably, $L_{11}$ is coupled to Ab via thiol group),

$L_{11}$ is selected from the group consisting of

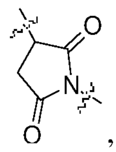,

and

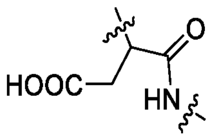,

with its N terminal linked to $L_{12}$,

$L_{12}$ is selected from the group consisting of:

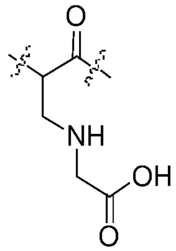

,

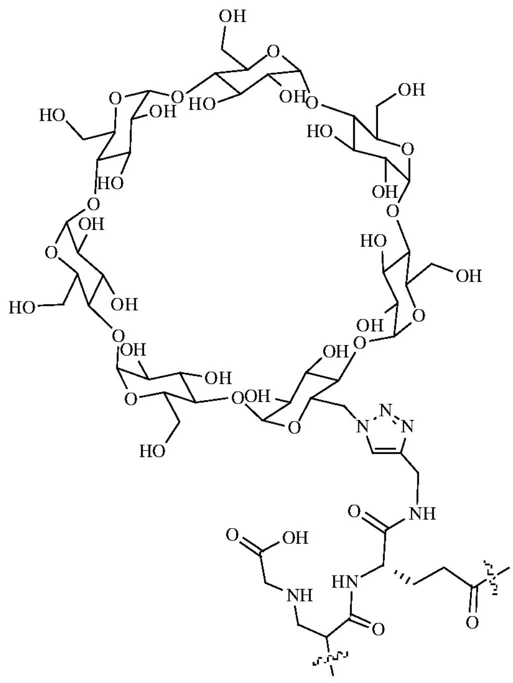

,

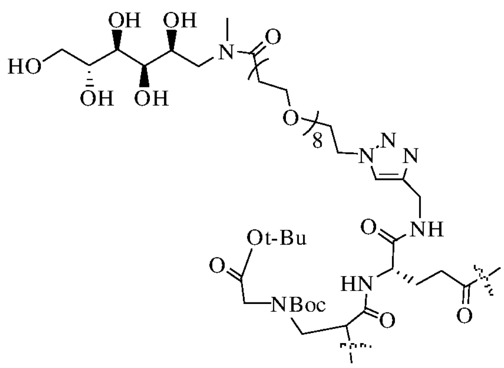

,

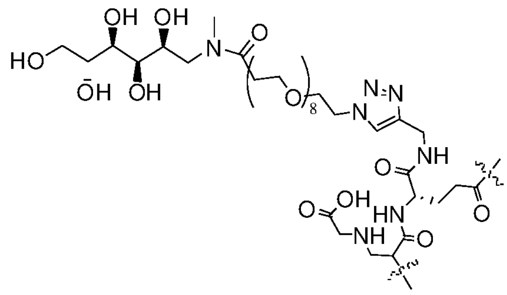

,

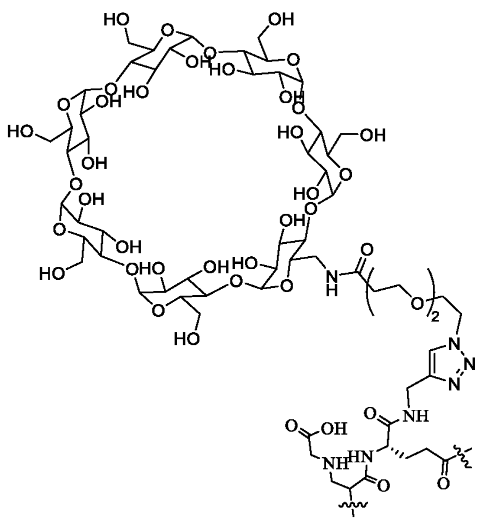

,

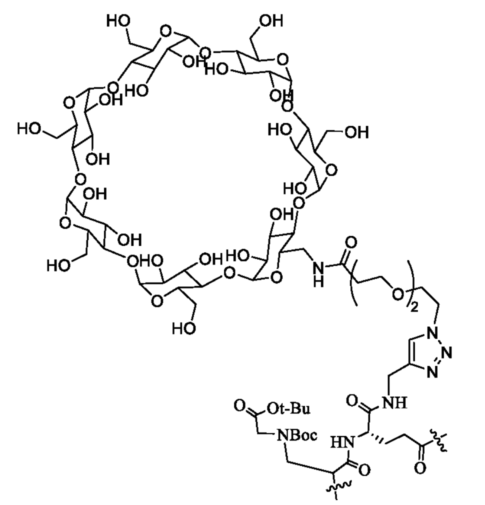

,

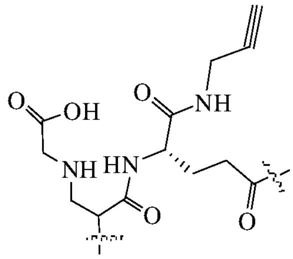

,

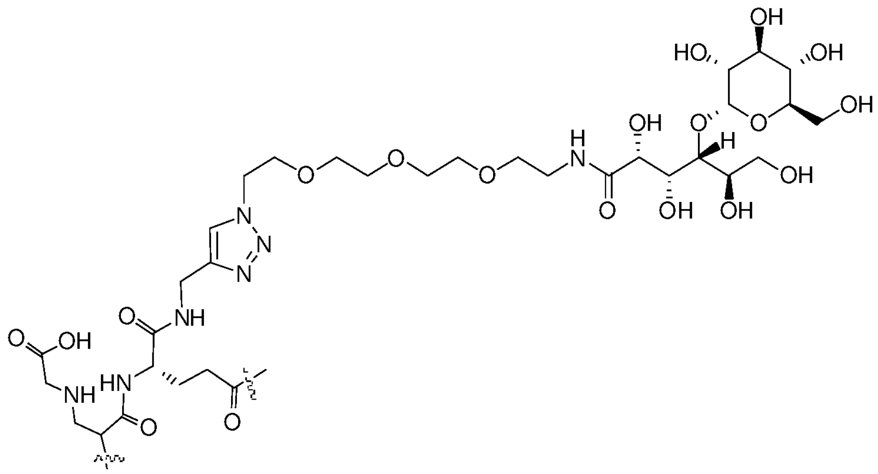

,

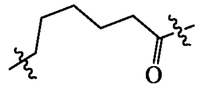

,

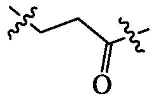

,

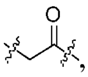

,

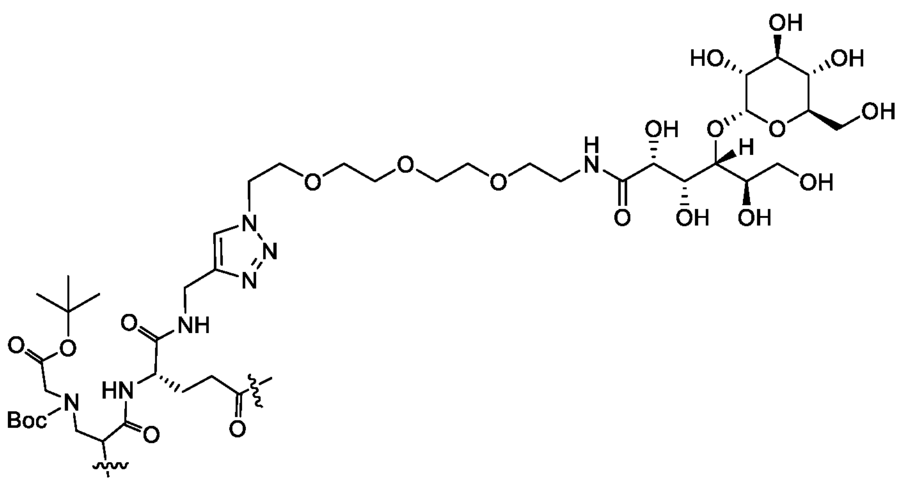

,

and

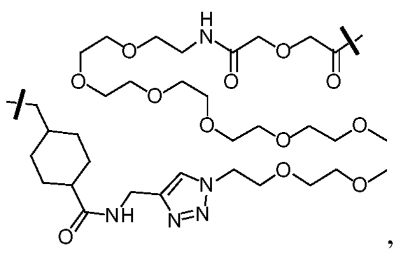

,

with its carbonyl terminal linked to $L_{13}$ or $L_{14}$;

$L_{13}$ is selected from a direct bond, an amino acid residue or a peptide residue consisting of 2-10 amino acid residues, and when $L_{13}$ is selected from an amino acid residue or a peptide residue consisting of 2-10 amino acid residues, its carbonyl terminal is linked to $L_{14}$;

preferably, $L_{13}$ is selected from a direct bond, an amino acid residue, a peptide residue consisting of 2-4 amino acid residues, and when $L_{13}$ is selected from an amino acid residue, or a peptide residue consisting of 2-4 amino acid residues, its carbonyl terminal is linked to $L_{14}$, preferably, the amino acid is selected from the group consisting of valine, alanine, phenylalanine, glycine, lysine, citrulline, serine, glutamic acid, and aspartic acid, more preferably, the amino acid is selected from the group consisting of valine, alanine, phenylalanine, glycine, citrulline, and lysine;

more preferably, $L_{13}$ is selected from the group consisting of a direct bond, lysine, glycine-glycine-phenylalanine-glycine (Gly-Gly-Phe-Gly), valine-alanine (Val-Ala), and valine-citrulline (Val-Cit), and when $L_{13}$ is selected from the group consisting of lysine, glycine-glycine-phenylalanine-glycine (Gly-Gly-Phe-Gly), valine-alanine (Val-Ala), and valine-citrulline (Val-Cit), its carbonyl terminal is linked to $L_{14}$;

most preferably, $L_{13}$ is selected from the group consisting of a direct bond,

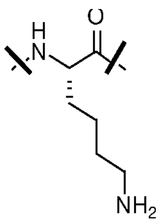,

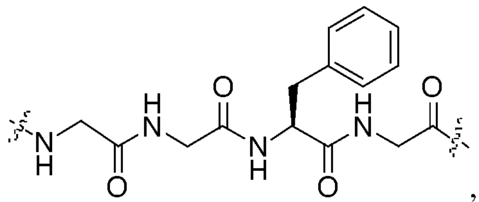, 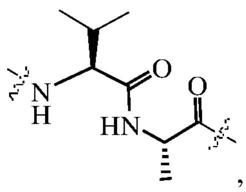, and 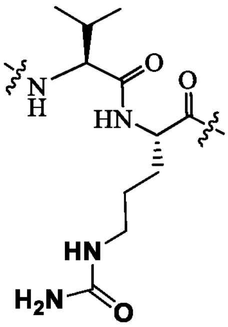,

and when $L_{13}$ is selected from the group consisting of

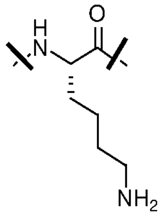, 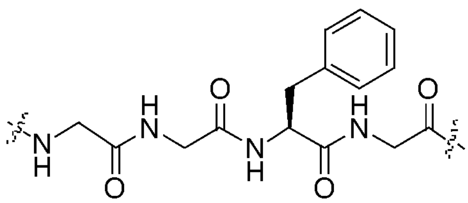,

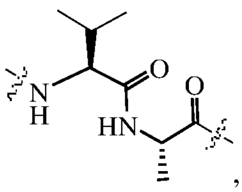, and 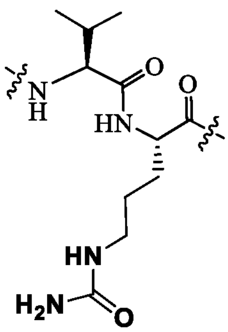,

its carbonyl terminal is linked to $L_{14}$;
$L_{14}$ is selected from the group consisting of

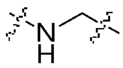, 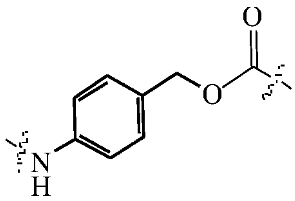,

and

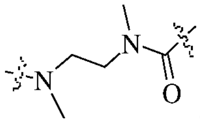,

with its amino terminal linked to $L_{13}$ or $L_{12}$, and its carbonyl terminal or carbon terminal linked to $D_1$;
preferably, $L_1$ is selected from the group consisting of:

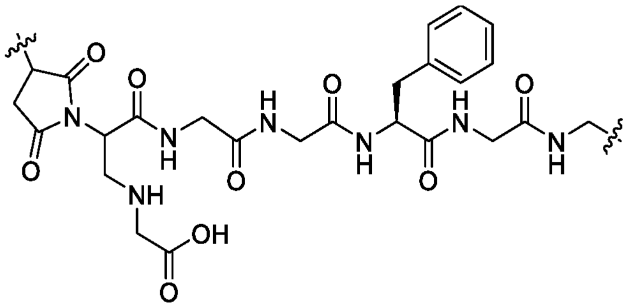,

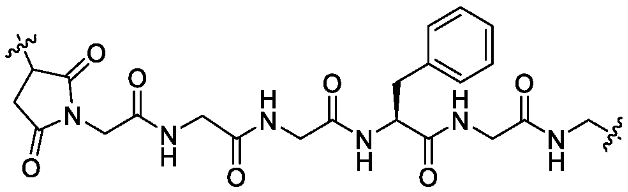,

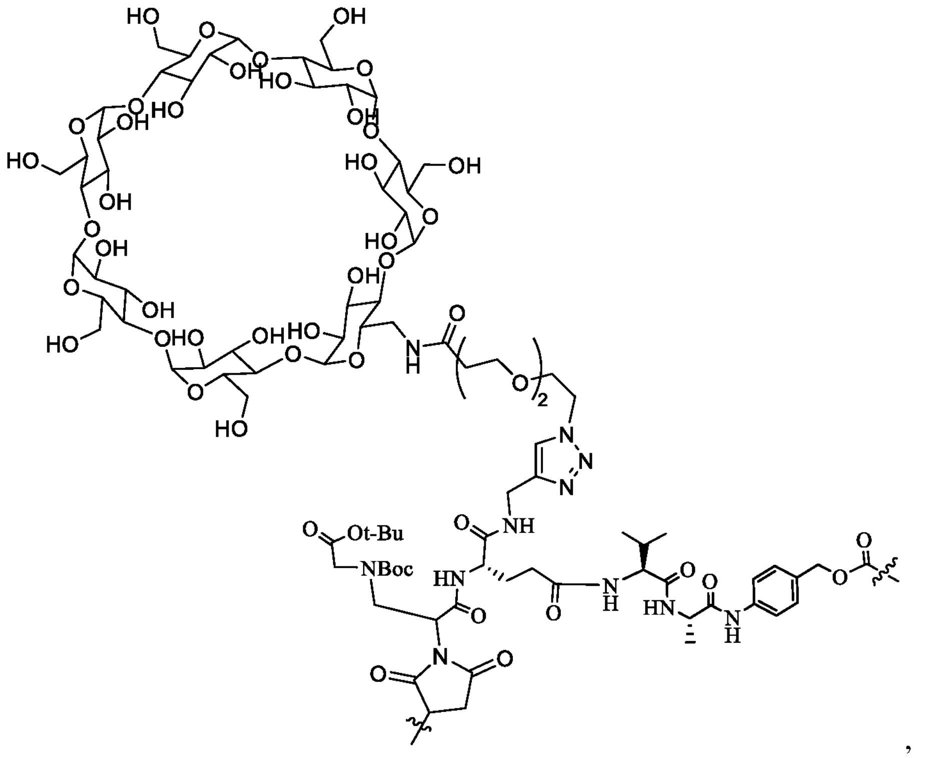

,

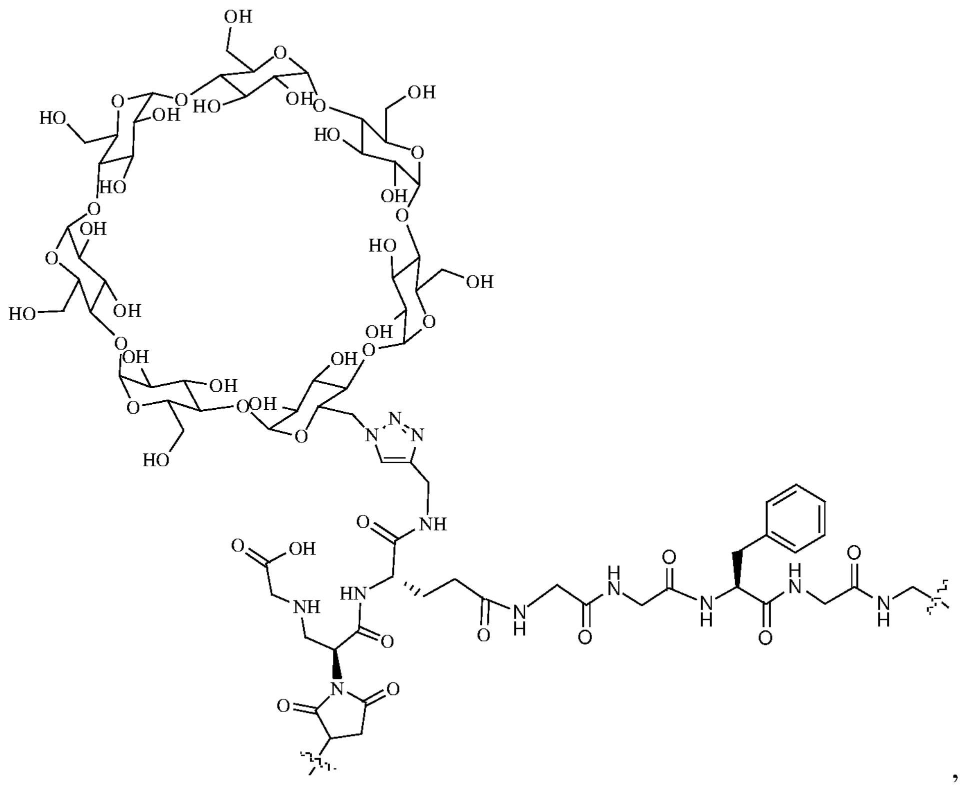

,

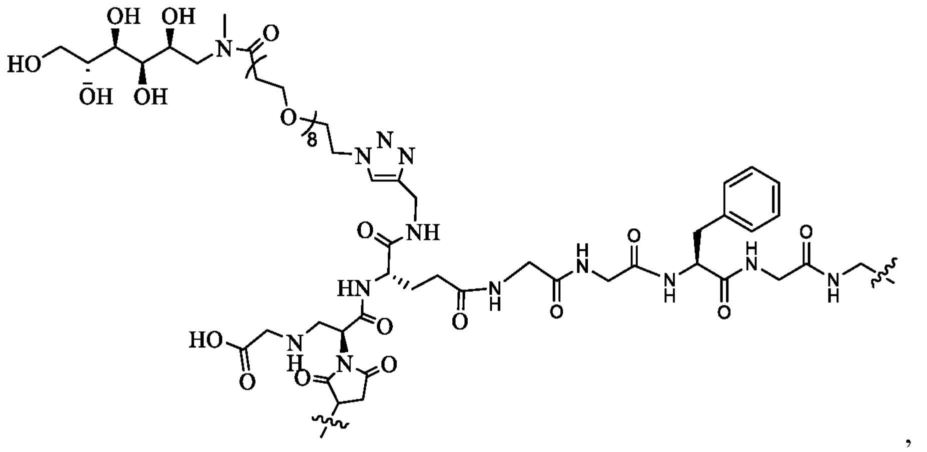

,

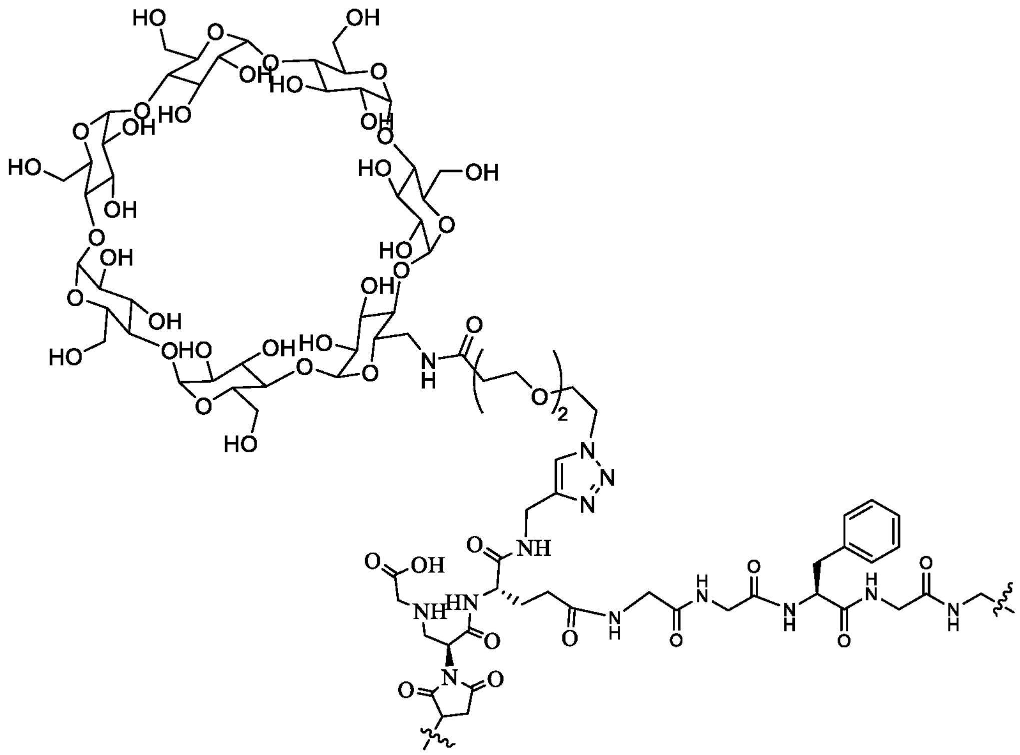

,

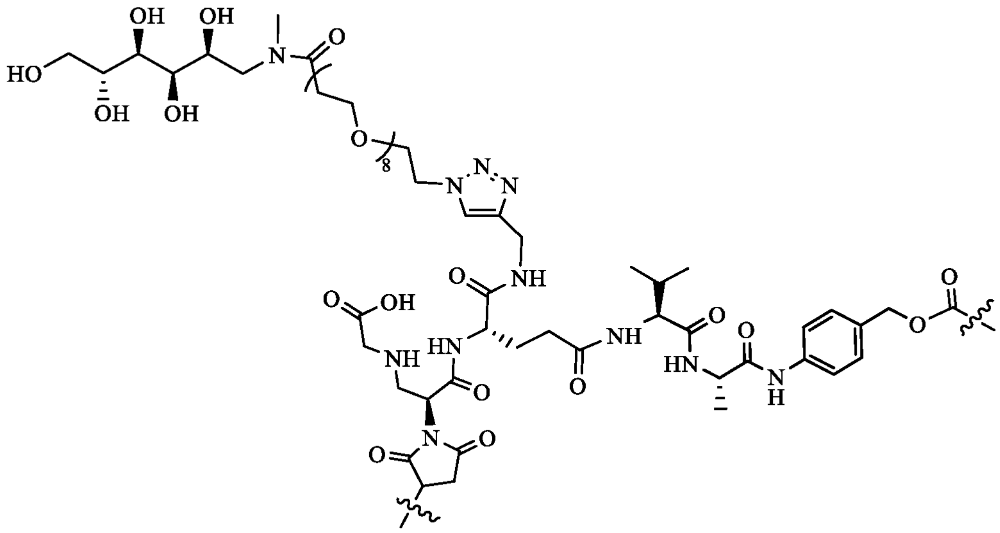

,

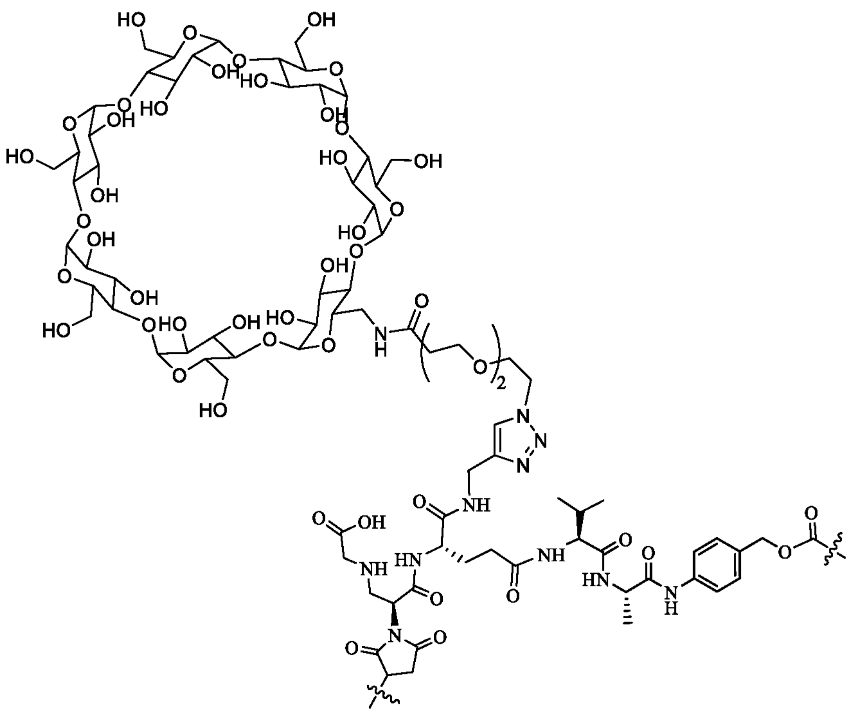

,

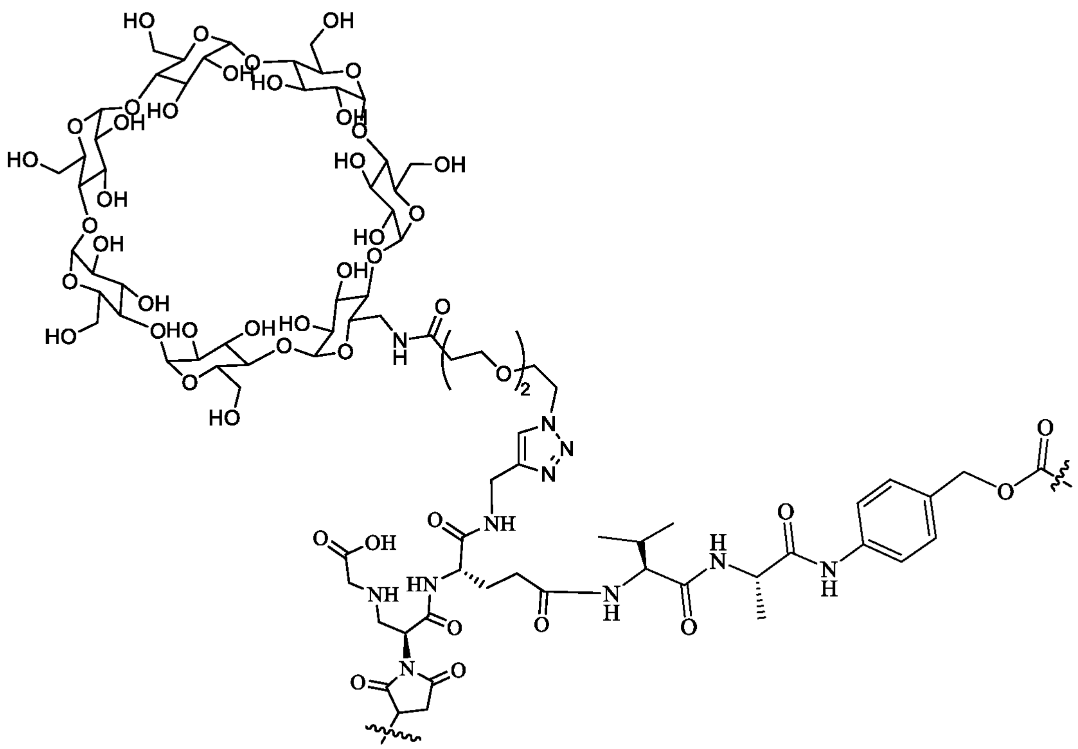

,

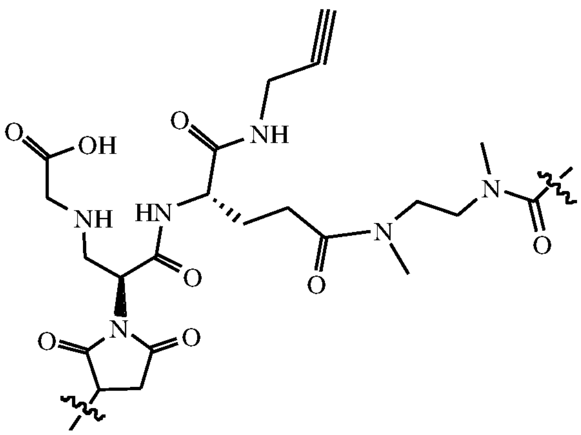

,

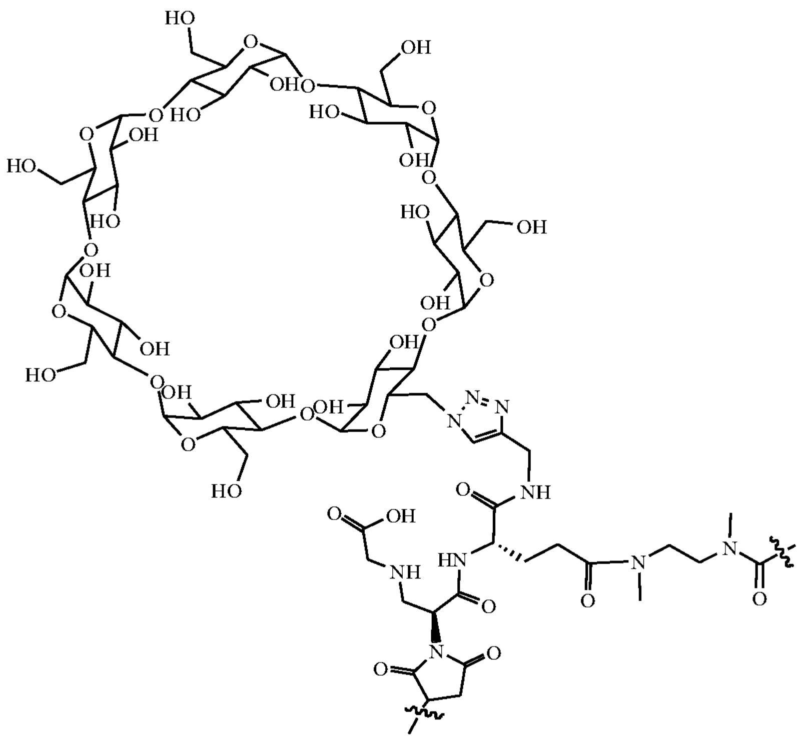

,

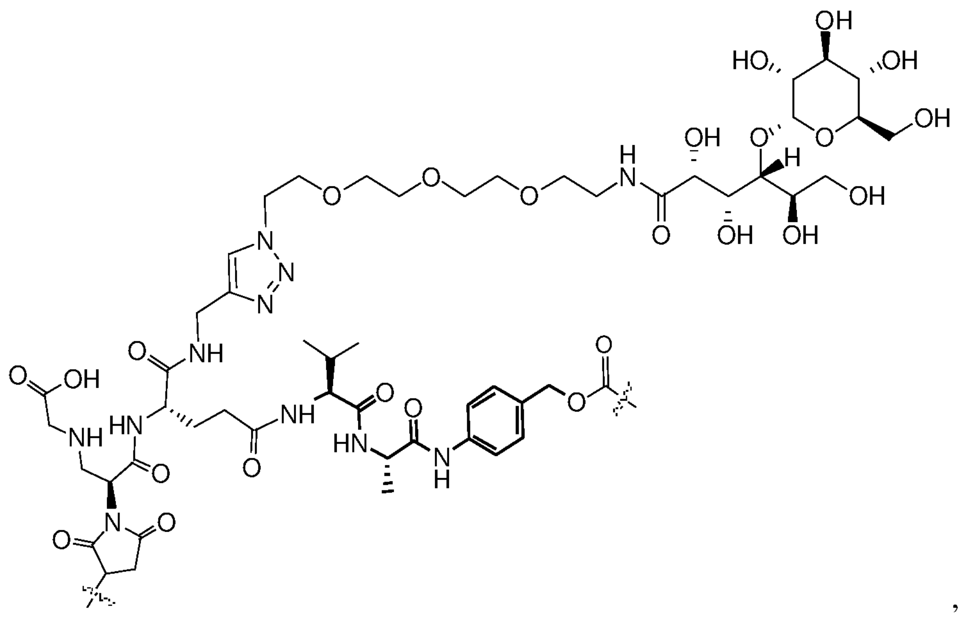

,

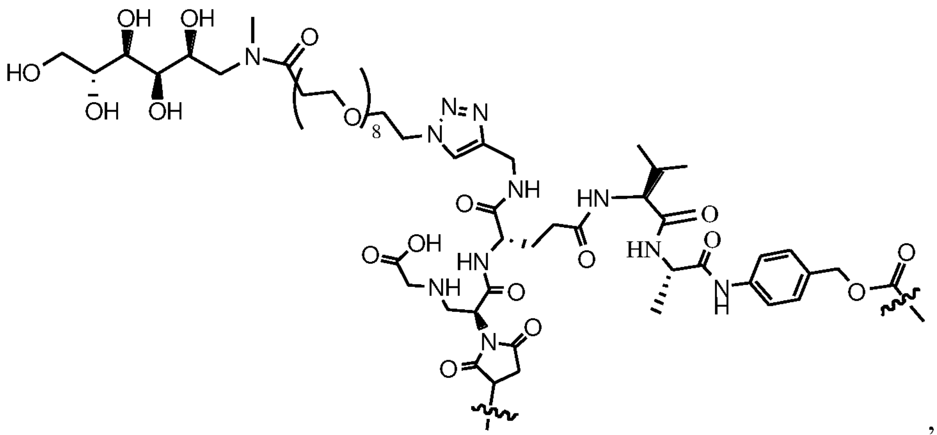

,

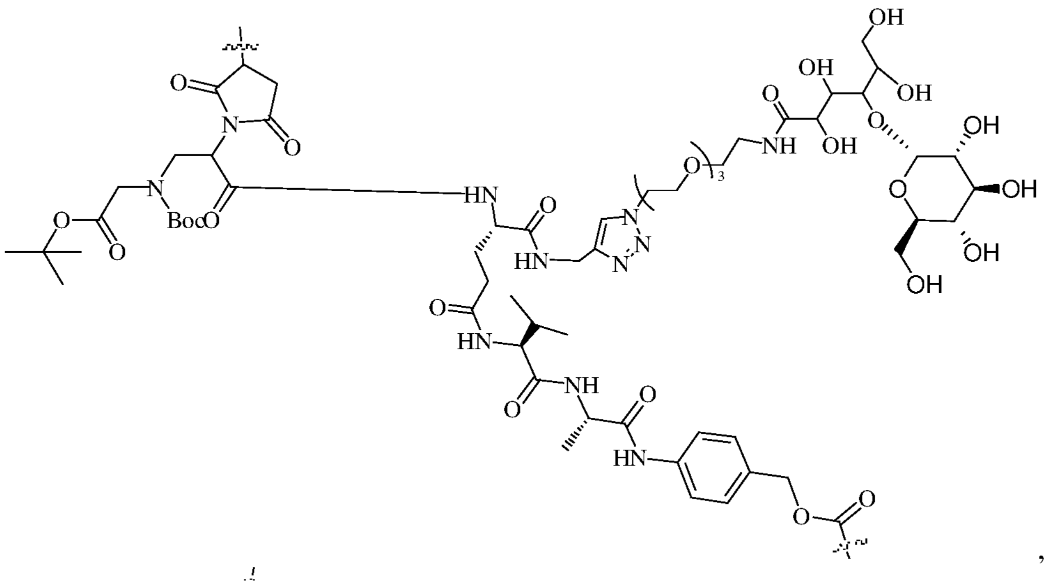

,

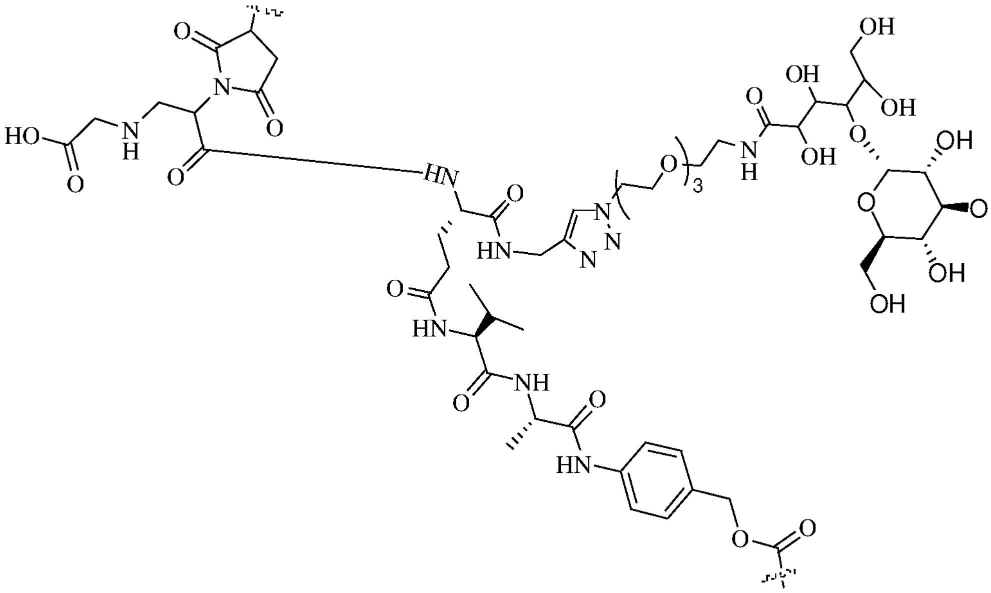

,

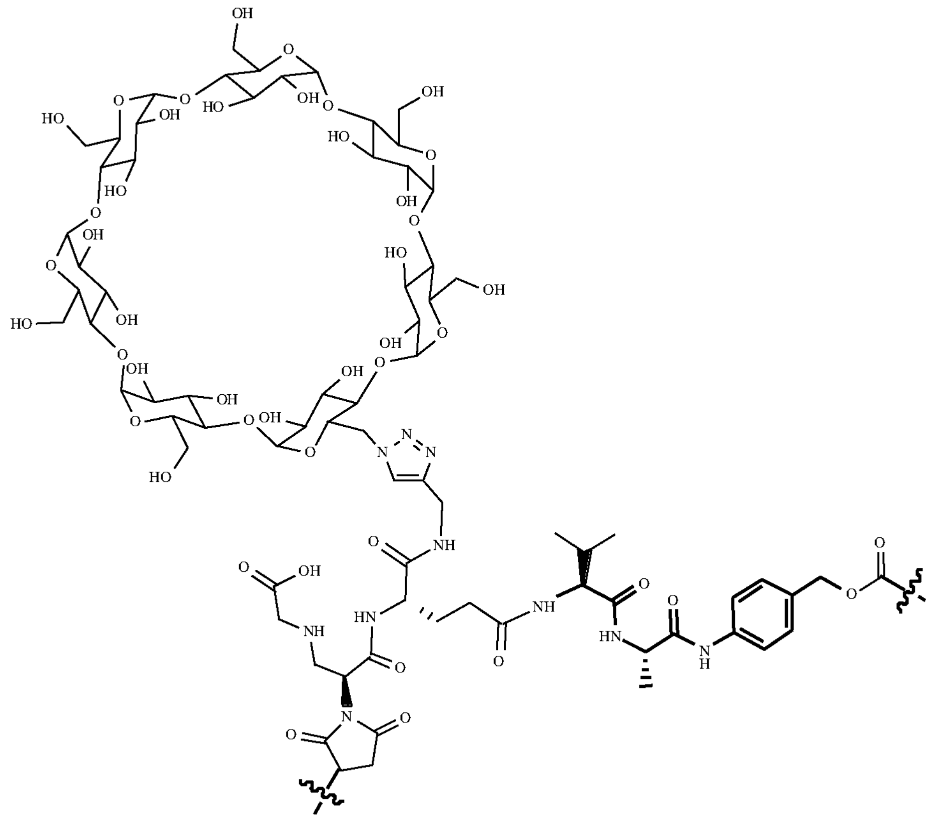

,

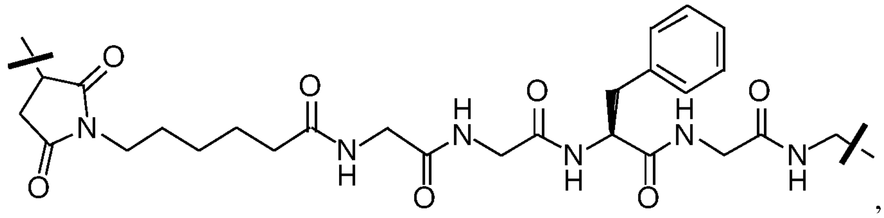

,

and

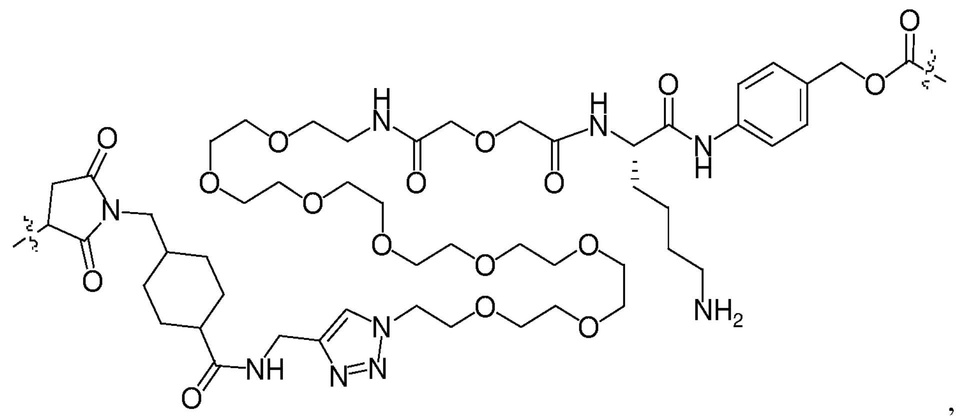

,

with its carbonyl terminal or carbon terminal of the end containing

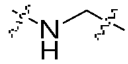

linked to $D_1$.

**7.** The ligand-drug conjugate, or a stereoisomer thereof, a prodrug thereof, a pharmaceutically acceptable salt thereof or a pharmaceutically acceptable solvate thereof according to any one of claims 1-6, wherein, $D_1$-$L_1$- is selected from the group consisting of:

| | |
|---|---|
| Ex01 | 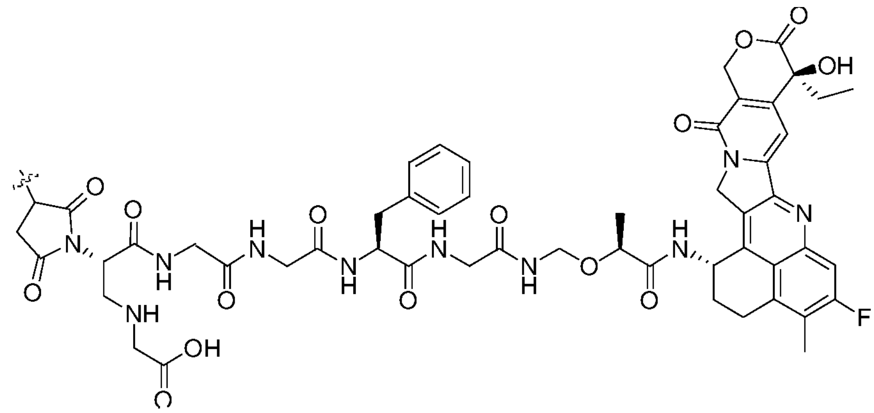 |
| Ex02 | 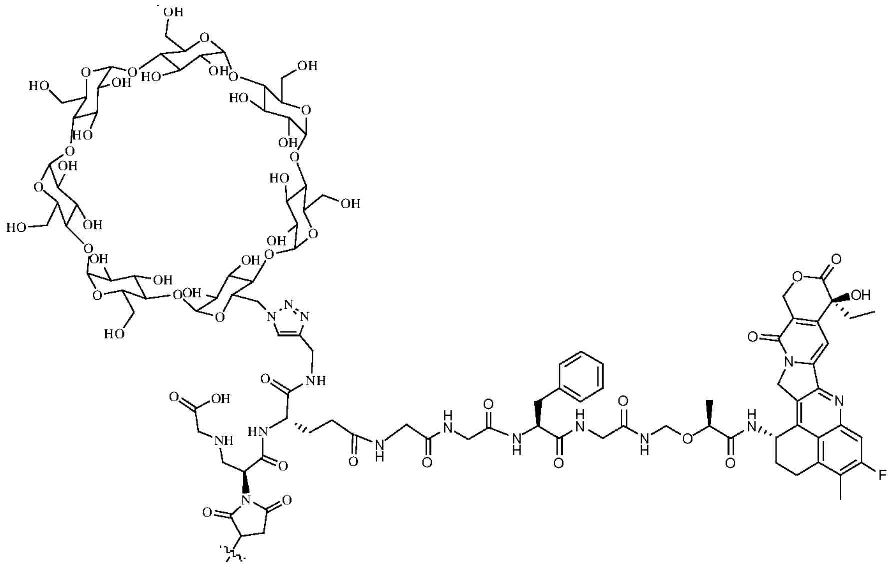 |
| Ex03 | 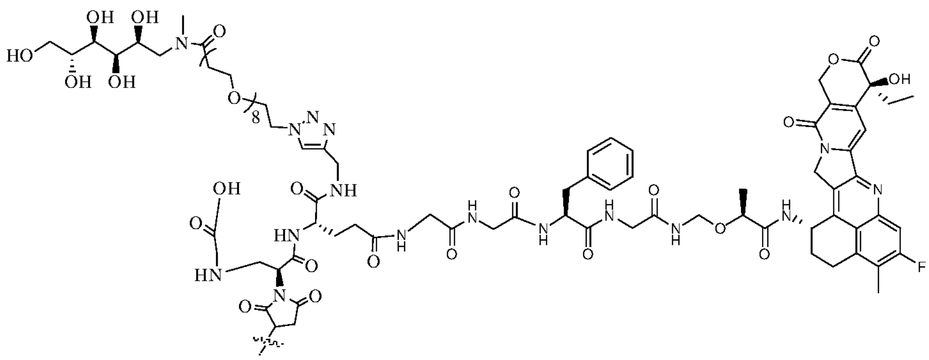 |

(continued)

| | |
|---|---|
| Ex04 | 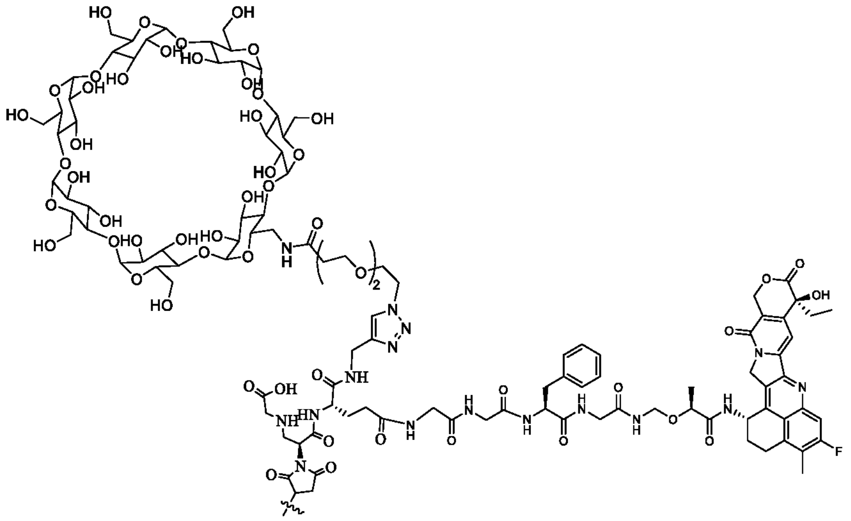 |
| Ex05 | 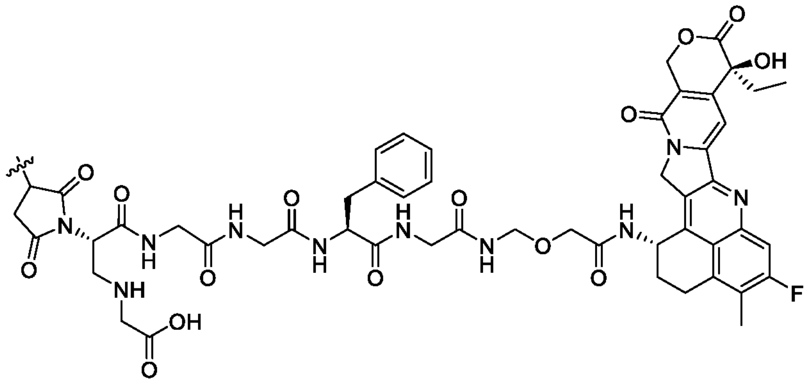 |
| Ex06 | 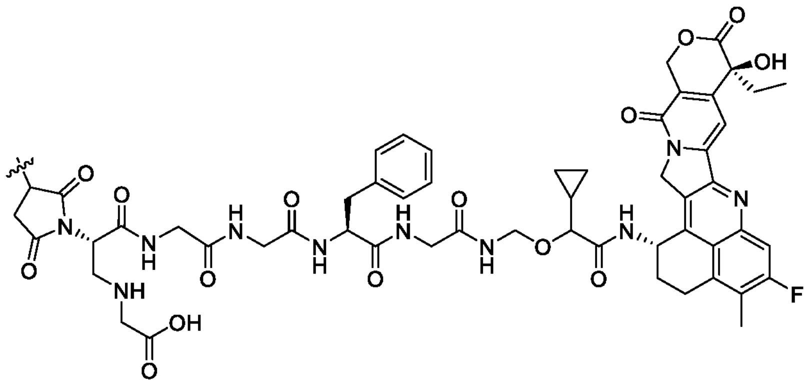 |
| Ex07 | 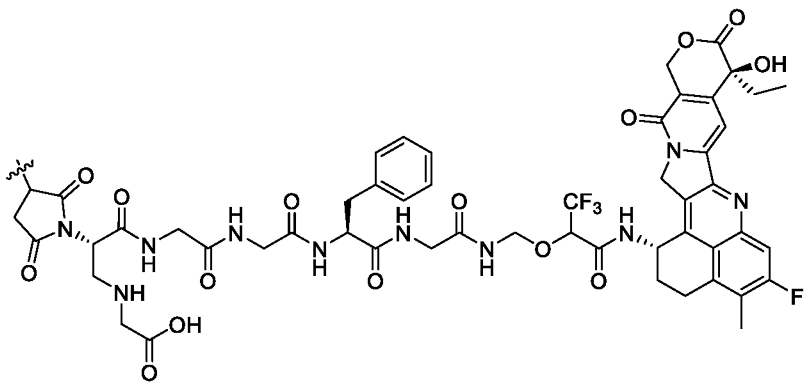 |

(continued)

| | |
|---|---|
| Ex08 | 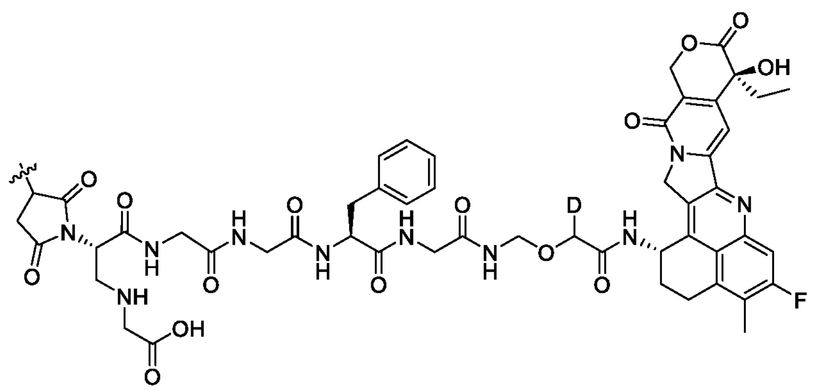 |
| Ex09 | 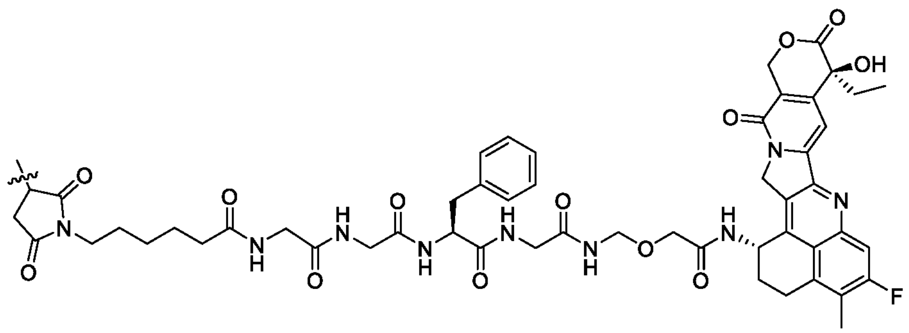 |
| SN01 | 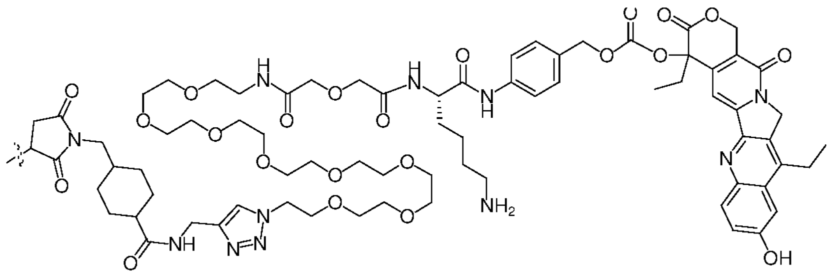 |
| PB01 | 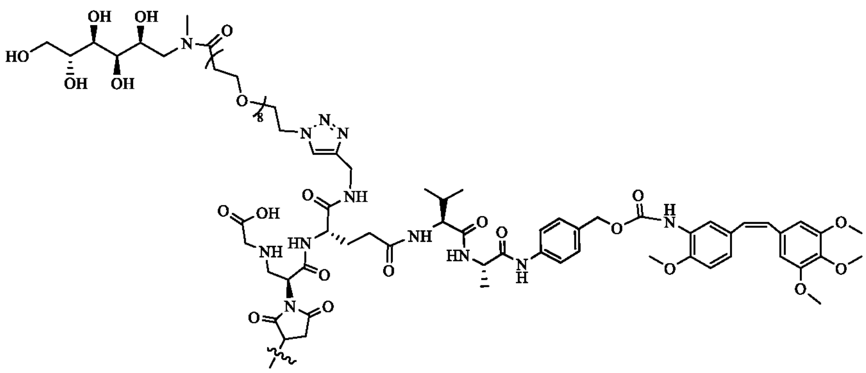 |

(continued)

| | |
|---|---|
| PB02 | 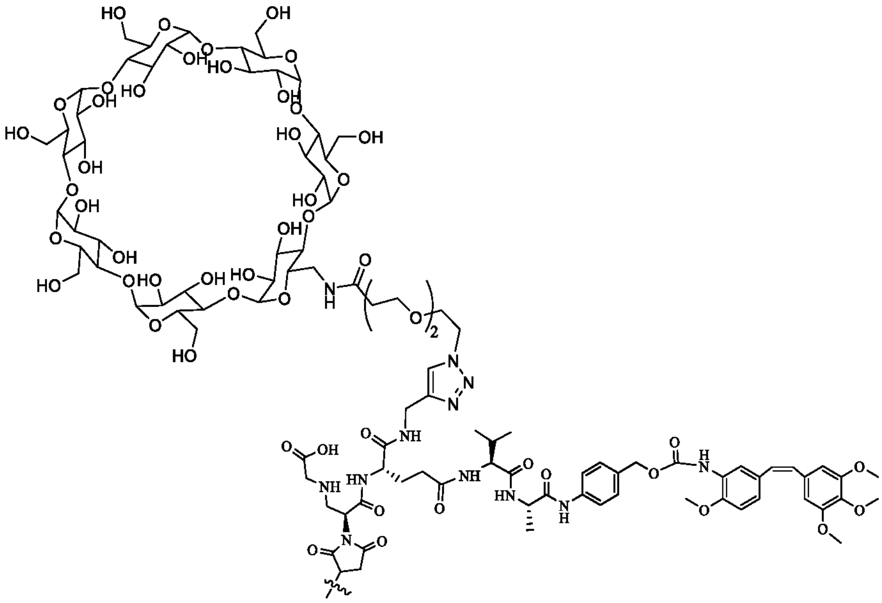 |
| PNU01 | 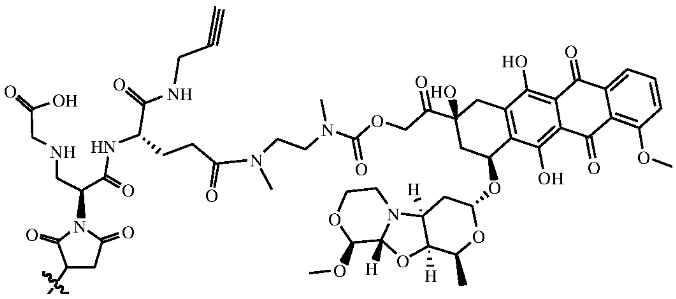 |
| PNU02 | 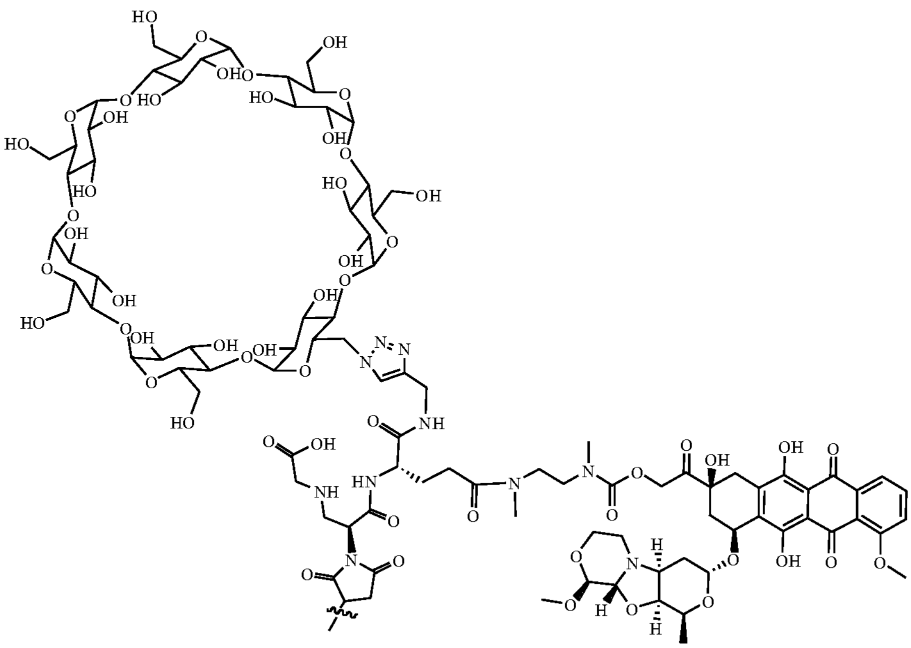 |

(continued)

| | |
|---|---|
| CBI01 | 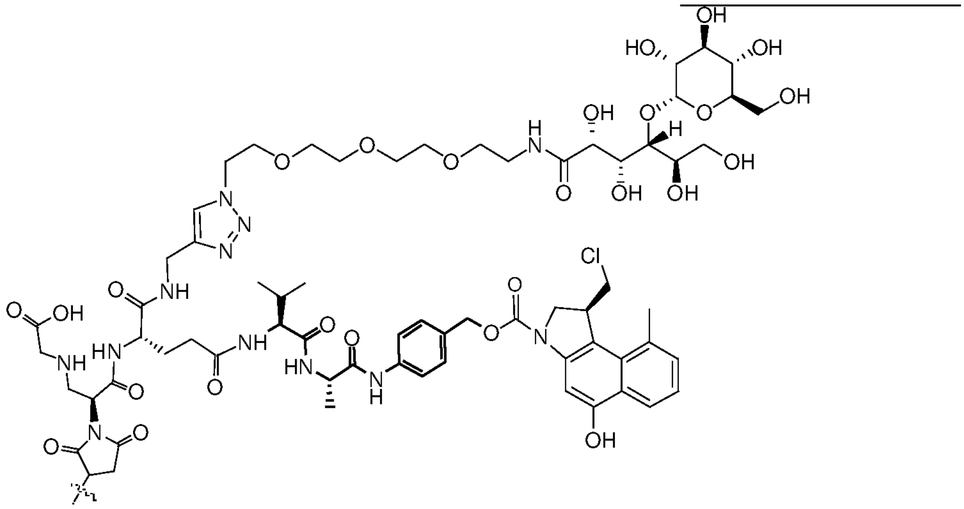 |
| CBI02 | 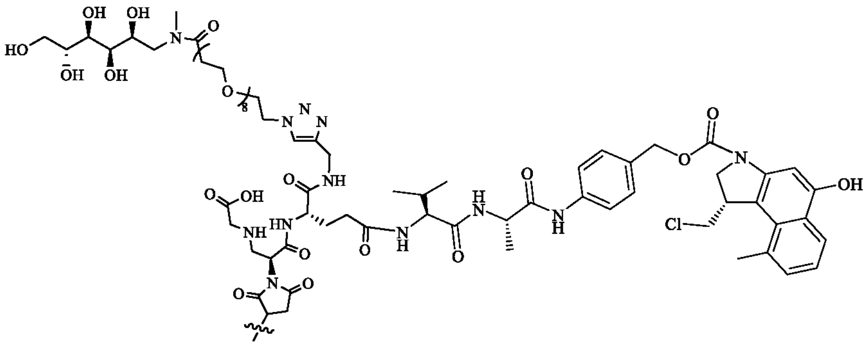 |
| CBI03 | 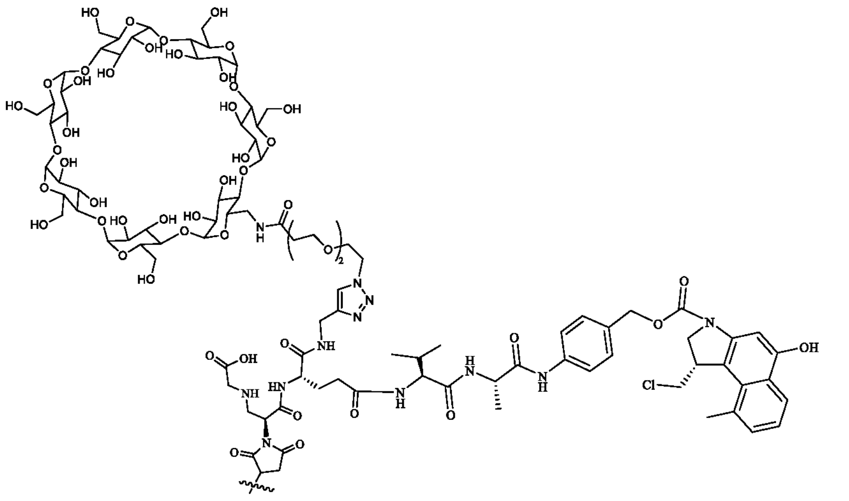 |
| PBD01 | 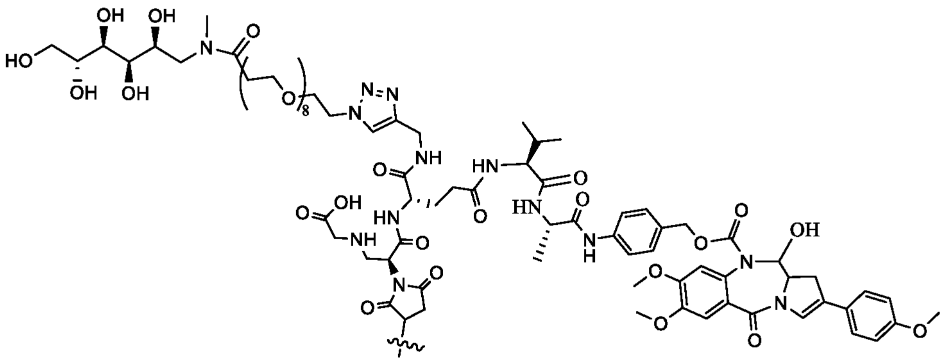 |

(continued)

| | |
|---|---|
| PBD02 | 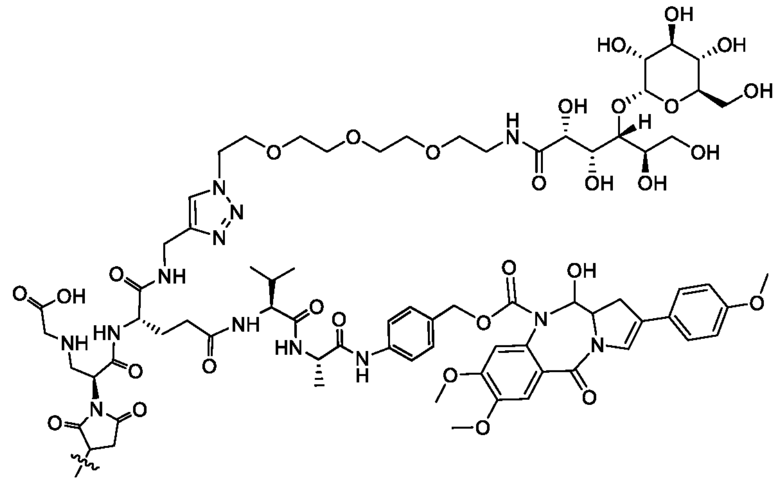 |
| PBD-CBI01 | 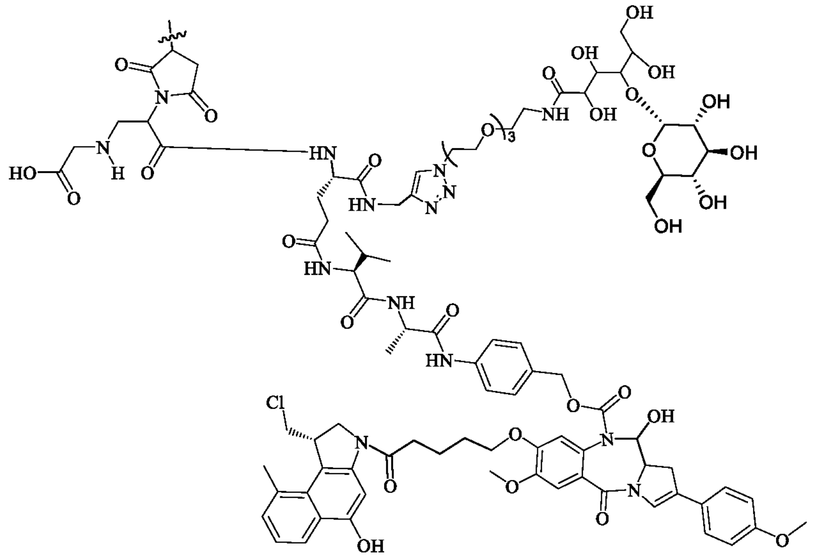 |
| PBD-CBI02 | 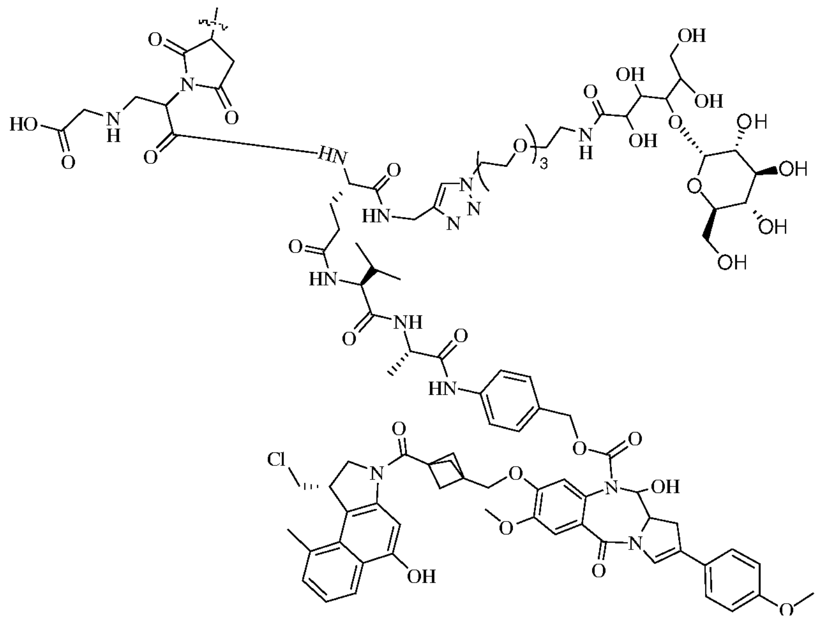 |

(continued)

| | |
|---|---|
| M01 | 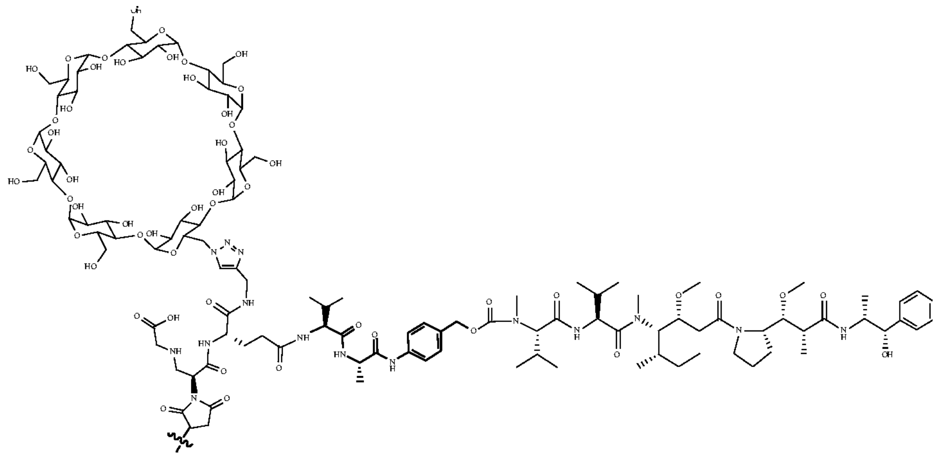 |
| M02 | 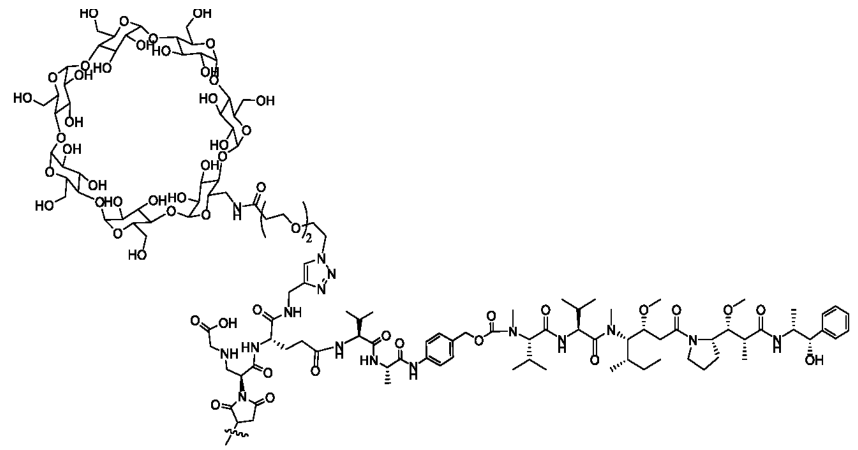 |
| M03 . | 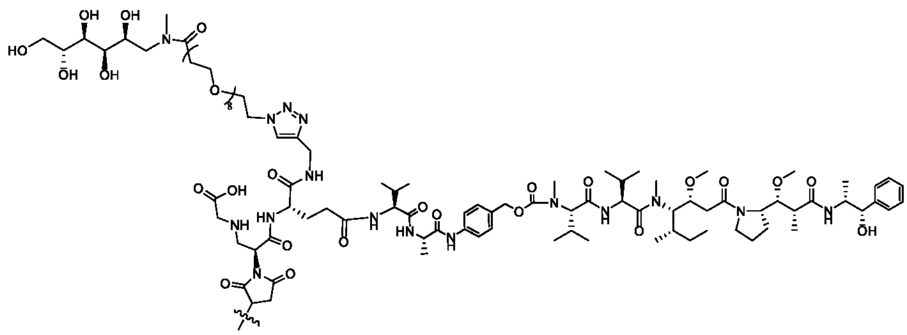 |

8. The ligand-drug conjugate, or a stereoisomer thereof, a prodrug thereof, a pharmaceutically acceptable salt thereof or a pharmaceutically acceptable solvate thereof according to any one of claims 1-7, wherein, m is an integer selected from 1 to 10;

   preferably, m is an integer selected from 2 to 8;
   more preferably, m is selected from the group consisting of 2, 4, and 8;
   or, n is an integer selected from 1 to 10;
   or preferably, n is an integer selected from 2 to 8;
   or more preferably, n is selected from the group consisting of 2, 4, and 8.

9. The ligand-drug conjugate, or a stereoisomer thereof, a prodrug thereof, a pharmaceutically acceptable salt thereof or a pharmaceutically acceptable solvate thereof according to any one of claims 1-8, wherein, Ab is selected from the group consisting of murine antibodies, chimeric antibodies, humanized antibodies, fully humanized antibodies, antibody fragments, bispecific antibodies, and multispecific antibodies, preferably, Ab forms a linking bond with the

linking unit via its cysteine thiol group (-SH);

preferably, Ab is an antibody, which is a monoclonal antibody selected from the group consisting of: anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-cladin 18.2 antibody, anti-Mesothelin antibody, anti-HER2(ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody and anti-CD123 antibody.

**10.** The ligand-drug conjugate, or a stereoisomer thereof, a prodrug thereof, a pharmaceutically acceptable salt thereof or a pharmaceutically acceptable solvate thereof according to any one of claims 1-9, wherein, $D_1$-$L_1$- and $D_2$-$L_2$- both are coupled to Ab via thiol group;

preferably, the source of the thiol group is selected from the group consisting of: the thiol group on a cysteine residue formed after reduction of an existing interchain disulfide bond in the targeting moiety, the thiol group on a cysteine residue formed by site-directed mutagenesis of certain original amino acids in the targeting moiety, the thiol group on a cysteine residue formed by site-directed insertion of a cysteine into the targeting moiety, and the thiol group on a cysteine residue formed by site-directed insertion of a cysteine-containing peptide into the targeting moiety;

preferably, $D_1$-$L_1$- and $D_2$-$L_2$- are respectively coupled to Ab via thiol groups from different sources;

preferably, any one of $D_1$-$L_1$- and $D_2$-$L_2$- is coupled to Ab via the thiol group on a cysteine residue formed after reduction of an existing interchain disulfide bond in the targeting moiety, and the other is coupled to Ab via the thiol group on a cysteine residue formed by site-directed mutagenesis of certain original amino acids in the targeting moiety, the thiol group on a cysteine residue formed by site-directed insertion of a cysteine into the targeting moiety, or the thiol group on a cysteine residue formed by site-directed insertion of a cysteine-containing peptide into the targeting moiety.

**11.** The ligand-drug conjugate, or a stereoisomer thereof, a prodrug thereof, a pharmaceutically acceptable salt thereof or a pharmaceutically acceptable solvate thereof according to any one of claims 1-10, wherein, the ligand-drug conjugate is selected from the group consisting of:

Ab-R01 (2) -Ex01 (8),
Ab-R01 (8) -Ex01 (2),
Ab-R01 (4) -Ex01 (4),
Ab-R01 (4) -Ex01 (8),
Ab-R01 (8) -Ex01 (4),
Ab-R01 (2) -Ex02 (8),
Ab-R01 (8) -Ex02 (2),
Ab-R01 (4) -Ex02 (4),
Ab-R01 (2) -Ex03 (8),
Ab-R01 (8) -Ex03 (2),
Ab-R01 (4) -Ex03 (4),
Ab-R01 (2) -Ex04 (8),
Ab-R01 (8) -Ex04 (2),
Ab-R01 (4) -Ex04 (4),
Ab-R01 (2) -Ex05 (8),
Ab-R01 (8) -Ex05 (2),
Ab-R01 (4) -Ex05 (4),
Ab-R01 (2) -Ex06 (8),
Ab-R01 (8) -Ex06 (2),

Ab-R01 (4) -Ex06 (4),
Ab-R01-1 (2) -Ex01 (8),
Ab-R01-1 (8) -Ex01 (2),
Ab-R01-1 (4) -Ex01 (4),
Ab-R01-1 (8) -Ex01 (4),
Ab-R01-1 (4) -Ex01 (8),
Ab-R01-1 (2) -Ex02 (8),
Ab-R01-1 (8) -Ex02 (2),
Ab-R01-1 (4) -Ex02 (4),
Ab-R01-1 (2) -Ex03 (8),
Ab-R01-1 (8) -Ex03 (2),
Ab-R01-1 (4) -Ex03 (4),
Ab-R01-1 (2) -Ex04 (8),
Ab-R01-1 (8) -Ex04 (2),
Ab-R01-1 (4) -Ex04 (4),
Ab-R01-1 (2) -Ex05 (8),
Ab-R01-1 (8) -Ex05 (2),
Ab-R01-1 (4) -Ex05 (4),
Ab-R01-1 (2) -Ex06 (8),
Ab-R01-1 (8) -Ex06 (2),
Ab-R01-1 (4) -Ex06 (4),
Ab-R01-1 (2) -PB01 (8),
Ab-R01-1 (2) -PB02 (8),
Ab-R01-1 (8) -PNU01 (2),
Ab-R01-1 (8) -PNU02 (2),
Ab-R01-1 (2) - CBI01 (8),
Ab-R01-1 (2) - CBI02 (8),
Ab-R01-1 (2) - CBI03 (8),
Ab-R01-1 (2) -PBD01 (8),
Ab-R01-1 (2) -PBD02 (8),
Ab-R01-1 (2) -PBD-CBI01 (8),
Ab-R01-1 (2) -PBD-CBI02 (8),
Ab-R01-1 (8) -M01 (2),
Ab-R01-1 (8) -M02 (2),
Ab-R01-1 (8) -M03 (2),
Ab-AT01 (2) -Ex01 (8),
Ab-AT02 (2) -Ex01 (8),
Ab-AT03 (2) -Ex01 (8),
Ab-AT04 (2) -Ex01 (8),
Ab-TA01 (2) -Ex01 (8),
Ab-VE01 (2) -Ex01 (8),
Ab-E01 (2) -Ex01 (8),
Ab-RU01 (2) -Ex01 (8),
Ab-PA01 (2) -Ex01 (8),
Ab-CEP01 (2) -Ex01 (8),
Ab-TM01 (2) -Ex01 (8),
Ab-TM02 (2) -Ex01 (8),
Ab-WE01 (2) -Ex01 (8),
Ab-CH01 (2) -Ex01 (8),
Ab-CH02 (2) -Ex01 (8),
Ab-CH03 (2) -Ex01 (8),
Ab-CH04 (2) -Ex01 (8),
Ab-DP01 (2) -Ex01 (8),
Ab-DP02 (2) -Ex01 (8), and
Ab-DP03 (2) -Ex01 (8).

**12.** A drug-linking unit conjugate, or a stereoisomer thereof, a prodrug thereof, a pharmaceutically acceptable salt thereof or a pharmaceutically acceptable solvate thereof, wherein, the drug-linking unit conjugate is selected from the group

consisting of:

| | |
|---|---|
| R01' | 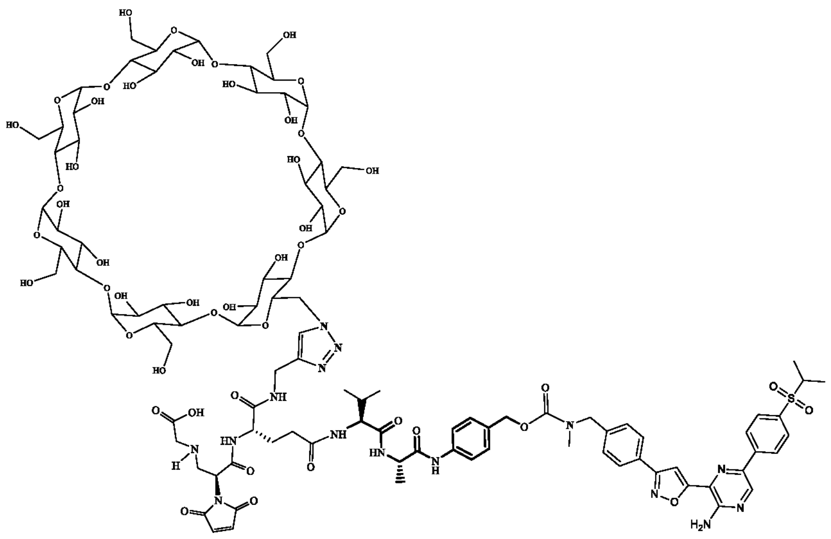 |
| R01-1' | 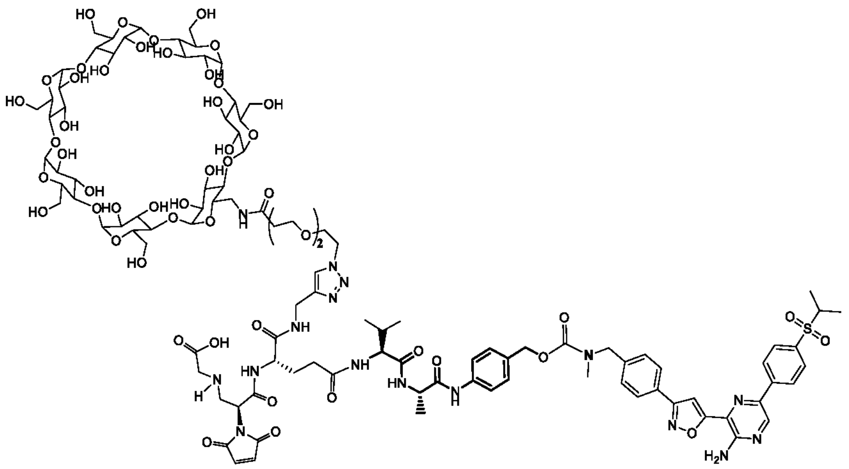 |
| R01-2' | 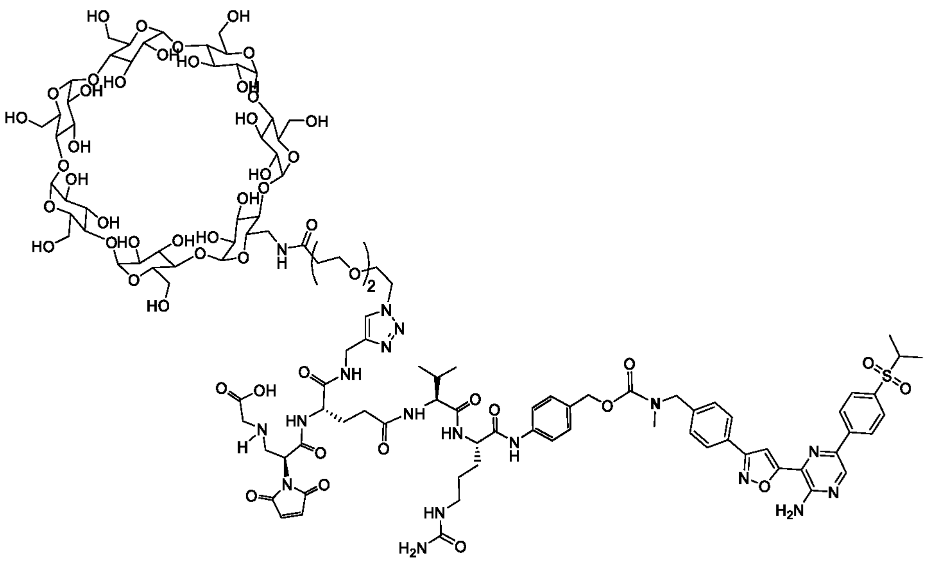 |

(continued)

| | |
|---|---|
| R01-4' | 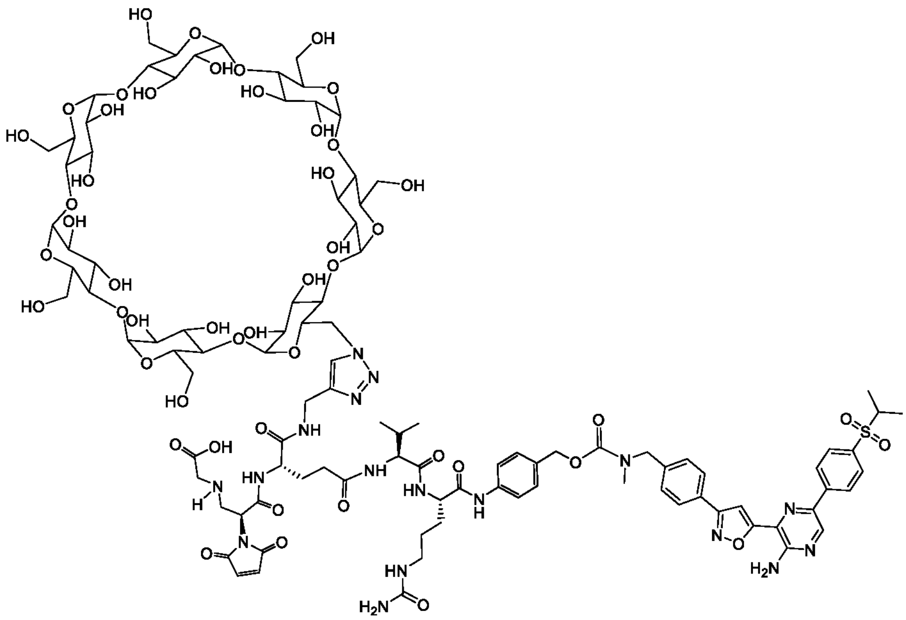 |
| R02' | 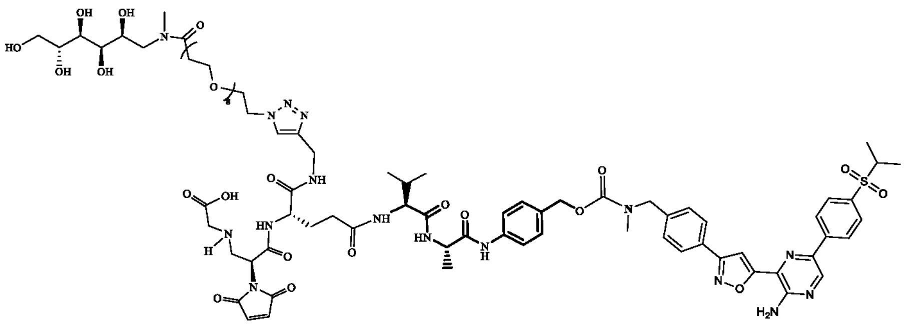 |
| R02-1' | 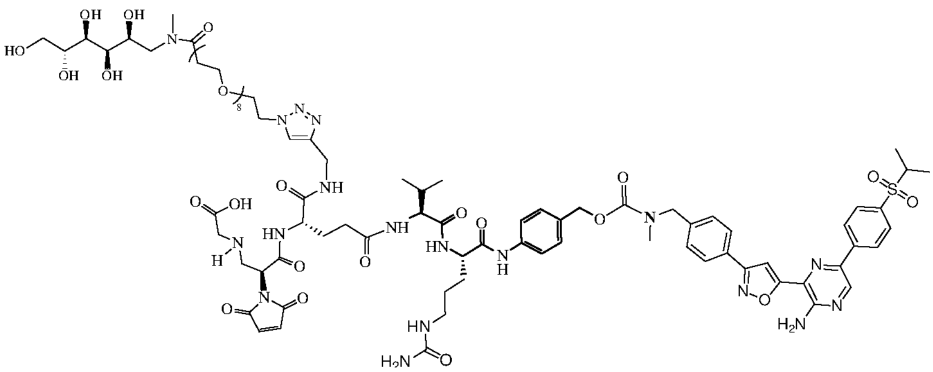 |
| R03' | 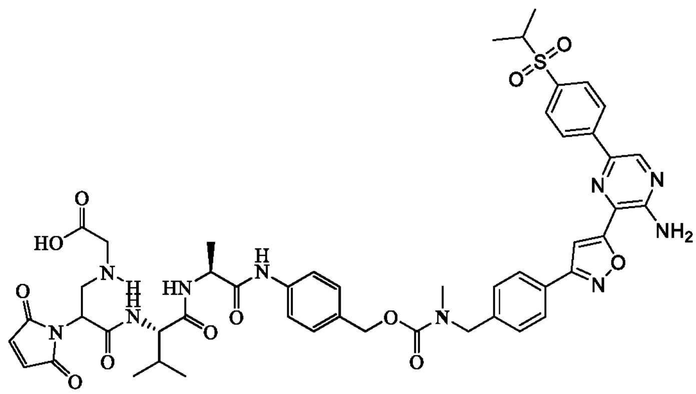 |

(continued)

| | |
|---|---|
| R03-1' | 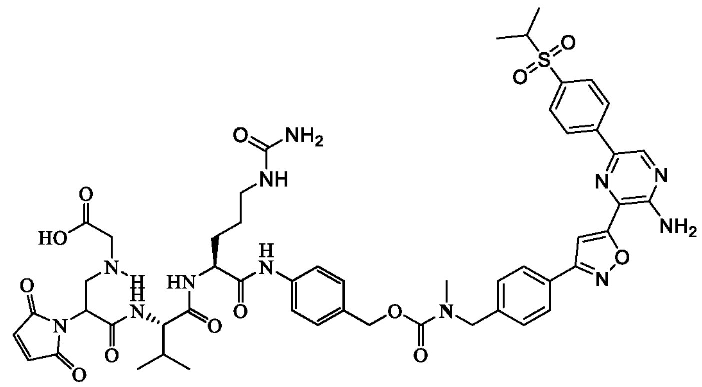 |
| AT01' | 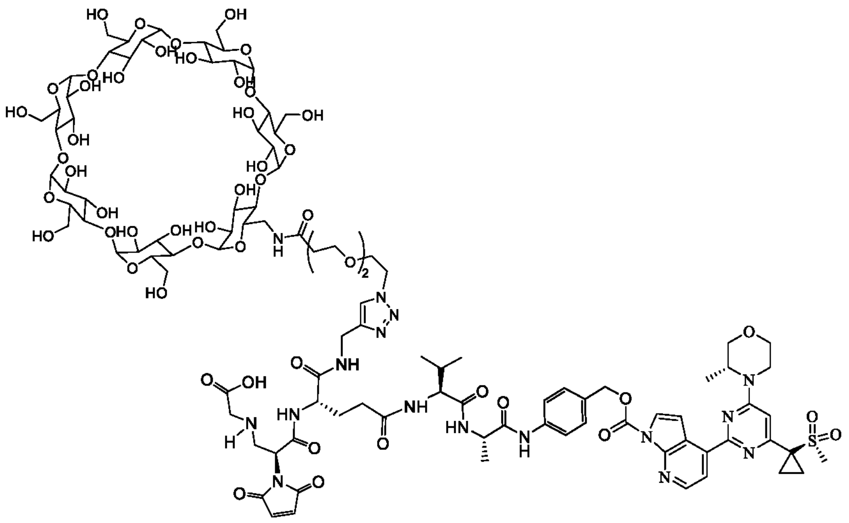 |
| AT02' | 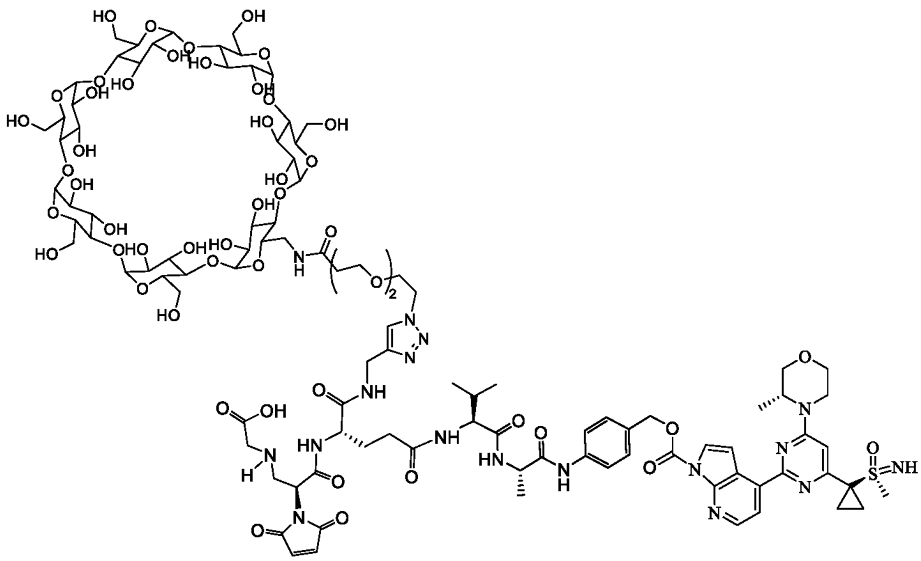 |

(continued)

| | |
|---|---|
| AT03' | 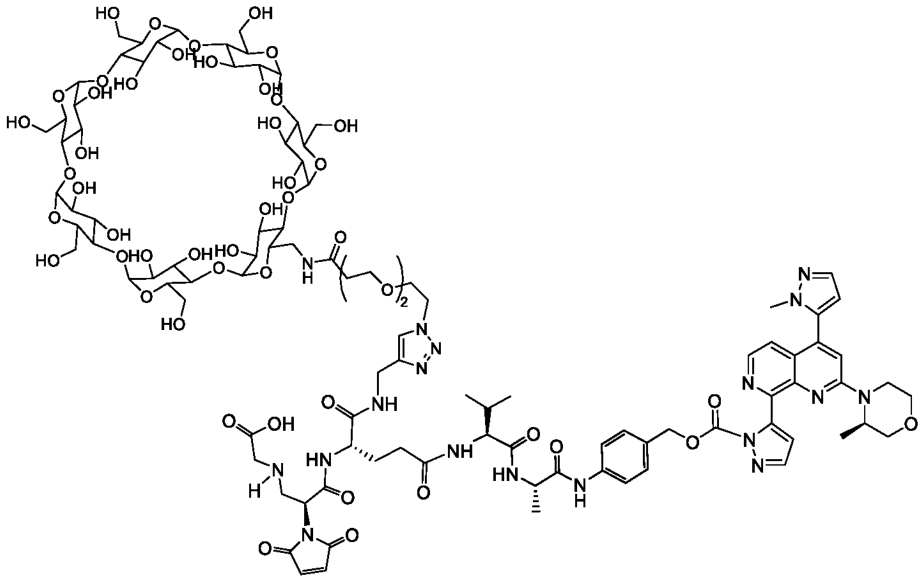 |
| AT04' | 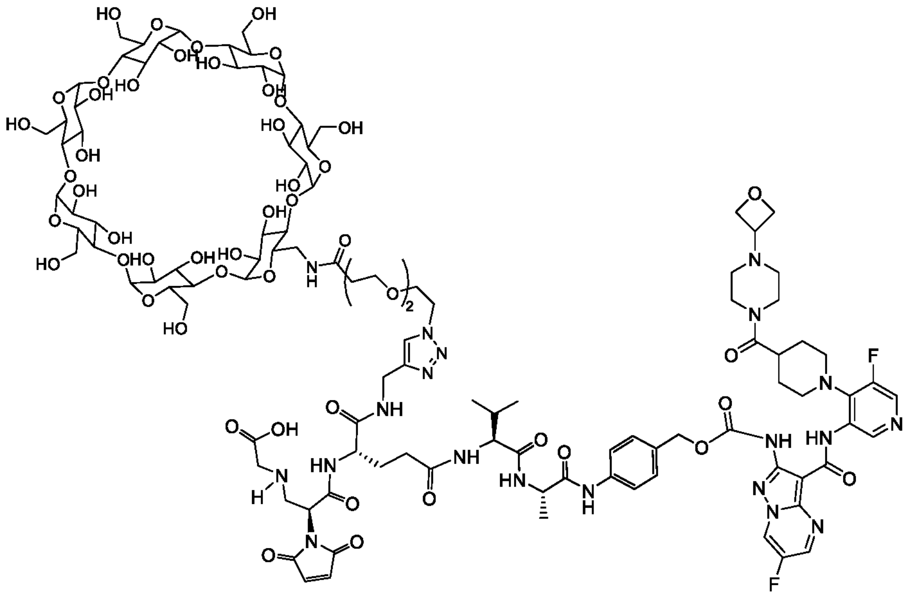 |
| TA01' | 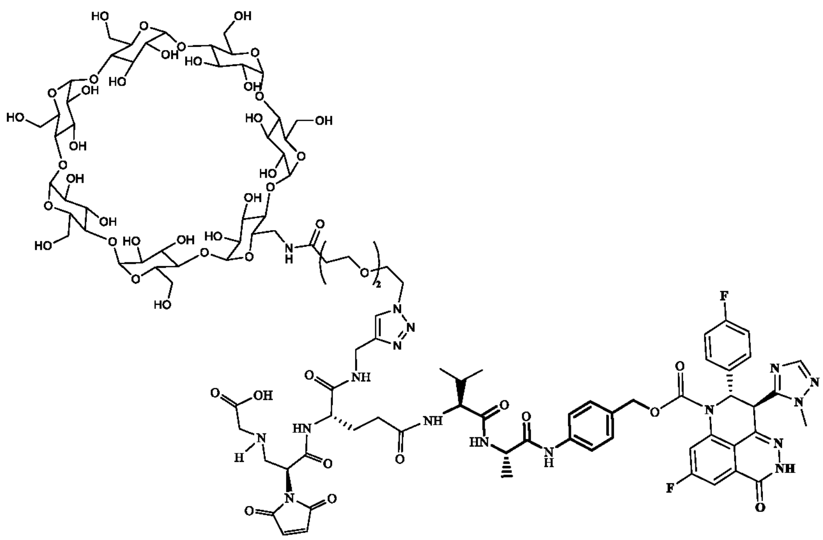 |

(continued)

| | |
|---|---|
| VE01' | 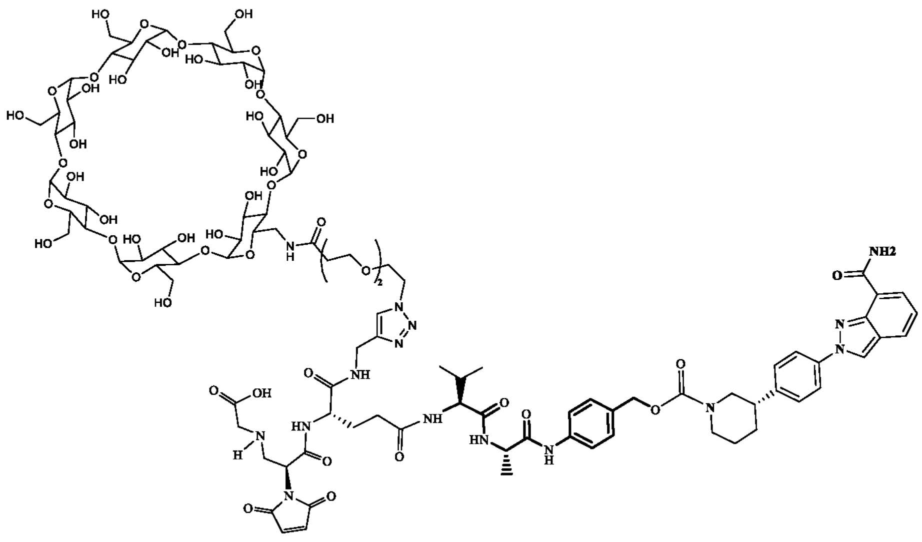 |
| E01' | 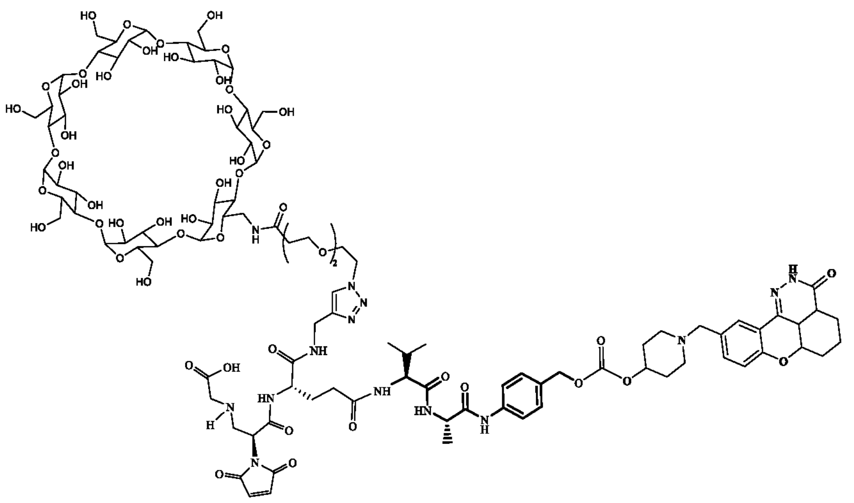 |
| RU01' | 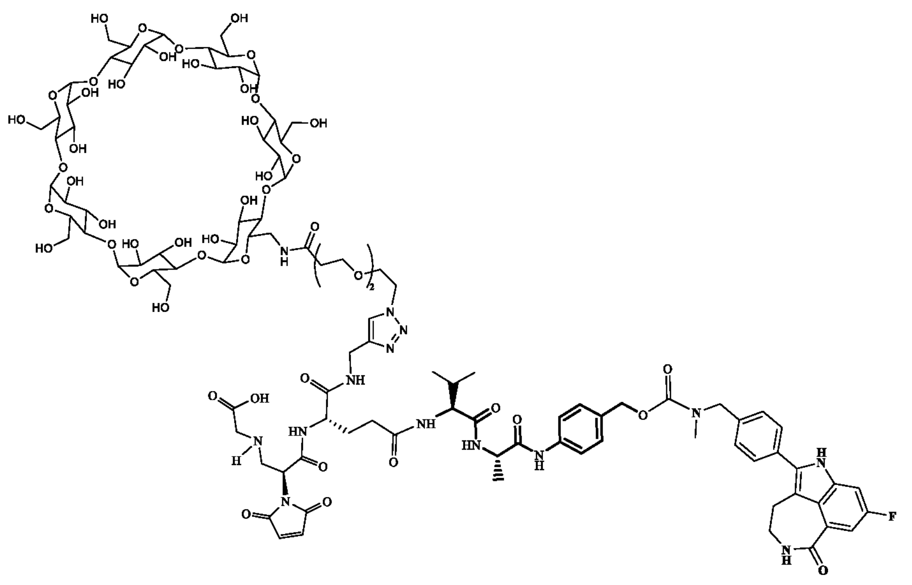 |

(continued)

| | |
|---|---|
| PA01' | 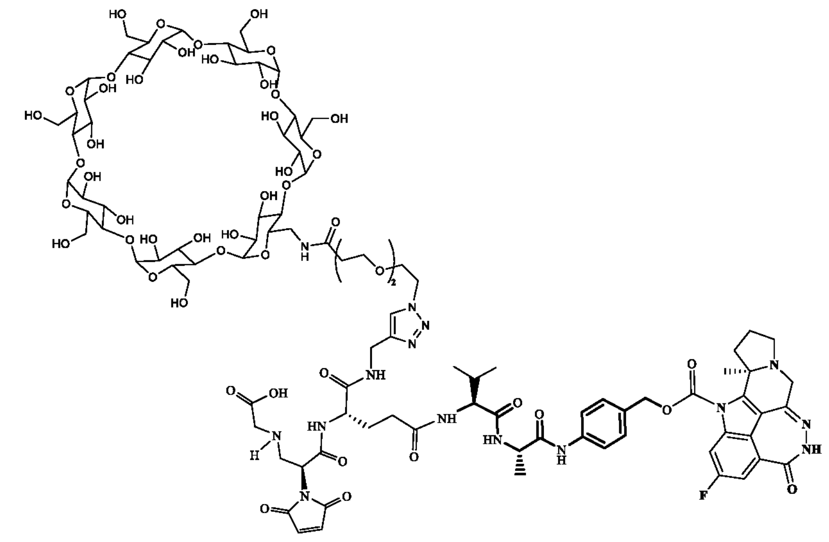 |
| CEP01' | 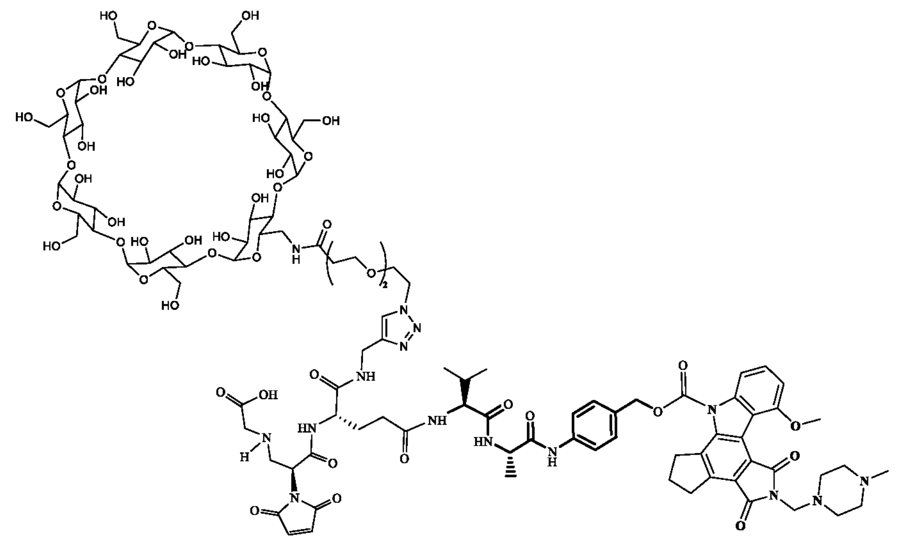 |
| TM01' | 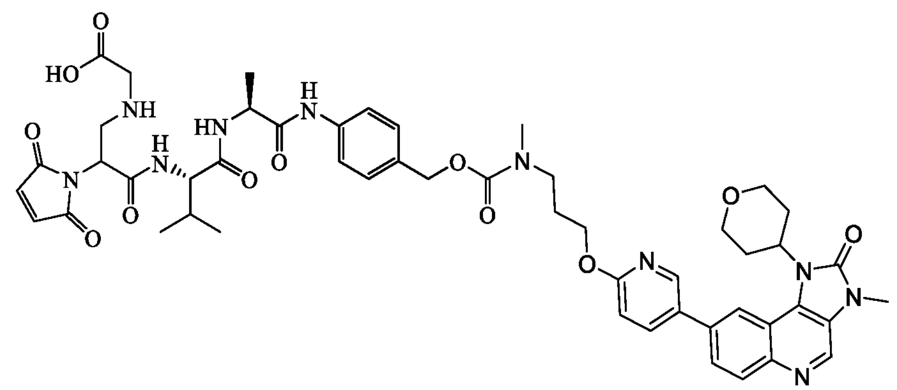 |

(continued)

| | |
|---|---|
| TM02' | 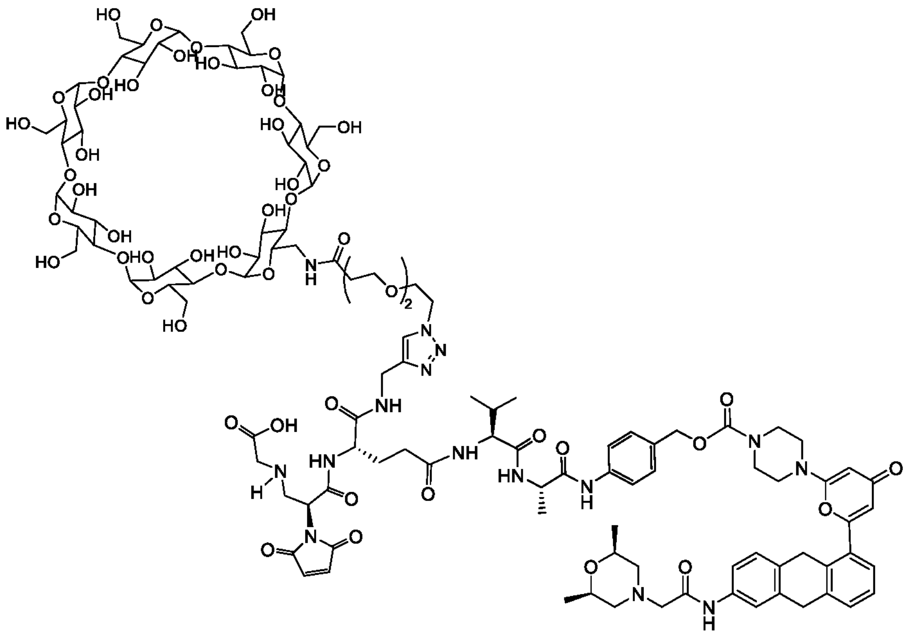 |
| WE01' | 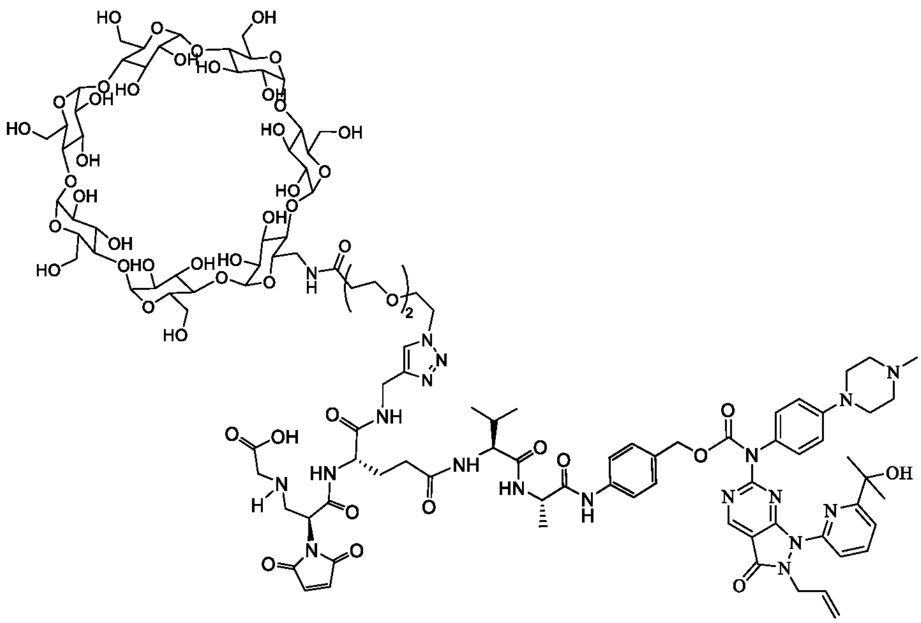 |
| CH01' | |

(continued)

| | |
|---|---|
| | 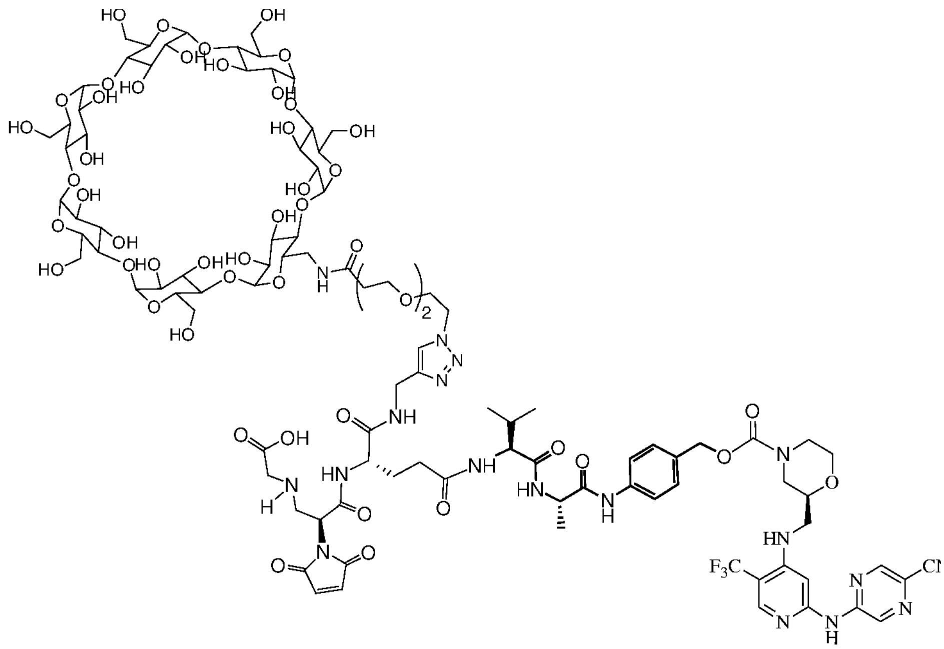 |
| CH02' | 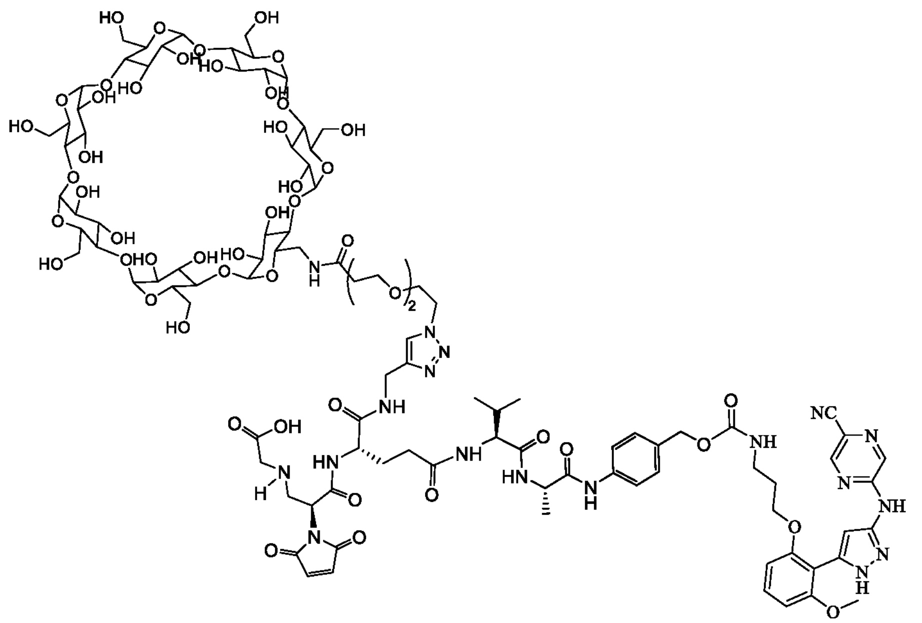 |

(continued)

| | |
|---|---|
| CH03' | 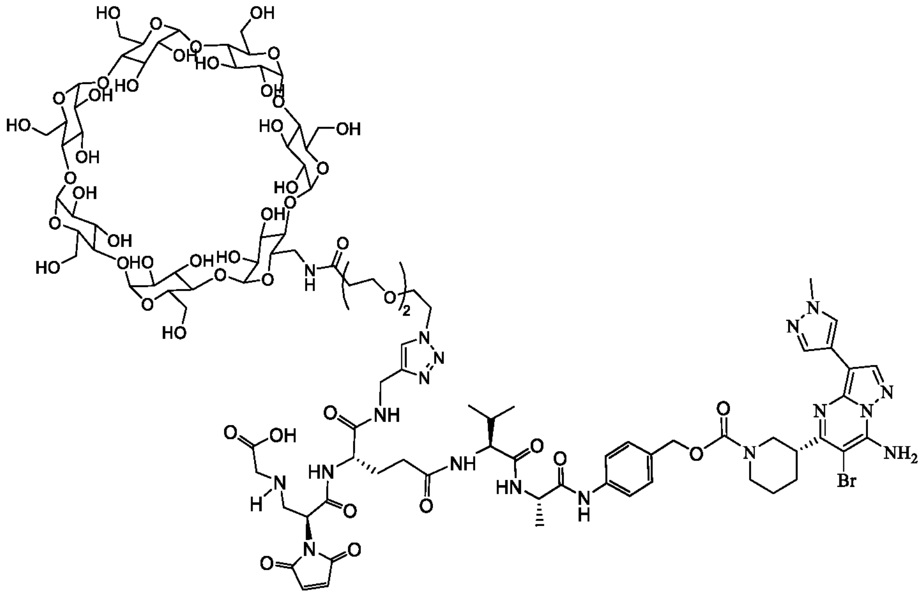 |
| CH04' | 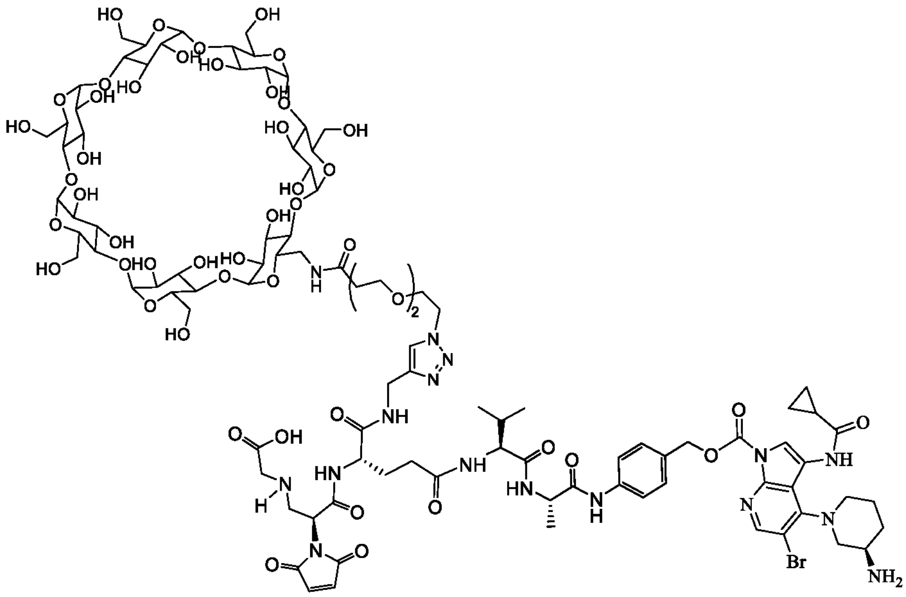 |

(continued)

| | |
|---|---|
| DP01' | 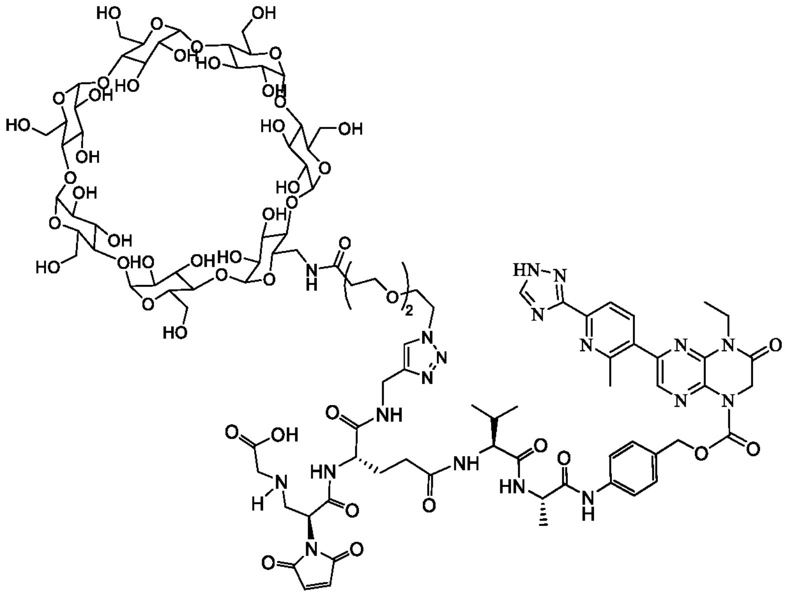 |
| DP02' | 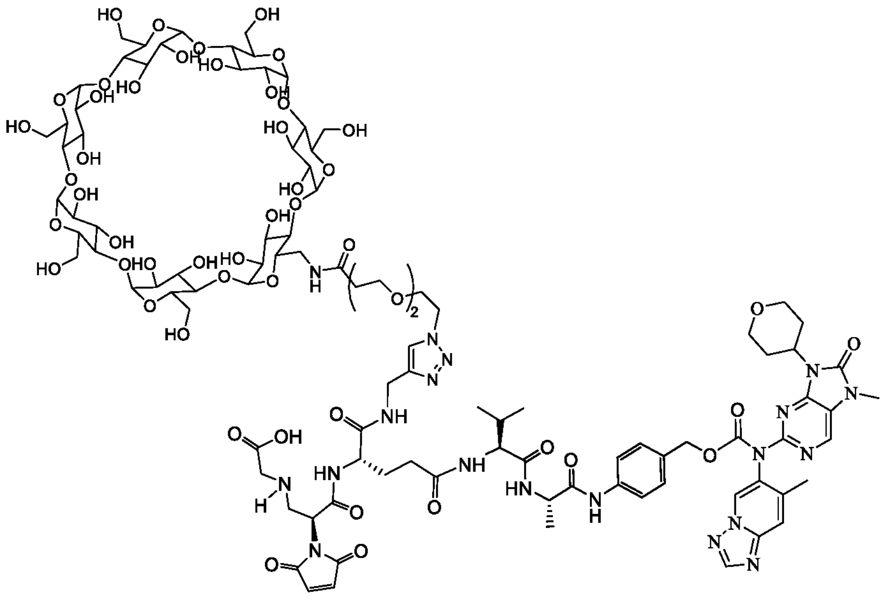 |

(continued)

| | |
|---|---|
| DP03' | 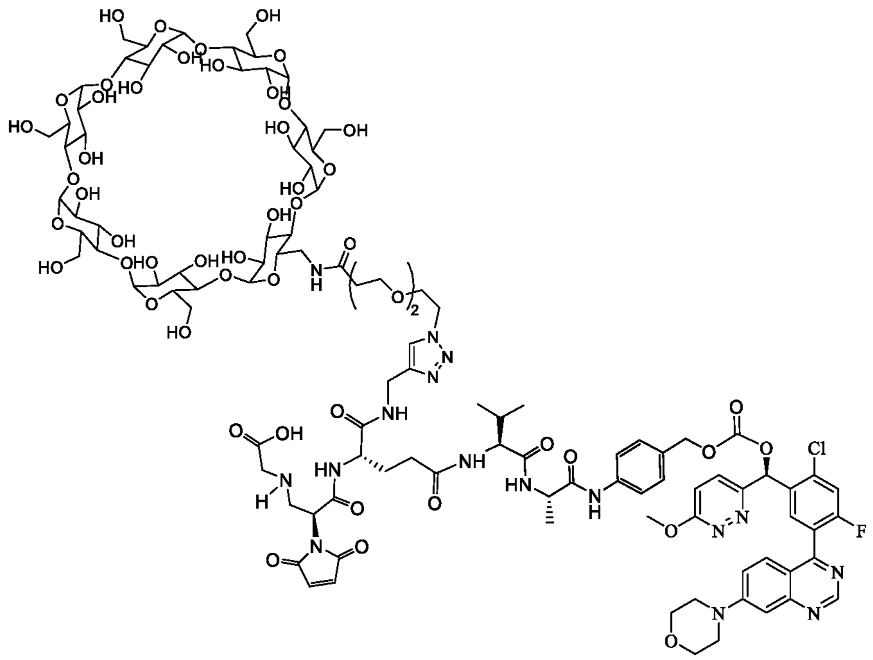 |

| | |
|---|---|
| Ex01' | 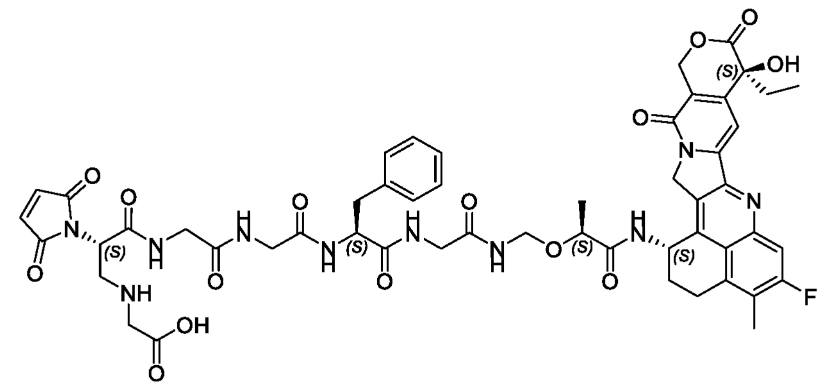 |
| Ex02' | 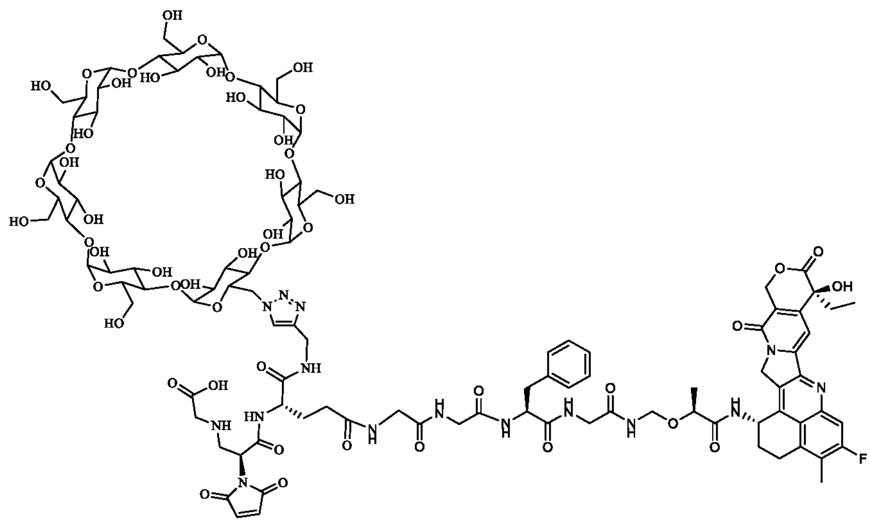 |

(continued)

| | |
|---|---|
| Ex03' | 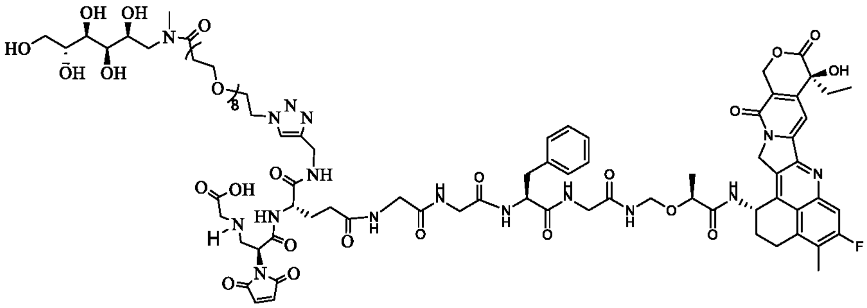 |
| Ex04' | 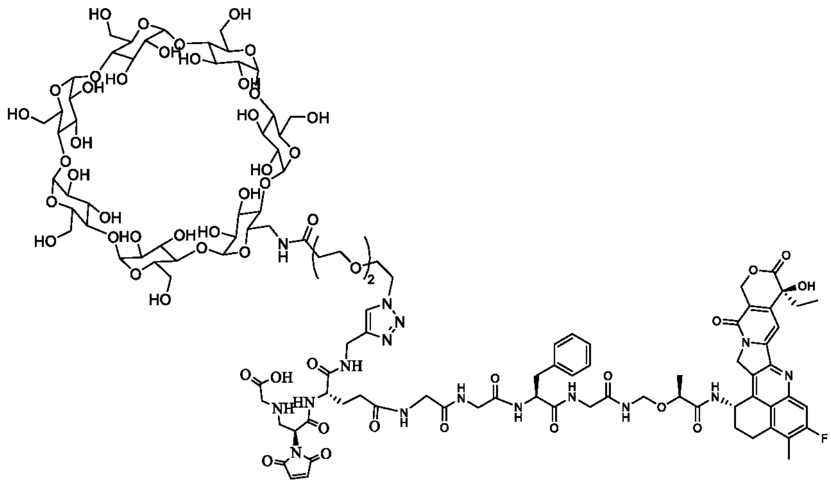 |
| Ex05' | 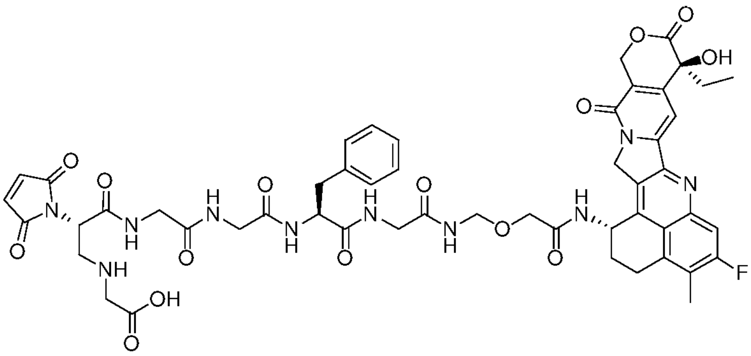 |
| Ex06' | 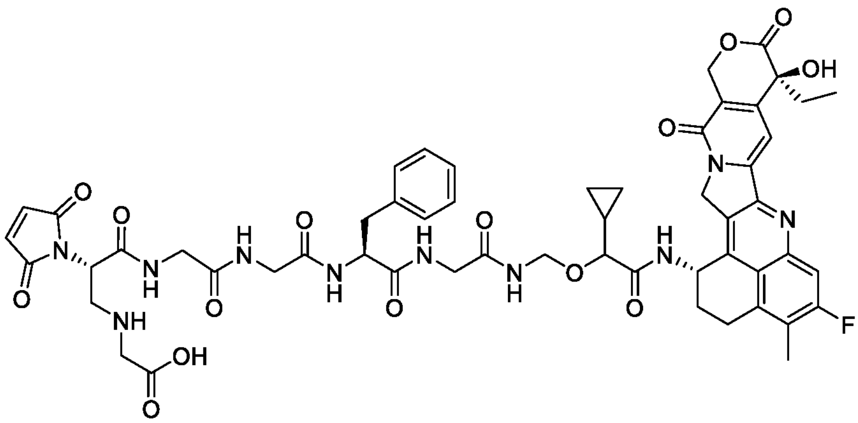 |

(continued)

| | |
|---|---|
| Ex07' | 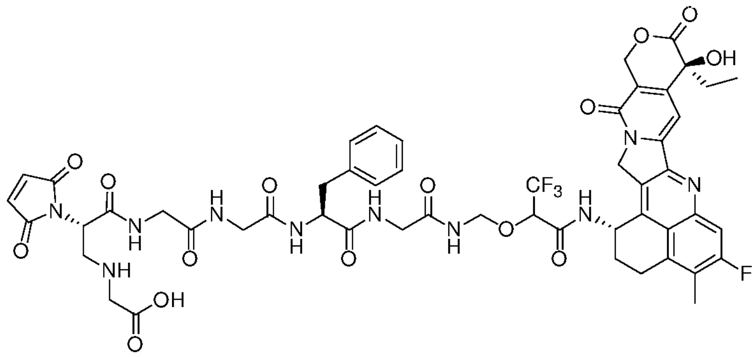 |
| Ex08' | 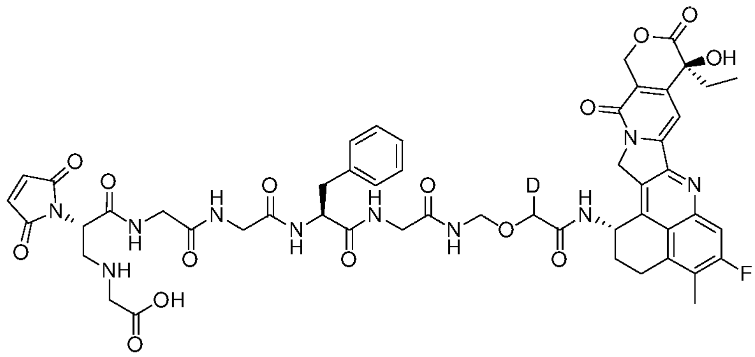 |
| Ex09' | 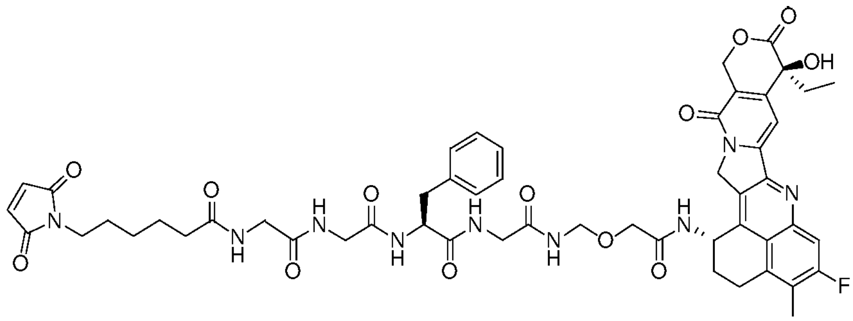 |
| SN01' | 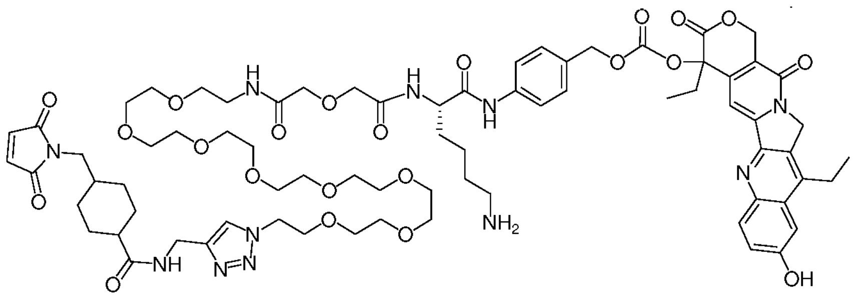 |
| PB01' | 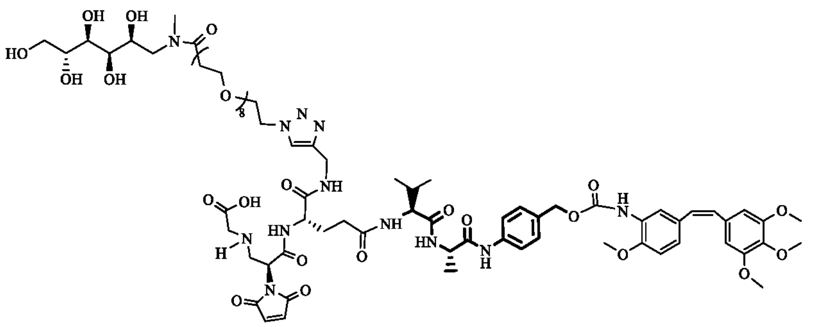 |

(continued)

| | |
|---|---|
| PB02' | 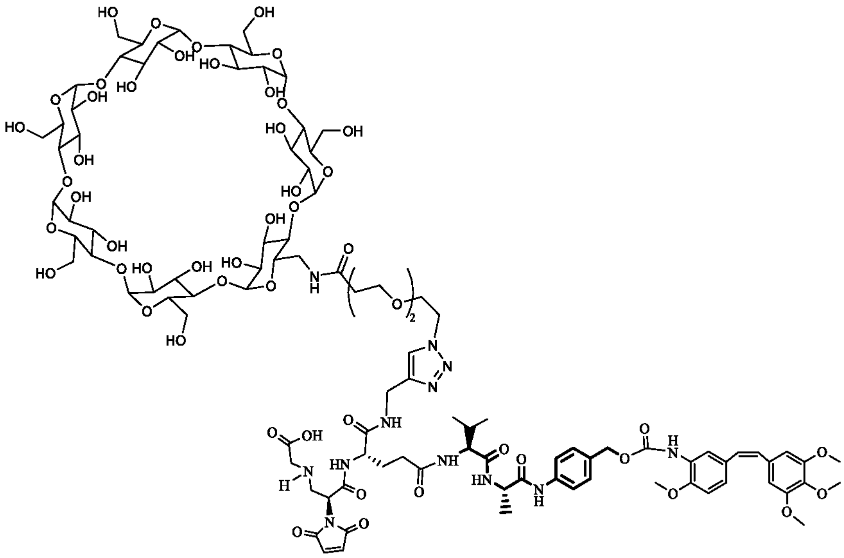 |
| PNU01' | 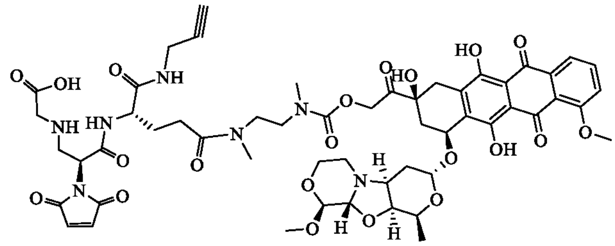 |
| PNU02' | 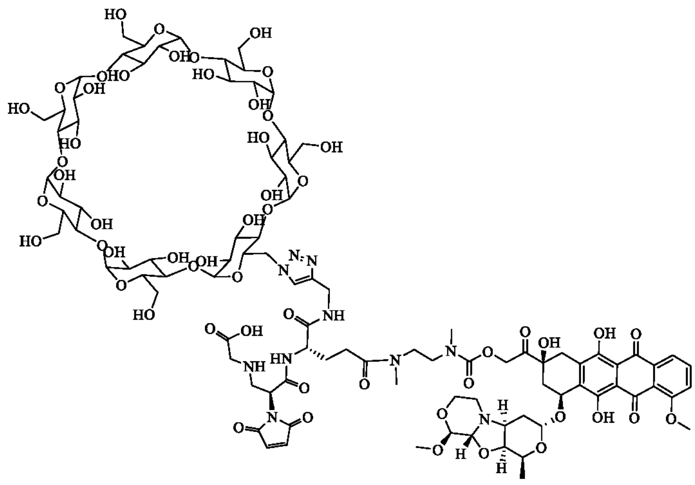 |

(continued)

| | |
|---|---|
| CBI01' | 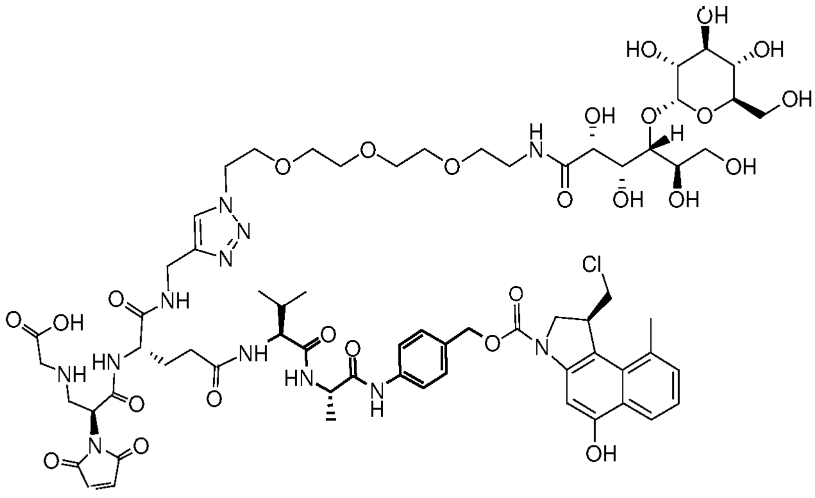 |
| CBI02' | 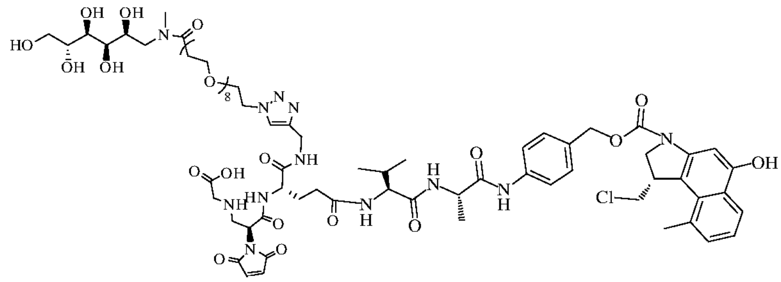 |
| CBI03' | 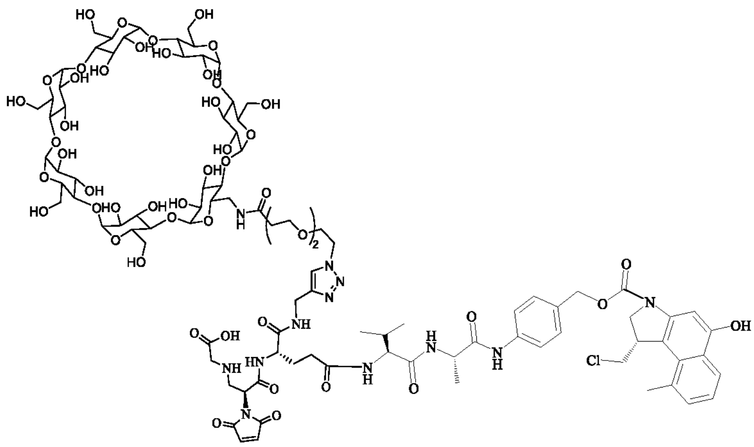 |
| PBD01' | 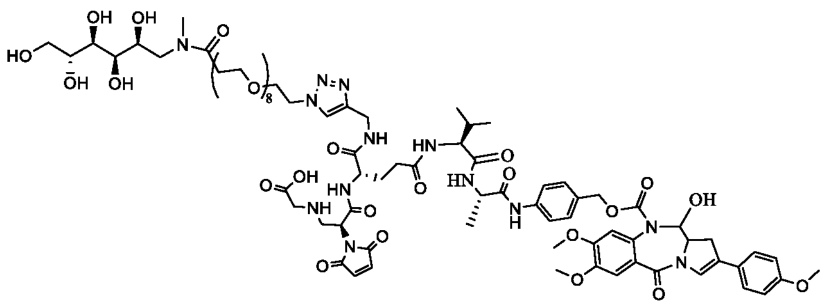 |

(continued)

| | |
|---|---|
| PBD02' | 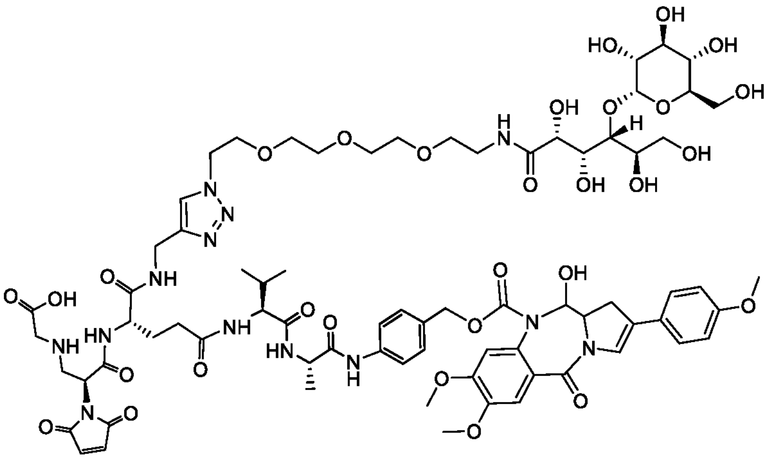 |
| PBD-CBI01' | 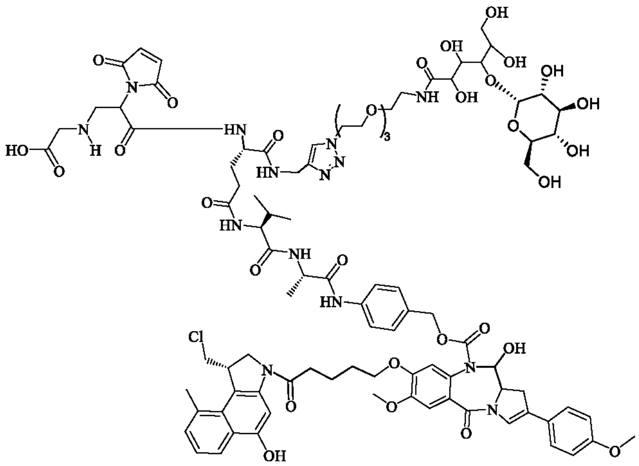 |
| PBD-CBI02' | 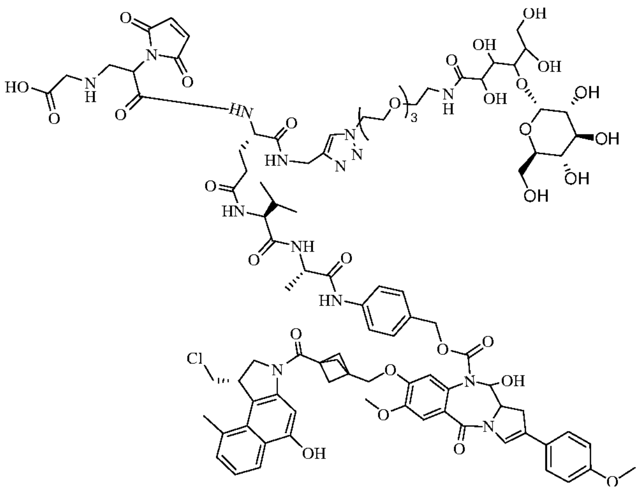 |

(continued)

| | |
|---|---|
| M01' | 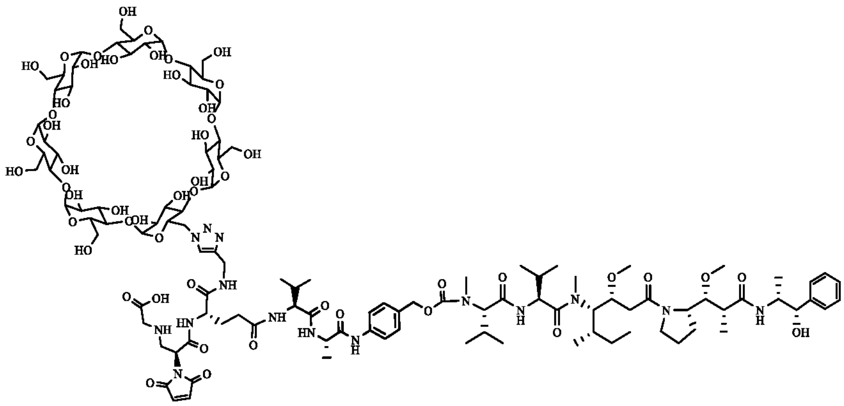 |
| M02' | 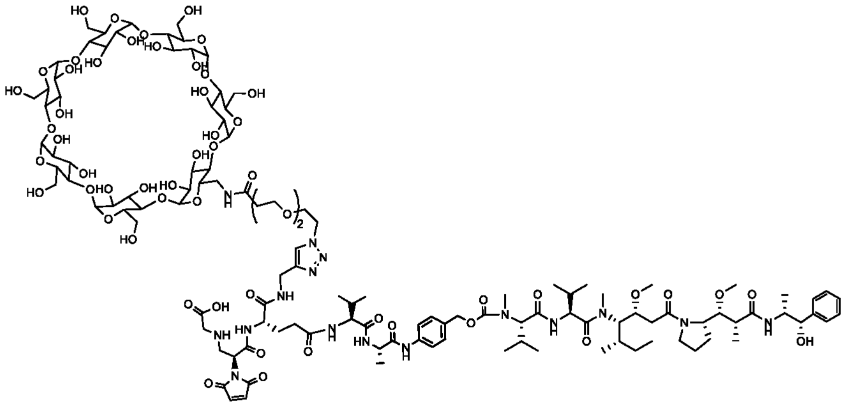 |
| M03' | 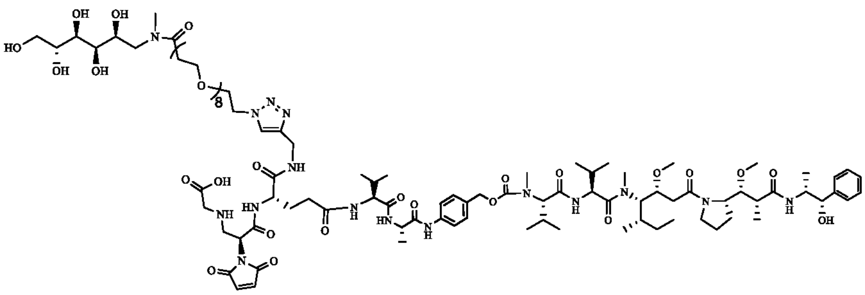 |

13. A pharmaceutical composition, comprising the ligand-drug conjugate, or a stereoisomer thereof, a prodrug thereof, a pharmaceutically acceptable salt thereof or a pharmaceutically acceptable solvate thereof according to any one of claims 1-11, or the drug-linking unit conjugate or a stereoisomer thereof, a prodrug thereof, a pharmaceutically acceptable salt thereof or a pharmaceutically acceptable solvate thereof according to claim 12, and optionally, one or more pharmaceutical adjuvants.

14. Use of the ligand-drug conjugate, or a stereoisomer thereof, a prodrug thereof, a pharmaceutically acceptable salt thereof or a pharmaceutically acceptable solvate thereof according to any one of claims 1-11, or the drug-linking unit conjugate or a stereoisomer thereof, a prodrug thereof, a pharmaceutically acceptable salt thereof or a pharmaceutically acceptable solvate thereof according to claim 12, or the pharmaceutical composition according to claim 13 in the preparation of a medicament for the treatment and/or prevention of a disease, and the disease is cancer;

preferably, the cancer is selected from the group consisting of skin cancer, lymphoma, esophageal cancer (e.g., esophageal adenocarcinoma and esophageal squamous cell carcinoma), brain tumor, lung cancer (e.g., small-cell lung cancer and non-small cell lung cancer), squamous cell cancer, bladder cancer, gastric cancer, ovarian cancer, peritoneal cancer, pancreatic cancer, breast cancer, head and neck cancer, cervical cancer, endometrial cancer, colon cancer, rectal cancer, colorectal cancer, liver cancer, kidney cancer, solid tumors, non-hodgkin's lymphoma, central nervous system tumors (e.g., glioma, glioblastoma multiforme, neuroblastoma, glioma or

sarcoma), melanoma, prostate cancer, thyroid cancer, bone cancer, urinary tract cancer, salivary gland cancer, leukemia (e.g., acute myeloid leukemia), and other solid tumors and hematological tumors;
more preferably, the cancer is selected from the group consisting of lung cancer, squamous cell cancer, bladder cancer, gastric cancer, ovarian cancer, breast cancer, colon cancer, rectal cancer, colorectal cancer, liver cancer, and kidney cancer.

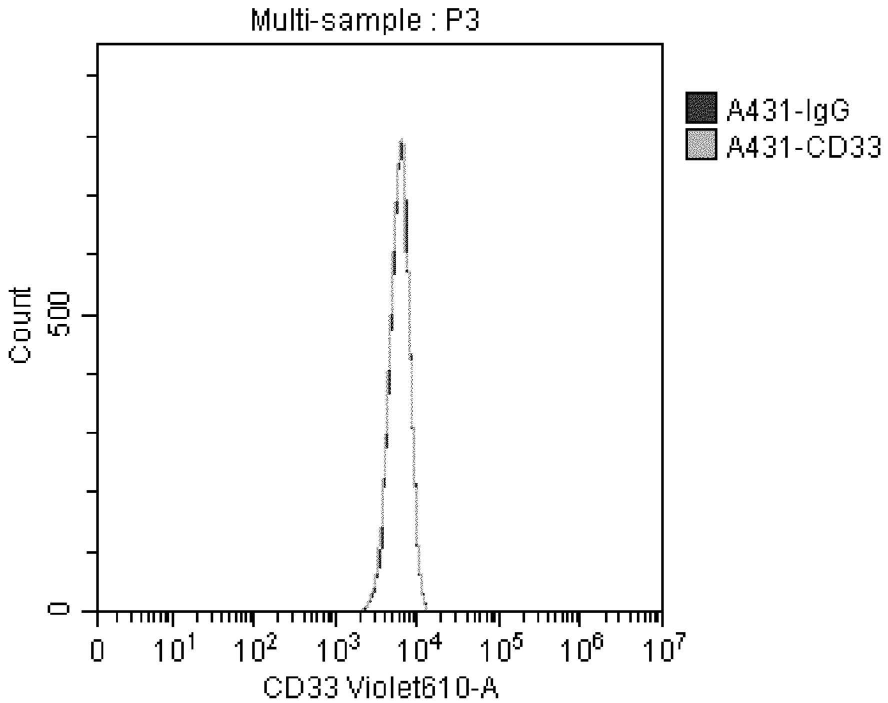
Multi-sample : P3
A431-IgG
A431-CD33
Count
500
0
0
$10^1$
$10^2$
$10^3$
$10^4$
$10^5$
$10^6$
$10^7$
CD33 Violet610-A

Fig. 1

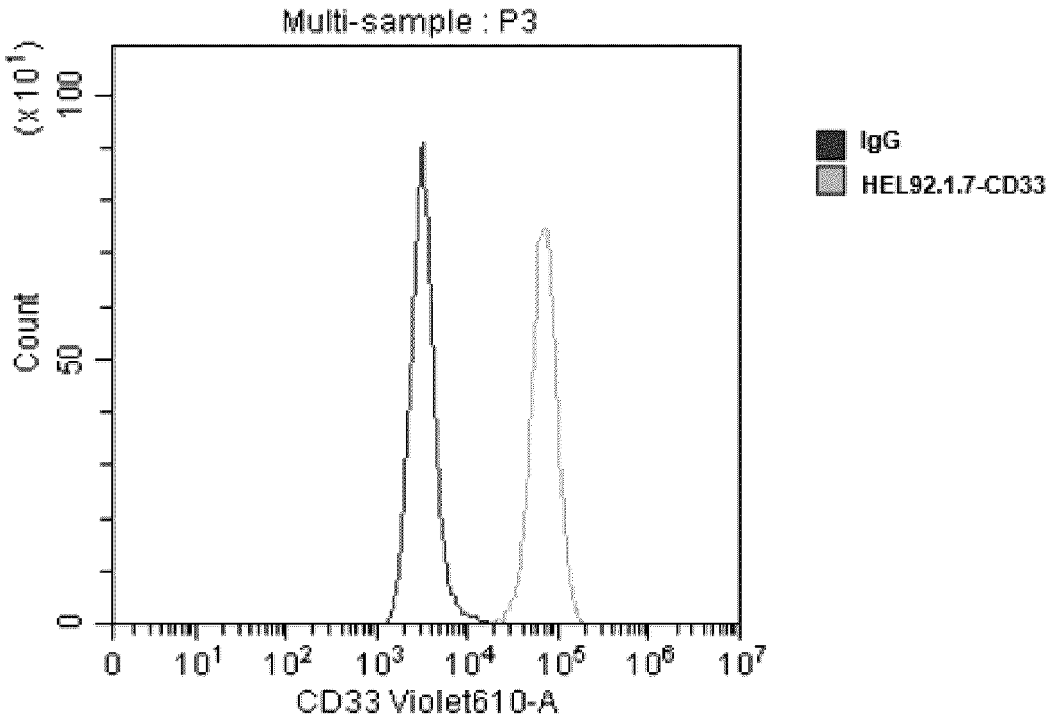
Multi-sample : P3
IgG
HEL92.1.7-CD33
(x $10^1$)
100
Count
50
0
0
$10^1$
$10^2$
$10^3$
$10^4$
$10^5$
$10^6$
$10^7$
CD33 Violet610-A

Fig. 2

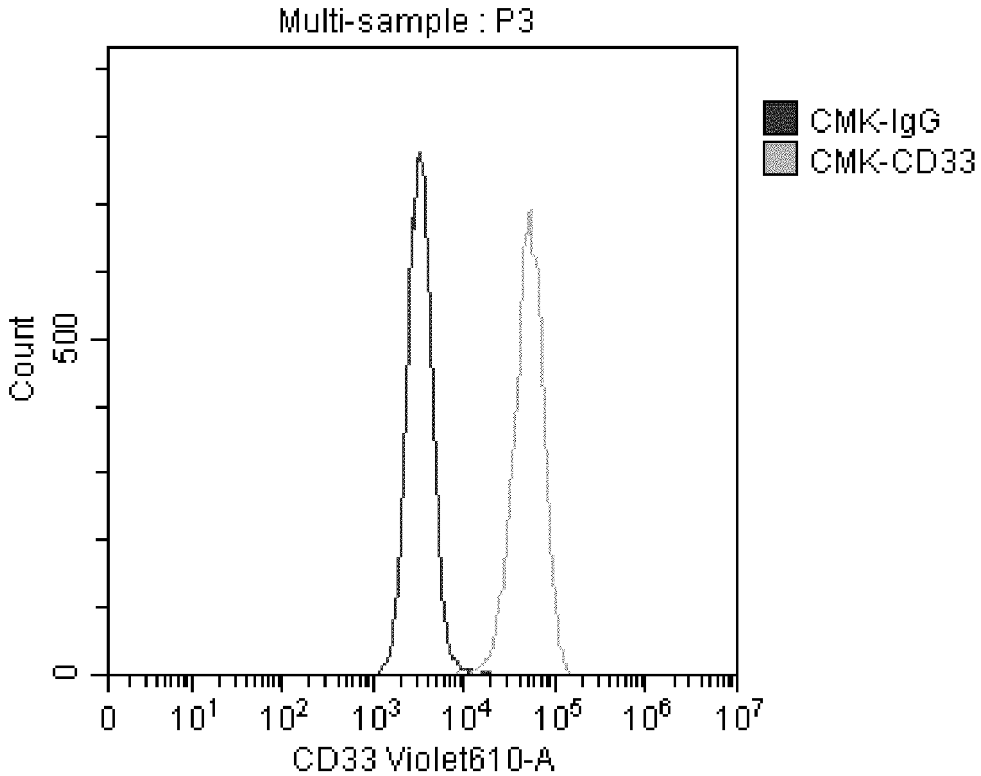
Multi-sample : P3
CMK-IgG
CMK-CD33
Count
500
0
0
$10^1$
$10^2$
$10^3$
$10^4$
$10^5$
$10^6$
$10^7$
CD33 Violet610-A

Fig. 3

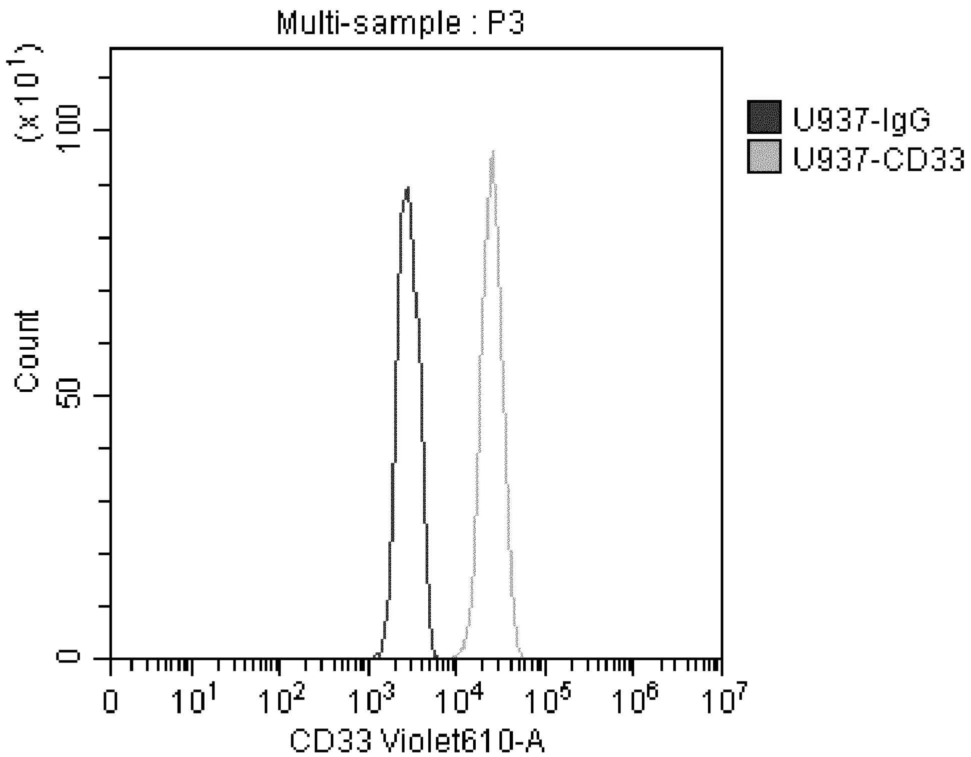
Multi-sample : P3
U937-IgG
U937-CD33
(x 10 1)
Count
100
50
0
0
$10^1$
$10^2$
$10^3$
$10^4$
$10^5$
$10^6$
$10^7$
CD33 Violet610-A

Fig. 4

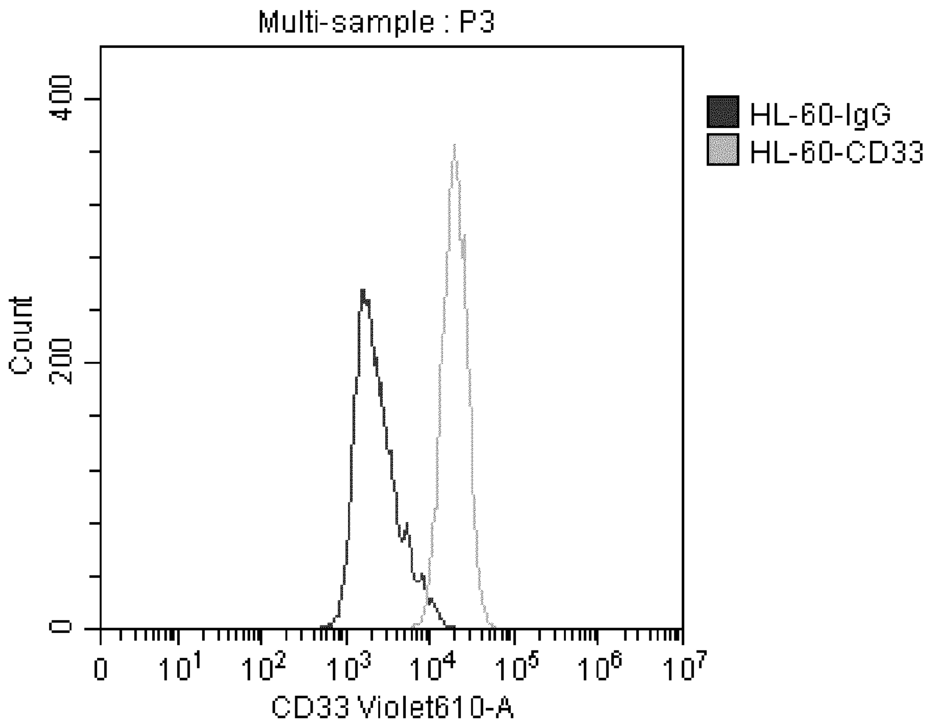
Multi-sample : P3
Count
400
200
0
HL-60-IgG
HL-60-CD33
0
10^1
10^2
10^3
10^4
10^5
10^6
10^7
CD33 Violet610-A

Fig. 5

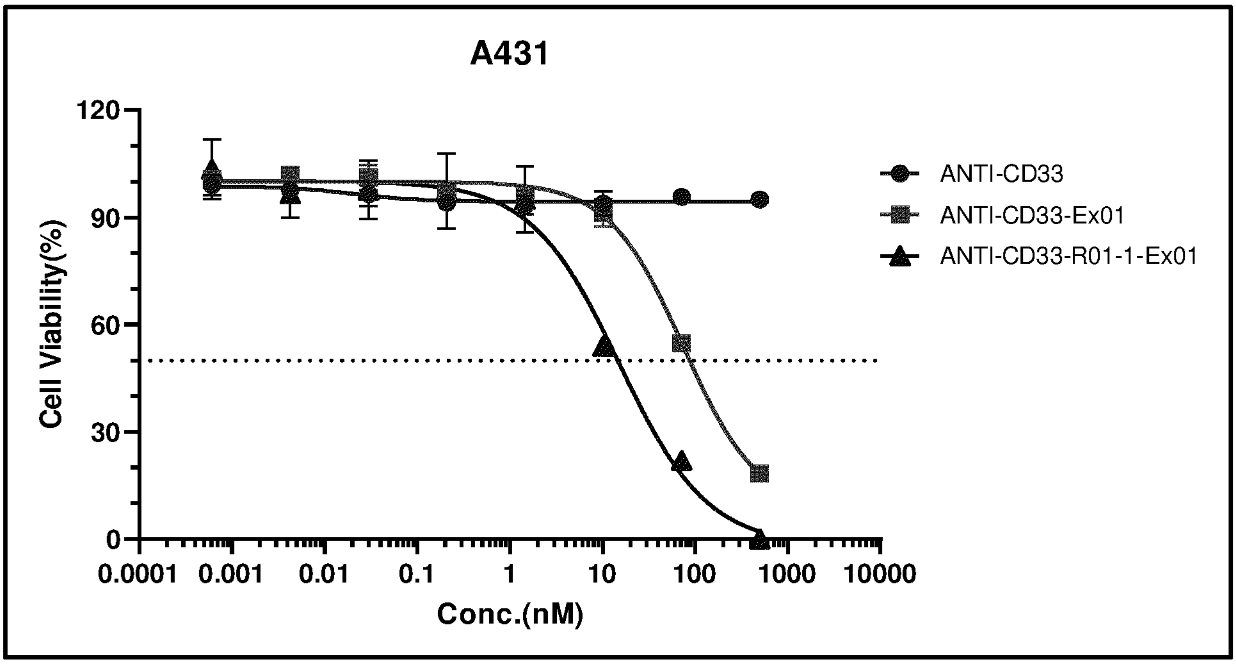
A431
Cell Viability(%)
120
90
60
30
0
0.0001
0.001
0.01
0.1
1
10
100
1000
10000
Conc.(nM)
ANTI-CD33
ANTI-CD33-Ex01
ANTI-CD33-R01-1-Ex01

Fig. 6

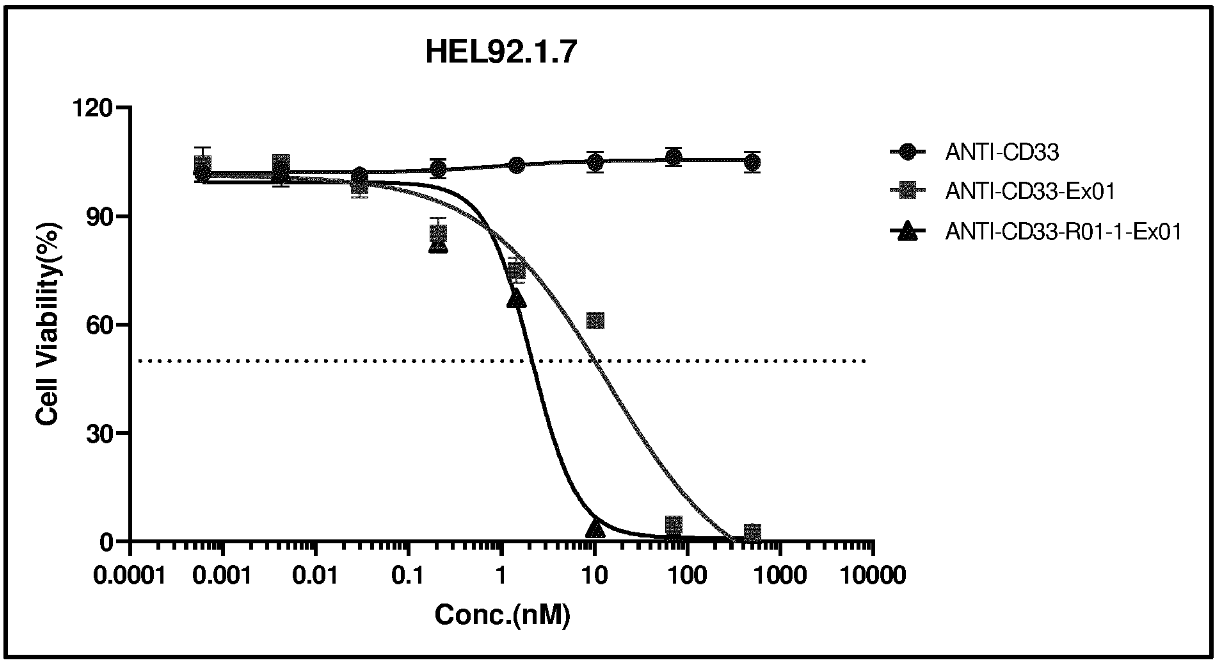
HEL92.1.7
Cell Viability(%)
120
90
60
30
0
0.0001
0.001
0.01
0.1
1
10
100
1000
10000
Conc.(nM)
ANTI-CD33
ANTI-CD33-Ex01
ANTI-CD33-R01-1-Ex01

Fig. 7

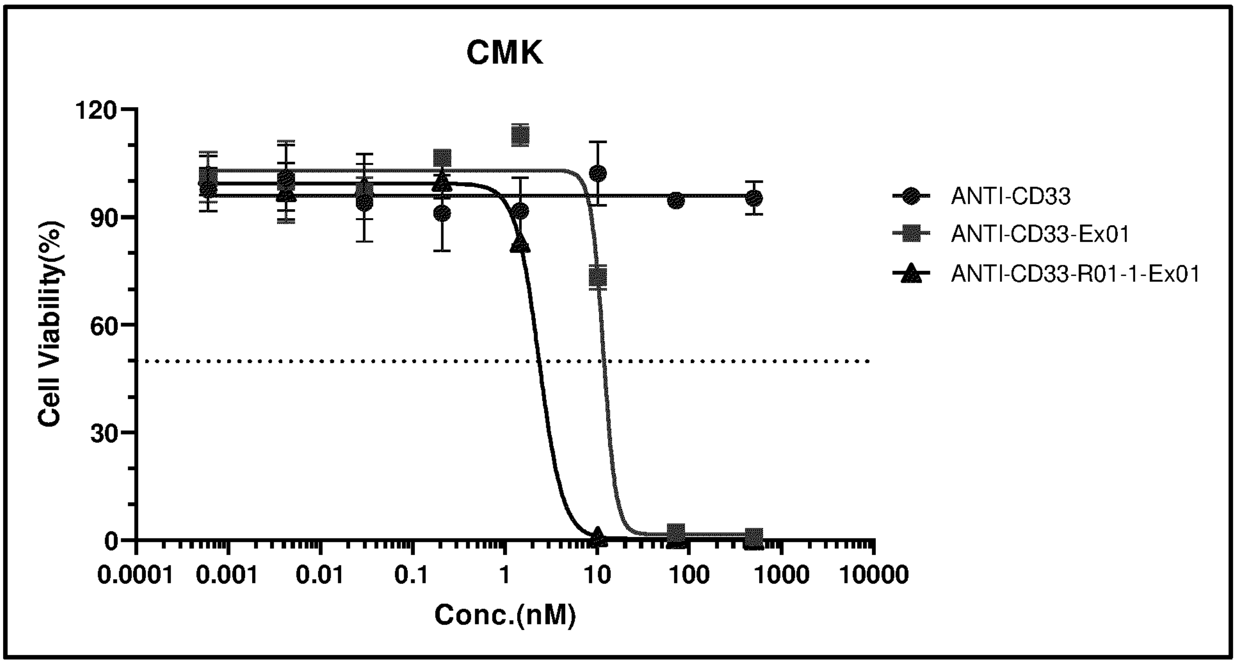
CMK
Cell Viability(%)
120
90
60
30
0
0.0001
0.001
0.01
0.1
1
10
100
1000
10000
Conc.(nM)
ANTI-CD33
ANTI-CD33-Ex01
ANTI-CD33-R01-1-Ex01

Fig. 8

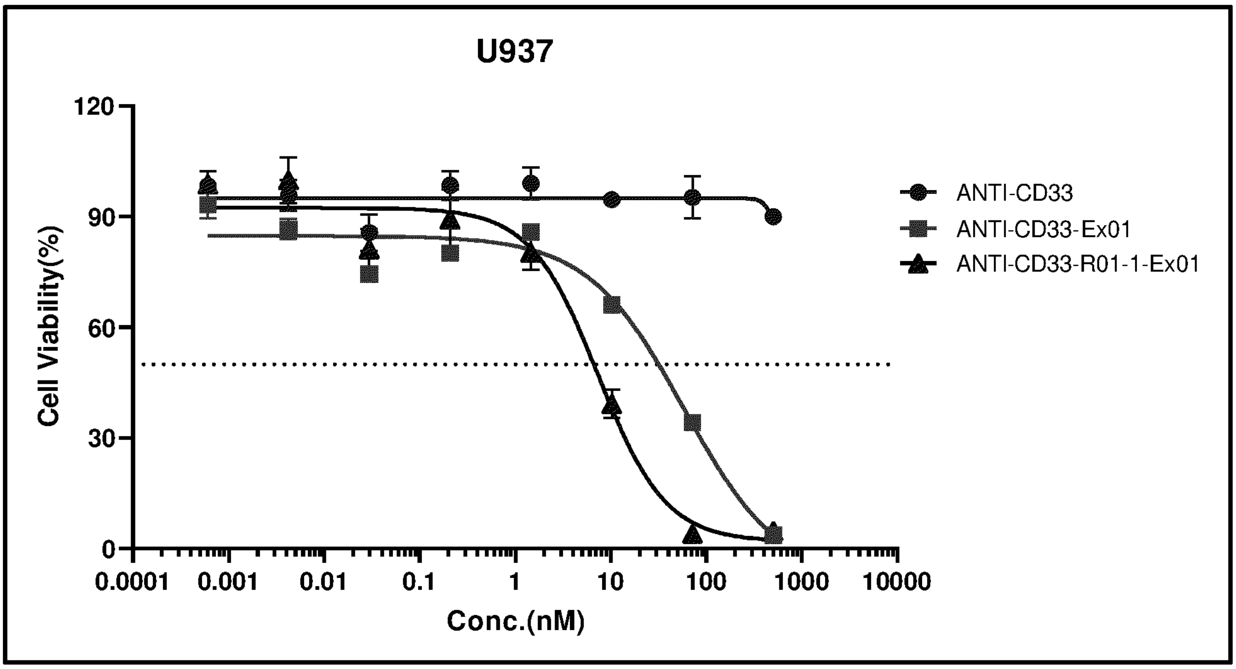
U937
120
90
60
30
0
Cell Viability(%)
0.0001
0.001
0.01
0.1
1
10
100
1000
10000
Conc.(nM)
ANTI-CD33
ANTI-CD33-Ex01
ANTI-CD33-R01-1-Ex01

Fig. 9

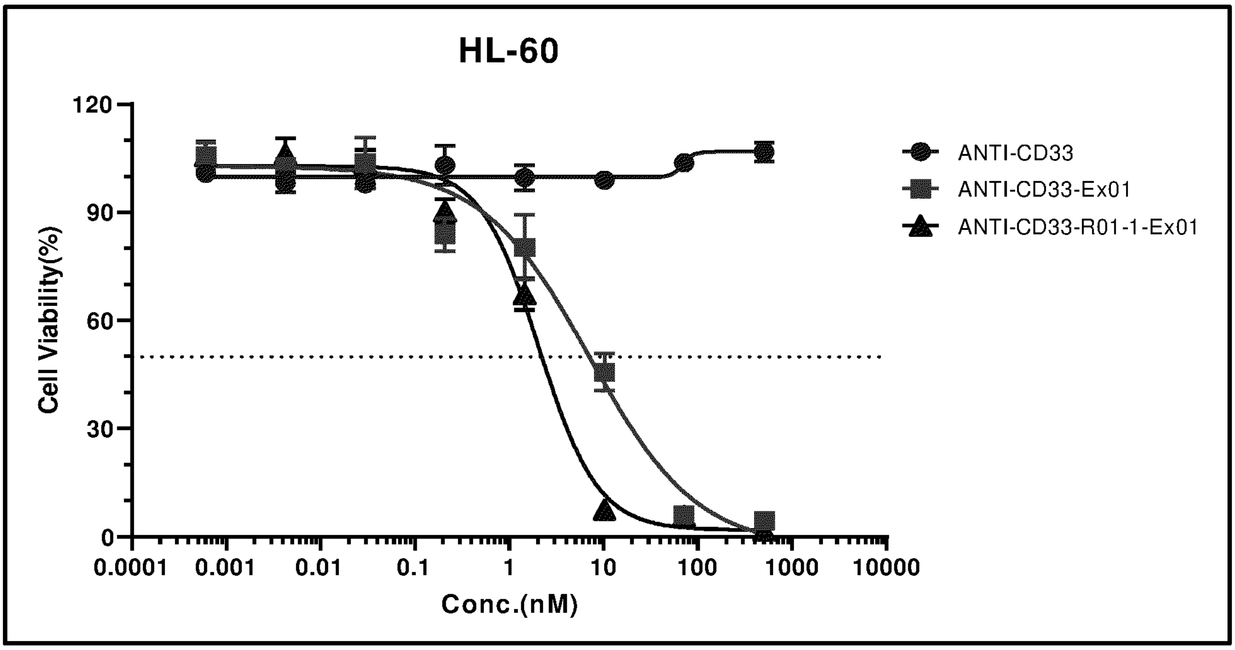
HL-60
120
90
60
30
0
Cell Viability(%)
0.0001
0.001
0.01
0.1
1
10
100
1000
10000
Conc.(nM)
ANTI-CD33
ANTI-CD33-Ex01
ANTI-CD33-R01-1-Ex01

Fig. 10

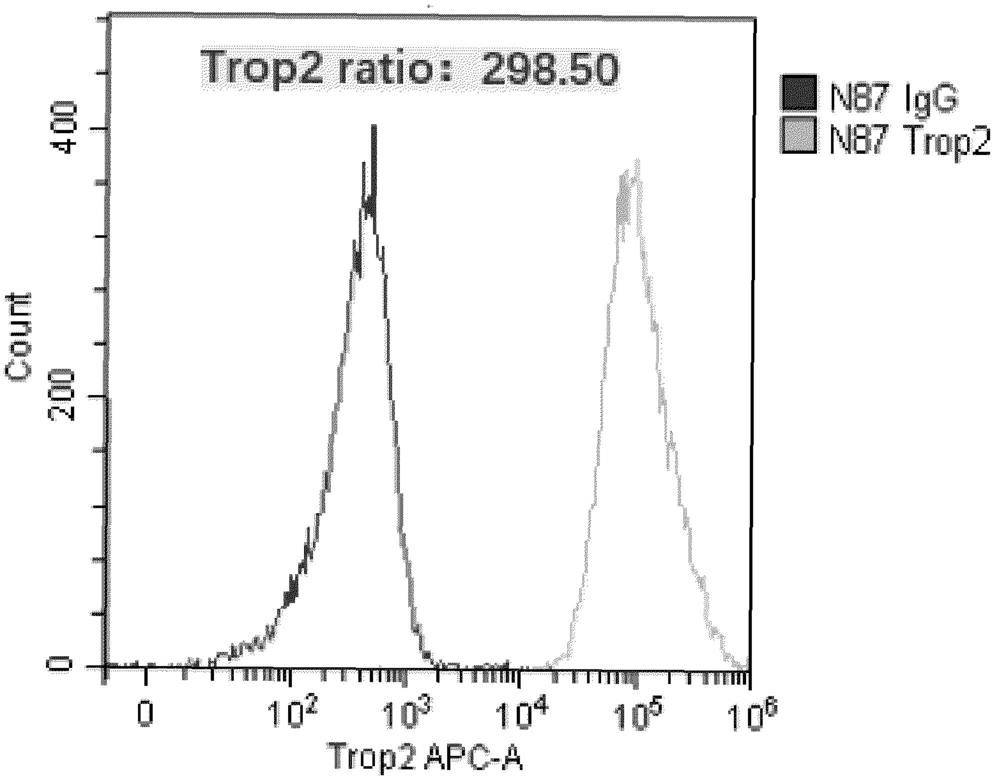
Trop2 ratio: 298.50
N87 IgG
N87 Trop2
Count
0
200
400
0
$10^2$
$10^3$
$10^4$
$10^5$
$10^6$
Trop2 APC-A

Fig. 11

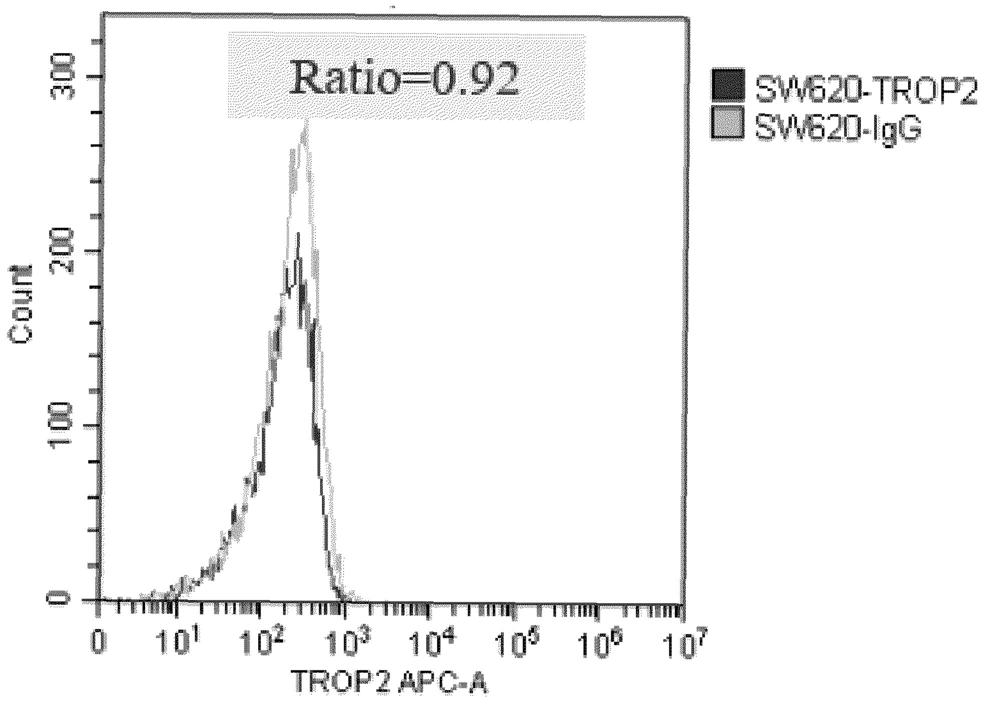
Ratio=0.92
SW620-TROP2
SW620-IgG
Count
0
100
200
300
0
$10^1$
$10^2$
$10^3$
$10^4$
$10^5$
$10^6$
$10^7$
TROP2 APC-A

Fig. 12

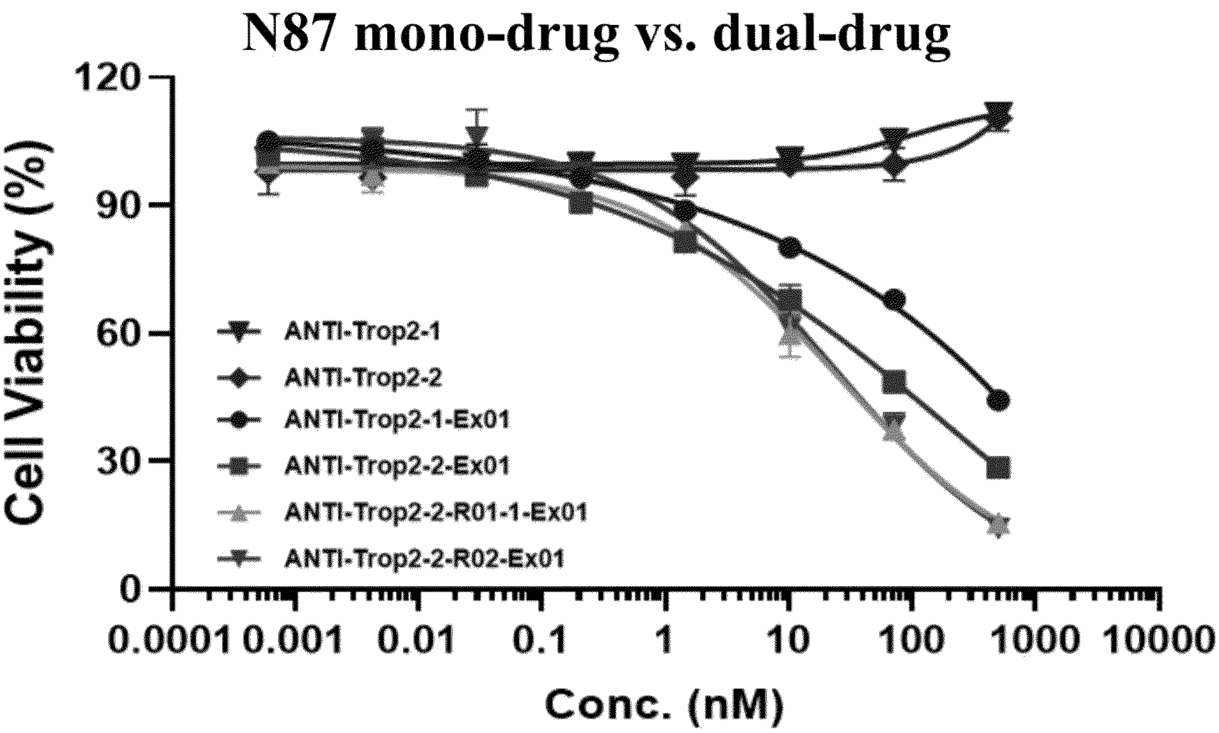

N87 mono-drug vs. dual-drug
Cell Viability (%)
120
90
60
30
0
ANTI-Trop2-1
ANTI-Trop2-2
ANTI-Trop2-1-Ex01
ANTI-Trop2-2-Ex01
ANTI-Trop2-2-R01-1-Ex01
ANTI-Trop2-2-R02-Ex01
0.0001 0.001 0.01 0.1 1 10 100 1000 10000
Conc. (nM)


Fig. 13

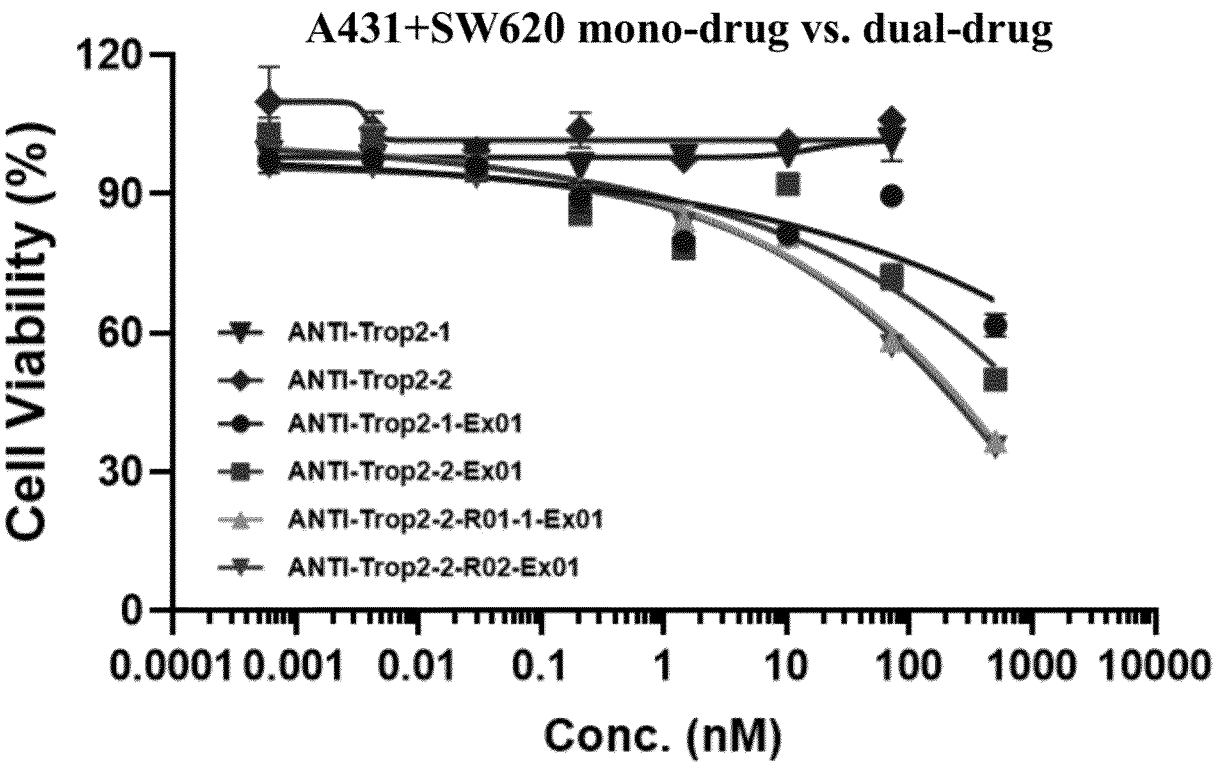

A431+SW620 mono-drug vs. dual-drug
Cell Viability (%)
120
90
60
30
0
ANTI-Trop2-1
ANTI-Trop2-2
ANTI-Trop2-1-Ex01
ANTI-Trop2-2-Ex01
ANTI-Trop2-2-R01-1-Ex01
ANTI-Trop2-2-R02-Ex01
0.0001 0.001 0.01 0.1 1 10 100 1000 10000
Conc. (nM)


Fig. 14

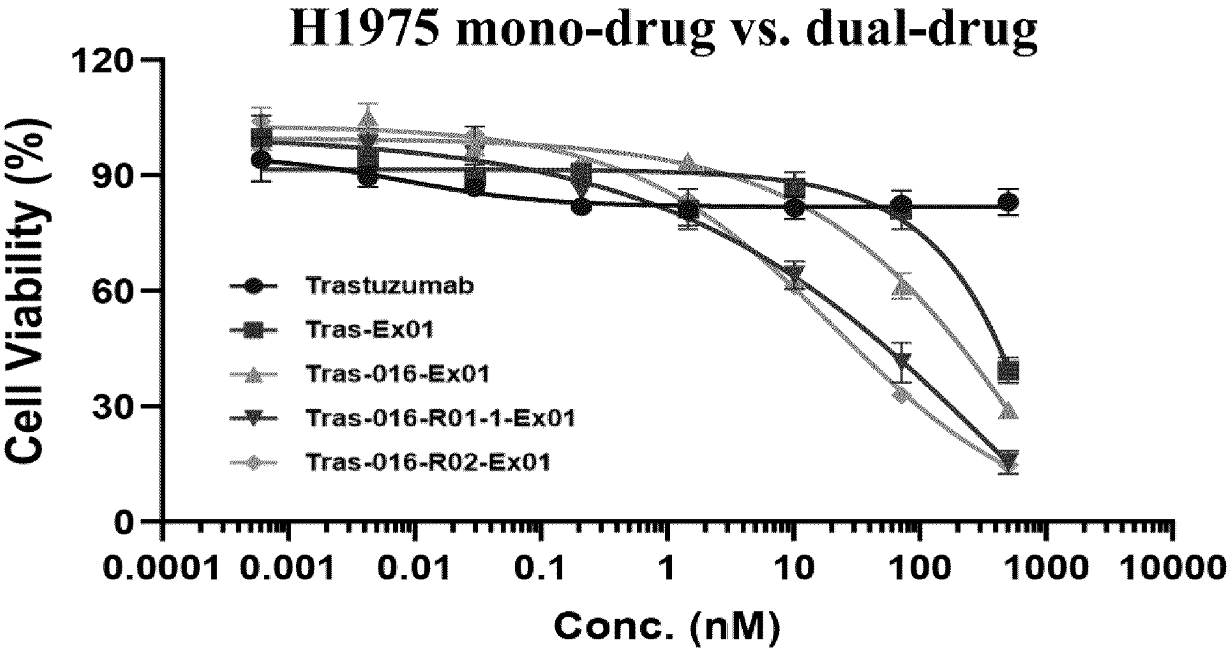
H1975 mono-drug vs. dual-drug
Cell Viability (%)
120
90
60
30
0
Trastuzumab
Tras-Ex01
Tras-016-Ex01
Tras-016-R01-1-Ex01
Tras-016-R02-Ex01
0.0001 0.001 0.01 0.1 1 10 100 1000 10000
Conc. (nM)

Fig. 15

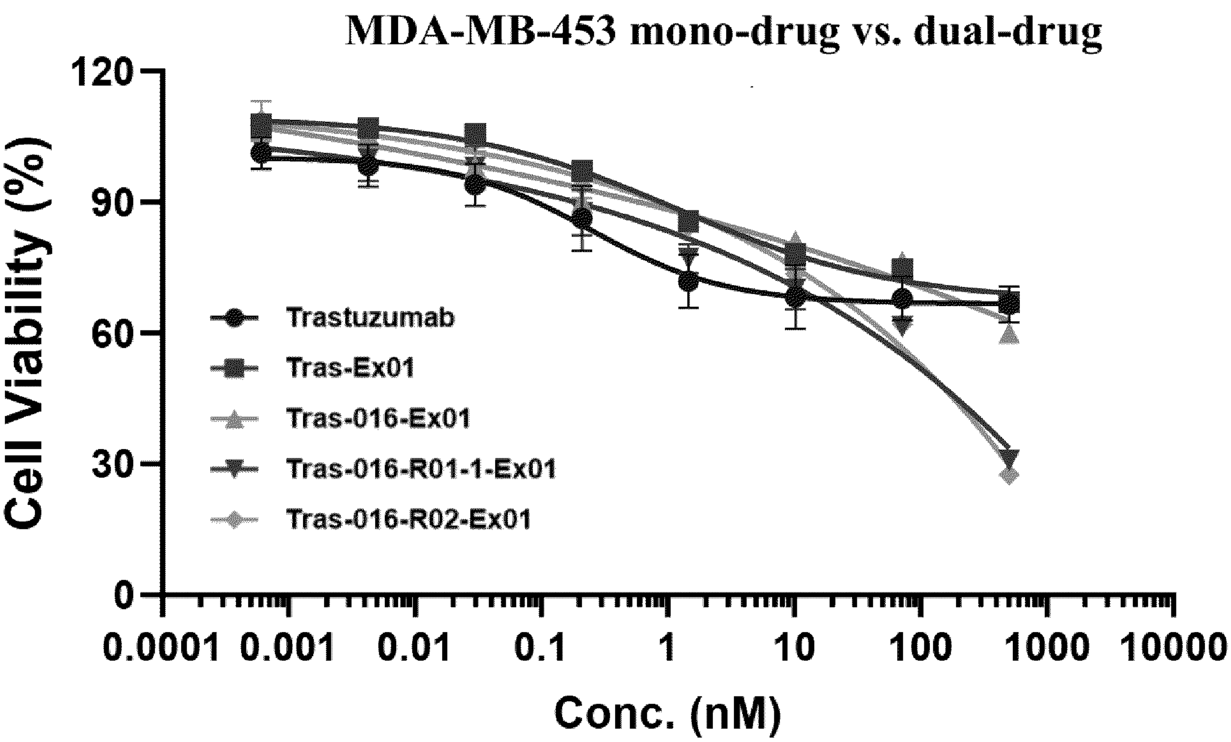
MDA-MB-453 mono-drug vs. dual-drug
Cell Viability (%)
120
90
60
30
0
Trastuzumab
Tras-Ex01
Tras-016-Ex01
Tras-016-R01-1-Ex01
Tras-016-R02-Ex01
0.0001 0.001 0.01 0.1 1 10 100 1000 10000
Conc. (nM)

Fig. 16

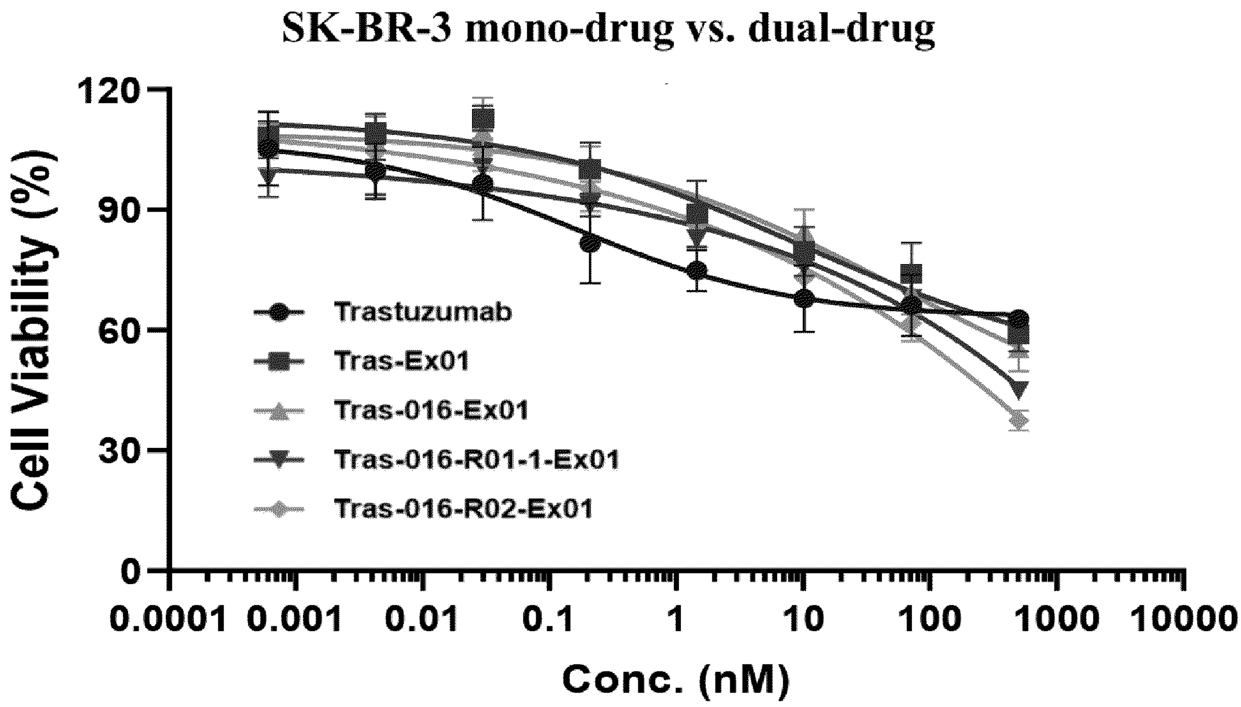
SK-BR-3 mono-drug vs. dual-drug
Cell Viability (%)
120
90
60
30
0
Trastuzumab
Tras-Ex01
Tras-016-Ex01
Tras-016-R01-1-Ex01
Tras-016-R02-Ex01
0.0001 0.001 0.01 0.1 1 10 100 1000 10000
Conc. (nM)

Fig. 17

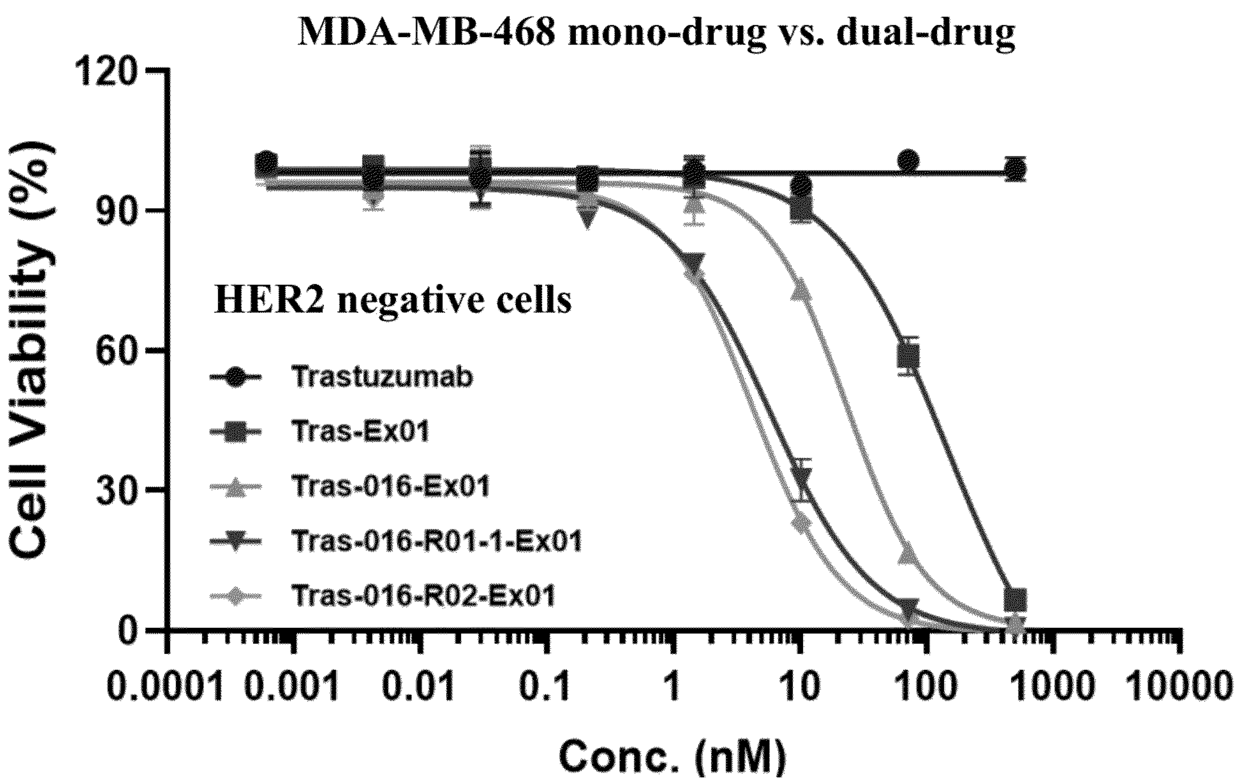
MDA-MB-468 mono-drug vs. dual-drug
Cell Viability (%)
120
90
60
30
0
HER2 negative cells
Trastuzumab
Tras-Ex01
Tras-016-Ex01
Tras-016-R01-1-Ex01
Tras-016-R02-Ex01
0.0001 0.001 0.01 0.1 1 10 100 1000 10000
Conc. (nM)

Fig. 18

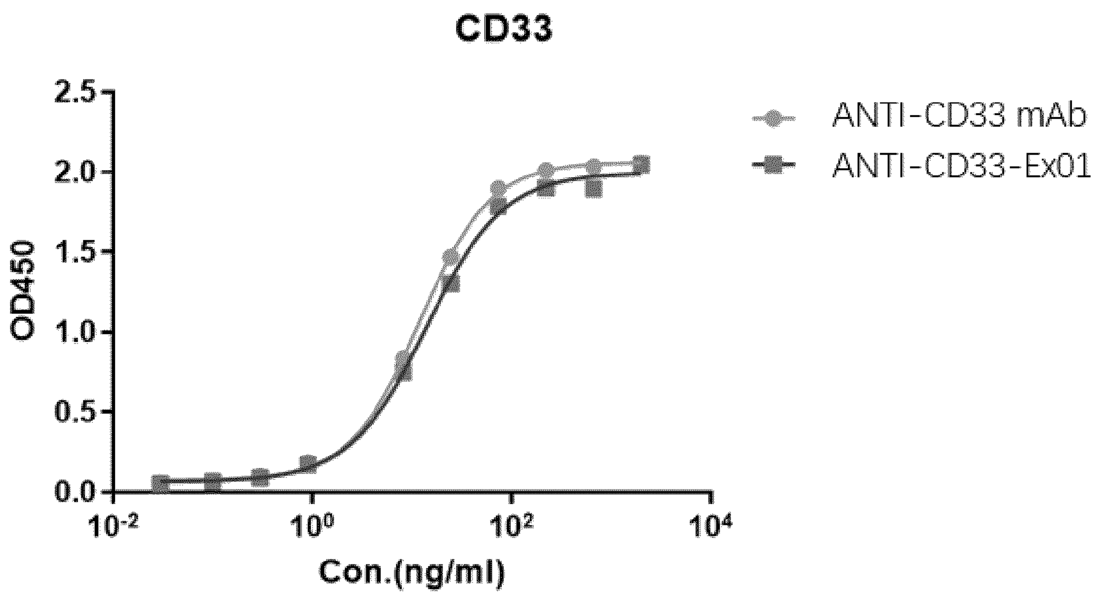
CD33
2.5
2.0
1.5
1.0
0.5
0.0
OD450
10-2
100
102
104
Con.(ng/ml)
ANTI-CD33 mAb
ANTI-CD33-Ex01

Fig. 19

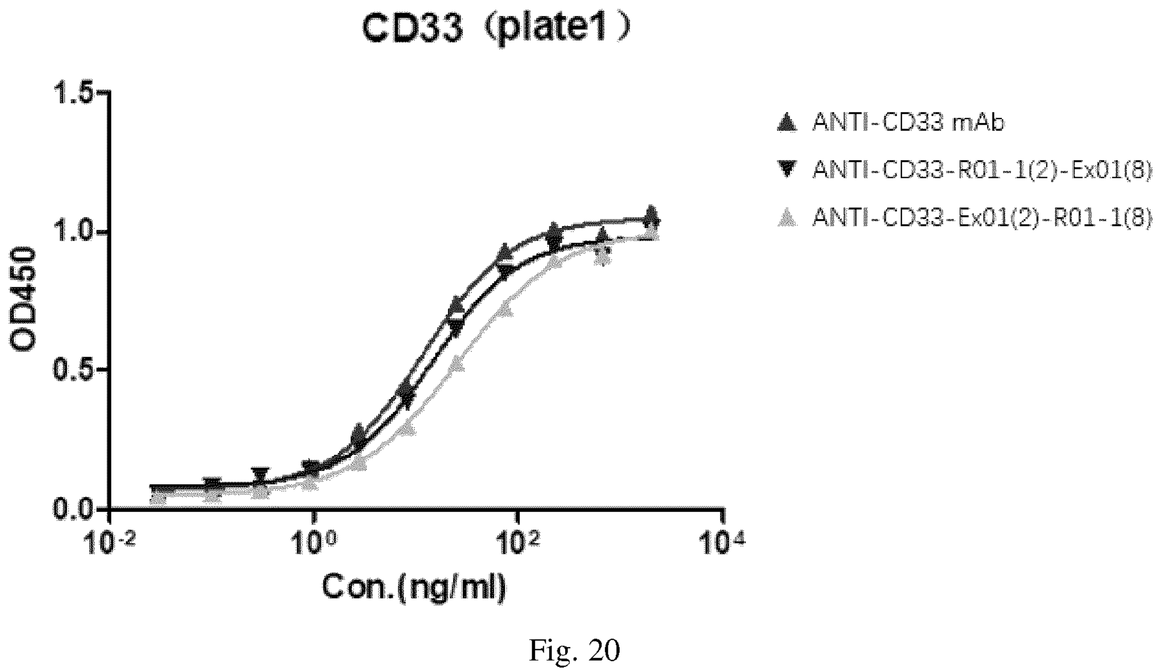
CD33（plate1）
1.5
1.0
0.5
0.0
OD450
10-2
100
102
104
Con.(ng/ml)
ANTI-CD33 mAb
ANTI-CD33-R01-1(2)-Ex01(8)
ANTI-CD33-Ex01(2)-R01-1(8)

Fig. 20

| INTERNATIONAL SEARCH REPORT | International application No. PCT/CN2023/094714 |
|---|---|

**A. CLASSIFICATION OF SUBJECT MATTER**

A61K47/68(2017.01)i; A61K39/00(2006.01)i; C07D401/00(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:A61K,C07D,A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pubmed, STN on web, genbank, NCBI, 中文专利序列检索系统, Chinese Patent Sequence Retrieval System: 成都百利多特生物药业有限责任公司, 朱义, DNA损伤修复抑制剂, DNA损伤反应抑制剂, DDRi, dna damage response inhibitor, ATR or ATM or PARP or WEE1 or CHK1 or PNA-PK, 第一药物, 第二药物, first drug, second drug, dual-drug, 双药, 偶联物, 缀合物, conjugate, ADC, antibody-drug-conjugate, 环糊精, 环糊精叠氮, CD-N3, N3-CD, Cyclodextrins, 组合, 协同, composition, synergism

**C. DOCUMENTS CONSIDERED TO BE RELEVANT**

| Category* | Citation of document, with indication, where appropriate, of the relevant passages | Relevant to claim No. |
|---|---|---|
| Y | CN 106132431 A (SORRENTO THERAPEUTICS INC.) 16 November 2016 (2016-11-16) abstract, and claims 1-15 | 1-14 |
| Y | CHEN, Tianen et al. "DNA damage response inhibition-based combination therapies in cancer treatment: Recent advances and future directions" *Aging and Cancer,* Vol. 3, No. 1, 07 March 2022 (2022-03-07), p. 44, and abstract | 1-14 |
| Y | CN 110291097 A (REGENERON PHARMACEUTICALS INC.) 27 September 2019 (2019-09-27) description, abstract, claims 29-38, and paragraphs 965-966 and 1009 | 3-14 |
| A | CN 111601619 A (REGENERON PHARMACEUTICALS INC.) 28 August 2020 (2020-08-28) description, abstract, and paragraph 3 | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 July 2023** | **21 July 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/094714**

**C. DOCUMENTS CONSIDERED TO BE RELEVANT**

| Category* | Citation of document, with indication, where appropriate, of the relevant passages | Relevant to claim No. |
|---|---|---|
| A | US 2021369705 A1 (MERCK PATENT GMBH) 02 December 2021 (2021-12-02)<br>entire document | 1-14 |
| A | WO 2022022649 A1 (CHENGDU KELINGYUAN MEDICINE TECHNOLOGY CO., LTD.) 03 February 2022 (2022-02-03)<br>entire document | 1-14 |
| A | WO 2020132655 A1 (SEATTLE GENETICS, INC.) 25 June 2020 (2020-06-25)<br>entire document | 1-14 |

Form PCT/ISA/210 (second sheet) (July 2022)

**INTERNATIONAL SEARCH REPORT**

International application No.
**PCT/CN2023/094714**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/094714**

| Patent document cited in search report | | | Publication date (day/month/year) | Patent family member(s) | | | Publication date (day/month/year) |
|---|---|---|---|---|---|---|---|
| CN | 106132431 | A | 16 November 2016 | IL | 246218 | A0 | 30 November 2016 |
| | | | | AU | 2014337317 | A1 | 15 September 2016 |
| | | | | CA | 2934030 | A1 | 23 April 2015 |
| | | | | RU | 2016134258 | A | 28 February 2018 |
| | | | | RU | 2016134258 | A3 | 25 June 2018 |
| | | | | US | 2021017274 | A1 | 21 January 2021 |
| | | | | US | 2015105540 | A1 | 16 April 2015 |
| | | | | US | 10836821 | B2 | 17 November 2020 |
| | | | | EP | 3057610 | A1 | 24 August 2016 |
| | | | | EP | 3057610 | A4 | 05 July 2017 |
| | | | | EP | 3057610 | B1 | 22 September 2021 |
| | | | | SG | 11201604879 | QA | 28 July 2016 |
| | | | | ZA | 201604375 | B | 24 April 2019 |
| | | | | WO | 2015057876 | A1 | 23 April 2015 |
| | | | | WO | 2015057876 | A8 | 10 November 2016 |
| CN | 110291097 | A | 27 September 2019 | US | 2021332080 | A1 | 28 October 2021 |
| | | | | US | 2021040144 | A1 | 11 February 2021 |
| | | | | AU | 2017359043 | A1 | 23 May 2019 |
| | | | | AU | 2017359043 | B2 | 16 June 2022 |
| | | | | MX | 2019005330 | A | 11 September 2019 |
| | | | | EP | 3538539 | A2 | 18 September 2019 |
| | | | | IL | 266353 | A | 30 June 2019 |
| | | | | KR | 20190074310 | A | 27 June 2019 |
| | | | | JP | 2019536765 | A | 19 December 2019 |
| | | | | CA | 3042428 | A1 | 17 May 2018 |
| | | | | CO | 2019005966 | A2 | 10 July 2019 |
| | | | | CL | 2019001259 | A1 | 19 July 2019 |
| | | | | PH | 12019501021 | A1 | 24 June 2019 |
| | | | | BR | 112019009019 | A2 | 09 July 2019 |
| | | | | SG | 10202104259 | RA | 29 June 2021 |
| | | | | US | 2018155389 | A1 | 07 June 2018 |
| | | | | US | 10711032 | B2 | 14 July 2020 |
| | | | | WO | 2018089373 | A2 | 17 May 2018 |
| | | | | WO | 2018089373 | A3 | 21 June 2018 |
| | | | | WO | 2018089373 | A9 | 12 July 2018 |
| CN | 111601619 | A | 28 August 2020 | AU | 2018365946 | A1 | 14 May 2020 |
| | | | | SG | 11202004151 | YA | 29 June 2020 |
| | | | | JP | 2021502341 | A | 28 January 2021 |
| | | | | MX | 2020004691 | A | 20 August 2020 |
| | | | | EP | 3706805 | A2 | 16 September 2020 |
| | | | | KR | 20200085807 | A | 15 July 2020 |
| | | | | EA | 202091135 | A1 | 14 October 2020 |
| | | | | WO | 2019094395 | A2 | 16 May 2019 |
| | | | | WO | 2019094395 | A3 | 22 August 2019 |
| | | | | IL | 274427 | A | 30 June 2020 |
| | | | | CA | 3080857 | A1 | 16 May 2019 |
| US | 2021369705 | A1 | 02 December 2021 | IL | 282312 | A | 31 May 2021 |
| | | | | EP | 3866785 | A1 | 25 August 2021 |
| | | | | WO | 2020078905 | A1 | 23 April 2020 |
| | | | | CA | 3116234 | A1 | 23 April 2020 |

Form PCT/ISA/210 (patent family annex) (July 2022)

**INTERNATIONAL SEARCH REPORT**
**Information on patent family members**

International application No.
**PCT/CN2023/094714**

| Patent document cited in search report | Publication date (day/month/year) | Patent family member(s) | Publication date (day/month/year) |
|---|---|---|---|
| | | AU 2019360608 A1 | 03 June 2021 |
| | | JP 2022505041 A | 14 January 2022 |
| WO 2022022649 A1 | 03 February 2022 | None | |
| WO 2020132655 A1 | 25 June 2020 | US 2022040320 A1 | 10 February 2022 |
| | | KR 20210141918 A | 23 November 2021 |
| | | MX 2021007548 A | 10 December 2021 |
| | | JP 2022514348 A | 10 February 2022 |
| | | CA 3122316 A1 | 25 June 2020 |
| | | SG 11202106122 QA | 29 July 2021 |
| | | EP 3897734 A1 | 27 October 2021 |
| | | EP 3897734 A4 | 07 December 2022 |
| | | IL 284147 A | 31 August 2021 |
| | | TW 202039008 A | 01 November 2020 |
| | | AU 2019403552 A1 | 17 June 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4816567 A
- CN 108452321
- CN 113827736 A
- WO 2020063676 A1
- CN 102933236 A


### Non-patent literature cited in the description


- Burger's Medicinal Chemistry and Drug Discovery. Manfred E. Wolff, 1995, vol. 172-178, 949-982
- **MORRISON et al.** *PNAS*, 1984, vol. 81, 6851-6855
- **JONES et al.** *Nature*, 1986, vol. 321, 522-525
- **RIECHMANN et al.** *Nature*, 1988, vol. 332, 323-329
- **PRESTA**. *Curr Op Struct Bwl*, 1992, vol. 2, 593-596
- **KOHLER et al.** *Nature*, 1975, vol. 256, 495
- **CLACKSON et al.** *Nature*, 1991, vol. 352, 624-628
- **MARKS et al.** *Journal of Molecular Biology*, 1991, vol. 222, 581-597